# EUROPEAN PATENT APPLICATION

(11) **EP 4 763 847 A1**
(43) Date of publication of application: **24.06.2026**
(21) Application number: 24855704.3
(22) Date of filing: 15.08.2024
(51) Int. Cl.: C07D 487/04, A61K 31/4985, A61P 35/00

(54) **PROTEIN ARGININE METHYLTRANSFERASE-5 INHIBITOR AND PHARMACEUTICAL USE THEREOF**

(30) Priority: 18.08.2023 CN 202311047559
(71) Applicant: Jiangsu Yahong Meditech Co., Ltd., Taizhou, Jiangsu 225316 (CN); Asieris Pharmaceuticals (Shanghai) Co., Ltd., Pudong, Shanghai 201203 (CN)
(72) Inventor: WU, Liang, Taizhou, Jiangsu 225316 (CN); ZHANG, Runtao, Taizhou, Jiangsu 225316 (CN); WANG, Tielin, Taizhou, Jiangsu 225316 (CN); LI, Lanli, Taizhou, Jiangsu 225316 (CN); PAN, Yaqiang, Taizhou, Jiangsu 225316 (CN)
(74) Representative: Regimbeau
(86) International application number: PCT/CN2024/112375
(87) International publication number: WO 2025/039979

(57) **Abstract**

The present invention relates to a protein arginine methyltransferase-5 (PRMT5) inhibitor and a pharmaceutical use thereof. Specifically, the present invention relates to a compound represented by general formula (I), a preparation method for the compound, a pharmaceutical composition comprising the compound, and a use of the compound as a PRMT5 inhibitor for treating diseases related to PRMT5 activity. The definitions of the groups in general formula (I) are the same as those in the description.

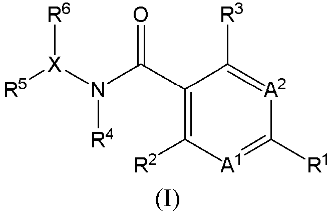

## Description

### FIELD OF THE INVENTION

The present invention relates to the field of pharmaceutical technology, and specifically relates to a class of novel protein arginine methyltransferase-5 (PRMT5) inhibitors, a pharmaceutical composition comprising the same, a method for preparing the same, and a use thereof as PRMT5 inhibitors for the treatment of diseases associated with PRMT5 activity.

### BACKGROUND OF THE INVENTION

Cancer treatment can be broadly categorized into two major types of cytotoxic therapy and targeted therapy. Cytotoxic therapies may produce widespread toxicity, whereas targeted therapies offer the advantage of selectively targeting tumor cells.

PRMT5 (Protein Arginine Methyltransferase-5) is a type II arginine methyltransferase that catalyzes the symmetric dimethylation of proteins involved in transcription and signal transduction by transferring a methyl group from S-adenosylmethionine (SAM). This process realizes various essential cellular functions, including the regulation of cell cycle progression, apoptosis, and DMA damage response. PRMT5 is overexpressed in various cancers, including glioblastoma, leukemia/lymphoma, prostate cancer, and colorectal cancer, and is associated with poor prognosis. Data from genome-wide genetic perturbation screens using shRNA have revealed the importance of PRMT5 activity for MTAP (methylthioadenosine phosphorylase)-deficient cancer cell lines (Kruykov et al, 2016; Marjon et al, 2016; and Markarov et al, 2016).

First-generation PRMT5 inhibitors are primarily SAM-competitive or non-competitive inhibitors, which lack selectivity for MTAP-deficient cells. Inhibition of PRMT5 in normal cells leads to dose-limiting toxicities such as thrombocytopenia, anemia, and neutropenia.

MTAP deficiency leads to the accumulation of the cellular metabolite methylthioadenosine (MTA) in cancer cells. MTA is an intermediate in the methionine salvage pathway and has a weak inhibitory effect on PRMT5. It competes with SAM and can form a PRMT5/MTA complex. There is a need to develop novel inhibitors that can enhance the inhibitory effect of MTA on PRMT5 by binding to and stabilizing the PRMT5/MTA complex, achieving inhibition of PRMT5 activity in MTAP-deficient cancer cells while maintaining PRMT5 activity in wild cells, and thereby effectively improve the therapeutic index.

Currently disclosed patent applications for second-generation PRMT5 inhibitors include: WO2021163344 A1, WO2022115377 A1, WO2022132914 A1, and WO2022169948 A1.

### SUMMARY OF THE INVENTION

Through dedicated research, the inventors have designed and synthesized a series of compounds that exhibit PRMT5 (Protein Arginine Methyltransferase-5) inhibition activity and can be developed as a medicament for the prevention or treatment of diseases associated with PRMT5 activity.

Therefore, an object of the present invention is to provide a compound of formula (I), or a tautomer, mesomer, racemate, enantiomer, diastereomer, or mixture thereof, or a pharmaceutically acceptable salt thereof: 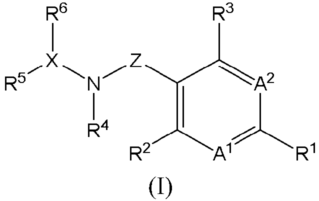 wherein:
Z is selected from the group consisting of C(=O), S(=O), S(=O)₂, 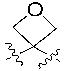 and 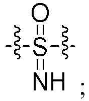
A¹ is selected from the group consisting of N and CR⁷;
A² is selected from the group consisting of N and CR⁸;
X is selected from the group consisting of N and CR⁹;
R¹ is selected from the group consisting of 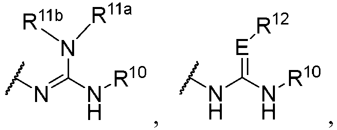 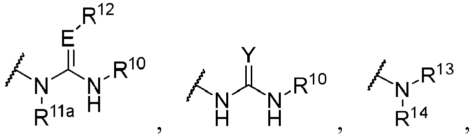 alkyl, alkenyl, alkynyl, cycloalkyl, heterocyclyl, aryl and heteroaryl, wherein the alkyl, alkenyl, alkynyl, cycloalkyl, heterocyclyl, aryl and heteroaryl are optionally further substituted by one or more groups selected from the group consisting of deuterium, halogen, amino, nitro, cyano, hydroxy, thiol, carboxy, -C(=O)OR^{a}, -C(=O)R^{a}, -C(=O)NR^{a}R^{b}, -OC(=O)R^{a}, -NR^{a}C(=O)R^{b}, -S(=O)₂R^{a}, -NR^{a}S(=O)₂R^{b}, -S(=O)₂NR^{a}R^{b}, -S(=O)R^{a}, -P(=O)R^{a}R^{b}, -NR^{a}S(=O)₂R^{b}, alkyl, -OR^{a}, haloalkyl, haloalkoxy, alkenyl, alkynyl, cycloalkyl, heterocyclyl, aryl and heteroaryl;
wherein,
E is selected from the group consisting of N and CR¹⁶;
Y is selected from the group consisting of O and S;
each R¹⁰ is independently selected from the group consisting of hydrogen, -(CH₂)ₚ-R^{a}, -OR^{a}, -(CH₂)ₚ-OR^{a}, -(CH₂)ₚ-C(=O)OR^{a}, -NR^{a}R^{b}, -(CH₂)ₚ-NR^{a}R^{b}, -C(=O)R^{a}, -C(=O)OR^{a}, -S(=O)₂R^{a}, halogen, nitro, cyano, hydroxy, thiol, alkyl, alkenyl, alkynyl, cycloalkyl, heterocyclyl, aryl and heteroaryl, wherein the alkyl, alkenyl, alkynyl, cycloalkyl, heterocyclyl, aryl and heteroaryl are optionally further substituted by one or more groups selected from the group consisting of deuterium, halogen, amino, nitro, cyano, hydroxy, thiol, carboxy, -C(=O)OR^{a}, -C(=O)R^{a}, -C(=O)NR^{a}R^{b}, -OC(=O)R^{a}, -NR^{a}C(=O)R^{b}, -S(=O)₂R^{a}, -NR^{a}S(=O)₂R^{b}, -S(=O)₂NR^{a}R^{b}, -S(=O)R^{a}, -P(=O)R^{a}R^{b}, -NR^{a}S(=O)₂R^{b}, alkyl, -OR^{a}, haloalkyl, haloalkoxy, alkenyl, alkynyl, cycloalkyl, heterocyclyl, aryl and heteroaryl;
R^{11a} and R^{11b} are each independently selected from the group consisting of hydrogen, -(CH₂)ₚ-R^{a}, -OR^{a}, -(CH₂)ₚ-OR^{a}, -(CH₂)ₚ-C(=O)OR^{a}, -NR^{a}R^{b}, -(CH₂)ₚ-NR^{a}R^{b}, -C(=O)R^{a}, -C(=O)OR^{a}, -S(=O)₂R^{a}, halogen, nitro, cyano, hydroxy, thiol, alkyl, alkenyl, alkynyl, cycloalkyl, heterocyclyl, aryl and heteroaryl, wherein the alkyl, alkenyl, alkynyl, cycloalkyl, heterocyclyl, aryl and heteroaryl are optionally further substituted by one or more groups selected from the group consisting of deuterium, halogen, amino, nitro, cyano, hydroxy, thiol, carboxy, -C(=O)OR^{a}, -C(=O)R^{a}, -C(=O)NR^{a}R^{b}, -OC(=O)R^{a}, -NR^{a}C(=O)R^{b}, -S(=O)₂R^{a}, -NR^{a}S(=O)₂R^{b}, -S(=O)₂NR^{a}R^{b}, -S(=O)R^{a}, -P(=O)R^{a}R^{b}, -NR^{a}S(=O)₂R^{b}, alkyl, -OR^{a}, haloalkyl, haloalkoxy, alkenyl, alkynyl, cycloalkyl, heterocyclyl, aryl and heteroaryl;
R¹² is selected from the group consisting of hydrogen, -(CH₂)ₚ-R^{a}, -OR^{a}, -(CH₂)ₚ-OR^{a}, -(CH₂)ₚ-C(=O)OR^{a}, -NR^{a}R^{b}, -(CH₂)ₚ-NR^{a}R^{b}, -C(=O)R^{a}, -C(=O)OR^{a}, -S(=O)₂R^{a}, halogen, nitro, cyano, hydroxy, thiol, alkyl, alkenyl, alkynyl, cycloalkyl, heterocyclyl, aryl and heteroaryl, wherein the alkyl, alkenyl, alkynyl, cycloalkyl, heterocyclyl, aryl and heteroaryl are optionally further substituted by one or more groups selected from the group consisting of deuterium, halogen, amino, nitro, cyano, hydroxy, thiol, carboxy, -C(=O)OR^{a}, -C(=O)R^{a}, -C(=O)NR^{a}R^{b}, -OC(=O)R^{a}, -NR^{a}C(=O)R^{b}, -S(=O)₂R^{a}, -NR^{a}S(=O)₂R^{b}, -S(=O)₂NR^{a}R^{b}, -S(=O)R^{a}, -P(=O)R^{a}R^{b}, -NR^{a}S(=O)₂R^{b}, alkyl, -OR^{a}, haloalkyl, haloalkoxy, alkenyl, alkynyl, cycloalkyl, heterocyclyl, aryl and heteroaryl;
   or,
R^{11a} or R^{11b} together with R¹⁰, or R¹² together with R¹⁰ and the atoms to which they are attached, form a heterocyclyl or heteroaryl, wherein the heterocyclyl or heteroaryl is optionally further substituted by one or more groups selected from the group consisting of halogen, amino, -NR^{a}R^{b}, nitro, cyano, hydroxy, thiol, carboxy, -C(=O)OR^{a}, -C(=O)R^{a}, -C(=O)NR^{a}R^{b}, -OC(=O)R^{a}, -NR^{a}C(=O)R^{b}, -S(=O)₂R^{a}, -S(=O)R^{a}, -P(=O)R^{a}R^{b}, -NR^{a}S(=O)₂R^{b}, alkyl, alkoxy, haloalkyl, haloalkoxy, alkenyl, alkynyl, cycloalkyl, heterocyclyl, aryl and heteroaryl;
R¹³ and R¹⁴ are each independently selected from the group consisting of hydrogen, -(CH₂)ₚ-R^{a}, -OR^{a}, -(CH₂)ₚ-OR^{a}, -(CH₂)ₚ-C(=O)OR^{a}, -NR^{a}R^{b}, -(CH₂)ₚ-NR^{a}R^{b}, -C(=O)R^{a}, -C(=O)OR^{a}, -S(=O)₂R^{a}, halogen, nitro, cyano, hydroxy, thiol, alkyl, alkenyl, alkynyl, cycloalkyl, heterocyclyl, aryl and heteroaryl, wherein the alkyl, alkenyl, alkynyl, cycloalkyl, heterocyclyl, aryl and heteroaryl are optionally further substituted by one or more groups selected from the group consisting of deuterium, halogen, amino, nitro, cyano, hydroxy, thiol, carboxy, -C(=O)OR^{a}, -C(=O)R^{a}, -C(=O)NR^{a}R^{b}, -OC(=O)R^{a}, -NR^{a}C(=O)R^{b}, -S(=O)₂R^{a}, -NR^{a}S(=O)₂R^{b}, -S(=O)₂NR^{a}R^{b}, -S(=O)R^{a}, -P(=O)R^{a}R^{b}, -NR^{a}S(=O)₂R^{b}, alkyl, -OR^{a}, haloalkyl, haloalkoxy, alkenyl, alkynyl, cycloalkyl, heterocyclyl, aryl and heteroaryl; or
R¹³ and R¹⁴ together with the nitrogen atom to which they are attached form a heteroaryl or heterocyclyl, wherein the heteroaryl or heterocyclyl is optionally further substituted by one or more groups selected from the group consisting of halogen, amino, -NR^{a}R^{b}, nitro, cyano, hydroxy, thiol, carboxy, -C(=O)OR^{a}, -C(=O)R^{a}, -C(=O)NR^{a}R^{b}, -OC(=O)R^{a}, -NR^{a}C(=O)R^{b}, -S(=O)₂R^{a}, -S(=O)R^{a}, -P(=O)R^{a}R^{b}, -NR^{a}S(=O)₂R^{b}, alkyl, alkoxy, haloalkyl, haloalkoxy, alkenyl, alkynyl, cycloalkyl, heterocyclyl, aryl and heteroaryl;
R¹⁶ is selected from the group consisting of hydrogen, -(CH₂)ₚ-R^{a}, -OR^{a}, -(CH₂)ₚ-OR^{a}, -(CH₂)ₚ-C(=O)OR^{a}, -NR^{a}R^{b}, -(CH₂)ₚ-NR^{a}R^{b}, -C(=O)R^{a}, -C(=O)OR^{a}, -S(=O)₂R^{a}, halogen, nitro, cyano, hydroxy, thiol, alkyl, alkenyl, alkynyl, cycloalkyl, heterocyclyl, aryl and heteroaryl, wherein the alkyl, alkenyl, alkynyl, cycloalkyl, heterocyclyl, aryl and heteroaryl are optionally further substituted by one or more groups selected from the group consisting of deuterium, halogen, amino, nitro, cyano, hydroxy, thiol, carboxy, -C(=O)OR^{a}, -C(=O)R^{a}, -C(=O)NR^{a}R^{b}, -OC(=O)R^{a}, -NR^{a}C(=O)R^{b}, -S(=O)₂R^{a}, -NR^{a}S(=O)₂R^{b}, -S(=O)₂NR^{a}R^{b}, -S(=O)R^{a}, -P(=O)R^{a}R^{b}, -NR^{a}S(=O)₂R^{b}, alkyl, -OR^{a}, haloalkyl, haloalkoxy, alkenyl, alkynyl, cycloalkyl, heterocyclyl, aryl and heteroaryl;
R², R³, R⁷ and R⁸ are each independently selected from the group consisting of hydrogen, -(CH₂)ₚ-R^{a}, -OR^{a}, -(CH₂)ₚ-OR^{a}, -(CH₂)ₚ-C(=O)OR^{a}, -NR^{a}R^{b}, -(CH₂)ₚ-NR^{a}R^{b}, -C(=O)R^{a}, -C(=O)OR^{a}, -S(=O)₂R^{a}, halogen, nitro, cyano, hydroxy, thiol, alkyl, alkenyl, alkynyl, cycloalkyl, heterocyclyl, aryl and heteroaryl, wherein the alkyl, alkenyl, alkynyl, cycloalkyl, heterocyclyl, aryl and heteroaryl are optionally further substituted by one or more groups selected from the group consisting of deuterium, halogen, amino, nitro, cyano, hydroxy, thiol, carboxy, -C(=O)OR^{a}, -C(=O)R^{a}, -C(=O)NR^{a}R^{b}, -OC(=O)R^{a}, -NR^{a}C(=O)R^{b}, -S(=O)₂R^{a}, -NR^{a}S(=O)₂R^{b}, -S(=O)₂NR^{a}R^{b}, -S(=O)R^{a}, -P(=O)R^{a}R^{b}, -NR^{a}S(=O)₂R^{b}, alkyl, -OR^{a}, haloalkyl, haloalkoxy, alkenyl, alkynyl, cycloalkyl, heterocyclyl, aryl and heteroaryl;
R⁵, R⁶ and R⁹ are each independently selected from the group consisting of hydrogen, -(CH₂)ₚ-R^{a}, -OR^{a}, -(CH₂)ₚ-OR^{a}, -(CH₂)ₚ-C(=O)OR^{a}, -NR^{a}R^{b}, -(CH₂)ₚ-NR^{a}R^{b}, -C(=O)R^{a}, -C(=O)OR^{a}, -S(=O)₂R^{a}, halogen, nitro, cyano, hydroxy, thiol, alkyl, alkenyl, alkynyl, cycloalkyl, heterocyclyl, aryl and heteroaryl, wherein the alkyl, alkenyl, alkynyl, cycloalkyl, heterocyclyl, aryl and heteroaryl are optionally further substituted by one or more groups selected from the group consisting of deuterium, halogen, amino, -NR^{a}R^{b}, nitro, cyano, hydroxy, thiol, carboxy, -C(=O)OR^{a}, -C(=O)R^{a}, -C(=O)NR^{a}R^{b}, -OC(=O)R^{a}, -NR^{a}C(=O)R^{b}, -S(=O)₂R^{a}, -NR^{a}S(=O)₂R^{b}, -S(=O)₂NR^{a}R^{b}, -S(=O)R^{a}, -P(=O)R^{a}R^{b}, -NR^{a}S(=O)₂R^{b}, -B(OR^{a})₂, alkyl, -OR^{a}, haloalkyl, haloalkoxy, alkenyl, alkynyl, cycloalkyl, heterocyclyl, aryl and heteroaryl;
R⁴ is selected from the group consisting of hydrogen, -(CH₂)ₚ-R¹⁵, -NR^{a}R^{b}, -C(=O)R^{a}, -S(=O)₂R^{a}, alkyl, alkenyl, alkynyl, cycloalkyl, heterocyclyl, aryl and heteroaryl, wherein the alkyl, alkenyl, alkynyl, cycloalkyl, heterocyclyl, aryl and heteroaryl are optionally further substituted by one or more groups selected from the group consisting of deuterium, halogen, amino, nitro, cyano, hydroxy, thiol, carboxy, -C(=O)OR^{a}, -C(=O)R^{a}, -C(=O)NR^{a}R^{b}, -OC(=O)R^{a}, -NR^{a}C(=O)R^{b}, -S(=O)₂R^{a}, -NR^{a}S(=O)₂R^{b}, -S(=O)₂NR^{a}R^{b}, -S(=O)R^{a}, -P(=O)R^{a}R^{b}, -NR^{a}S(=O)₂R^{b}, alkyl, -OR^{a}, haloalkyl, haloalkoxy, alkenyl, alkynyl, cycloalkyl, heterocyclyl, aryl and heteroaryl;
   or
any two of R⁴, R⁵, R⁶ and R⁹ together with the atoms to which they are attached, form a cycloalkyl, heterocyclyl, aryl, or heteroaryl, wherein the cycloalkyl, heterocyclyl, aryl, or heteroaryl is optionally further substituted by one or more groups selected from the group consisting of halogen, amino, -NR^{a}R^{b}, nitro, cyano, hydroxy, thiol, carboxy, -C(=O)OR^{a}, -C(=O)R^{a}, -C(=O)NR^{a}R^{b}, -OC(=O)R^{a}, -NR^{a}C(=O)R^{b}, -S(=O)₂R^{a}, -S(=O)R^{a}, -P(=O)R^{a}R^{b}, -NR^{a}S(=O)₂R^{b}, alkyl, -OR^{a}, haloalkyl, haloalkoxy, alkenyl, alkynyl, cycloalkyl, heterocyclyl, aryl and heteroaryl, wherein the cycloalkyl, heterocyclyl, aryl and heteroaryl are optionally further substituted by one or more groups selected from the group consisting of halogen, amino, -NR^{a}R^{b}, nitro, cyano, hydroxy, thiol, carboxy, -C(=O)OR^{a}, -C(=O)R^{a}, -C(=O)NR^{a}R^{b}, -OC(=O)R^{a}, -NR^{a}C(=O)R^{b}, -S(=O)₂R^{a}, -S(=O)R^{a}, -P(=O)R^{a}R^{b}, -NR^{a}S(=O)₂R^{b}, alkyl, -OR^{a}, haloalkyl, haloalkoxy, alkenyl, alkynyl, cycloalkyl, heterocyclyl, aryl and heteroaryl;
R¹⁵ is selected from the group consisting of heteroaryl, aryl, heterocyclyl and cycloalkyl, wherein the heteroaryl, aryl, heterocyclyl and cycloalkyl are optionally further substituted by one or more groups selected from the group consisting of deuterium, halogen, amino, -NR^{a}R^{b}, nitro, cyano, hydroxy, thiol, carboxy, -C(=O)OR^{a}, -C(=O)R^{a}, -C(=O)NR^{a}R^{b}, -OC(=O)R^{a}, -NR^{a}C(=O)R^{b}, -S(=O)₂R^{a}, -NR^{a}S(=O)₂R^{b}, -S(=O)ZNR^{a}R^{b}, -S(=O)R^{a}, -P(=O)R^{a}R^{b}, -NR^{a}S(=O)₂R^{b}, alkyl, -OR^{a}, haloalkyl, haloalkoxy, alkenyl, alkynyl, cycloalkyl, heterocyclyl, aryl and heteroaryl, wherein the cycloalkyl, heterocyclyl, aryl and heteroaryl are optionally further substituted by one or more groups selected from the group consisting of deuterium, halogen, amino, -NR^{a}R^{b}, nitro, cyano, hydroxy, thiol, carboxy, -C(=O)OR^{a}, -C(=O)R^{a}, -C(=O)NR^{a}R^{b}, -OC(=O)R^{a}, -NR^{a}C(=O)R^{b}, -S(=O)₂R^{a}, -NR^{a}S(=O)₂R^{b}, -S(=O)₂NR^{a}R^{b}, -S(=O)R^{a}, -P(=O)R^{a}R^{b}, -NR^{a}S(=O)₂R^{b}, alkyl, -OR^{a}, haloalkyl, haloalkoxy, alkenyl, alkynyl, cycloalkyl, heterocyclyl, aryl and heteroaryl;
R^{a} and R^{b} are each independently selected from the group consisting of hydrogen, halogen, hydroxy, alkyl, alkenyl, alkynyl, cycloalkyl, heterocyclyl, aryl and heteroaryl, wherein the alkyl, alkenyl, alkynyl, cycloalkyl, heterocyclyl, aryl and heteroaryl are optionally further substituted by one or more groups selected from the group consisting of halogen, amino, nitro, cyano, hydroxy, thiol, carboxy, -C(=O)OR^{c}, -C(=O)R^{c}, -C(=O)NR^{c}R^{d}, -OC(=O)R^{c}, -NR^{c}C(=O)R^{d}, -S(=O)₂R^{c}, -NR^{c}S(=O)₂R^{d}, -S(=O)₂NR^{c}R^{d}, -S(=O)R^{c}, -P(=O)R^{c}R^{d}, -NR^{c}S(=O)₂R^{d}, alkyl, -OR^{c}, alkenyl, alkynyl, cycloalkyl, heterocyclyl, aryl and heteroaryl;
or, R^{a} and R^{b} together with the atoms to which they are attached, form a heterocyclyl, wherein the heterocyclyl is optionally further substituted by one or more groups selected from the group consisting of halogen, amino, nitro, cyano, oxo, hydroxy, thiol, carboxy, alkoxycarbonyl, alkyl, alkoxy, alkenyl, alkynyl, cycloalkyl, heterocyclyl, aryl and heteroaryl;
R^{c} and R^{d} are each independently selected from the group consisting of hydrogen, halogen, hydroxy, alkyl, alkenyl, alkynyl, cycloalkyl, heterocyclyl, aryl and heteroaryl, wherein the alkyl, alkenyl, alkynyl, cycloalkyl, heterocyclyl, aryl and heteroaryl are optionally further substituted by one or more groups selected from the group consisting of halogen, amino, nitro, cyano, hydroxy, thiol, carboxy, alkyl, alkoxy, alkenyl, alkynyl, cycloalkyl, heterocyclyl, aryl and heteroaryl;
or, R^{c} and R^{d} together with the atoms to which they are attached form a heterocyclyl, wherein the heterocyclyl is optionally further substituted by one or more groups selected from the group consisting of halogen, amino, nitro, cyano, oxo, hydroxy, thiol, carboxy, ester, alkyl, alkoxy, alkenyl, alkynyl, cycloalkyl, heterocyclyl, aryl and heteroaryl;
p is an integer from 1 to 6.

In a specific embodiment, the compound of formula (I) or a tautomer, mesomer, racemate, enantiomer, diastereomer, or mixture thereof, or a pharmaceutically acceptable salt thereof according to the present invention is a compound of formula (II) or formula (III), or a tautomer, mesomer, racemate, enantiomer, diastereomer, or mixture thereof, or a pharmaceutically acceptable salt thereof, 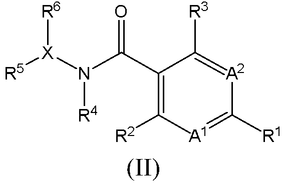 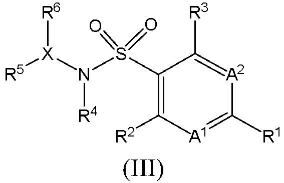 wherein, A¹, A², X, R¹~R⁶ are as defined in formula (I).

In another specific embodiment, the compound of formula (I) or a tautomer, mesomer, racemate, enantiomer, diastereomer, or mixture thereof, or a pharmaceutically acceptable salt thereof according to the present invention is a compound of formula (I-1), or a tautomer, mesomer, racemate, enantiomer, diastereomer, or mixture thereof, or a pharmaceutically acceptable salt thereof, 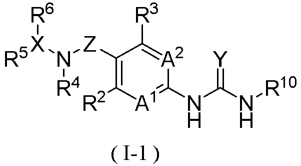 wherein, A¹, A², X, Y, Z, R²~R⁶, R¹⁰ are as defined in formula (I).

In another specific embodiment, the compound of formula (I) or a tautomer, mesomer, racemate, enantiomer, diastereomer, or mixture thereof, or a pharmaceutically acceptable salt thereof according to the present invention is a compound of formula (I-2), or a tautomer, mesomer, racemate, enantiomer, diastereomer, or mixture thereof, or a pharmaceutically acceptable salt thereof, 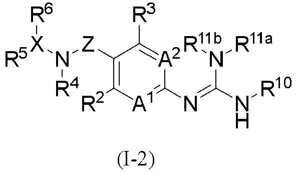 wherein, A¹, A², X, Z, R²~R⁶, R¹⁰, R^{11a}, R^{11b} are as defined in formula (I).

In another specific embodiment, the compound of formula (I) or a tautomer, mesomer, racemate, enantiomer, diastereomer, or mixture thereof, or a pharmaceutically acceptable salt thereof according to the present invention is a compound of formula (IV) or formula (V), or a tautomer, mesomer, racemate, enantiomer, diastereomer, or mixture thereof, or a pharmaceutically acceptable salt thereof, 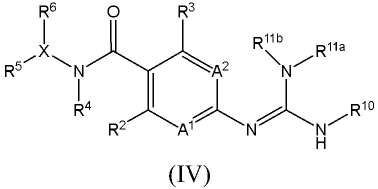 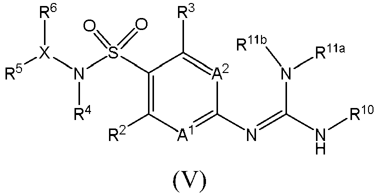 wherein,
A¹, A², X, R²~R⁶, R¹⁰, R^{11a}, R^{11b} are as defined in formula (I).

In another specific embodiment, the compound of formula (I) or a tautomer, mesomer, racemate, enantiomer, diastereomer, or mixture thereof, or a pharmaceutically acceptable salt thereof according to the present invention is a compound of formula (IIA) or formula (IIIA), or a tautomer, mesomer, racemate, enantiomer, diastereomer, or mixture thereof, or a pharmaceutically acceptable salt thereof, 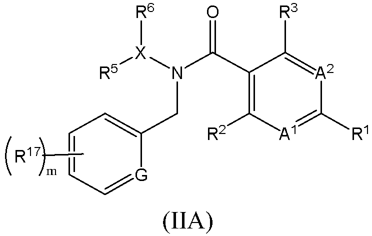 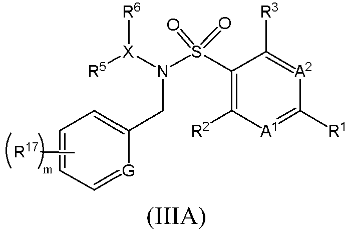 wherein:
G is selected from the group consisting of N and CH;
R¹⁷ is selected from the group consisting of hydrogen, deuterium, halogen, amino, -NR^{a}R^{b}, nitro, cyano, hydroxy, thiol, carboxy, -C(=O)OR^{a}, -C(=O)R^{a}, -C(=O)NR^{a}R^{b}, -OC(=O)R^{a}, -NR^{a}C(=O)R^{b}, -S(=O)₂R^{a}, -NR^{a}S(=O)₂R^{b}, -S(=O)₂NR^{a}R^{b}, -S(=O)R^{a}, -P(=O)R^{a}R^{b}, -NR^{a}S(=O)₂R^{b}, alkyl, -OR^{a}, haloalkyl, haloalkoxy, alkenyl, alkynyl, cycloalkyl, heterocyclyl, aryl and heteroaryl, preferably halogen, alkyl, cycloalkyl and haloalkyl; wherein the cycloalkyl, heterocyclyl, aryl and heteroaryl are optionally further substituted by one or more groups selected from the group consisting of deuterium, halogen, amino, -NR^{a}R^{b}, nitro, cyano, hydroxy, thiol, carboxy, -C(=O)OR^{a}, -C(=O)R^{a}, -C(=O)NR^{a}R^{b}, -OC(=O)R^{a}, -NR^{a}C(=O)R^{b}, -S(=O)₂R^{a}, -NR^{a}S(=O)₂R^{b}, -S(=O)ZNR^{a}R^{b}, -S(=O)R^{a}, -P(=O)R^{a}R^{b}, -NR^{a}S(=O)₂R^{b}, alkyl, -OR^{a}, haloalkyl, haloalkoxy, alkenyl, alkynyl, cycloalkyl, heterocyclyl, aryl and heteroaryl;
m is an integer from 0 to 4, preferably 1 or 2;
A¹, A², X, R¹~R³, R⁵, R⁶, R^{a}, R^{b} are as defined in formula (I).

In another specific embodiment, the compound of formula (I) or a tautomer, mesomer, racemate, enantiomer, diastereomer, or mixture thereof, or a pharmaceutically acceptable salt thereof according to the present invention is a compound of formula (IVA) or formula (VA), or a tautomer, mesomer, racemate, enantiomer, diastereomer, or mixture thereof, or a pharmaceutically acceptable salt thereof, 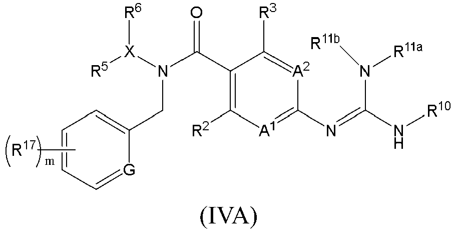 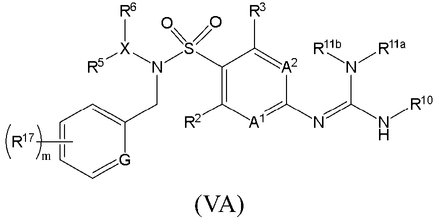 wherein,
G is selected from the group consisting of N and CH;
R¹⁷ is selected from the group consisting of hydrogen, deuterium, halogen, amino, -NR^{a}R^{b}, nitro, cyano, hydroxy, thiol, carboxy, -C(=O)OR^{a}, -C(=O)R^{a}, -C(=O)NR^{a}R^{b}, -OC(=O)R^{a}, -NR^{a}C(=O)R^{b}, -S(=O)₂R^{a}, -NR^{a}S(=O)₂R^{b}, -S(=O)₂NR^{a}R^{b}, -S(=O)R^{a}, -P(=O)R^{a}R^{b}, -NR^{a}S(=O)₂R^{b}, alkyl, -OR^{a}, haloalkyl, haloalkoxy, alkenyl, alkynyl, cycloalkyl, heterocyclyl, aryl and heteroaryl; wherein the cycloalkyl, heterocyclyl, aryl and heteroaryl are optionally further substituted by one or more groups selected from the group consisting of deuterium, halogen, amino, -NR^{a}R^{b}, nitro, cyano, hydroxy, thiol, carboxy, -C(=O)OR^{a}, -C(=O)R^{a}, -C(=O)NR^{a}R^{b}, -OC(=O)R^{a}, -NR^{a}C(=O)R^{b}, -S(=O)₂R^{a}, -NR^{a}S(=O)₂R^{b}, -S(=O)₂NR^{a}R^{b}, -S(=O)R^{a}, -P(=O)R^{a}R^{b}, -NR^{a}S(=O)₂R^{b}, alkyl, -OR^{a}, haloalkyl, haloalkoxy, alkenyl, alkynyl, cycloalkyl, heterocyclyl, aryl and heteroaryl, preferably halogen, alkyl, cycloalkyl and haloalkyl;
m is an integer from 0 to 4, preferably 1 or 2;
A¹, A², X, R²~R³, R⁵, R⁶, R¹⁰, R^{11a}, R^{11b}, R^{a}, R^{b} are as defined in formula (I).

In a preferred embodiment, in the compound of formula, or a tautomer, mesomer, racemate, enantiomer, diastereomer, or mixture thereof, or a pharmaceutically acceptable salt thereof according to the present invention, A¹ is CR⁷ and A² is N; or A¹ is CR⁷ and A² is CR⁸; or A' is N and A² is N; R⁷ and R⁸ are as defined in claim 1, preferably, R⁷ and R⁸ are each independently selected from the group consisting of hydrogen, halogen and C₁₋₆ alkyl.

In another preferred embodiment, in the compound of formula, or a tautomer, mesomer, racemate, enantiomer, diastereomer, or mixture thereof, or a pharmaceutically acceptable salt thereof according to the present invention,
X is selected from the group consisting of N and CR⁹;
R⁵ and R⁶ are each independently selected from the group consisting of hydrogen, C₁₋₆ alkyl, C₃₋₆ cycloalkyl, 4- to 6-membered heterocyclyl, C₆₋₁₀ aryl and 5- to 10-membered heteroaryl, wherein the C₁₋₆ alkyl, C₃₋₆ cycloalkyl, 4- to 6-membered heterocyclyl, C₆₋₁₀ aryl and 5- to 10-membered heteroaryl are optionally further substituted by one or more groups selected from the group consisting of deuterium, halogen, C₁₋₆ alkyl and C₁₋₆ haloalkyl;
R⁹ is selected from the group consisting of hydrogen and C₁₋₆ alkyl.

In another preferred embodiment, in the compound of formula, or a tautomer, mesomer, racemate, enantiomer, diastereomer, or mixture thereof, or a pharmaceutically acceptable salt thereof according to the present invention,
X is selected from the group consisting of N and CR⁹;
any two of R⁴, R⁵, R⁶ and R⁹ together with the atoms to which they are attached, form a C₃₋₁₀ cycloalkyl, 5- to 10-membered heterocyclyl, C₆₋₁₀ aryl, or 5- to 10-membered heteroaryl, wherein the C₃₋₁₀ cycloalkyl, 5- to 10-membered heterocyclyl, C₆₋₁₀ aryl, or 5- to 10-membered heteroaryl is optionally further substituted by one or more groups selected from the group consisting of halogen, amino, -NR^{a}R^{b}, nitro, cyano, hydroxy, thiol, carboxy, -C(=O)OR^{a}, -NR^{a}C(=O)R^{b}, -OR^{a}, haloalkoxy, C₃₋₆ cycloalkyl, 5- to 10-membered heterocyclyl, C₁₋₆ alkyl, C₁₋₆ haloalkyl, C₆₋₁₀ aryl and 5-to 10-membered heteroaryl; wherein the C₆₋₁₀ aryl and 5- to 10-membered heteroaryl are optionally further substituted by C₁₋₆ alkyl;
wherein R^{a} and R^{b} are as defined in formula (I).

In another preferred embodiment, in the compound of formula, or a tautomer, mesomer, racemate, enantiomer, diastereomer, or mixture thereof, or a pharmaceutically acceptable salt thereof according to the present invention,
X is selected from the group consisting of N and CR⁹;
R⁴ and one of R⁵, R⁶, R⁹, together with the atoms to which they are attached, form a 5- to 10-membered heterocyclyl or 5- to 10-membered heteroaryl, wherein the 5- to 10-membered heterocyclyl or 5- to 10-membered heteroaryl is optionally further substituted by one or more groups selected from the group consisting of halogen, amino, -NR^{a}R^{b}, nitro, cyano, hydroxy, thiol, carboxy, -C(=O)OR^{a}, -NR^{a}C(=O)R^{b}, -OR^{a}, haloalkoxy, C₃₋₆ cycloalkyl, 5- to 10-membered heterocyclyl, C₁₋₆ alkyl, C₁₋₆ haloalkyl, C₆₋₁₀ aryl and 5- to 10-membered heteroaryl; wherein the C₆₋₁₀ aryl and 5- to 10-membered heteroaryl are optionally further substituted by C₁₋₆ alkyl;
wherein R^{a} and R^{b} are as defined in claim 1.

In another specific embodiment, the compound of formula (I) or a tautomer, mesomer, racemate, enantiomer, diastereomer, or mixture thereof, or a pharmaceutically acceptable salt thereof according to the present invention is a compound of formula (IVB) or formula (VB), or a tautomer, mesomer, racemate, enantiomer, diastereomer, or mixture thereof, or a pharmaceutically acceptable salt thereof, 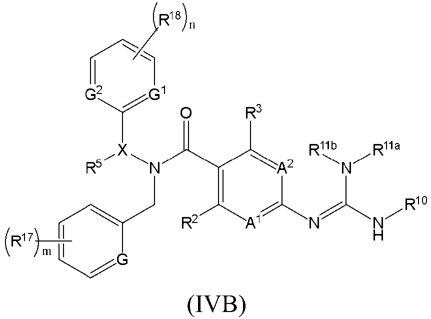 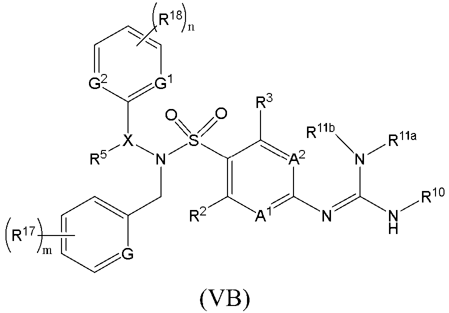 wherein,
X is selected from the group consisting of N and CR⁹;
G is selected from the group consisting of N and CH;
G¹ and G² are each independently selected from the group consisting of N and CH;
R⁵ is selected from the group consisting of hydrogen and C₁₋₆ alkyl;
R⁹ is selected from the group consisting of hydrogen and C₁₋₆ alkyl;
each R¹⁷ is independently selected from the group consisting of hydrogen, deuterium, halogen, amino, -NR^{a}R^{b}, nitro, cyano, hydroxy, thiol, carboxy, -C(=O)OR^{a}, -C(=O)R^{a}, -C(=O)NR^{a}R^{b}, -OC(=O)R^{a}, -NR^{a}C(=O)R^{b}, -S(=O)₂R^{a}, -NR^{a}S(=O)₂R^{b}, -S(=O)ZNR^{a}R^{b}, -S(=O)R^{a}, -P(=O)R^{a}R^{b}, -NR^{a}S(=O)₂R^{b}, alkyl, -OR^{a}, haloalkyl, haloalkoxy, alkenyl, alkynyl, cycloalkyl, heterocyclyl, aryl and heteroaryl; wherein the heterocyclyl, aryl and heteroaryl are optionally further substituted by one or more groups selected from the group consisting of deuterium, halogen, amino, -NR^{a}R^{b}, nitro, cyano, hydroxy, thiol, carboxy, -C(=O)OR^{a}, -C(=O)R^{a}, -C(=O)NR^{a}R^{b}, -OC(=O)R^{a}, -NR^{a}C(=O)R^{b}, -S(=O)₂R^{a}, -NR^{a}S(=O)₂R^{b}, -S(=O)₂NR^{a}R^{b}, -S(=O)R^{a}, -P(=O)R^{a}R^{b}, -NR^{a}S(=O)₂R^{b}, alkyl, -OR^{a}, haloalkyl, haloalkoxy, alkenyl, alkynyl, cycloalkyl, heterocyclyl, alkyl substituted heterocyclyl, aryl and heteroaryl, preferably halogen, C₁₋₆ alkyl, C₁₋₆ alkoxy, C₃₋₆ cycloalkyl, C₁₋₆ haloalkoxy, C₁₋₆ haloalkyl, 5- to 6-membered heterocyclyl, phenyl and 5- to 6-membered heteroaryl, wherein the 5- to 6-membered heterocyclyl, phenyl and 5- to 6-membered heteroaryl are optionally substituted by one or more groups selected from the group consisting of -NR^{a}R^{b}, C₁₋₆ alkyl, C₁₋₆ alkoxy, C₁₋₆ haloalkyl, C₁₋₆ haloalkoxy and 5- to 6-membered heterocyclyl;
each R¹⁸ is independently selected from the group consisting of hydrogen, deuterium, halogen, amino, nitro, cyano, hydroxy, thiol, carboxy, -C(=O)OR^{a}, -C(=O)R^{a}, -C(=O)NR^{a}R^{b}, -OC(=O)R^{a}, -NR^{a}C(=O)R^{b}, -S(=O)₂R^{a}, -NR^{a}S(=O)₂R^{b}, -S(=O)₂NR^{a}R^{b}, -S(=O)R^{a}, -P(=O)R^{a}R^{b}, -NR^{a}S(=O)₂R^{b}, C₁₋₆ alkyl, -OR^{a}, haloalkyl, haloalkoxy, alkenyl, alkynyl, cycloalkyl, heterocyclyl, aryl and heteroaryl, preferably hydrogen, deuterium, C₁₋₆ alkyl and C₁₋₆ haloalkyl;
or, two adjacent R¹⁸, together with the atoms to which they are attached, form a 5-to 6-membered cycloalkyl, 5- to 6-membered heterocyclyl, 6-membered aryl, or 5- to 6-membered heteroaryl, wherein the 5- to 6-membered cycloalkyl, 5- to 6-membered heterocyclyl, 6-membered aryl, or 5- to 6-membered heteroaryl is optionally further substituted by one or more groups selected from the group consisting of deuterium, halogen, amino, -NR^{a}R^{b}, nitro, cyano, hydroxy, thiol, carboxy, -C(=O)OR^{a}, -C(=O)R^{a}, -C(=O)NR^{a}R^{b}, -OC(=O)R^{a}, -NR^{a}C(=O)R^{b}, -S(=O)₂R^{a}, -NR^{a}S(=O)₂R^{b}, -S(=O)₂NR^{a}R^{b}, -S(=O)R^{a}, -P(=O)R^{a}R^{b}, -NR^{a}S(=O)₂R^{b}, -B(OR^{a})₂, alkyl, -OR^{a}, haloalkyl, haloalkoxy, alkenyl, alkynyl, cycloalkyl, heterocyclyl, aryl and heteroaryl;
R^{a} and R^{b} are each independently selected from the group consisting of hydrogen and C₁₋₆ alkyl; or
R^{a} and R^{b}, together with the atoms to which they are attached, form a 5- to 6-membered heterocyclyl, wherein the 5- to 6-membered heterocyclyl is optionally further substituted by C₁₋₆ alkyl;
m is an integer from 0 to 4, preferably 1 or 2;
n is an integer from 0 to 4, preferably 1 or 2;
A¹, A², R², R³, R¹⁰, R^{11a}, R^{11b} are as defined in formula (I).

In another specific embodiment, the compound of formula (I) or a tautomer, mesomer, racemate, enantiomer, diastereomer, or mixture thereof, or a pharmaceutically acceptable salt thereof according to the present invention is a compound of formula (IVC) or formula (VC), or a tautomer, mesomer, racemate, enantiomer, diastereomer, or mixture thereof, or a pharmaceutically acceptable salt thereof, 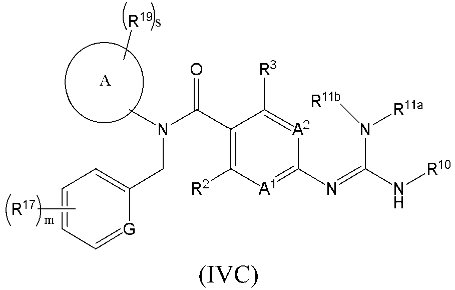 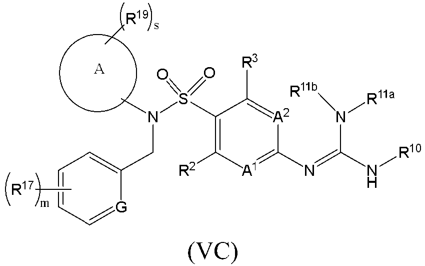 wherein,
ring A is selected from the group consisting of 5- to 10-membered heteroaryl, C₆₋₁₀ aryl, 3- to 10-membered heterocyclyl and 3- to 10-membered cycloalkyl, the 5- to 10-membered heteroaryl, C₆₋₁₀ aryl, 3- to 10-membered heterocyclyl and 3- to 10-membered cycloalkyl; preferably 9- to 10-membered fused heteroaryl, naphthyl, 9-to 10-membered fused heterocyclyl, 9- to 10-membered fused cycloalkyl and C₃₋₆ cycloalkyl;
each R¹⁹ is independently selected from the group consisting of hydrogen, halogen, amino, -NR^{a}R^{b}, nitro, cyano, hydroxy, thiol, carboxy, -C(=O)OR^{a}, -C(=O)R^{a}, -C(=O)NR^{a}R^{b}, -OC(=O)R^{a}, -NR^{a}C(=O)R^{b}, -S(=O)₂R^{a}, -S(=O)R^{a}, -P(=O)R^{a}R^{b}, -NR^{a}S(=O)₂R^{b}, alkyl, -OR^{a}, haloalkyl, haloalkoxy, alkenyl, alkynyl, cycloalkyl, heterocyclyl, aryl and heteroaryl; wherein the cycloalkyl, heterocyclyl, aryl and heteroaryl are optionally further substituted by one or more groups selected from the group consisting of halogen, amino, -NR^{a}R^{b}, nitro, cyano, hydroxy, thiol, carboxy, -C(=O)OR^{a}, -C(=O)R^{a}, -C(=O)NR^{a}R^{b}, -OC(=O)R^{a}, -NR^{a}C(=O)R^{b}, -S(=O)₂R^{a}, -S(=O)R^{a}, -P(=O)R^{a}R^{b}, -NR^{a}S(=O)₂R^{b}, alkyl, -OR^{a}, haloalkyl, haloalkoxy, alkenyl, alkynyl, cycloalkyl, heterocyclyl, aryl and heteroaryl; preferably hydrogen, halogen, C₁₋₆ alkyl, C₁₋₆ haloalkyl, phenyl and 5- to 6-membered heteroaryl, wherein the 5- to 6-membered heteroaryl is optionally substituted by C₁₋₆ alkyl;
G is selected from the group consisting of N and CH;
each R¹⁷ is independently selected from the group consisting of hydrogen, deuterium, halogen, amino, -NR^{a}R^{b}, nitro, cyano, hydroxy, thiol, carboxy, -C(=O)OR^{a}, -C(=O)R^{a}, -C(=O)NR^{a}R^{b}, -OC(=O)R^{a}, -NR^{a}C(=O)R^{b}, -S(=O)₂R^{a}, -NR^{a}S(=O)₂R^{b}, -S(=O)ZNR^{a}R^{b}, -S(=O)R^{a}, -P(=O)R^{a}R^{b}, -NR^{a}S(=O)₂R^{b}, alkyl, -OR^{a}, haloalkyl, haloalkoxy, alkenyl, alkynyl, cycloalkyl, heterocyclyl, aryl and heteroaryl, preferably halogen, C₁₋₆ alkyl, C₃₋₆ cycloalkyl, C₁₋₆ haloalkyl, 5- to 6-membered heterocyclyl, phenyl and 5- to 6-membered heteroaryl;
m is an integer from 0 to 4, preferably 1 or 2;
s is an integer from 0 to 4, preferably 1 or 2;
A¹, A², R², R³, R¹⁰, R^{11a}, R^{11b}, R^{a}, R^{b} are as defined in formula (I).

In a preferred embodiment, in the compound of formula (IVC) or formula (VC) or a tautomer, mesomer, racemate, enantiomer, diastereomer, or mixture thereof, or a pharmaceutically acceptable salt thereof according to the present invention, ring A is 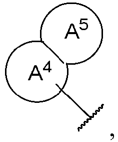 wherein ring A⁴ and A⁵ are each independently selected from the group consisting of C₅₋₆ cycloalkyl, 5- to 6-membered heterocyclyl, 5- to 6-membered heteroaryl and phenyl; specifically, ring A⁴ is selected from the group consisting of C₅₋₆ cycloalkyl, 5- to 6-membered heterocyclyl, 5- to 6-membered heteroaryl and phenyl and ring A⁵ is selected from the group consisting of 5- to 6-membered heteroaryl and phenyl;
preferably, ring A is selected from the group consisting of: 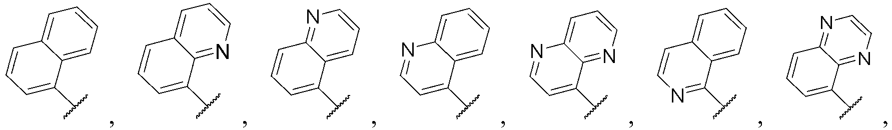 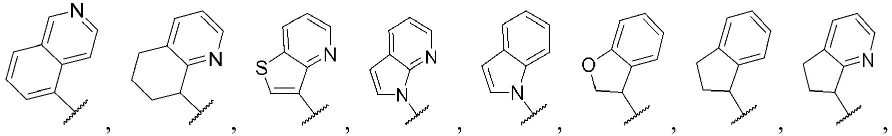 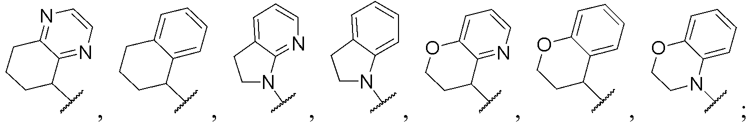
ring A is optionally substituted by one or more R¹⁹, wherein R¹⁹ is as defined in formula (IVC) or formula (VC).

In another preferred embodiment, the compound of formula (I) or a tautomer, mesomer, racemate, enantiomer, diastereomer, or mixture thereof, or a pharmaceutically acceptable salt thereof according to the present invention is a compound of formula (IVD) or formula (VD), or a tautomer, mesomer, racemate, enantiomer, diastereomer, or mixture thereof, or a pharmaceutically acceptable salt thereof, 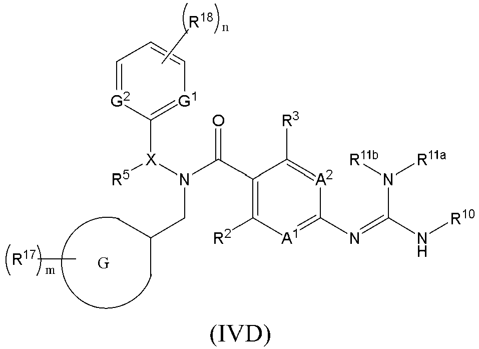 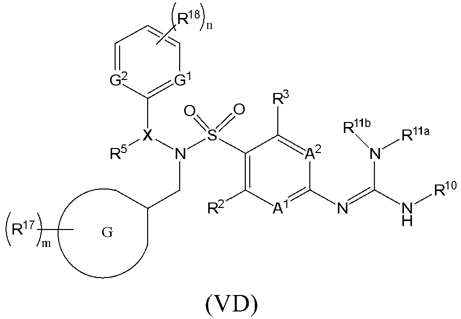 wherein,
X is selected from the group consisting of N and CR⁹;
ring G is selected from the group consisting of 5- to 10-membered heteroaryl, C₆₋₁₀ aryl and 5- to 10-membered heterocyclyl, preferably 5- to 10-membered heteroaryl, phenyl and or 5- to 6-membered heterocyclyl;
G¹ and G² are each independently selected from the group consisting of N and CH;
R⁵ is selected from the group consisting of hydrogen, C₁₋₆ alkyl and C₃₋₆ cycloalkyl;
R⁹ is selected from the group consisting of hydrogen and C₁₋₆ alkyl;
each R¹⁷ is independently selected from the group consisting of hydrogen, deuterium, halogen, amino, -NR^{a}R^{b}, nitro, cyano, hydroxy, thiol, carboxy, -C(=O)OR^{a}, -C(=O)R^{a}, -C(=O)NR^{a}R^{b}, -OC(=O)R^{a}, -NR^{a}C(=O)R^{b}, -S(=O)₂R^{a}, -NR^{a}S(=O)₂R^{b}, -S(=O)ZNR^{a}R^{b}, -S(=O)R^{a}, -P(=O)R^{a}R^{b}, -NR^{a}S(=O)₂R^{b}, alkyl, -OR^{a}, haloalkyl, haloalkoxy, alkenyl, alkynyl, cycloalkyl, heterocyclyl, aryl and heteroaryl, wherein the heterocyclyl, aryl and heteroaryl are optionally further substituted by one or more groups selected from the group consisting of deuterium, halogen, amino, -NR^{a}R^{b}, nitro, cyano, hydroxy, thiol, carboxy, -C(=O)OR^{a}, -C(=O)R^{a}, -C(=O)NR^{a}R^{b}, -OC(=O)R^{a}, -NR^{a}C(=O)R^{b}, -S(=O)₂R^{a}, -NR^{a}S(=O)₂R^{b}, -S(=O)₂NR^{a}R^{b}, -S(=O)R^{a}, -P(=O)R^{a}R^{b}, -NR^{a}S(=O)₂R^{b}, alkyl, -OR^{a}, haloalkyl, haloalkoxy, alkenyl, alkynyl, cycloalkyl, heterocyclyl, aryl and heteroaryl; preferably halogen, C₁₋₆ alkyl, C₁₋₆ alkoxy, C₃₋₆ cycloalkyl, C₁₋₆ haloalkyl, C₁₋₆ haloalkoxy, 5- to 6-membered heterocyclyl, phenyl, 5-to 6-membered heteroaryl, wherein the 5- to 6-membered heterocyclyl, phenyl and 5- to 6-membered heteroaryl are optionally substituted by one or more groups selected from the group consisting of -NR^{a}R^{b}, C₁₋₆ alkyl, C₁₋₆ alkoxy, C₁₋₆ haloalkyl, C₁₋₆ haloalkoxy and 5- to 6-membered heterocyclyl;
each R¹⁸ is independently selected from the group consisting of hydrogen, deuterium, halogen, amino, nitro, cyano, hydroxy, thiol, carboxy, -C(=O)OR^{a}, -C(=O)R^{a}, -C(=O)NR^{a}R^{b}, -OC(=O)R^{a}, -NR^{a}C(=O)R^{b}, -S(=O)₂R^{a}, -NR^{a}S(=O)₂R^{b}, -S(=O)₂NR^{a}R^{b}, -S(=O)R^{a}, -P(=O)R^{a}R^{b}, -NR^{a}S(=O)₂R^{b}, C₁₋₆ alkyl, -OR^{a}, haloalkyl, haloalkoxy, alkenyl, alkynyl, cycloalkyl, heterocyclyl, aryl and heteroaryl, preferably hydrogen, deuterium, C₁₋₆ alkyl, C₁₋₆ haloalkyl;
or, two adjacent R¹⁸, together with the atoms to which they are attached, form a 5-to 6-membered cycloalkyl, 5- to 6-membered heterocyclyl, 6-membered aryl, or 5- to 6-membered heteroaryl, wherein the 5- to 6-membered cycloalkyl, 5- to 6-membered heterocyclyl, 6-membered aryl, or 5- to 6-membered heteroaryl is optionally further substituted by one or more groups selected from the group consisting of deuterium, halogen, amino, -NR^{a}R^{b}, nitro, cyano, hydroxy, thiol, carboxy, alkyl, -OR^{a}, haloalkyl, haloalkoxy, alkenyl, alkynyl, cycloalkyl, heterocyclyl, aryl and heteroaryl;
R^{a} and R^{b} are each independently selected from the group consisting of hydrogen and C₁₋₆ alkyl; or
R^{a} and R^{b}, together with the atoms to which they are attached, form a 5- to 6-membered heterocyclyl, wherein the 5- to 6-membered heterocyclyl is optionally further substituted by C₁₋₆ alkyl;
m is an integer from 0 to 4, preferably 1 or 2;
n is an integer from 0 to 4, preferably 1 or 2;
A¹, A², R², R³, R¹⁰, R^{11a}, R^{11b} are as defined in formula (I).

In another preferred embodiment, in the compound of formula (IVD) or formula (VD) or a tautomer, mesomer, racemate, enantiomer, diastereomer, or mixture thereof, or a pharmaceutically acceptable salt thereof according to the present invention, ring G is selected from the group consisting of

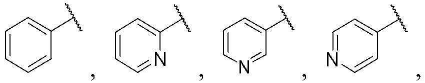

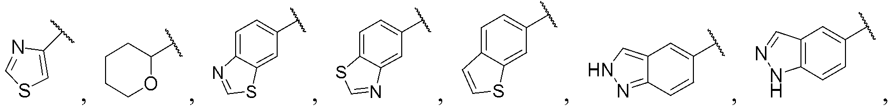

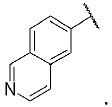

In another preferred embodiment, in the compound of formula or a tautomer, mesomer, racemate, enantiomer, diastereomer, or mixture thereof, or a pharmaceutically acceptable salt thereof according to the present invention,
R¹⁰ is selected from the group consisting of hydrogen, -C(=O)R^{a} and C₁₋₆ alkyl;
R^{11a} is selected from the group consisting of hydrogen, -OR^{a}, -(CH₂)ₚ-OR^{a}, -(CH₂)ₚ-C(=O)OR^{a}, -(CH₂)ₚ-NR^{a}R^{b}, -C(=O)OR^{a}, nitro, C₁₋₆ alkyl, C₃₋₆ cycloalkyl, 4- to 6-membered heterocyclyl, C₆₋₁₀ aryl and 5- to 10-membered heteroaryl, wherein the C₁₋₆ alkyl, C₃₋₆ cycloalkyl, 4- to 6-membered heterocyclyl, C₆₋₁₀ aryl and 5- to 10-membered heteroaryl are optionally further substituted by one or more groups selected from the group consisting of deuterium, halogen, amino, nitro, cyano, hydroxy, thiol, carboxy, haloalkyl, haloalkoxy, alkenyl, alkynyl, cycloalkyl, heterocyclyl, aryl and heteroaryl;
R^{11b} is selected from the group consisting of hydrogen and C₁₋₆ alkyl;
R^{a} and R^{b} are each independently selected from the group consisting of hydrogen and C₁₋₆ alkyl;
or, one of R^{11a} and R^{11b}, together with R¹⁰ and the atoms to which they are attached, form a 5- to 6-membered heterocyclyl, and the other one of R^{11a} and R^{11b} is hydrogen, wherein the 5- to 6-membered heterocyclyl is optionally further substituted by one or more groups selected from the group consisting of halogen, amino, nitro, cyano, hydroxy, thiol, carboxy, alkyl, alkoxy, haloalkyl, haloalkoxy, alkenyl, alkynyl, cycloalkyl, heterocyclyl, aryl and heteroaryl;
p is an integer from 1 to 6, preferably 1.

In another preferred embodiment, in the compound of formula or a tautomer, mesomer, racemate, enantiomer, diastereomer, or mixture thereof, or a pharmaceutically acceptable salt thereof according to the present invention,
R¹⁰ is selected from the group consisting of hydrogen and C₁₋₆ alkyl;
R^{11a} is selected from the group consisting of C₁₋₆ alkyl and hydroxy;
R^{11b} is selected from the group consisting of hydrogen and C₁₋₆ alkyl;
R^{a} and R^{b} are each independently selected from the group consisting of hydrogen and C₁₋₆ alkyl;
or, one of R^{11a} and R^{11b}, together with R¹⁰ and the atoms to which they are attached, form a 5- to 6-membered heterocyclyl, and the other one of R^{11a} and R^{11b} is hydrogen, wherein the 5- to 6-membered heterocyclyl is optionally further substituted by one or more groups selected from the group consisting of halogen, amino, nitro, cyano, hydroxy, thiol, carboxy, alkyl, alkoxy, haloalkyl, haloalkoxy, alkenyl, alkynyl, cycloalkyl, heterocyclyl, aryl and heteroaryl;
p is an integer from 1 to 6, preferably 1, 2, or 3.

In another specific embodiment, the compound of formula (I) or a tautomer, mesomer, racemate, enantiomer, diastereomer, or mixture thereof, or a pharmaceutically acceptable salt thereof according to the present invention is a compound of formula (VIA) or formula (VIB), or a tautomer, mesomer, racemate, enantiomer, diastereomer, or mixture thereof, or a pharmaceutically acceptable salt thereof, 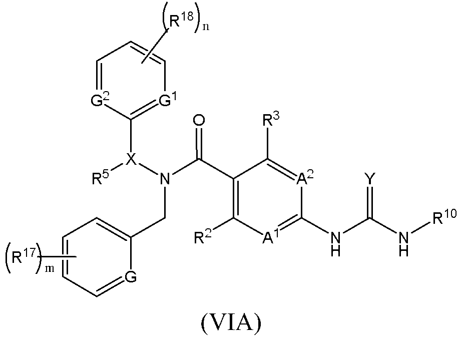 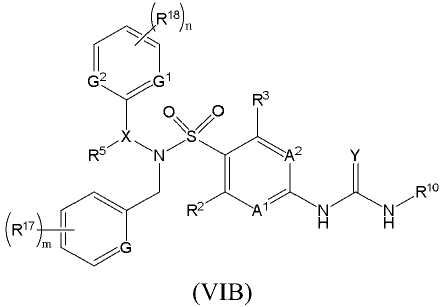 wherein,
Y is selected from the group consisting of O and S;
X is selected from the group consisting of N and CR⁹;
G is selected from the group consisting of N and CH;
G¹ and G² are each independently selected from the group consisting of N and CH;
R⁵ is selected from the group consisting of hydrogen and C₁₋₆ alkyl;
R⁹ is selected from the group consisting of hydrogen and C₁₋₆ alkyl;
R¹⁰ is selected from the group consisting of hydrogen and C₁₋₆ alkyl;
each R¹⁷ is independently selected from the group consisting of hydrogen, deuterium, halogen, amino, -NR^{a}R^{b}, nitro, cyano, hydroxy, thiol, carboxy, -C(=O)OR^{a}, -C(=O)R^{a}, -C(=O)NR^{a}R^{b}, -OC(=O)R^{a}, -NR^{a}C(=O)R^{b}, -S(=O)₂R^{a}, -NR^{a}S(=O)₂R^{b}, -S(=O)ZNR^{a}R^{b}, -S(=O)R^{a}, -P(=O)R^{a}R^{b}, -NR^{a}S(=O)₂R^{b}, alkyl, -OR^{a}, haloalkyl, haloalkoxy, alkenyl, alkynyl, cycloalkyl, heterocyclyl, aryl and heteroaryl, preferably halogen, C₁₋₆ alkyl, C₃₋₆ cycloalkyl or C₁₋₆ haloalkyl;
each R¹⁸ is independently selected from the group consisting of hydrogen, deuterium, halogen, amino, nitro, cyano, hydroxy, thiol, carboxy, -C(=O)OR^{a}, -C(=O)R^{a}, -C(=O)NR^{a}R^{b}, -OC(=O)R^{a}, -NR^{a}C(=O)R^{b}, -S(=O)₂R^{a}, -NR^{a}S(=O)₂R^{b}, -S(=O)₂NR^{a}R^{b}, -S(=O)R^{a}, -P(=O)R^{a}R^{b}, -NR^{a}S(=O)₂R^{b}, C₁₋₆ alkyl, -OR^{a}, haloalkyl, haloalkoxy, alkenyl, alkynyl, cycloalkyl, heterocyclyl, aryl and heteroaryl, preferably hydrogen, deuterium, C₁₋₆ alkyl, C₁₋₆ haloalkyl;
m is an integer from 0 to 4, preferably 1 or 2;
n is an integer from 0 to 4, preferably 1 or 2;
A¹, A², R², R³ are as defined in formula (I).

In another specific embodiment, the compound of formula (I) or a tautomer, mesomer, racemate, enantiomer, diastereomer, or mixture thereof, or a pharmaceutically acceptable salt thereof according to the present invention is a compound of formula (VIIA), formula (VIIB), formula (VIIC), or formula (VIID), or a tautomer, mesomer, racemate, enantiomer, diastereomer, or mixture thereof, or a pharmaceutically acceptable salt thereof, 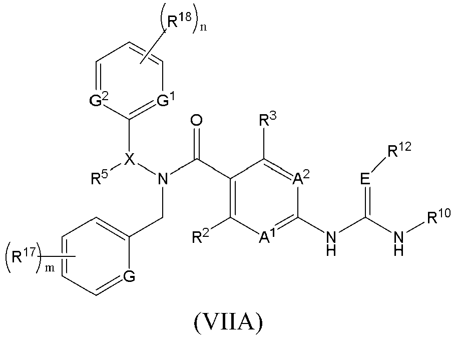 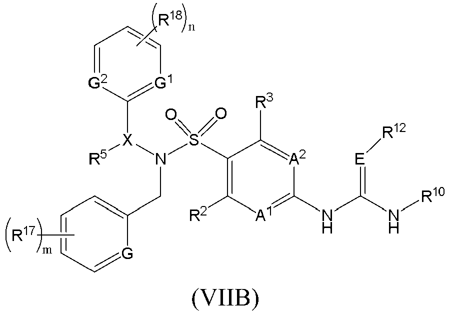 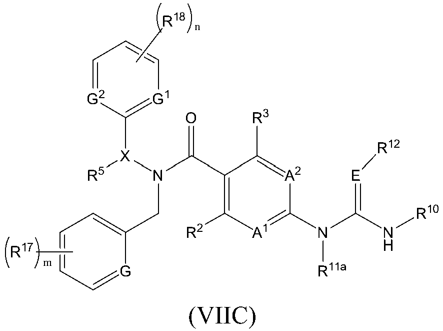 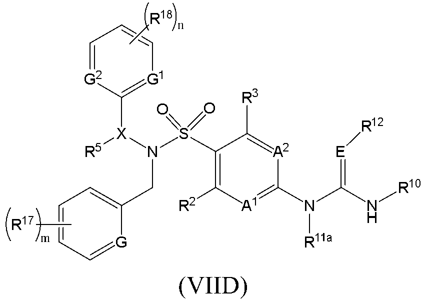 wherein,
E is selected from the group consisting of N and CR¹⁶;
X is selected from the group consisting of N and CR⁹;
G is selected from the group consisting of N and CH;
G¹ and G² are each independently selected from the group consisting of N and CH;
R⁵ is selected from the group consisting of hydrogen and C₁₋₆ alkyl;
R⁹ is selected from the group consisting of hydrogen and C₁₋₆ alkyl;
R¹⁰ is selected from the group consisting of hydrogen and C₁₋₆ alkyl;
R^{11a} is C₁₋₆ alkyl;
R¹² is selected from the group consisting of hydrogen and C₁₋₆ alkyl;
R¹⁶ is selected from the group consisting of hydrogen, C₁₋₆ alkoxy and C₁₋₆ alkyl;
each R¹⁷ is independently selected from the group consisting of hydrogen, deuterium, halogen, amino, -NR^{a}R^{b}, nitro, cyano, hydroxy, thiol, carboxy, -C(=O)OR^{a}, -C(=O)R^{a}, -C(=O)NR^{a}R^{b}, -OC(=O)R^{a}, -NR^{a}C(=O)R^{b}, -S(=O)₂R^{a}, -NR^{a}S(=O)₂R^{b}, -S(=O)ZNR^{a}R^{b}, -S(=O)R^{a}, -P(=O)R^{a}R^{b}, -NR^{a}S(=O)₂R^{b}, alkyl, -OR^{a}, haloalkyl, haloalkoxy, alkenyl, alkynyl, cycloalkyl, heterocyclyl, aryl and heteroaryl, wherein the aryl and heteroaryl are optionally substituted by C₁₋₆ alkyl; preferably halogen, C₁₋₆ alkyl, C₃₋₆ cycloalkyl, C₁₋₆ haloalkyl, 5- to 6-membered heteroaryl, wherein the 5- to 6-membered heteroaryl is optionally substituted by C₁₋₆ alkyl;
each R¹⁸ is independently selected from the group consisting of hydrogen, deuterium, halogen, amino, nitro, cyano, hydroxy, thiol, carboxy, -C(=O)OR^{a}, -C(=O)R^{a}, -C(=O)NR^{a}R^{b}, -OC(=O)R^{a}, -NR^{a}C(=O)R^{b}, -S(=O)₂R^{a}, -NR^{a}S(=O)₂R^{b}, -S(=O)₂NR^{a}R^{b}, -S(=O)R^{a}, -P(=O)R^{a}R^{b}, -NR^{a}S(=O)₂R^{b}, C₁₋₆ alkyl, -OR^{a}, haloalkyl, haloalkoxy, alkenyl, alkynyl, cycloalkyl, heterocyclyl, aryl and heteroaryl, preferably hydrogen, deuterium, C₁₋₆ alkyl, C₁₋₆ haloalkyl;
m is an integer from 0 to 4, preferably 1 or 2;
n is an integer from 0 to 4, preferably 1 or 2;
A¹, A², R², R³ are as defined in formula (I).

In another specific embodiment, the compound of formula (I) or a tautomer, mesomer, racemate, enantiomer, diastereomer, or mixture thereof, or a pharmaceutically acceptable salt thereof according to the present invention is a compound of formula (VIIIA) or formula (VIIIB), or a tautomer, mesomer, racemate, enantiomer, diastereomer, or mixture thereof, or a pharmaceutically acceptable salt thereof, 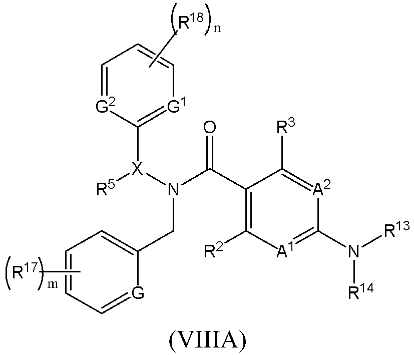 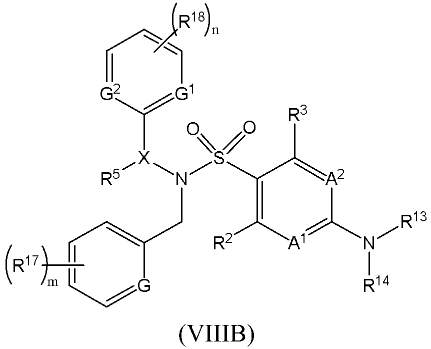 wherein,
X is selected from the group consisting of N and CR⁹;
G is selected from the group consisting of N and CH;
G¹ and G² are each independently selected from the group consisting of N and CH;
R⁵ is selected from the group consisting of hydrogen and C₁₋₆ alkyl;
R⁹ is selected from the group consisting of hydrogen and C₁₋₆ alkyl;
R¹³ is selected from the group consisting of hydrogen and C₁₋₆ alkyl;
R¹⁴ is selected from the group consisting of 5- to 6-membered heteroaryl, wherein the 5- to 6-membered heteroaryl is optionally further substituted by one or more groups selected from the group consisting of halogen, amino, nitro, cyano, hydroxy, thiol, carboxy, alkyl, alkoxy, haloalkyl, haloalkoxy, alkenyl, alkynyl, cycloalkyl, heterocyclyl, aryl and heteroaryl;
or, R¹³ and R¹⁴, together with the nitrogen atom to which they are attached, form a 5- to 6-membered heteroaryl, wherein the 5- to 6-membered heteroaryl is optionally further substituted by one or more groups selected from the group consisting of halogen, amino, nitro, cyano, hydroxy, thiol, carboxy, alkyl, alkoxy, haloalkyl, haloalkoxy, alkenyl, alkynyl, cycloalkyl, heterocyclyl, aryl and heteroaryl;
each R¹⁷ is independently selected from the group consisting of hydrogen, deuterium, halogen, amino, -NR^{a}R^{b}, nitro, cyano, hydroxy, thiol, carboxy, -C(=O)OR^{a}, -C(=O)R^{a}, -C(=O)NR^{a}R^{b}, -OC(=O)R^{a}, -NR^{a}C(=O)R^{b}, -S(=O)₂R^{a}, -NR^{a}S(=O)₂R^{b}, -S(=O)ZNR^{a}R^{b}, -S(=O)R^{a}, -P(=O)R^{a}R^{b}, -NR^{a}S(=O)₂R^{b}, alkyl, -OR^{a}, haloalkyl, haloalkoxy, alkenyl, alkynyl, cycloalkyl, heterocyclyl, aryl and heteroaryl, preferably halogen, C₁₋₆ alkyl, C₃₋₆ cycloalkyl or C₁₋₆ haloalkyl;
each R¹⁸ is independently selected from the group consisting of hydrogen, deuterium, halogen, amino, nitro, cyano, hydroxy, thiol, carboxy, -C(=O)OR^{a}, -C(=O)R^{a}, -C(=O)NR^{a}R^{b}, -OC(=O)R^{a}, -NR^{a}C(=O)R^{b}, -S(=O)₂R^{a}, -NR^{a}S(=O)₂R^{b}, -S(=O)₂NR^{a}R^{b}, -S(=O)R^{a}, -P(=O)R^{a}R^{b}, -NR^{a}S(=O)₂R^{b}, C₁₋₆ alkyl, -OR^{a}, haloalkyl, haloalkoxy, alkenyl, alkynyl, cycloalkyl, heterocyclyl, aryl and heteroaryl, preferably hydrogen, deuterium, C₁₋₆ alkyl, C₁₋₆ haloalkyl;
m is an integer from 0 to 4, preferably 1 or 2;
n is an integer from 0 to 4, preferably 1 or 2;
A¹, A², R², R³ are as defined in formula (I).

In a preferred embodiment, in the compound of formula or a tautomer, mesomer, racemate, enantiomer, diastereomer, or mixture thereof, or a pharmaceutically acceptable salt thereof according to the present invention, R², R³, Rand R⁸ are each independently selected from the group consisting of hydrogen, halogen and C₁₋₆ alkyl.

In another preferred embodiment, in the compound of formula or a tautomer, mesomer, racemate, enantiomer, diastereomer, or mixture thereof, or a pharmaceutically acceptable salt thereof according to the present invention, R¹ is selected from the group consisting of C₁₋₆ alkyl, C₂₋₆ alkenyl, C₂₋₆ alkynyl, C₃₋₆ cycloalkyl, 4- to 6-membered heterocyclyl, C₆₋₁₀ aryl and 5- to 10-membered heteroaryl, wherein the C₁₋₆ alkyl, C₂₋₆ alkenyl, C₂₋₆ alkynyl, C₃₋₆ cycloalkyl, 4- to 6-membered heterocyclyl, C₆₋₁₀ aryl and 5-to 10-membered heteroaryl are optionally further substituted by one or more groups selected from the group consisting of deuterium, halogen, amino, nitro, cyano, hydroxy, thiol, carboxy, alkyl, alkoxy, haloalkyl, haloalkoxy, alkenyl, alkynyl, cycloalkyl, heterocyclyl, aryl and heteroaryl.

In one embodiment, according to the present invention, R⁶ is selected from the group consisting of C₆₋₁₀ aryl, 5- to 10-membered heteroaryl, C₃₋₆ cycloalkyl and 4- to 6-membered heterocyclyl, which are optionally further substituted by substituents selected from the group consisting of halogen and C₁₋₆ alkyl.

In another embodiment, according to the present invention, R⁶ is selected from the group consisting of phenyl, naphthyl, pyridinyl, pyrimidinyl, cyclopropyl, cyclobutyl, cyclopentyl and cyclohexyl, which are optionally further substituted by substituents selected from the group consisting of halogen and C₁₋₆ alkyl.

In another embodiment, according to the present invention, ring A is selected from the group consisting of C₃₋₆ cycloalkyl or 4- to 6-membered heterocyclyl.

In another embodiment, in the compound of formula or a tautomer, mesomer, racemate, enantiomer, diastereomer, or mixture thereof, or a pharmaceutically acceptable salt thereof according to the present invention, X is CR⁹; R⁶ is selected from the group consisting of C₆₋₁₀ aryl, 5- to 10-membered heteroaryl, C₃₋₆ cycloalkyl and 4-to 6-membered heterocyclyl, which are optionally further substituted by substituents selected from the group consisting of halogen and C₁₋₆ alkyl; R⁵ is selected from the group consisting of hydrogen and C₁₋₆ alkyl; and R⁹ is selected from hydrogen.

In another embodiment, in the compound of formula or a tautomer, mesomer, racemate, enantiomer, diastereomer, or mixture thereof, or a pharmaceutically acceptable salt thereof according to the present invention, X is N; R⁶ is selected from the group consisting of C₆₋₁₀ aryl, 5- to 10-membered heteroaryl, C₃₋₆ cycloalkyl and 4-to 6-membered heterocyclyl, which are optionally further substituted by substituents selected from the group consisting of halogen and C₁₋₆ alkyl; and R⁵ is selected from the group consisting of hydrogen and C₁₋₆ alkyl.

In another embodiment, according to the present invention, R⁴ is selected from the group consisting of C₁₋₆ alkyl.

In another embodiment, in the compound of formula or a tautomer, mesomer, racemate, enantiomer, diastereomer, or mixture thereof, or a pharmaceutically acceptable salt thereof according to the present invention,
R¹⁰ is selected from the group consisting of hydrogen and C₁₋₆ alkyl;
R^{11a} is selected from the group consisting of hydrogen, -OR^{a}, -(CH₂)ₚ-OR^{a}, -(CH₂)ₚ-C(=O)OR^{a}, -(CH₂)ₚ-NR^{a}R^{b}, -C(=O)OR^{a}, nitro, C₁₋₆ alkyl and C₃₋₆ cycloalkyl;
R^{11b} is selected from the group consisting of hydrogen and C₁₋₆ alkyl;
R^{a} and R^{b} are each independently selected from the group consisting of hydrogen and C₁₋₆ alkyl;
p is 1.

In another embodiment, in the compound of formula or a tautomer, mesomer, racemate, enantiomer, diastereomer, or mixture thereof, or a pharmaceutically acceptable salt thereof according to the present invention,
R¹⁰ is selected from -C(=O)R^{a};
R^{11a} is selected from the group consisting of hydrogen and C₁₋₆ alkyl;
R^{11b} is selected from the group consisting of hydrogen and C₁₋₆ alkyl;
R^{a} is selected from C₁₋₆ alkyl.

In another embodiment, in the compound of formula or a tautomer, mesomer, racemate, enantiomer, diastereomer, or mixture thereof, or a pharmaceutically acceptable salt thereof according to the present invention,
R¹³ is selected from hydrogen;
R¹⁴ is selected from the group consisting of 5- to 6-membered heteroaryl.

In another embodiment, in the compound of formula or a tautomer, mesomer, racemate, enantiomer, diastereomer, or mixture thereof, or a pharmaceutically acceptable salt thereof according to the present invention, R¹³ and R¹⁴, together with the nitrogen atom to which they are attached, form a 5- to 6-membered heteroaryl, wherein the 5- to 6-membered heteroaryl is optionally further substituted by amino.

Typical compounds of the present invention include, but are not limited to: 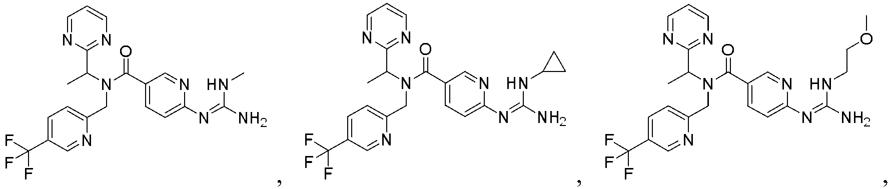 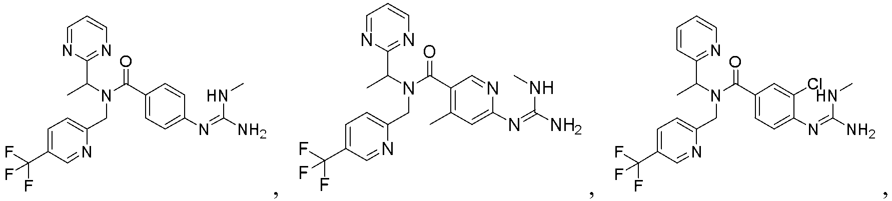 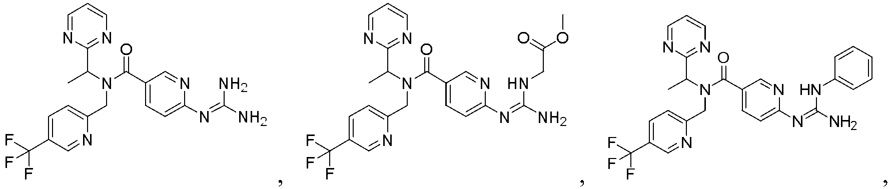 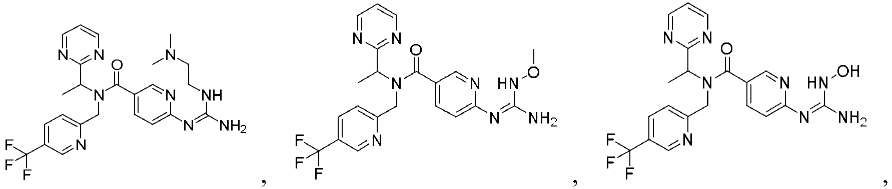 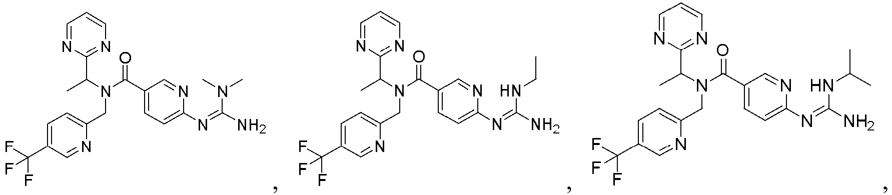 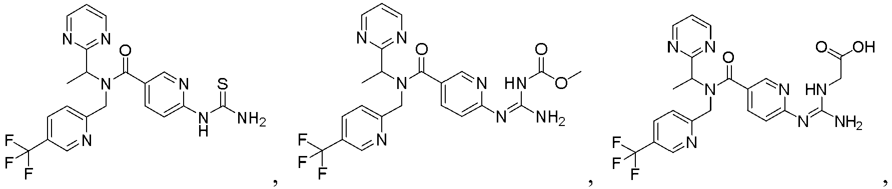 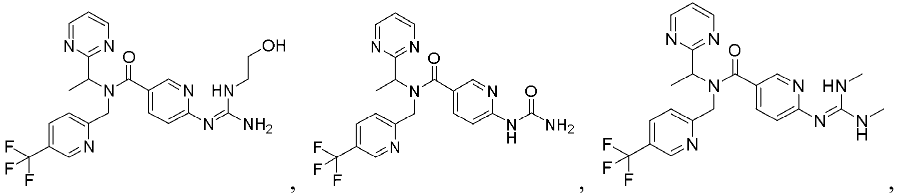 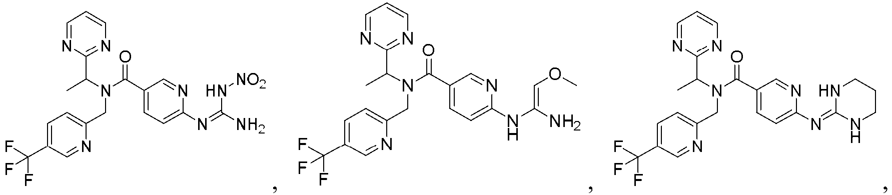 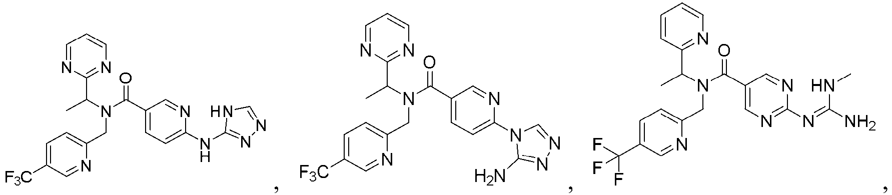 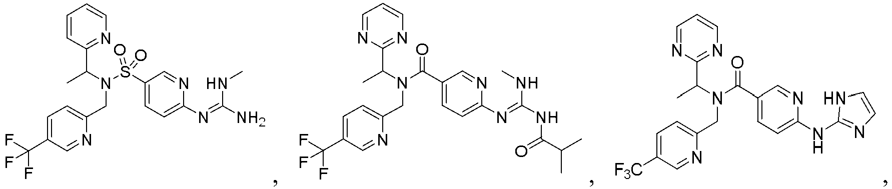 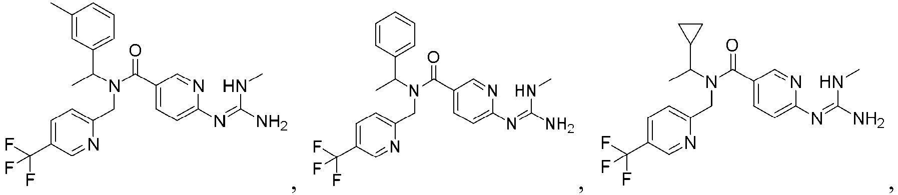 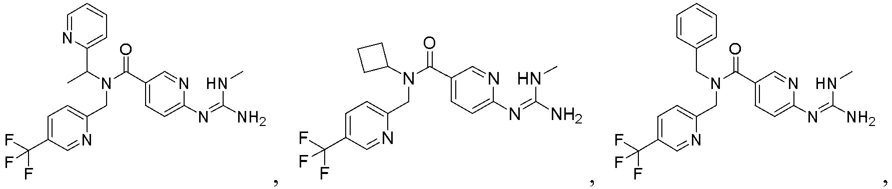 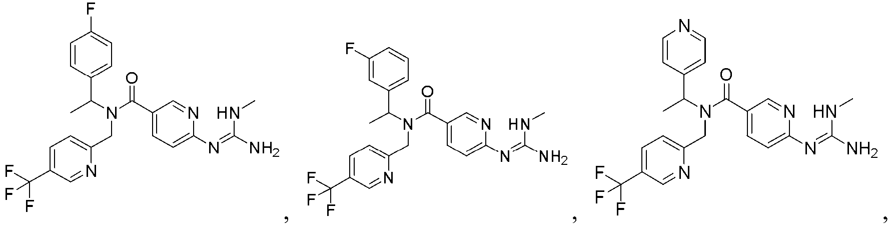 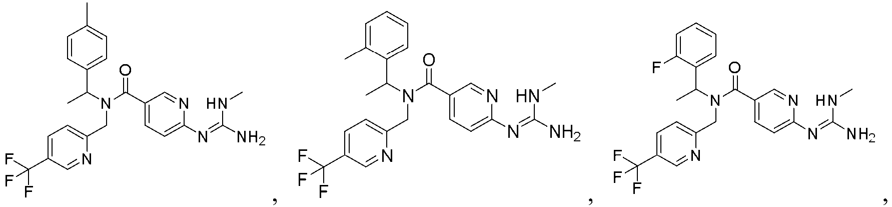 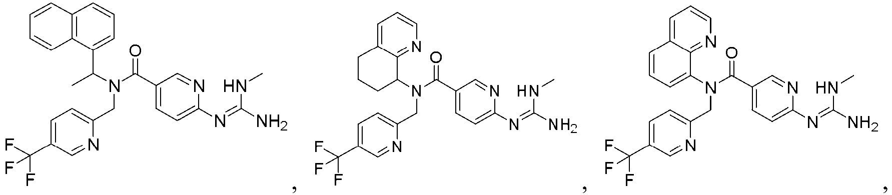 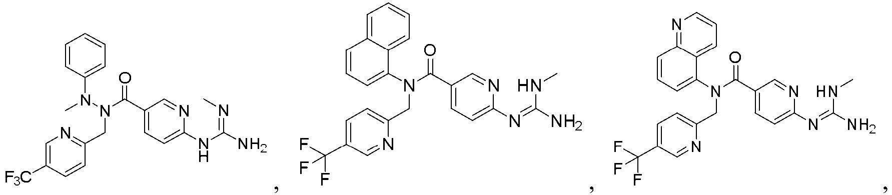 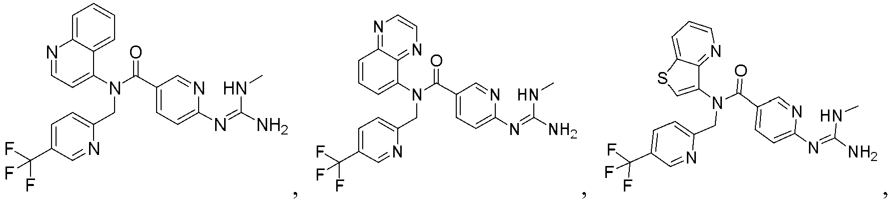 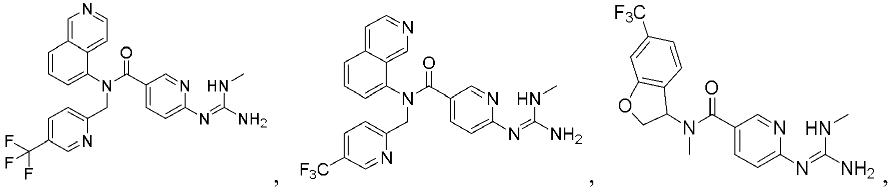 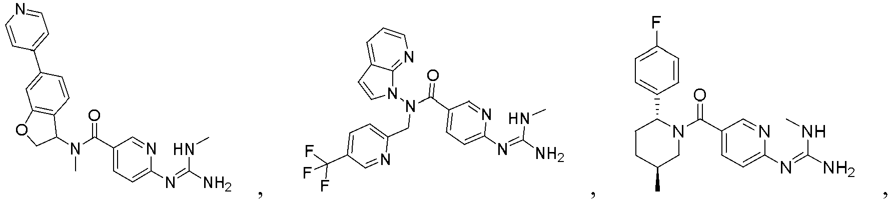 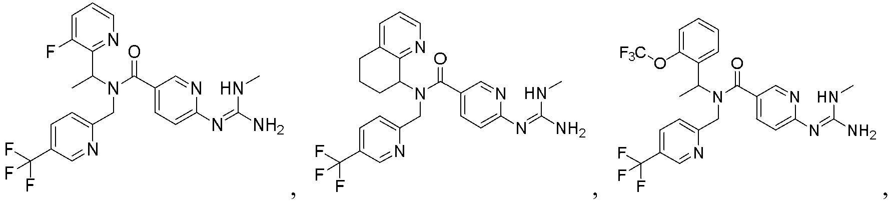 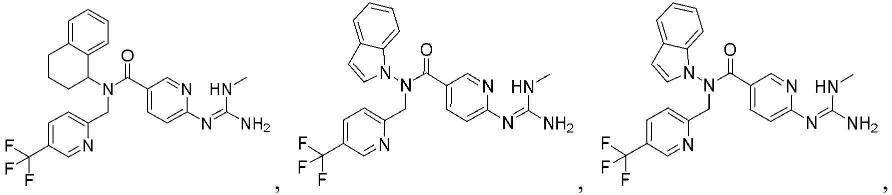 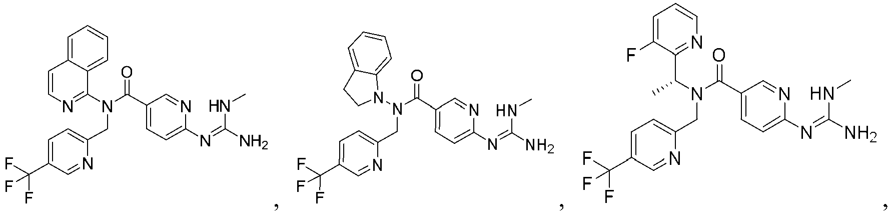 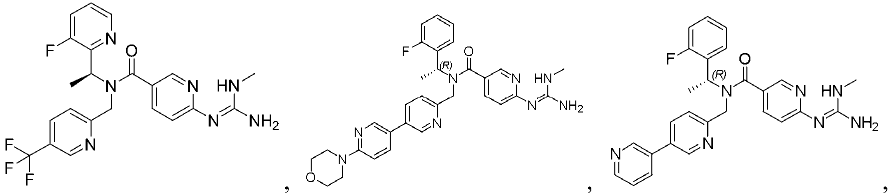 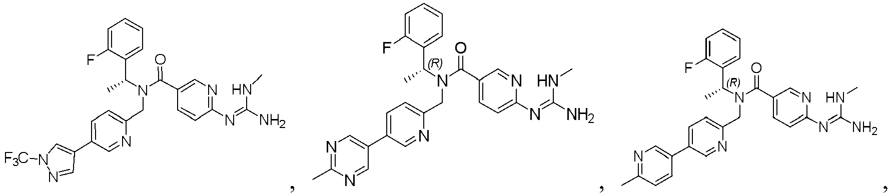 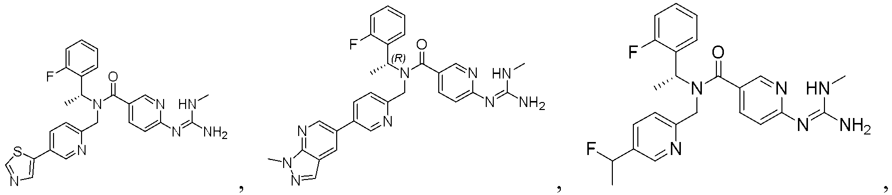 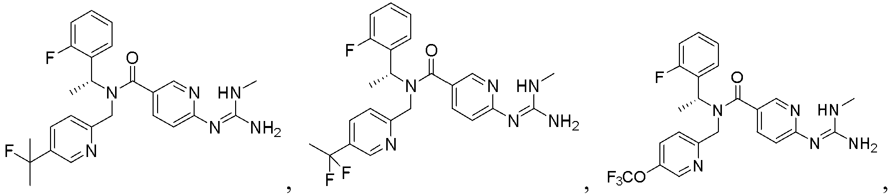 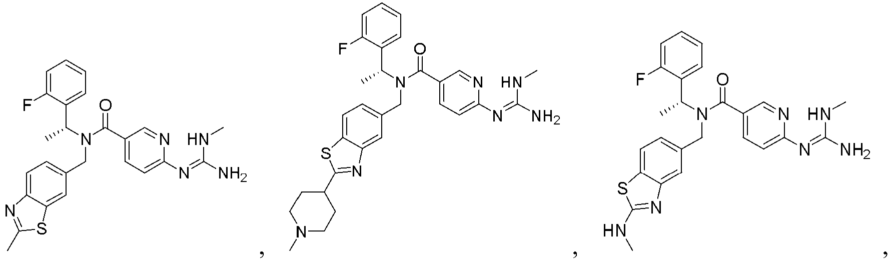 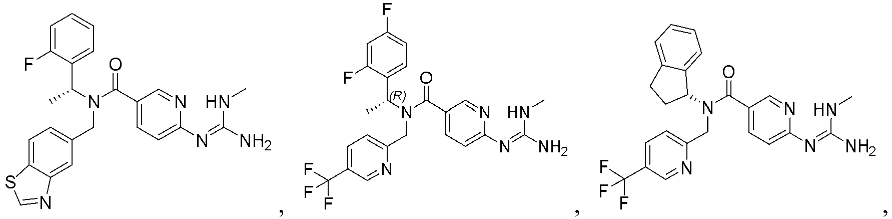 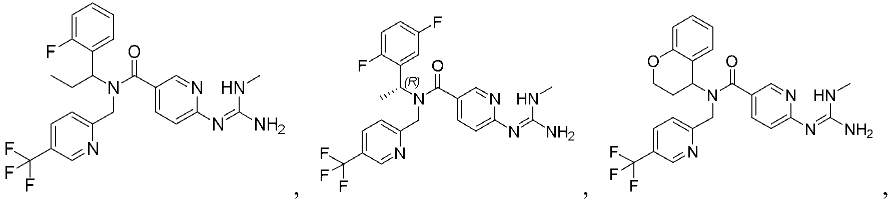 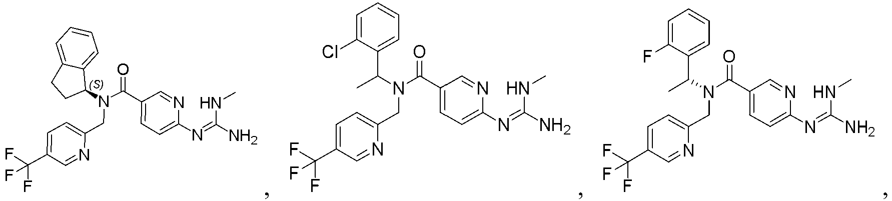 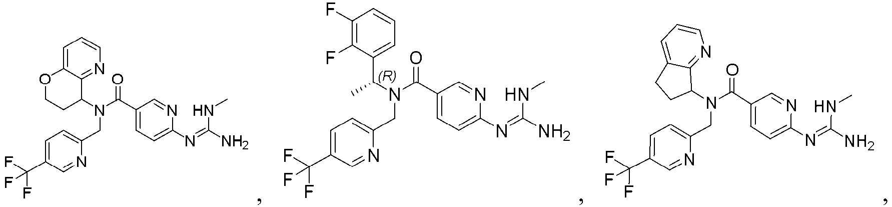 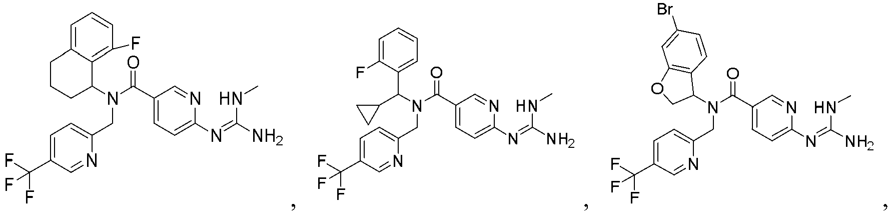 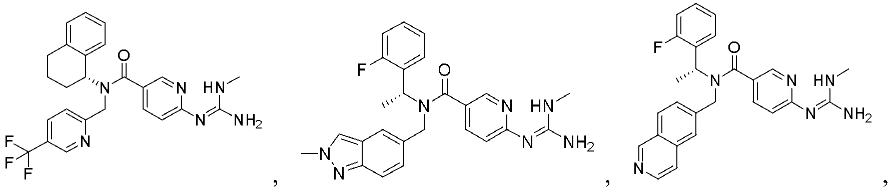 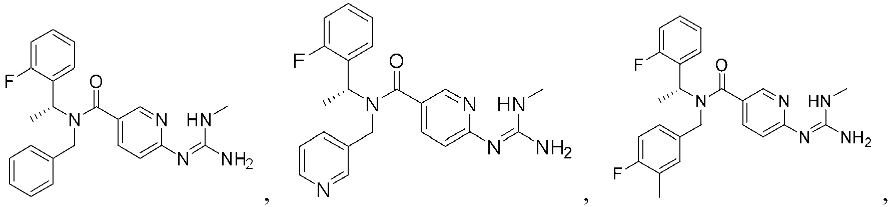 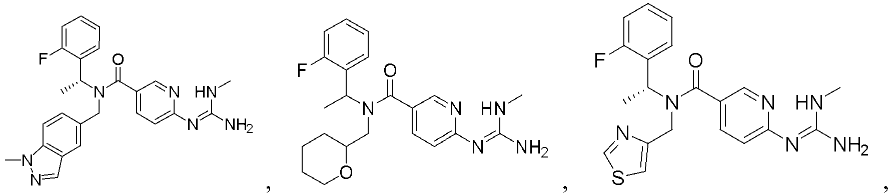 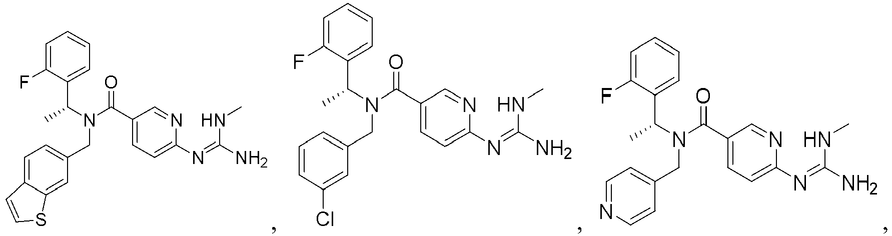 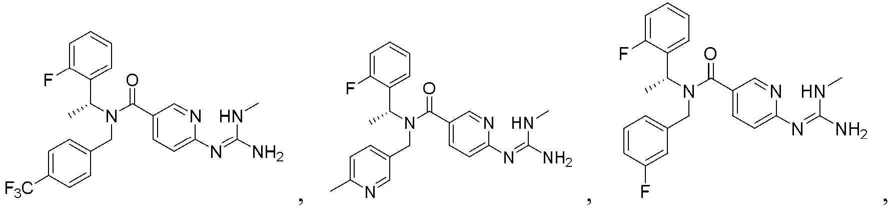 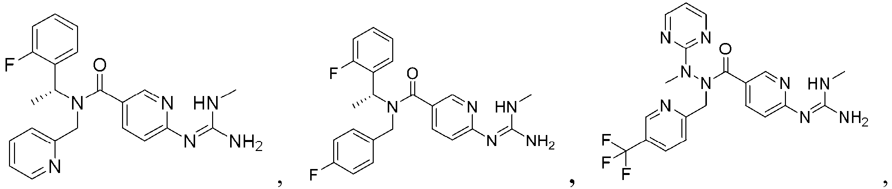 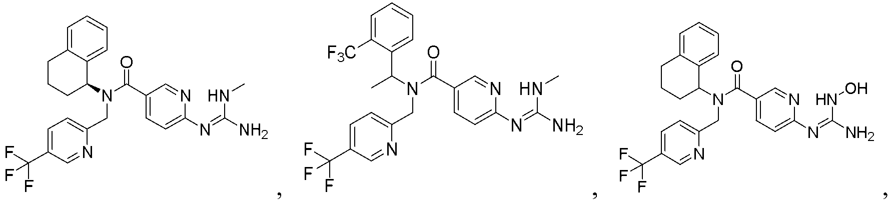 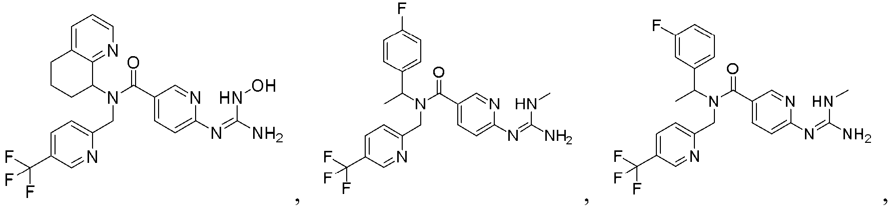 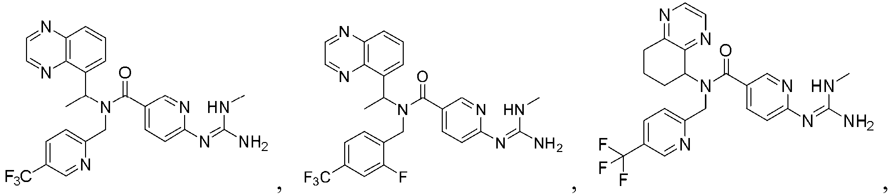 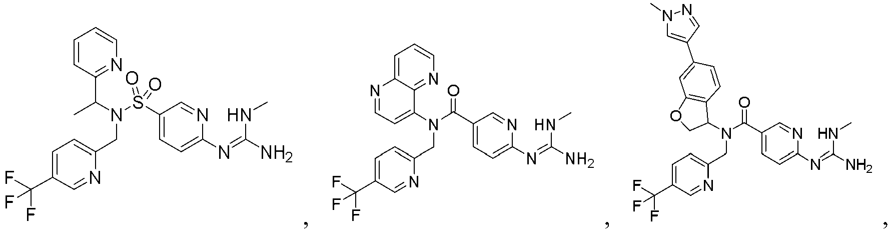 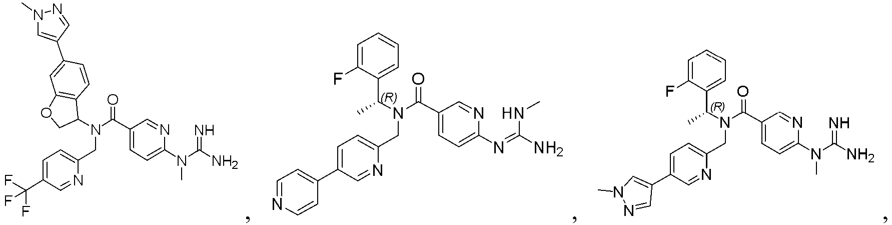 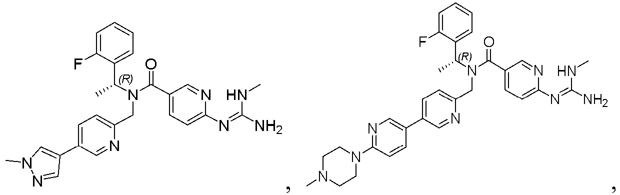 or a mesomer, racemate, enantiomer, diastereomer, or mixture thereof, or a pharmaceutically acceptable salt thereof.

The present invention further provides a method for preparing the compound of formula (I) or a mesomer, racemate, enantiomer, diastereomer, or mixture thereof, or a pharmaceutically acceptable salt thereof according to the present invention, comprising the following step of: 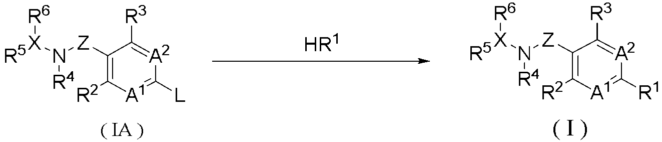
reacting a compound of formula (IA) with a compound HR¹ via a substitution reaction or a coupling reaction to obtain the compound of formula (I) or a mesomer, racemate, enantiomer, diastereomer, or mixture thereof, or a pharmaceutically acceptable salt thereof;
wherein, L is halogen;
A¹, A², R¹~R⁶, X and Z are as defined in formula (I).

The present invention further provides a method for preparing the compound of formula (I-1) or a mesomer, racemate, enantiomer, diastereomer, or mixture thereof, or a pharmaceutically acceptable salt thereof according to the present invention, comprising the following step of: 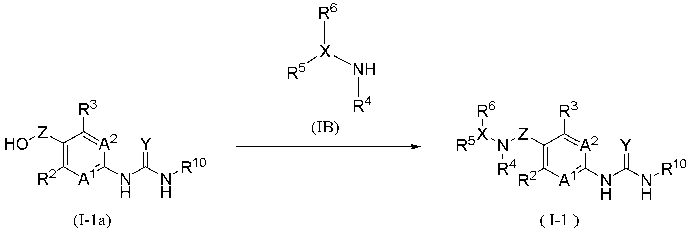
reacting a compound of formula (I-1a) with a compound of formula (IB) via a condensation reaction to obtain the compound of formula (I-1) or a mesomer, racemate, enantiomer, diastereomer, or mixture thereof, or a pharmaceutically acceptable salt thereof;
wherein A¹, A², X, Y, Z, R²~R⁶ and R¹⁰ are as defined in formula (I-1).

The present invention further provides a method for preparing the compound of formula (I-2) or a mesomer, racemate, enantiomer, diastereomer, or mixture thereof, or a pharmaceutically acceptable salt thereof according to the present invention, comprising the following steps of: 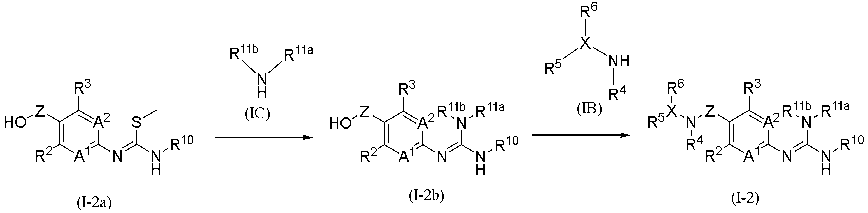
reacting a compound of formula (I-2a) with a compound of formula (IC) via a substitution reaction to obtain a compound of formula (I-2b); and then reacting the compound of formula (I-2b) with a compound of formula (IB) to obtain the compound of formula (I-2) or a mesomer, racemate, enantiomer, diastereomer, or mixture thereof, or a pharmaceutically acceptable salt thereof;
wherein A¹, A², X, Z, R²~R⁶, R¹⁰, R^{11a} and R^{11b} are as defined in formula (I-2).

The present invention further provides a method for preparing the compound of formula (I-2) or a mesomer, racemate, enantiomer, diastereomer, or mixture thereof, or a pharmaceutically acceptable salt thereof according to the present invention, comprising the following steps of: 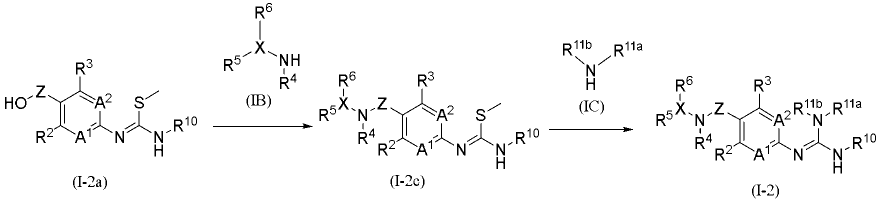
reacting a compound of formula (I-2a) with a compound of formula (IB) via a substitution reaction to obtain a compound of formula (I-2c); and then reacting the compound of formula (I-2c) with a compound of formula (IC) to obtain the compound of formula (I-2) or a mesomer, racemate, enantiomer, diastereomer, or mixture thereof, or a pharmaceutically acceptable salt thereof;
wherein A¹, A², X, Z, R²~R⁶, R¹⁰, R^{11a} and R^{11b} are as defined in formula (I-2).

The present invention further provides a method for preparing the compound of formula (I-2) or a mesomer, racemate, enantiomer, diastereomer, or mixture thereof, or a pharmaceutically acceptable salt thereof according to the present invention, comprising the following step of: 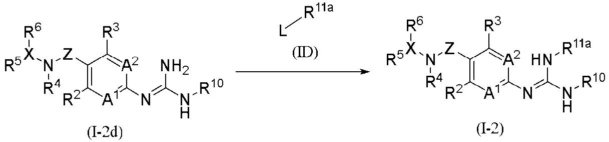
reacting a compound of formula (I-2d) with a compound of formula (ID) via a condensation or substitution reaction to obtain the compound of formula (I-2) or a mesomer, racemate, enantiomer, diastereomer, or mixture thereof, or a pharmaceutically acceptable salt thereof;
wherein, L is halogen; A¹, A², X, Z, R²~R⁶, R¹⁰, R^{11a} and R^{11b} are as defined in formula (I-2).

The present invention further provides a method for preparing the compound of formula (I-2) or a mesomer, racemate, enantiomer, diastereomer, or mixture thereof, or a pharmaceutically acceptable salt thereof according to the present invention, comprising the following step of: 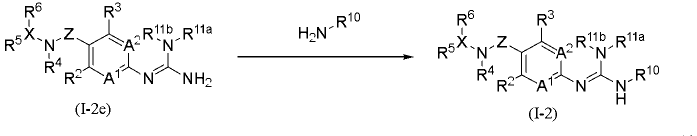
reacting a compound of formula (I-2e) with a compound H₂NR¹⁰ via a condensation or substitution reaction to obtain the compound of formula (I-2) or a mesomer, racemate, enantiomer, diastereomer, or mixture thereof, or a pharmaceutically acceptable salt thereof;
wherein A¹, A², X, Z, R²~R⁶, R¹⁰, R^{11a} and R^{11b} are as defined in formula (I-2).

The present invention further provides a pharmaceutical composition comprising the compound or a mesomer, racemate, enantiomer, diastereomer, or mixture thereof, or a pharmaceutically acceptable salt thereof according to the present invention, and a pharmaceutically acceptable carrier or excipient.

The present invention further relates to a use of the compound or a mesomer, racemate, enantiomer, diastereomer, or mixture thereof, or a pharmaceutically acceptable salt thereof according to the present invention, or a pharmaceutical composition comprising the same in the preparation of a PRMT5 inhibitor.

The present invention further relates to a use of the compound or a mesomer, racemate, enantiomer, diastereomer, or mixture thereof, or a pharmaceutically acceptable salt thereof according to the present invention, or a pharmaceutical composition comprising the same in the preparation of a medicament for the prevention and/or treatment of diseases associated with PRMT5 activity, wherein the diseases are preferably cancer and tumor-related diseases.

The present invention further relates to a compound or a mesomer, racemate, enantiomer, diastereomer, or mixture thereof, or a pharmaceutically acceptable salt thereof according to the present invention, or a pharmaceutical composition comprising the same, for use as a PRMT5 inhibitor.

The present invention further relates to a compound or a mesomer, racemate, enantiomer, diastereomer, or mixture thereof, or a pharmaceutically acceptable salt thereof according to the present invention, or a pharmaceutical composition comprising the same, for use as a medicament for the prevention and/or treatment of diseases associated with PRMT5 activity, wherein the diseases are preferably cancer and tumor-related diseases.

The present invention further relates to a method for inhibiting PRMT5, comprising administering to a patient in need thereof an effective amount of the compound or a mesomer, racemate, enantiomer, diastereomer, or mixture thereof, or a pharmaceutically acceptable salt thereof according to the present invention, or a pharmaceutical composition comprising the same.

The present invention further relates to a method for preventing and/or treating diseases associated with PRMT5 activity, comprising administering to a patient in need thereof a prophylactically or therapeutically effective amount of the compound or a mesomer, racemate, enantiomer, diastereomer, or mixture thereof, or a pharmaceutically acceptable salt thereof according to the present invention, or a pharmaceutical composition comprising the same.

In a preferred embodiment, the cancer and tumor-related diseases according to the present invention are bladder cancer.

The pharmaceutical composition containing the active ingredient can be in a form suitable for oral administration, for example, a tablet, troche, lozenge, aqueous or oily suspension, dispersible powder or granule, emulsion, hard or soft capsule, syrup or elixir. An oral composition can be prepared according to any known method in the art for the preparation of pharmaceutical composition. Such composition can contain one or more ingredients selected from the group consisting of sweeteners, flavoring agents, colorants and preservatives, in order to provide a pleasing and palatable pharmaceutical formulation. The tablet contains the active ingredient in admixture with nontoxic, pharmaceutically acceptable excipients suitable for the preparation of tablets. These excipients can be inert excipients, such as calcium carbonate, sodium carbonate, lactose, calcium phosphate or sodium phosphate; granulating and disintegrating agents, such as microcrystalline cellulose, cross-linked sodium carboxymethyl cellulose, corn starch or alginic acid; binders, such as starch, gelatin, polyvinylpyrrolidone or acacia; and lubricants, such as magnesium stearate, stearic acid or talc. The tablet can be uncoated or coated by means of known techniques which can mask drug taste or delay the disintegration and absorption of the active ingredient in the gastrointestinal tract, thereby providing sustained release over an extended period. For example, a water-soluble taste masking material can be used, such as hydroxypropyl methylcellulose or hydroxypropyl cellulose, or an extended release material can be used, such as ethyl cellulose, cellulose acetate butyrate.

An oral formulation can also be provided as hard gelatin capsules in which the active ingredient is mixed with an inert solid diluent such as calcium carbonate, calcium phosphate or kaolin, or as soft gelatin capsules in which the active ingredient is mixed with a water-soluble carrier such as polyethylene glycol or an oil medium such as peanut oil, liquid paraffin or olive oil.

An aqueous suspension contains the active ingredient in admixture with an excipient suitable for the preparation of aqueous suspension. Such excipient is a suspending agent, such as sodium carboxymethylcellulose, methylcellulose, hydroxypropylmethylcellulose, sodium alginate, polyvinylpyrrolidone and acacia; a dispersant or wetting agent. The aqueous suspension can also contain one or more preservatives such as ethylparaben or *n*-propylparaben, one or more colorants, one or more flavoring agents, and one or more sweeteners such as sucrose, saccharin or aspartame.

An oil suspension can be formulated by suspending the active ingredient in a vegetable oil such as peanut oil, olive oil, sesame oil or coconut oil, or in a mineral oil such as liquid paraffin. The oil suspension can contain a thickener, such as beeswax, hard paraffin or cetyl alcohol. The above sweetener and flavoring agent can be added to provide a palatable formulation. These compositions can be preserved by addition of an antioxidant, such as butylated hydroxyanisole or α-tocopherol.

The pharmaceutical composition of the present invention can also be in the form of an oil-in-water emulsion. The oil phase can be a vegetable oil such as olive oil or peanut oil, or a mineral oil such as liquid paraffin or a mixture thereof. Suitable emulsifying agent can be naturally occurring phosphatides, such as soy bean lecithin, and esters or partial esters derived from fatty acids and hexitol anhydrides such as sorbitan monooleate, and condensation products of said partial esters with ethylene oxide such as polyoxyethylene sorbitol monooleate. The emulsion can also contain a sweetener, flavoring agent, preservative and antioxidant. Syrup and elixir can be formulated with a sweetener, such as glycerol, propylene glycol, sorbitol or sucrose. Such formulations can also contain a moderator, a preservative, a colorant and an antioxidant.

The pharmaceutical composition of the present invention can also be in the form of a sterile injectable aqueous solution. The acceptable vehicles and solvents that can be employed include water, Ringer's solution and isotonic sodium chloride solution. The sterile injectable formulation can be a sterile injectable oil-in-water microemulsion in which the active ingredient is dissolved in the oil phase. For example, the active ingredient can be firstly dissolved in a mixture of soybean oil and lecithin, then the oil solution is introduced into a mixture of water and glycerol and processed to form a microemulsion. The injectable solution or microemulsion can be injected into a patient's bloodstream by local bolus injection. Alternatively, it can be advantageous to administrate the solution or microemulsion in such a way as to maintain a constant circulating concentration of the compound of the present invention. In order to maintain such a constant concentration, a continuous intravenous delivery device can be utilized.

The pharmaceutical composition of the present invention can be in the form of a sterile injectable aqueous or oily suspension for intramuscular and subcutaneous administration. Such a suspension can be formulated with suitable dispersants or wetting agents and suspending agents as described above according to known techniques. The sterile injectable formulation can also be a sterile injectable solution or suspension prepared in a nontoxic and parenterally acceptable diluent or solvent, such as a solution prepared in 1,3-butanediol. Moreover, sterile fixed oils can easily be used as a solvent or suspending medium. For this purpose, any blending fixed oils including synthetic mono- or di-glyceride can be employed. Moreover, fatty acids such as oleic acid can also be employed in the preparation of an injection.

The compound of the present invention can be administrated in the form of a suppository for rectal administration. These pharmaceutical compositions can be prepared by mixing the drug with a suitable non-irritating excipient that is solid at ordinary temperatures, but liquid in the rectum, thereby melting in the rectum to release the drug. Such materials include cocoa butter, glycerin gelatin, hydrogenated vegetable oils, mixtures of polyethylene glycols of various molecular weights and fatty acid esters of polyethylene glycols.

It is well known to those skilled in the art that the dosage of a drug depends on a variety of factors including, but not limited to the following factors of activity of a specific compound, age of the patient, weight of the patient, general health of the patient, behavior of the patient, diet of the patient, administration time, administration route, excretion rate, drug combination and the like. In addition, the optimal treatment, such as treatment mode, daily dose of the active compound or the type of pharmaceutically acceptable salt can be verified according to the traditional therapeutic regimens.

The present invention can contain a composition comprising the compound or the pharmaceutically acceptable salt, hydrate or solvate as an active ingredient, and a pharmaceutically acceptable carrier or excipient, which is formulated into a clinically acceptable formulation. The derivatives of the present invention can be used in combination with other active ingredients as long as they do not cause other adverse effects such as allergic reactions and the like. The compound of the present invention can be used as the sole active ingredient, and can also be used in combination with other anticancer agents or immune checkpoint inhibitors. A combination therapy is achieved by administrating the individual therapeutic components simultaneously, separately or sequentially.

### Definition of the terms

Unless otherwise stated, the terms used in the specification and claims have the meanings described below.

The term "alkyl" refers to a saturated aliphatic hydrocarbon group, which is a straight or branched chain group comprising 1 to 20 carbon atoms, preferably an alkyl having 1 to 12 carbon atoms, and more preferably an alkyl having 1 to 6 carbon atoms. Non-limiting examples include methyl, ethyl, *n*-propyl, isopropyl, *n*-butyl, isobutyl, *tert-*butyl, *sec-*butyl, *n*-pentyl, 1,1-dimethylpropyl, 1,2-dimethylpropyl, 2,2-dimethylpropyl, 1-ethylpropyl, 2-methylbutyl, 3-methylbutyl, *n*-hexyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2-ethylbutyl, 2-methylpentyl, 3-methylpentyl, 4-methylpentyl, 2,3-dimethylbutyl, *n*-heptyl, 2-methylhexyl, 3-methylhexyl, 4-methylhexyl, 5-methylhexyl, 2,3-dimethylpentyl, 2,4-dimethylpentyl, 2,2-dimethylpentyl, 3,3-dimethylpentyl, 2-ethylpentyl, 3-ethylpentyl, *n*-octyl, 2,3-dimethylhexyl, 2,4-dimethylhexyl, 2,5-dimethylhexyl, 2,2-dimethylhexyl, 3,3-dimethylhexyl, 4,4-dimethylhexyl, 2-ethylhexyl, 3-ethylhexyl, 4-ethylhexyl, 2-methyl-2-ethylpentyl, 2-methyl-3-ethylpentyl, *n*-nonyl, 2-methyl-2-ethylhexyl, 2-methyl-3-ethylhexyl, 2,2-diethylpentyl, *n*-decyl, 3,3-diethylhexyl, 2,2-diethylhexyl and various branched isomers thereof. More preferably, the alkyl group is a lower alkyl having 1 to 6 carbon atoms and non-limiting examples include methyl, ethyl, *n*-propyl, isopropyl, *n*-butyl, isobutyl, *tert-*butyl, *sec*-butyl, *n*-pentyl, 1,1-dimethylpropyl, 1,2-dimethylpropyl, 2,2-dimethylpropyl, 1-ethylpropyl, 2-methylbutyl, 3-methylbutyl, *n*-hexyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2-ethylbutyl, 2-methylpentyl, 3-methylpentyl, 4-methylpentyl, 2,3-dimethylbutyl and the like. The alkyl group can be substituted or unsubstituted. When substituted, the substituent group(s) can be substituted at any available connection point. The substituent group(s) is preferably one or more group(s) independently selected from the group consisting of alkyl, alkenyl, alkynyl, alkoxy, alkylthio, alkylamino, halogen, thiol, hydroxy, nitro, cyano, cycloalkyl, heterocyclyl, aryl, heteroaryl, cycloalkoxy, heterocycloalkoxy, cycloalkylthio, heterocyclylthio, oxo, carboxy and alkoxycarbonyl.

The term "alkenyl" refers to an alkyl group as defined above consisting of at least two carbon atoms and at least one carbon-carbon double bond, such as vinyl, 1-propenyl, 2-propenyl, 1-, 2- or 3-butenyl and the like. The alkenyl group can be substituted or unsubstituted. When substituted, the substituent group(s) is preferably one or more group(s) independently selected from the group consisting of alkyl, alkenyl, alkynyl, alkoxy, alkylthio, alkylamino, halogen, thiol, hydroxy, nitro, cyano, cycloalkyl, heterocyclyl, aryl, heteroaryl, cycloalkoxy, heterocycloalkoxy, cycloalkylthio and heterocyclylthio.

The term "alkynyl" refers to an alkyl group as defined above consisting of at least two carbon atoms and at least one carbon-carbon triple bond, such as ethynyl, propynyl, butynyl and the like. The alkynyl group can be substituted or unsubstituted. When substituted, the substituent group(s) is preferably one or more group(s) independently selected from the group consisting of alkyl, alkenyl, alkynyl, alkoxy, alkylthio, alkylamino, halogen, thiol, hydroxy, nitro, cyano, cycloalkyl, heterocyclyl, aryl, heteroaryl, cycloalkoxy, heterocycloalkoxy, cycloalkylthio and heterocyclylthio.

The term "cycloalkyl" refers to a saturated or partially unsaturated monocyclic or polycyclic hydrocarbon group having 3 to 20 carbon atoms, preferably 3 to 12 carbon atoms, and more preferably 3 to 6 carbon atoms. Non-limiting examples of monocyclic cycloalkyl include cyclopropyl, cyclobutyl, cyclopentyl, cyclopentenyl, cyclohexyl, cyclohexenyl, cyclohexadienyl, cycloheptyl, cycloheptatrienyl, cyclooctyl and the like. Polycyclic cycloalkyl includes a cycloalkyl having a spiro ring, fused ring or bridged ring.

The term "spiro cycloalkyl" refers to a 5- to 20-membered polycyclic group with individual rings sharing one commom carbon atom (called a spiro atom), wherein one or more rings can contain one or more double bonds, but none of the rings has a completely conjugated π-electron system. The spiro cycloalkyl is preferably a 6- to 14-membered spiro cycloalkyl, and more preferably a 6- to 10-membered spiro cycloalkyl. According to the number of the spiro atoms shared between the rings, the spiro cycloalkyl can be divided into a mono-spiro cycloalkyl, a di-spiro cycloalkyl, or a poly-spiro cycloalkyl, and the spiro cycloalkyl is preferably a mono-spiro cycloalkyl or di-spiro cycloalkyl, and more preferably a 4-membered/4-membered, 4-membered/5-membered, 4-membered/6-membered, 5-membered/5-membered, or 5-membered/6-membered mono-spiro cycloalkyl. Non-limiting examples of spiro cycloalkyl include:

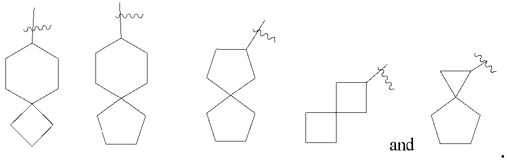

The term "fused cycloalkyl" refers to a 5- to 20-membered all-carbon polycyclic group, wherein each ring in the system shares an adjacent pair of carbon atoms with another ring, and one or more rings can contain one or more double bonds, but none of the rings has a completely conjugated π-electron system. The fused cycloalkyl is preferably a 6- to 14-membered fused cycloalkyl, and more preferably a 6- to 10-membered fused cycloalkyl. According to the number of membered rings, the fused cycloalkyl can be divided into a bicyclic, tricyclic, tetracyclic or polycyclic fused cycloalkyl, and the fused cycloalkyl is preferably a bicyclic or tricyclic fused cycloalkyl, and more preferably a 5-membered/5-membered, or 5-membered/6-membered bicyclic fused cycloalkyl. Non-limiting examples of fused cycloalkyl include:

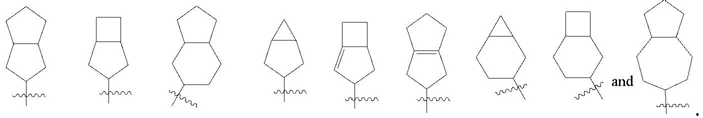

The term "bridged cycloalkyl" refers to a 5- to 20-membered all-carbon polycyclic group, wherein any two rings in the system share two disconnected carbon atoms, and one or more rings can have one or more double bonds, but none of the rings has a completely conjugated π-electron system. The bridged cycloalkyl is preferably a 6- to 14-membered bridged cycloalkyl, and more preferably a 6- to 10-membered bridged cycloalkyl. According to the number of membered rings, the bridged cycloalkyl can be divided into a bicyclic, tricyclic, tetracyclic or polycyclic bridged cycloalkyl, and the bridged cycloalkyl is preferably a bicyclic, tricyclic or tetracyclic bridged cycloalkyl, and more preferably a bicyclic or tricyclic bridged cycloalkyl. Non-limiting examples of bridged cycloalkyl include:

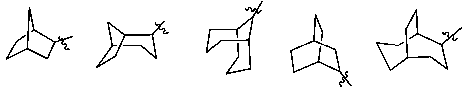

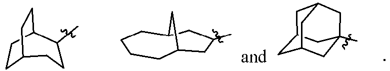

The cycloalkyl ring can be fused to the ring of aryl, heteroaryl or heterocyclyl, wherein the ring linking to the parent structure is cycloalkyl. Non-limiting examples include indanyl, tetrahydronaphthyl, benzocycloheptyl and the like. The cycloalkyl can be optionally substituted or unsubstituted. When substituted, the substituent group(s) is preferably one or more group(s) independently selected from the group consisting of alkyl, alkenyl, alkynyl, alkoxy, alkylthio, alkylamino, halogen, thiol, hydroxy, nitro, cyano, cycloalkyl, heterocyclyl, aryl, heteroaryl, cycloalkoxy, heterocycloalkoxy, cycloalkylthio, heterocyclylthio, oxo, carboxy and carboxylic ester group.

The term "heterocyclyl" refers to a 3- to 20-membered saturated or partially unsaturated monocyclic or polycyclic hydrocarbon group, wherein one or more ring atoms are heteroatoms selected from the group consisting of N, O and S(O)ₘ (wherein m is an integer of 0 to 2), but excluding -O-O-, -O-S- or -S-S- in the ring, with the remaining ring atoms being carbon atoms. Preferably, the heterocyclyl comprises 3 to 12 ring atoms wherein 1 to 4 atoms are heteroatoms; most preferably, 3 to 8 ring atoms wherein 1 to 3 atoms are heteroatoms; and most preferably 5 to 6 ring atoms wherein 1 to 2 or 1 to 3 atoms are heteroatoms. Non-limiting examples of monocyclic heterocyclyl include pyrrolidinyl, imidazolyl, tetrahydrofuranyl, tetrahydrothienyl, dihydroimidazolyl, dihydrofuranyl, dihydropyrazolyl, dihydropyrrolyl, piperidinyl, piperazinyl, morpholinyl, thiomorpholinyl, homopiperazinyl, pyranyl and the like, and preferably, 1,2,5-oxadiazolyl, pyranyl or morpholinyl. Polycyclic heterocyclyl includes a heterocyclyl having a spiro ring, fused ring or bridged ring.

The term "spiro heterocyclyl" refers to a 5- to 20-membered polycyclic heterocyclyl group with individual rings connected through one common atom (called a spiro atom), wherein one or more ring atoms are heteroatoms selected from the group consisting of N, O and S(O)ₘ (wherein m is an integer of 0 to 2), with the remaining ring atoms being carbon atoms, and the rings can contain one or more double bonds, but none of the rings has a completely conjugated π-electron system. The spiro heterocyclyl is preferably a 6- to 14-membered spiro heterocyclyl, and more preferably a 6- to 10-membered spiro heterocyclyl. According to the number of the spiro atoms shared between the rings, the spiro heterocyclyl can be divided into a mono-spiro heterocyclyl, di-spiro heterocyclyl, or poly-spiro heterocyclyl, and the spiro heterocyclyl is preferably a mono-spiro heterocyclyl or di-spiro heterocyclyl, and more preferably a 4-membered/4-membered, 4-membered/5-membered, 4-membered/6-membered, 5-membered/5-membered, or 5-membered/6-membered mono-spiro heterocyclyl. Non-limiting examples of spiro heterocyclyl include:

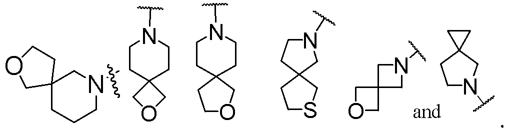

The term "fused heterocyclyl" refers to a 5- to 20-membered polycyclic heterocyclyl group, wherein each ring in the system shares an adjacent pair of atoms with another ring, one or more rings can contain one or more double bonds, but none of the rings has a completely conjugated π-electron system, and one or more ring atoms are heteroatoms selected from the group consisting of N, O and S(O)ₘ (wherein m is an integer of 0 to 2), with the remaining ring atoms being carbon atoms. The fused heterocyclyl is preferably a 6- to 14-membered fused heterocyclyl, and more preferably a 8- to 10-membered fused heterocyclyl. According to the number of membered rings, the fused heterocyclyl can be divided into a bicyclic, tricyclic, tetracyclic or polycyclic fused heterocyclyl, and preferably a bicyclic or tricyclic fused heterocyclyl, and more preferably a 5-membered/5-membered or 5-membered/6-membered bicyclic fused heterocyclyl. Non-limiting examples of fused heterocyclyl include: 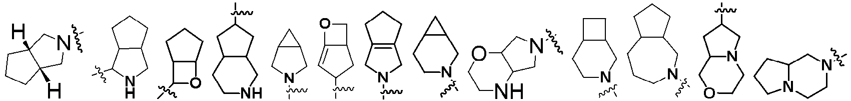 and

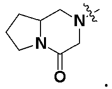

The term "bridged heterocyclyl" refers to a 5- to 14-membered polycyclic heterocyclyl group with any two of rings thereof sharing two atoms that are not directly connected to each other, which may have one or more double bonds, but none of the rings has a completely conjugated π-electron system, and in which one or more ring atoms are heteroatoms selected from the group consisting of N, O and S(O)m (wherein m is an integer from 0 to 2), with the remaining ring atoms being carbon atoms. The bridged heterocyclyl is preferably a 6- to 14-membered bridged heterocyclyl, and more preferably a 8- to 10-membered bridged heterocyclyl. According to the number of membered rings, the bridged heterocyclyl can be divided into a bicyclic, tricyclic, tetracyclic or polycyclic bridged heterocyclyl, and the bridged heterocyclyl is preferably a bicyclic, tricyclic or tetracyclic bridged heterocyclyl, and more preferably a bicyclic or tricyclic bridged heterocyclyl. Non-limiting examples of bridged heterocyclyl include:

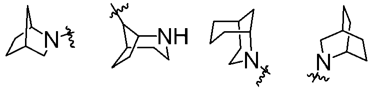

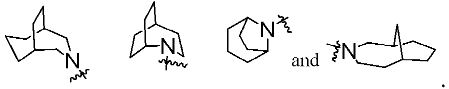

The heterocyclyl ring can be fused to the ring of aryl, heteroaryl or cycloalkyl, wherein the ring bound to the parent structure is heterocyclyl. Non-limiting examples thereof include: 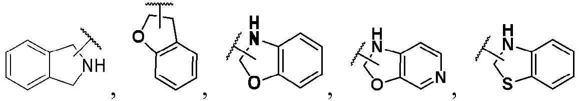 and the like.

The heterocyclyl can be optionally substituted or unsubstituted. When substituted, the substituent group(s) is preferably one or more group(s) independently selected from the group consisting of alkyl, alkenyl, alkynyl, alkoxy, alkylthio, alkylamino, halogen, thiol, hydroxy, nitro, cyano, cycloalkyl, heterocyclyl, aryl, heteroaryl, cycloalkoxy, heterocycloalkoxy, cycloalkylthio, heterocyclylthio, oxo, carboxy and carboxylic ester group.

The term "aryl" refers to a 6- to 14-membered full-carbon monocyclic ring or polycyclic fused ring (*i.e.* each ring in the system shares an adjacent pair of carbon atoms with another ring in the system) having a conjugated π-electron system, preferably a 6- to 10-membered aryl, for example, phenyl and naphthyl. The aryl ring can be fused to the ring of heteroaryl, heterocyclyl or cycloalkyl to provide a fused aryl, wherein the ring bound to the parent structure is aryl ring. Non-limiting examples thereof include:

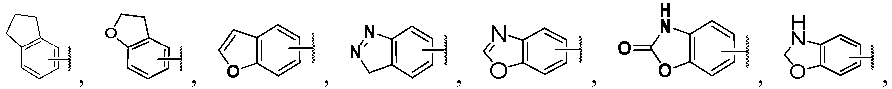

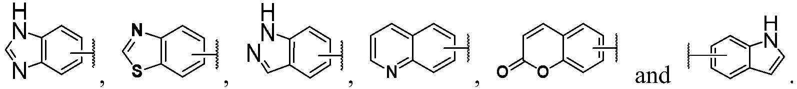

The aryl can be substituted or unsubstituted. When substituted, the substituent group(s) is preferably one or more group(s) independently selected from the group consisting of alkyl, alkenyl, alkynyl, alkoxy, alkylthio, alkylamino, halogen, thiol, hydroxy, nitro, cyano, cycloalkyl, heterocyclyl, aryl, heteroaryl, cycloalkoxy, heterocycloalkoxy, cycloalkylthio, heterocyclylthio, carboxy and carboxylic ester group.

The term "heteroaryl" refers to a 5- to 14-membered heteroaromatic system having 1 to 4 heteroatoms selected from the group consisting of O, S and N. The heteroaryl is preferably a 5- to 10-membered heteroaryl having 1 to 3 heteroatom(s), and more preferably a 5- or 6-membered heteroaryl having 1 to 2 heteroatom(s), for example imidazolyl, furyl, thienyl, thiazolyl, pyrazolyl, oxazolyl, pyrrolyl, tetrazolyl, pyridyl, pyrimidinyl, thiadiazolyl, pyrazinyl and the like, preferably imidazolyl, thiazolyl, pyrazolyl, pyrimidinyl or thiazolyl, and more preferably pyrazolyl or thiazolyl. The heteroaryl ring can be fused to the ring of aryl, heterocyclyl or cycloalkyl to provide a fused heteroaryl, wherein the ring bound to the parent structure is heteroaryl ring. Non-limiting examples thereof include:

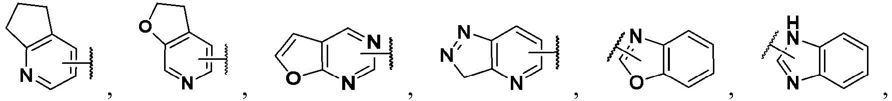

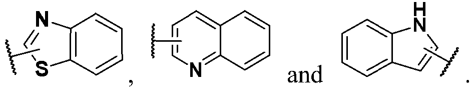

The heteroaryl can be optionally substituted or unsubstituted. When substituted, the substituent group(s) is preferably one or more group(s) independently selected from the group consisting of alkyl, alkenyl, alkynyl, alkoxy, alkylthio, alkylamino, halogen, thiol, hydroxy, nitro, cyano, cycloalkyl, heterocyclyl, aryl, heteroaryl, cycloalkoxy, heterocycloalkoxy, cycloalkylthio, heterocyclylthio, carboxy and carboxylic ester group.

The term "alkoxy" refers to an -O-(alkyl) or an -O-(unsubstituted cycloalkyl) group, wherein the alkyl and cycloalkyl are as defined above. Non-limiting examples of alkoxy include methoxy, ethoxy, propoxy, butoxy, cyclopropyloxy, cyclobutyloxy, cyclopentyloxy, cyclohexyloxy. The alkoxy can be optionally substituted or unsubstituted. When substituted, the substituent group(s) is preferably one or more group(s) independently selected from the group consisting of alkyl, alkenyl, alkynyl, alkoxy, alkylthio, alkylamino, halogen, thiol, hydroxy, nitro, cyano, cycloalkyl, heterocyclyl, aryl, heteroaryl, cycloalkoxy, heterocycloalkoxy, cycloalkylthio, heterocyclylthio, carboxy and carboxylic ester group.

In the chemical structure of the compound of the present disclosure, the bond " 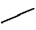 " represents an unspecified configuration. That is, if a chiral isomer exists in the chemical structure, the bond " 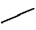 " can be " 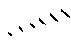 " or " 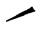 ", or can contain both " 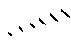 " and " 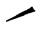 " configurations.

The term "haloalkyl" refers to an alkyl group substituted by one or more halogen(s), wherein the alkyl is as defined above.

The term "haloalkoxy" refers to an alkoxy group substituted by one or more halogen(s), wherein the alkoxy is as defined above.

The term "deuterated alkyl" refers to an alkyl group substituted by one or more deuterium atoms, wherein the alkyl is as defined above.

The term "deuterated alkoxy" refers to an alkoxy group substituted by one or more deuterium atoms, wherein the alkoxy is as defined above.

The term "hydroxyalkyl" refers to an alkyl group substituted by one or more hydroxy groups, wherein the alkyl is as defined above.

The term "hydroxy" refers to an -OH group.

The term "halogen" refers to fluorine, chlorine, bromine or iodine.

The term "amino" refers to a -NH₂ group.

The term "cyano" refers to a -CN group.

The term "nitro" refers to a -NO₂ group.

The term "oxo" refers to a =O group.

The term "thio" refers to a =S group.

The term "carboxy" refers to a -C(O)OH group.

The term "thiol" refers to a -SH group.

The term "ester" refers to a -C(O)O(alkyl) or a -C(O)O(cycloalkyl) group, wherein the alkyl and cycloalkyl are as defined above.

The compound of the present invention can be in deuterated form. Each available hydrogen atom bonded to a carbon atom can be independently replaced by a deuterium atom. Those skilled in the art are able to synthesize the deuterated form of the compounds by referring to relevant literature. Commercially available deuterated starting materials can be used to prepare the deuterated forms of the compound, or they can be synthesized by conventional techniques with deuterating reagents.

"Optional" or "optionally" means that the event or circumstance described subsequently can, but need not, occur, and such a description includes the situation in which the event or circumstance does or does not occur. For example, "the heterocyclyl optionally substituted by an alkyl" means that an alkyl group can be, but need not be, present, and such a description includes the situation of the heterocyclyl being substituted by an alkyl and the situation of the heterocyclyl being not substituted by an alkyl.

"Substituted" means one or more hydrogen atoms in a group, preferably up to 5, and more preferably 1 to 3 hydrogen atoms in a group, are independently replaced by a corresponding number of substituents. It goes without saying that the substituents only exist in their possible chemical position. The person skilled in the art is able to determine whether the substitution is possible or impossible by experiments or theory without excessive efforts. For example, the combination of amino or hydroxy having free hydrogen and carbon atoms having unsaturated bonds (such as olefinic) may be unstable.

A "pharmaceutical composition" refers to a mixture of one or more of the compounds according to the present invention or physiologically/pharmaceutically acceptable salts or prodrugs thereof with other chemical components, and other components such as physiologically/pharmaceutically acceptable carriers and excipients. The purpose of the pharmaceutical composition is to facilitate administration of a compound to an organism, which is conducive to the absorption of the active ingredient so as to exert biological activity.

A "pharmaceutically acceptable salt" refers to a salt of the compound of the present invention, which is safe and effective in mammals and has the desired biological activity.

### DETAILED DESCRIPTION OF THE INVENTION

The compounds of the present invention and their preparation are further illustrated with reference to the following examples. These examples illustrate certain methods for preparing or using said compounds. However, it should be understood that the scope of the present invention is not limited to these examples.. All currently known or further developed variations of the present invention are considered to fall within the scope of the present invention described and claimed herein.

The compound of the present invention is prepared by using convenient starting materials and general preparation procedures. The present invention provides typical or preferential reaction conditions, such as reaction temperature, duration, solvent, pressure and molar ratio of reactants. However, unless otherwise specified, other reaction conditions can also be adopted. Optimal conditions may vary with the use of specific reactants or solvents, but under normal circumstances, reaction optimization steps and conditions can be determined.

In addition, some protecting groups may be used in the present invention to protect certain functional groups from unnecessary reactions. The protecting groups suitable for various functional groups and their protection or deprotection conditions are well known to those skilled in the art. For example, "Protective Groups in Organic Synthesis" by T. W. Greene and G. M. Wuts (3rd edition, Wiley, New York, 1999) and citations in the book describes the protection or deprotection of a large number of protecting groups in detail.

The isolation and purification of compounds and intermediates are carried out by appropriate methods and steps according to specific needs, such as filtration, extraction, distillation, crystallization, column chromatography, preparative thin-layer chromatography, preparative high performance liquid chromatography or a combination of the above methods. The specific method of use may be referred to the examples described in the present invention. Of course, other similar isolation and purification methods can also be used. They can be characterized using conventional methods (including physical constants and spectral data).

The structures of the compounds are identified by nuclear magnetic resonance (NMR) and/or mass spectrometry (MS). NMR shifts are given in 10⁻⁶ (ppm). NMR is determined by a Q. One WNMR-I-400 MHz NMR spectrometer. The solvents for determination are deuterated-dimethyl sulfoxide (DMSO-*d₆*), deuterated-chloroform (CDCl₃) and deuterated-methanol (CD₃OD), and the internal standard is tetramethylsilane (TMS).

MS is determined by a 1260 Infinity II 6125B single quadrupole LC-MS system (manufacturer: Agilent Technologies), with a Kinetex XB-C18 100A 1.7µm (30×3mm) column (manufacturer: Phenomenex), and the mobile phase is acetonitrile/water (0.1% FA). Preparative liquid chromatography is conducted on a 1260 Infinity II preparative liquid chromatography (manufacturer: Agilent Technologies), with an Xtimate C18 5µm (21.2×250mm) column (manufacturer: Welch), and the mobile phase is acetonitrile/water. GF254 silica gel plate of Qingdao Haiyang Chemical is used for the thin-layer silica gel chromatography (TLC). The dimension of the silica gel plate used in TLC is 0.20 mm to 0.25 mm, and the dimension of the silica gel plate used in product purification is 0.5 mm.

Silica gel of 100 to 200 mesh, 200 to 300 mesh or 300 to 400 mesh of Qingdao Haiyang Chemical is used as a carrier for gel column chromatography.

The known starting materials of the present invention can be prepared by the known methods in the art, or can be purchased from Wanghua Mall, Tansoole, ChemicalBook, Labnetwork, Beijing Ouhe Technology, Sigma, J&K Scientific, Yishiming, Shanghai Shuya Chemical, Innochem Science & Technology, Energy Chemical, Shanghai Bide Pharmatech, Shanghai Leyan, Pharmablock Sciences (Nanjing), Inc. and the like.

Unless otherwise stated, the reactions are carried out under a nitrogen atmosphere.

Argon atmosphere, nitrogen atmosphere or hydrogen atmosphere means that a reaction flask is equipped with an argon, nitrogen or hydrogen balloon (about 1 L).

The reaction solvent, organic solvent or inert solvent is each expressed as the solvent used that does not participate in the reaction under the described reaction conditions, which includes for example, benzene, toluene, acetonitrile, tetrahydrofuran (THF), dimethylformamide (DMF), chloroform, dichloromethane (DCM), ether, methanol (MeOH), ethanol (EtOH), dimethyl sulfoxide (DMSO), 1,4-dioxane, N-methylpyrrolidone (NMP), pyridine, water and the like. Unless otherwise specified in the examples, a solution means an aqueous solution.

The chemical reaction described in the present invention is generally carried out under normal pressure. The reaction duration and condition are, for example, between -78°C and 200°C under one atmospheric pressure, and completed within about 1 to 24 hours. If the reaction is carried out overnight, the reaction duration is generally 16 hours. Unless otherwise specified in the examples, the reaction temperature is room temperature, which is 20°C to 30°C.

Reaction processes in the examples are monitored by thin-layer chromatography (TLC). The developing systems used for the reactions are: A: dichloromethane-methanol system, B: petroleum ether-ethyl acetate system, and C: acetone, and the volume ratios of the solvents are adjusted depending on the polarity of the compounds.

The eluent systems of the column chromatography and the developing systems of the thin-layer chromatography used for purifying the compounds include: A: dichloromethane-methanol system, and B: petroleum ether-ethyl acetate system, and the volume ratios of the solvents are adjusted depending on the polarity of the compounds. A small amount of alkaline or acidic reagents such as triethylamine and trifluoroacetic acid can also be added for adjustment.

Unless otherwise defined, all professional and scientific terms used herein have the same meaning as those familiar to those skilled in the art. In addition, any methods and materials similar or equivalent to the content described herein can be applied to the method of the present invention.

### Examples

### Example 1 : Synthesis of 6-((amino(methylamino)methylene)amino)-N-(1-(pyrimidin-2-yl)ethyl)-N-(5-(trifluoro methyl)pyridin-2-yl)methyl)nicotinamide (1)

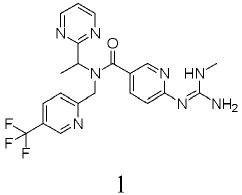

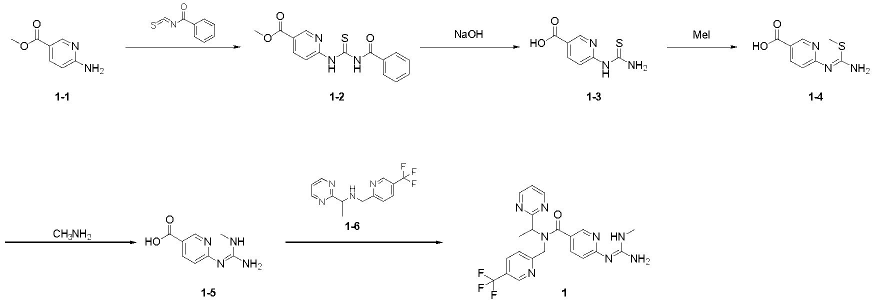

### Step 1: Preparation of methyl 6-(3-benzoylthioureido)nicotinate (1-2)

Benzoyl isothiocyanate (540 mg, 3.3 mmol) was added to a single-neck flask containing a solution of methyl 6-aminonicotinate (1-1) (500 mg, 3.3 mmol) in THF (10 mL). The resulting mixture was stirred at room temperature overnight. Ice water (50 mL) was added to the reaction solution, and the mixture was extracted with EtOAc (20 mL × 3). The organic phases were combined, dried over anhydrous sodium sulfate, and filtered. The filtrate was concentrated under reduced pressure. The residues were purified by silica gel column chromatography (PE/EtOAc = 3/1) to obtain compound 1-2 (700 mg, yield 67%, yellow solid).

LC-MS (ESI+): 316.0 m/z [M+H]⁺.

### Step 2: Preparation of 6-thioureidonicotinic acid (1-3)

An aqueous NaOH solution (1.1 mL, 4 M) was added to a single-neck flask containing a solution of compound 1-2 (700 mg, 2.22 mmol) in THF (7 mL). The resulting mixture was stirred at room temperature for 18 hours. The reaction solution was filtered, and the filter cake was washed with THF. The filter cake was dried under vacuum to obtain the crude compound 6-thioureidonicotinic acid (400 mg, yield 93%, white solid).

LC-MS (ESI+): 198.0 m/z [M+H]⁺.

### Step 3: Preparation of 6-((amino(methylthio)methylene)amino)nicotinic acid (1-4)

Iodomethane (60 mg, 0.42 mmol) was added to a single-neck flask containing a solution of compound 1-3 (55 mg, 0.28 mmol) in acetone (2 mL). The resulting mixture was stirred at room temperature for 18 hours. The reaction solution was filtered, and the filter cake was dried under vacuum to obtain crude compound 1-4 (60 mg, yield 99%, white solid).

LC-MS (ESI+): 211.95 m/z [M+H]⁺.

### Step 4: Preparation of 6-((amino(methylamino)methylene)amino)nicotinic acid (1-5)

A solution of methylamine in tetrahydrofuran (2 M, 2.6 mL, 5.21 mmol) was added to a sealed tube containing a solution of compound 1-4 (1.0 g, 4.73 mmol) in isopropanol (10 mL). The resulting mixture was stirred at 80°C for 18 hours. The reaction solution was cooled to room temperature and concentrated to dryness under reduced pressure. The residues were purified by Prep-HPLC (ACN/H₂O = 5-95%) and lyophilized to obtain compound 1-5 (0.2 g, yield 22%, white solid).

LC-MS (ESI+): 195.0 m/z [M+H]⁺.

### Step 5: Preparation of 6-((amino(methylamino)methylene)amino)-N-(1-(pyrimidin-2-yl)ethyl)-N-(5-(trifluoro methyl)pyridin-2-yl)methyl)nicotinamide (1)

1-(Pyrimidin-2-yl)-N-((5-(trifluoromethyl)pyridin-2-yl)methyl)ethan-1-amine **(1-6)** (70 mg, 0.25 mmol) (prepared according to the synthesis method of intermediate 139 on page 93/311 of patent application WO2021163344A1) and TEA (68 mg, 0.67 mmol) were added to a single-neck flask containing a solution of compound 1-5 (40 mg, 0.21 mmol) in DMF (2 mL) successively. The resulting mixture was cooled to 0°C under a nitrogen atmosphere, BOPCl (0.13 g, 0.53 mmol) was added thereto, and and the mixture was allowed to naturally warm to room temperature and stirred for 18 hours. EtOAc (50 mL) was added to the reaction solution, and the mixture was washed with saturated brine (10 mL × 3). The organic phase was dried over anhydrous sodium sulfate and filtered. The filtrate was concentrated to dryness under reduced pressure. The residues were purified by Prep-HPLC (ACN/H₂O = 5-95%) and lyophilized to obtain compound 1 (11.1 mg, yield 22%, white solid).

LC-MS (ESI+): 459.1 m/z [M+H]⁺.

¹H NMR (400 MHz, CH₃OH-d₄) δ 8.80 - 8.46 (m, 4H), 8.12 - 7.98 (m, 2H), 7.53 (d, J = 8.9 Hz, 1H), 7.32 (t, J = 4.9 Hz, 1H), 7.15 - 6.89 (m, 1H), 5.46 - 5.28 (m, 1H), 4.98 (d, J = 17.0 Hz, 1H), 4.68 (d, J = 16.9 Hz, 1H), 3.01 (s, 3H), 1.74 - 1.63 (m, 3H).

The following compounds were obtained according to the synthesis method of Example 1 using corresponding starting materials:

| Compound No. | Structure | MS data (ESI+): m/z | NMR data |
|---|---|---|---|
| 2 | 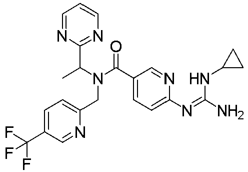 | 485.2 | ¹H NMR (400 MHz, CH₃OH-d₄) δ 8.73 - 8.48 (m, 4H), 8.12 - 7.99 (m, 2H), 7.56 - 7.46 (m, 1H), 7.32 (t, J = 4.9 Hz, 1H), 7.18 - 6.92 (m, 1H), 5.42 - 5.29 (m, 1H), 4.97 (d, J = 16.9 Hz, 1H), 4.68 (d, J = 16.9 Hz, 1H), 2.72 - 2.60 (m, 1H), 1.73 - 1.55 (m, 3H), 1.03 - 0.92 (m, 2H), 0.81 - 0.69 (m, 2H). |
| 3 | 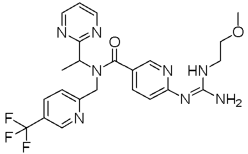 | 503.2 | ¹H NMR (400 MHz, CH₃OH-d₄) δ 8.75 - 8.66 (m, 3H), 8.65 - 8.44 (m, 2H), 8.17 - 7.97 (m, 2H), 7.56 - 7.48 (m, 1H), 7.32 (t, J = 4.9 Hz, 1H), 7.14 - 6.94 (m, 1H), 5.43 - 5.25 (m, 1H), 4.98 (d, J = 17.0 Hz, 1H), 4.74 - 4.57 (m, 1H), 3.65 - 3.50 (m, 4H), 3.41 (s, 3H), 1.68 (s, 3H). |

### Example 4: Synthesis of 4-((amino(methylamino)methylene)amino)-N-(1-(pyrimidin-2-yl)ethyl)-N-(5-(trifluoro methyl)pyridin-2-yl)methyl)benzamide (4)

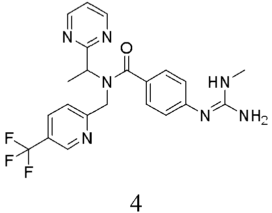

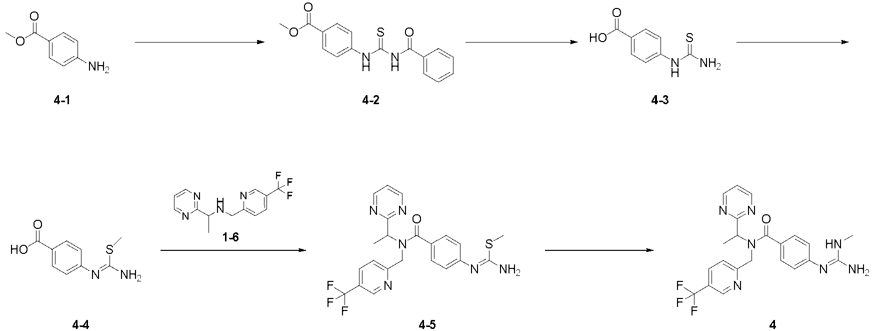

### Step 1: Preparation of methyl 4-(3-benzoylthioureido)benzoate (4-2)

Benzoyl isothiocyanate (5.4 g, 33 mmol) was added to a single-neck flask containing a solution of methyl 4-aminobenzoate (5.0 g, 33 mmol) in THF (50 mL). The resulting mixture was stirred at room temperature overnight. Ice water (50 mL) was added to the reaction solution, and the mixture was extracted with EtOAc (20 mL × 3). The organic phases were combined, dried over anhydrous sodium sulfate, and filtered. The filtrate was concentrated under reduced pressure. The residues were purified by silica gel column chromatography (PE/EtOAc = 3/1) to obtain compound 4-2 (6.5 g, yield 63%, pale yellow solid).

LC-MS (ESI+): 315.0 m/z [M+H]⁺.

### Step 2: Preparation of 4-thioureidobenzoic acid (4-3)

An aqueous NaOH solution (4 M, 10.5 mL) was added to a single-neck flask containing a solution of compound 4-2 (6.5 g, 20.7 mmol) in THF (70 mL). The resulting mixture was stirred at room temperature for 18 hours. The reaction solution was filtered, and the filter cake was washed with THF. The filter cake was collected and dried under vacuum to obtain compound 4-3 (4.2 g, yield 95%, white solid), which was used directly in the next reaction.

LC-MS (ESI+): 197.0 m/z [M+H]⁺.

### Step 3: Preparation of 4-((amino(methylthio)methylene)amino)benzoic acid (4-4)

Iodomethane (2.28 g, 16.07 mmol) was added to a single-neck flask containing a solution of compound 4-3 (2.1 g, 10.7 mmol) in acetone (42 mL). The resulting mixture was stirred at room temperature for 18 hours. The reaction solution was filtered, and the filter cake was collected and dried under vacuum to obtain compound 4-4 (1.8 g, yield 80%, white solid).

LC-MS (ESI+): 211.0 m/z [M+H]⁺.

### Step 4: Preparation of methyl (Z)-N'-(4-((1-(pyrimidin-2-yl)ethyl)((5-(trifluoromethyl)pyridin-2-yl)methyl)carbamoyl )phenyl)carbamimidothioate (4-5)

Compound 1-6 (120 mg, 0.43 mmol) and TEA (129 mg, 1.3 mmol) were added to a single-neck flask containing a solution of compound 4-4 (107 mg, 0.5 mmol) in DMF (2 mL) successively. The resulting mixture was cooled to 0°C under a nitrogen atmosphere, BOPCl (151 mg, 0.60 mmol) was added thereto, and the mixture was allowed to naturally warm to room temperature and stirred for 18 hours. EtOAc (50 mL) was added to the reaction solution, and the mixture was washed with saturated brine (10 mL × 3). The organic phase was dried over anhydrous sodium sulfate and filtered. The filtrate was concentrated under reduced pressure. The residues were purified by Prep-HPLC (ACN/H₂O = 5-95%) and lyophilized to obtain compound 4-5 (40 mg, yield 20%, white solid).

LC-MS (ESI+): 475.1 m/z [M+H]⁺.

### Step 5: Preparation of 4-((amino(methylamino)methylene)amino)-N-(1-(pyrimidin-2-yl)ethyl)-N-((5-(trifluoro methyl)pyridin-2-yl)methyl)benzamide (4)

A solution of methylamine in tetrahydrofuran (2.8 M, 0.06 mL, 0.17 mmol) was added to a microwave tube containing a solution of compound 4-5 (40 mg, 0.21 mmol) in isopropanol (3 mL). The resulting mixture was heated to 80°C and stirred for 18 hours. The reaction solution was cooled to room temperature and concentrated under reduced pressure. The residues were purified by Prep-HPLC (ACN/H₂O = 5-95%) and lyophilized to obtain compound 4 (5.4 mg, yield 22%, white solid).

LC-MS (ESI+): 458.0 m/z [M+H]⁺.

¹H NMR (400 MHz, CH₃OH-d₄) δ 8.81 - 8.66 (m, 3H), 8.51 (s, 1H), 8.01 (d, J = 8.3 Hz, 1H), 7.67 (d, J = 8.0 Hz, 1H), 7.55 (d, J = 8.4 Hz, 1H), 7.40 - 7.15 (m, 3H), 5.46 - 5.32 (m, 1H), 5.03 (d, J = 17.0 Hz, 1H), 4.69 (d, J = 16.7 Hz, 1H), 2.91 (s, 3H), 1.72 - 1.57 (m, 3H).

The following compounds were obtained according to the synthesis method of Example 4 using corresponding starting materials:

| Compound No. | Structure | MS data (ESI+): m/z | NMR data |
|---|---|---|---|
| 5 | 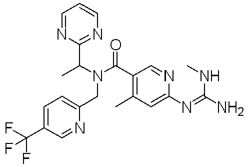 | 517.2 | ¹H NMR (400 MHz, CH₃OH-d₄) δ 8.74 - 8.45 (m, 4H), 8.18 - 7.89 (m, 2H), 7.53 (d, J = 7.5 Hz, 1H), 7.32 (t, J = 4.9 Hz, 1H), 7.18 - 6.94 (s, 1H), 5.50 - 5.32 (m, 1H), 4.98 (d, J = 16.8 Hz, 1H), 4.68 (d, J = 16.5 Hz, 1H), 4.23 (s, 2H), 3.29 (s, 3H), 1.76 - 1.60 (m, 3H). |
| 6 | 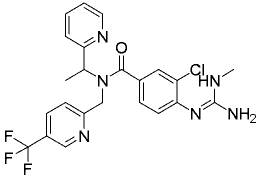 | 492.1 | ¹H NMR (400 MHz, DMSO-d₆) δ 8.83 (s, 1H), 8.54 (s, 1H), 8.28 (s, 1H), 8.07 (d, *J =* 8.0 Hz, 1H), 7.77 (s, 1H), 7.64 (s, 1H), 7.51 - 7.44 (m, 2H), 7.36 (s, 1H), 7.28 (s, 1H), 7.15 (s, 1H), 6.41 (s, 1H), 5.32 (s, 1H), 4.80 (d, *J =* 16.0 Hz, 1H), 4.44 - 4.40 (m, 1H), 2.72 (s, 3H), 1.57 (s, 3H). |

### Example 7: Synthesis of 6-(diaminomethylene)amino)-N-(1-(pyrimidin-2-yl)ethyl)-N-((5-(trifluoromethyl)pyridi n-2-yl)methyl)nicotinamide (7)

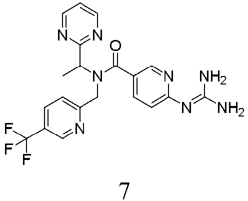

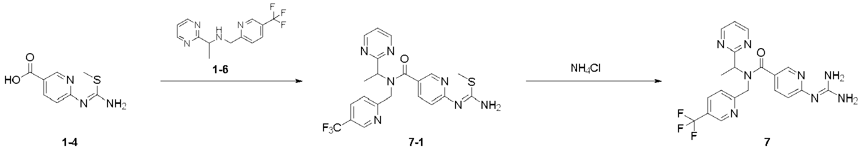

### Step 1: Preparation of methyl N'-(5-((1-(pyrimidin-2-yl)ethyl)((5-(trifluoromethyl)pyridin-2-yl)methyl)carbamoyl)pyr idin-2-ylcarbamimidothioate (7-1)

Compound 1-6 (0.3 g, 1.1 mmol) and TEA (0.28 g, 2.8 mmol) were added to a single-neck flask containing a solution of compound 1-4 (0.27 g, 1.3 mmol) in DMF (6 mL) successively. The resulting mixture was cooled to 0°C under a nitrogen atmosphere, BOPCl (0.33 g, 1.3 mmol) was added thereto, and the mixture was allowed to naturally warm to room temperature and stirred for 12 hours. Water (25 mL) was added to the reaction solution, and the mixture was extracted with EtOAc (25 mL × 3). The organic phases were combined, washed with saturated brine, dried over anhydrous sodium sulfate, and filtered. The filtrate was concentrated under reduced pressure. The residues were purified by silica gel column chromatography (DCM/EtOAc = 1/1) to obtain compound 7-1 (0.2 g, yield 39%, yellow liquid).

LC-MS (ESI+): 476.2 m/z [M+H]⁺.

### Step 2: Preparation of 6-(diaminomethylene)amino)-N-(1-(pyrimidin-2-yl)ethyl)-N-((5-(trifluoromethyl)pyridi n-2-yl)methyl)nicotinamide (7)

Ammonium chloride (12 mg, 0.2 mmol) was added to a sealed tube containing a solution of compound 7-1 (25 mg, 0.05 mmol) in isopropanol (2 mL). The resulting mixture was heated to 90°C and stirred for 12 hours. The reaction solution was cooled to room temperature and concentrated under reduced pressure. The residues were purified by Prep-HPLC (ACN/H₂O = 5-95%) and lyophilized to obtain compound 7 (12.6 mg, yield 52%, white solid).

LC-MS (ESI+): 445.0 m/z [M+H]⁺.

¹H NMR (400 MHz, CH₃OH-d₄) δ 8.87 - 8.53 (m, 4H), 8.29 - 8.05 (m, 1H), 8.03 - 7.92 (m, 1H), 7.58 - 7.46 (m, 1H), 7.38 - 7.06 (m, 2H), 5.47 - 5.23 (m, 1H), 4.98 (d, J = 17.4 Hz, 1H), 4.69 (d, J = 17.5 Hz, 1H), 2.77 (s, 3H), 1.76 - 1.60 (m, 3H).

The following compounds were obtained according to the synthesis method of Example 7 using corresponding starting materials:

| Compound No. | Structure | MS data (ESI+): m/z | NMR data |
|---|---|---|---|
| 8 | 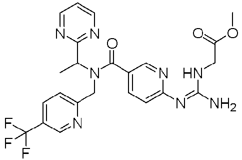 | 517.2 | ¹H NMR (400 MHz, CH₃OH-d₄) δ 8.74 - 8.45 (m, 4H), 8.18 - 7.89 (m, 2H), 7.53 (d, J = 7.5 Hz, 1H), 7.32 (t, J = 4.9 Hz, 1H), 7.18 - 6.94 (s, 1H), 5.50 - 5.32 (m, 1H), 4.98 (d, J = 16.8 Hz, 1H), 4.68 (d, J = 16.5 Hz, 1H), 4.23 (s, 2H), 3.29 (s, 3H), 1.76 - 1.60 (m, 3H). |
| 9 | 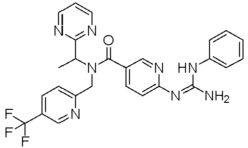 | 521.2 | |
| 10 | 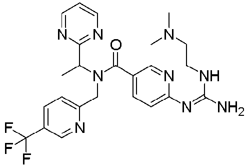 | 516.2 | ¹H NMR (400 MHz, CH₃OH-d₄) δ 8.76 - 8.66 (m, 3H), 8.66 - 8.58 (m, 1H), 8.20 - 8.09 (m, 1H), 8.02 - 7.96 (m, 1H), 7.56 - 7.48 (m, 1H), 7.32 (t, J = 4.9 Hz, 1H), 7.24 - 7.15 (m, 1H), 5.42 - 5.30 (m, 1H), 4.98 (d, J = 17.1 Hz, 1H), 4.68 (d, J = 16.8 Hz, 1H), 3.86 - 3.77 (m, 2H), 3.51 - 3.40 (m, 2H), 3.02 - 2.89 (m, 6H), 1.73 - 1.57 (m, 3H). |
| 11 | 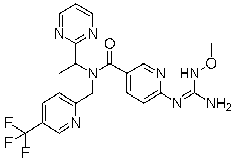 | 475.2 | ¹H NMR (400 MHz, CH₃OH-d₄) δ 9.00 - 8.51 (m, 3H), 8.20 - 7.94 (m, 2H), 7.60 - 7.48 (m, 1H), 7.32 (t, J = 4.9 Hz, 1H), 7.25 - 7.14 (m, 1H), 5.43 - 5.28 (m, 1H), 4.98 (d, J = 17.1 Hz, 1H), 4.69 (d, J = 16.4 Hz, 1H), 3.84 (s, 3H), 1.76 - 1.58 (m, 3H). |
| 12 | 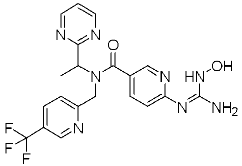 | 461.2 | ¹H NMR (400 MHz, CH₃OH-d₄) δ 8.78 - 8.71 (m, 3H), 8.52 - 8.41 (m, 1H), 8.04 - 7.91 (m, 2H), 7.52 (d, J = 8.3 Hz, 1H), 7.31 (t, J = 4.9 Hz, 1H), 7.14 - 7.01 (m, 1H), 5.52 - 5.36 (m, 1H), 4.97 (d, J = 16.5 Hz, 2H), 4.75 - 4.62 (m, 1H), 1.76 - 1.54 (m, 3H). |
| 13 | 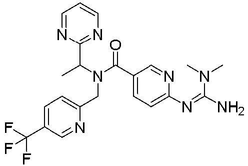 | 473.1 | ¹H NMR (400 MHz, CHCl₃-d) δ 9.18 (brs, 2H), 8.78 - 8.54 (m, 3H), 8.47 (s, 1H), 8.00 - 7.70 (m, 2H), 7.41 (d, J = 8.2 Hz, 2H), 7.15 (t, J = 4.9 Hz, 1H), 5.34 (s, 1H), 4.95 (d, J = 16.8 Hz, 1H), 4.60 (d, J = 16.4 Hz, 1H), 3.07 (s, 6H), 1.61 (d, J = 7.0 Hz, 3H). |
| 14 | 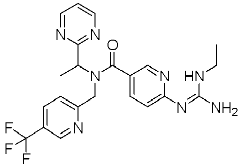 | 473.0 | ¹H NMR (400 MHz, DMSO-d₆) δ 8.84 - 8.78 (m, 3H), 8.51 (s, 1H), 8.41 (s, 1H), 8.16 - 8.09 (m, 1H), 8.01 (s, 1H), 7.71 - 7.55 (m, 1H), 7.42 - 7.38 (m, 1H), 7.11 (s, 1H), 5.66 - 5.38 (m, 1H), 4.89 (d, J = 16.1 Hz, 1H), 4.52 (d, J = 16.2 Hz, 1H), 3.93 (q, J = 6.4 Hz, 2H), 1.64 (s, 2H), 1.39 (d, J = 6.8 Hz, 1H), 1.24 - 1.14 (m, 3H). |
| 15 | 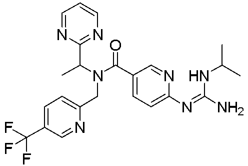 | 487.2 | ¹H NMR (400 MHz, DMSO-d₆) δ 8.84 (s, 1H), 8.80 (d, J = 4.8 Hz, 2H), 8.47 (s, 1H), 8.38 (s, 1H), 8.19 - 8.03 (m, 1H), 7.92 (s, 1H), 7.55 (d, J = 8.4 Hz, 1H), 7.42 (t, J = 4.8 Hz, 1H), 6.99 (s, 1H), 5.40 (s, 1H), 4.88 (d, J = 16.4 Hz, 1H), 4.51 (s, 1H), 3.90 (s, 1H), 1.63 (s, 3H), 1.20 (d, J = 6.4 Hz, 6H). |

### Example 16: Synthesis of N-(1-(pyrimidin-2-yl)ethyl)-6-thioureido-N-(5-(trifluoromethyl)pyridin-2-yl)methyl)nic otinamide (16)

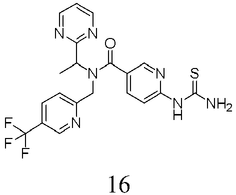

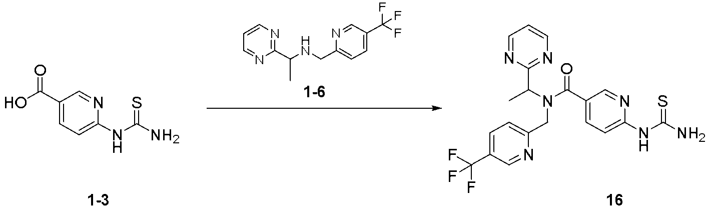

Compound 1-6 (50 mg, 0.18 mmol) and TEA (55 mg, 0.54 mmol) were added to a single-neck flask containing a solution of compound 1-3 (45 mg, 0.21 mmol) in DMF (2 mL) successively. The resulting mixture was cooled to 0°C under a nitrogen atmosphere, BOPCl (68 mg, 0.27 mmol) was added thereto, and the mixture was allowed to warm to room temperature and stirred for 18 hours. EtOAc (25 mL) was added to the reaction solution, and the mixture was washed with saturated brine (10 mL × 3). The organic phase was dried over anhydrous sodium sulfate and filtered. The filtrate was concentrated under reduced pressure. The residues were purified by Prep-HPLC (ACN/H₂O = 5-95%) and lyophilized to obtain compound 16 (14.9 mg, yield 15%, white solid).

LC-MS (ESI+): 462.0 m/z [M+H]⁺.

¹H NMR (400 MHz, CH₃OH-d₄) δ 8.76 - 8.66 (m, 3H), 8.56 (s, 1H), 8.06 - 7.96 (m, 2H), 7.52 (d, J = 8.4 Hz, 1H), 7.31 (t, J = 4.9 Hz, 1H), 7.12 - 7.02 (m, 1H), 5.47 - 5.35 (m, 1H), 4.97 (d, J = 17.0 Hz, 1H), 4.73 - 4.60 (m, 1H), 1.77 - 1.56 (m, 3H).

### Example 17: Synthesis of 6-((amino(methoxycarbonylamino)methylene)amino)-N-(1-(pyrimidin-2-yl)ethyl)-N-(( 5-(trifluoromethyl)pyridin-2-yl)methyl)nicotinamide (17)

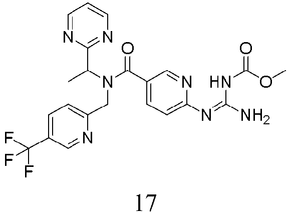

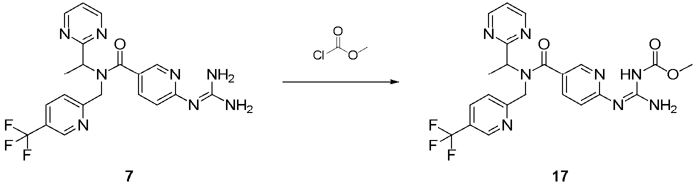

TEA (15 mg, 0.12 mmol) was added to a single-neck flask containing a solution of compound 7 (20 mg, 0.04 mmol) in DCM (4 mL). The resulting mixture was cooled to 0°C under an ice bath, and methyl chloroformate (5 mg, 0.04 mmol) was added thereto. The mixture was allowed to naturally warm to room temperature and stirred for 2 hours. Water (10 mL) was added to the reaction solution, and the mixture was extracted with DCM (10 mL × 3). The organic phases were combined, dried over anhydrous sodium sulfate, and filtered. The filtrate was concentrated under reduced pressure. The residues were purified by Prep-HPLC (ACN/H₂O = 5-95%) and lyophilized to obtain compound 17 (3 mg, yield 13%, white solid).

LC-MS (ESI+): 503.5 m/z [M+H]⁺.

¹H NMR (400 MHz, CH₃OH-d₄) δ 8.76 - 8.65 (m, 4H), 8.60 - 8.50 (m, 1H), 8.04 - 7.95 (m, 2H), 7.52 (d, J = 8.3 Hz, 1H), 7.31 (t, J = 4.9 Hz, 1H), 5.44 - 5.42 (m, 1H), 4.97 (d, J = 16.9 Hz, 1H), 4.72 - 4.53 (m, 1H), 3.67 (s, 3H), 1.70 - 1.65 (m, 3H).

### Example 18: Synthesis of tert-butyl (N'-(5-(1-(pyrimidin-2-yl)ethyl)((5-(trifluoromethyl)pyridin-2-yl)methyl)carbamoyl)pyr idin-2-ylcarbamimidoyl)glycinate (18)

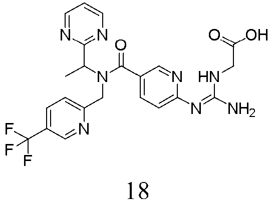

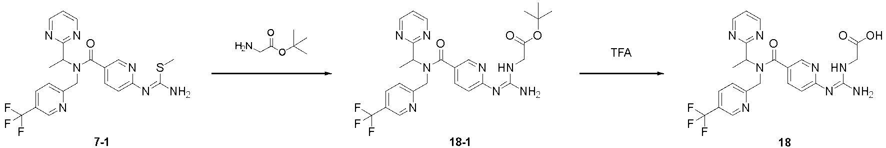

### Step 1: Preparation of tert-butyl (N'-(5-(1-(pyrimidin-2-yl)ethyl)((5-(trifluoromethyl)pyridin-2-yl)methyl)carbamoyl)pyr idin-2-ylcarbamimidoyl)glycinate (18-1)

*Tert*-butyl glycinate (66 mg, 0.5 mmol) was added to a sealed tube containing a solution of compound 7-1 (60 mg, 0.12 mmol) in isopropanol (2 mL). The resulting mixture was heated to 90°C and stirred for 12 hours. The reaction solution was cooled to room temperature and concentrated under reduced pressure. The residues were purified by Prep-TLC (DCM/EtOAc = 1/1) to obtain compound 18-1 (22 mg, yield 62%, white solid).

LC-MS (ESI+): 559.2 m/z [M+H]⁺.

### Step 2: Preparation of tert-butyl (N'-(5-(1-(pyrimidin-2-yl)ethyl)((5-(trifluoromethyl)pyridin-2-yl)methyl)carbamoyl)pyr idin-2-ylcarbamimidoyl)glycinate (18)

TFA (1 mL) was added to a single-neck flask containing a solution of compound 18-1 (22 mg, 0.04 mmol) in DCM (2 mL). The resulting mixture was stirred at room temperature for 2 hours. The reaction solution was concentrated under reduced pressure. The residues were purified by Prep-HPLC (ACN/H₂O = 5-95%) and lyophilized to obtain compound 18 (3.7 mg, yield 19%, white solid).

LC-MS (ESI+): 503.0 m/z [M+H]⁺.

¹H NMR (400 MHz, CH₃OH-d₄) δ 8.81 - 8.53 (m, 4H), 8.20 - 8.07 (m, 1H), 7.99 (d, J = 8.1 Hz, 1H), 7.62 - 7.44 (m, 1H), 7.32 (t, J = 4.9 Hz, 1H), 7.19 - 6.98 (m, 1H), 5.50 - 5.27 (m, 1H), 4.98 (d, J = 17.1 Hz, 1H), 4.69 (d, J = 17.6 Hz, 1H), 4.19 (s, 2H), 1.73 - 1.57 (m, 3H).

### Example 19: Synthesis of 6-((amino((2-hydroxyethyl)amino)methylene)amino)-N-(1-(pyrimidin-2-yl)ethyl)-N-(5-(trifluoromethyl)pyridin-2-yl)methyl)nicotinamide (19)

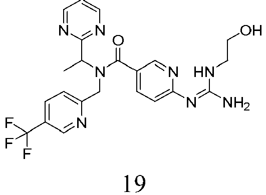

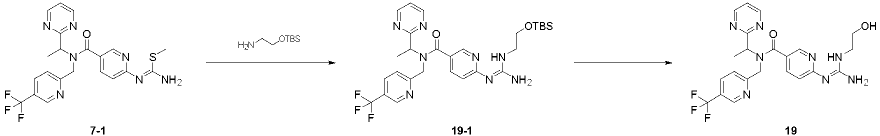

### Step 1: Preparation of 6-((amino((2-((tert-butyldimethylsilyl)oxy)ethyl)amino)methylene)amino)-N-(1-(pyrim idin-2-yl)ethyl)-N-(5-(trifluoromethyl)pyridin-2-yl)methyl)nicotinamide (19-1)

2-((*Tert*-butyldimethylsilyl)oxy)ethan-1-amine (46 mg, 0.26 mmol) was added to a microwave tube containing a solution of compound 7-1 (61.8 mg, 0.13 mmol) in isopropanol (3 mL). The resulting mixture was heated to 80°C and stirred overnight. The reaction solution was cooled to room temperature and concentrated under reduced pressure. The residues were purified by Prep-TLC (DCM/MeOH = 15/1) to obtain compound 19-1 (15.2 mg, yield 19%, white solid).

LC-MS (ESI+): 603.3 m/z [M+H]⁺.

### Step 2: Preparation of (Z)-6-((amino((2-hydroxyethyl)amino)methylene)amino)-N-(1-(pyrimidin-2-yl)ethyl)-N -(5-(trifluoromethyl)pyridin-2-yl)methyl)nicotinamide (19)

Triethylamine trihydrofluoride (65 mg, 0.4 mmol) was added to a single-neck flask containing a solution of compound 19-1 (25 mg, 0.04 mmol) in THF (6 mL). The resulting mixture was stirred at room temperature overnight. Water (10 mL) and saturated aqueous NaHCO₃ solution (10 mL) were added to the reaction solution, and the mixture was extracted with EtOAc (20 mL × 3). The organic phases were combined, dried over anhydrous sodium sulfate, and filtered. The filtrate was concentrated under reduced pressure. The residues were purified by Prep-HPLC (ACN/H₂O = 5-95%) and lyophilized to obtain compound 19 (4.3 mg, yield 21%, purity 99%, brown solid).

LC-MS (ESI+): 489.15 m/z [M+H]⁺.

¹H NMR (400 MHz, CH₃OH-d₄): δ 8.85 - 8.41 (m, 5H), 8.12 (d, J = 8.2 Hz, 1H), 7.99 (dd, J = 8.3, 2.2 Hz, 1H), 7.52 (d, J = 8.2 Hz, 1H), 7.31 (t, J = 4.9 Hz, 1H), 7.18 - 6.93 (m, 1H), 5.46 - 5.32 (m, 1H), 4.97 (d, J = 17.1 Hz, 1H), 4.67 (d, J = 16.8 Hz, 1H), 3.85 - 3.70 (m, 2H), 3.55 - 3.40 (m, 2H), 1.77 - 1.56 (m, 3H).

### Example 20: Synthesis of N-(1-(pyrimidin-2-yl)ethyl)-N-((5-(trifluoromethyl)pyridin-2-yl)methyl)-6-ureidonicoti namide (20)

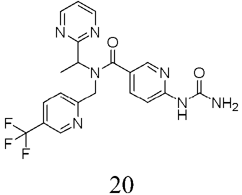

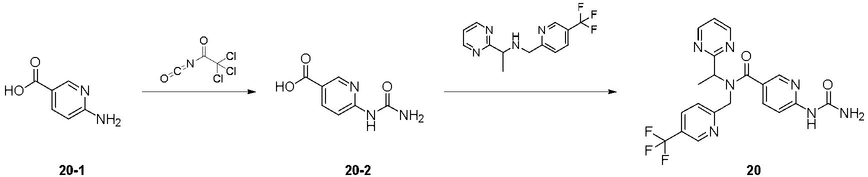

### Step 1: Preparation of 6-ureidonicotinic acid (20-2)

6-Aminonicotinic acid (300 mg, 2.17 mmol) was dissolved in THF (10 mL) in a three-neck flask. NaH (347 mg, 8.68 mmol) was slowly added in portions after cooling to 0°C under an ice bath. The resulting mixture was stirred at 0°C for 1 hour, and then trichloroacetyl isocyanate (613 mg, 3.26 mmol) was slowly added dropwise. After the addition was complete, the ice bath was removed, and the mixture was allowed to naturally warm to room temperature and stirred overnight. Water (10 mL) was added to the reaction solution, and the mixture was extracted with EtOAc (10 mL × 3). The aqueous phase was purified by Prep-HPLC (ACN/H₂O = 5-95%) and lyophilized to obtain compound 20-2 (96.0 mg, yield 24%, colorless oil).

LC-MS (ESI+): 182.0 m/z [M+H]⁺.

### Step 2: Preparation of N-(1-(pyrimidin-2-yl)ethyl)-N-((5-(trifluoromethyl)pyridin-2-yl)methyl)-6-ureidonicoti namide (20)

Compound 1-6 (77.8 mg, 0.28 mmol) and DIEA (71 mg, 0.55 mmol) were added to a single-neck flask containing a solution of 6-ureidonicotinic acid (50 mg, 0.28 mmol) in DMF (5 mL) successively. The resulting mixture was cooled to 0 °C in an ice bath, BOPCl (105 mg, 0.41 mmol) was slowly added thereto, and after addition, the mixture was allowed to naturally warm to room temperature and stirred overnight. Water (10 mL) was added to the reaction solution, and the mixture was extracted with EtOAc (10 mL × 3). The organic phases were combined, dried over anhydrous sodium sulfate, and filtered. The filtrate was concentrated under reduced pressure. The residues were purified by Prep-HPLC (ACN/H₂O = 5-95%) and lyophilized to obtain compound 20 (14 mg, yield 11.4%, white solid).

LC-MS (ESI+): 446.2 m/z [M+H]⁺.

¹H NMR (400 MHz, CH₃OH-d₄) δ 8.77 - 8.65 (m, 3H), 8.60 - 8.50 (m, 1H), 8.13 - 8.04 (m, 1H), 8.03 - 7.91 (m, 1H), 7.52 (d, J = 8.3 Hz, 1H), 7.35 - 7.21 (m, 2H), 5.50 - 5.37 (m, 1H), 4.96 (d, J = 16.2 Hz, 1H), 4.75 - 4.63 (m, 1H), 1.74 - 1.60 (m, 3H).

### Example 21: Synthesis of 6-((bis(methyl-amino)methylene)amino)-N-(1-(pyrimidin-2-yl)ethyl)-N-((5-(trifluorom ethyl)pyridin-2-yl)methyl)nicotinamide (21)

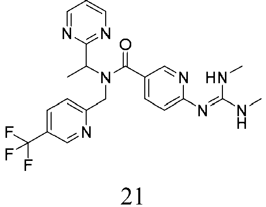

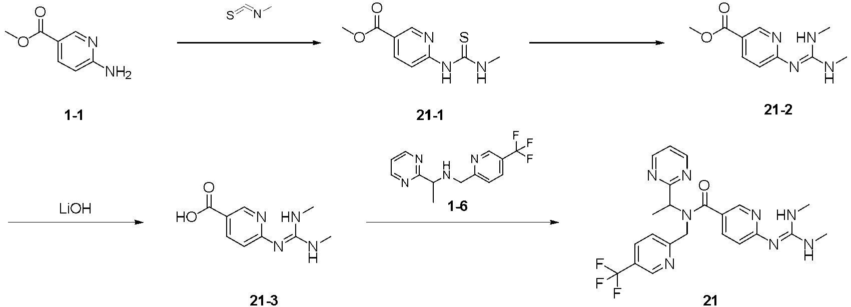

### Step 1: Preparation of methyl 6-(3-methylthioureido)nicotinate (21-1)

Methyl isothiocyanate (1.1 g, 15.8 mmol) was added to a single-neck flask containing a solution of compound 1-1 (2.0 g, 13.1 mmol) in toluene (20 mL). The resulting mixture was heated to 110°C and stirred for 48 hours. The reaction solution was cooled to room temperature and filtered. The filter cake was purified by silica gel column chromatography (DCM/EtOAc = 1/1) to obtain compound 21-1 (1.3 g, yield 40%, white solid).

LC-MS (ESI+): 225.9 m/z [M+H]⁺.

### Step 2: Preparation of methyl 6-(bis(methylamino)methylene)amino)nicotinate (21-2)

MeNH₂ (3 M in THF, 0.57 mL, 1.5 mmol) and HgO (0.25 g, 1.1 mmol) were added to a sealed tube containing a solution of compound 21-1 (260 mg, 1.1 mmol) in ethanol (5 mL) successively. The resulting mixture was heated to 100°C and stirred for 12 hours. The reaction solution was cooled to room temperature and filtered, and the filtrate was concentrated under reduced pressure. The residues were purified by Prep-TLC (DCM/MeOH = 15/1) to obtain compound 21-2 (110 mg, yield 43%, white solid).

LC-MS (ESI+): 223.0 m/z [M+H]⁺.

### Step 3: Preparation of 6-(bis(methylamino)methylene)amino)nicotinic acid (21-3)

An aqueous LiOH solution (4 M, 0.5 mL, 2.0 mmol) was slowly added to a single-neck flask containing a solution of 21-2 (0.11 g, 0.5 mmol) in MeOH (2 mL). The resulting mixture was stirred at room temperature for 18 hours. Dilute hydrochloric acid (0.5 M) was added to the reaction solution to adjust the pH to about 5. The resulting solution was directly purified by Prep-HPLC (ACN/H₂O = 5-95%) and lyophilized to obtain compound 21-3 (100 mg, yield 96%, white solid).

LC-MS (ESI+): 209.0 m/z [M+H]⁺.

### Step 4: Preparation of 6-((bis(methyl-amino)methylene)amino)-N-(1-(pyrimidin-2-yl)ethyl)-N-((5-(trifluorom ethyl)pyridin-2-yl)methyl)nicotinamide (21)

TEA (97 mg, 0.96 mmol) and compound 1-6 (140 mg, 0.48 mmol) were added to a single-neck flask containing a solution of compound 21-3 (100 mg, 0.48 mmol) in DMF (2 mL) successively. The resulting mixture was cooled to 0°C in an ice bath, BOPCl (0.13 g, 0.53 mmol) was slowly added thereto, and the mixture was allowed to naturally warm to room temperature and stirred for 18 hours. EtOAc (50 mL) was added to the reaction solution, and the mixture was washed with saturated brine (10 mL × 3). The organic phase was dried over anhydrous sodium sulfate and filtered. The filtrate was concentrated under reduced pressure. The residues were purified by Prep-HPLC (ACN/H₂O = 5-95%) and lyophilized to obtain compound 21 (23.5 mg, yield 10%, white solid).

LC-MS (ESI+): 473.15 m/z [M+H]⁺.

¹H NMR (400 MHz, CH₃OH-d₄) δ 8.75 - 8.66 (m, 2H), 8.64 - 8.58 (m, 1H), 8.54 - 8.43 (m, 1H), 8.19 - 8.10 (m, 1H), 8.00 (d, J = 7.2 Hz, 1H), 7.53 (d, J = 7.6 Hz, 1H), 7.42 (d, J = 8.0 Hz, 1H), 7.32 (t, J = 4.9 Hz, 1H), 5.45 - 5.30 (m, 1H), 4.98 (d, J = 17.2 Hz, 1H), 4.68 (d, J = 16.7 Hz, 1H), 3.04 (s, 6H), 1.74 - 1.57 (m, 3H).

### Example 22: Synthesis of 6-((amino(nitroamino)methylene)amino)-N-(1-(pyrimidin-2-yl)ethyl)-N-((5-(trifluorom ethyl)pyridin-2-yl)methyl)nicotinamide (22)

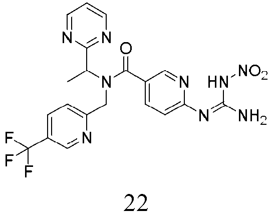

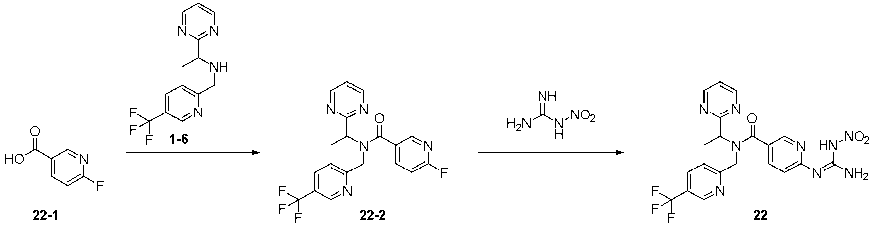

### Step 1: Preparation of 6-fluoro-N-(1-(pyrimidin-2-yl)ethyl)-N-((5-(trifluoromethyl)pyridin-2-yl)methyl)nicoti namide (22-2)

6-Fluoronicotinic acid (100 mg, 0.71 mmol), HATU (324 mg, 0.85 mmol), and DIEA (275 mg, 2.13 mmol) were added to a single-neck flask containing a solution of compound **1-6** (200 mg, 0.71 mmol) in DMF (5 mL) successively. The resulting mixture was stirred at room temperature under a nitrogen atmosphere for 18 hours. Water (10 mL) was added to the reaction solution, and the mixture was extracted with EtOAc (10 mL × 3). The organic phases were combined, dried over anhydrous sodium sulfate, and filtered. The filtrate was concentrated under reduced pressure. The residues were purified by Prep-TLC (DCM/EtOAc = 1/1) to obtain compound 22-2 (0.14 g, yield 49%, pale yellow solid).

LC-MS (ESI+): 406.1 m/z [M+H]⁺.

### Step 2: Preparation of 6-((amino(nitroamino)methylene)amino)-N-(1-(pyrimidin-2-yl)ethyl)-N-((5-(trifluorom ethyl)pyridin-2-yl)methyl)nicotinamide (22)

1-Nitroguanidine (23 mg, 0.22 mmol) and Cs₂CO₃ (181 mg, 0.55 mmol) were added to a single-neck flask containing a solution of compound 22-2 (75 mg, 0.18 mmol) in DMF (2 mL) successively. The resulting mixture was heated to 40°C and stirred for 18 hours. The reaction solution was cooled to room temperature, and water (10 mL) was added. The mixture was extracted with EtOAc (10 mL × 3). The organic phases were combined, dried over anhydrous sodium sulfate, and filtered. The filtrate was concentrated to dryness under reduced pressure. The residues were purified by Prep-HPLC (ACN/H₂O = 5-95%) and lyophilized to obtain compound **22** (17.8 mg, yield 20%, white solid).

LC-MS (ESI+): 490.1 m/z [M+H]⁺.

¹H NMR (400 MHz, CH₃OH-d₄) δ 8.85 - 8.42 (m, 4H), 8.17 - 7.86 (m, 2H), 7.59 - 7.47 (m, 1H), 7.31 (t, J = 4.9 Hz, 1H), 7.21 - 6.94 (m, 1H), 5.45 - 5.37 (m, 1H), 4.97 (d, J = 16.9 Hz, 1H), 4.77 - 4.56 (m, 1H), 1.71 - 1.60 (m, 3H).

The following compounds were obtained according to the synthesis method of Example 22 using corresponding starting materials:

| Compound No. | Structure | MS data (ESI+): m/z | NMR data |
|---|---|---|---|
| 23 | 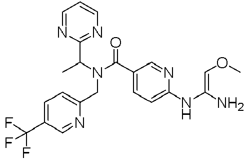 | 474.4 | |
| 24 | 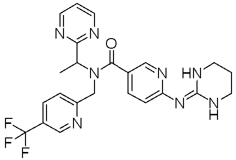 | 485.4 | |
| 25 | 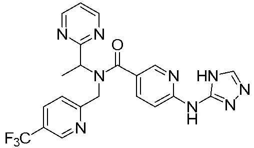 | 469.2 | ¹H NMR (400 MHz, DMSO-d₆) δ 8.97 (s, 1H), 8.85 - 8.81 (m, 3H), 8.70 (s, 1H), 8.23 - 8.11 (m, 2H), 7.71 - 7.60 (m, 2H), 7.46 - 7.44 (m, 1H), 5.97 (s, 2H), 5.36 (q, J = 7.6 Hz, 1H), 4.92 (d, J = 16.0 Hz, 1H), 4.56 (d, J = 16.8 Hz, 1H), 1.66 - 1.58 (m, 3H). |
| 26 | 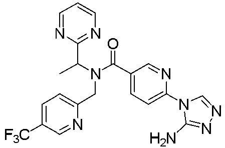 | 469.2 | ¹H NMR (400 MHz, DMSO-d₆) δ 8.91 - 8.66 (m, 3H), 8.27 - 7.27 (m, 2H), 7.70 - 7.60 (m, 4H), 7.45 - 7.43 (m, 1H), 5.38 - 5.36 (m, 1H), 4.92 (d, J = 16.0 Hz, 1H),4.54 (d, J = 16.8 Hz, 1H), 1.67 - 1.60 (m, 3H). |
| 27 | 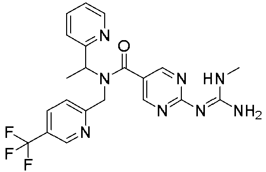 | 459.5 | |
| 28 | 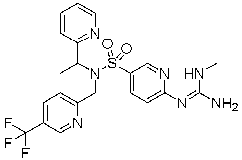 | 494.0 | |

### Example 29: Synthesis of 6-((isobutyrylamido(methylamino)methylene)amino)-N-(1-(pyrimidin-2-yl)ethyl)-N-((5 -(trifluoromethyl)pyridin-2-yl)methyl)nicotinamide (29)

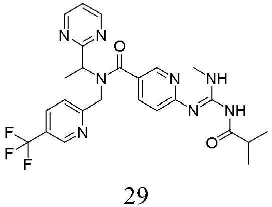

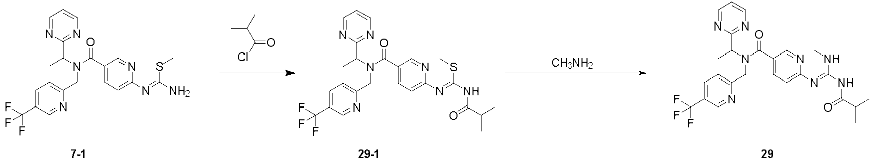

### Step 1: Preparation of methyl N-isobutyryl-N'-(5-((1-(pyrimidin-2-yl)ethyl)((5-(trifluoromethyl)pyridin-2-yl)methyl)c arbamoyl)pyridin-2-yl)carbamimidothioate (29-1)

A solution of compound 7-1 (200 mg, 0.42 mmol) in DCM (5.0 mL) was cooled to 0°C under an ice bath, and isobutyryl chloride (59.4 mg, 0.56 mmol) and triethylamine (200 µL, 1.42 mmol) were slowly added thereto. The resulting mixture was stirred at room temperature for 1 hour. The reaction solution was poured into water (30.0 mL) and extracted with DCM (30.0 mL x 2). The organic phase was dried over anhydrous sodium sulfate, filtered, and concentrated under reduced pressure to obtain crude compound 29-1 (200 mg, yield 87%).

### Step 2: Preparation of 6-((isobutyrylamido(methylamino)methylene)amino)-N-(1-(pyrimidin-2-yl)ethyl)-N-((5 -(trifluoromethyl)pyridin-2-yl)methyl)nicotinamide (29)

The crude compound 29-1 (200 mg, 0.36 mmol) and a solution of methylamine in tetrahydrofuran (2 M, 100 µL) were added to isopropanol (10.0 mL) successively at room temperature. The resulting mixture was reacted in a microwave at 80°C for 2 hours. The reaction solution was filtered and concentrated under reduced pressure. The obtained residues were purified by Prep-HPLC (ACN/H₂O = 5-95%) to obtain compound **29** (16 mg, yield 31%).

LC-MS (ESI+): 528.2 m/z [M+H]⁺.

### Example 30: Synthesis of 6-((1H-imidazol-2-yl)amino)-N-(1-(pyrimidin-2-yl)ethyl)-N-((5-(trifluoromethyl)pyridi n-2-yl)methyl)nicotinamide (30)

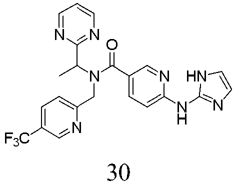

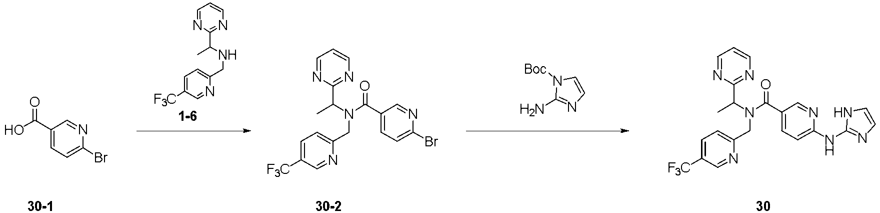

### Step 1: Preparation of 6-bromo-N-(1-(pyrimidin-2-yl)ethyl)-N-((5-(trifluoromethyl)pyridin-2-yl)methyl)nicoti namide (30-2)

6-Bromonicotinic acid (100 mg, 0.50 mmol) and compound 1-6 (140 mg, 0.60 mmol) were added to N,N-dimethylacetamide (2.0 mL) at room temperature, followed by the addition of tri-pyrrolidinylphosphonium hexafluorophosphate (278 mg, 0.60 mmol) and N,N-diisopropylethylamine (248 µL, 1.50 mmol) successively. The resulting mixture was stirred at 25°C for 2 hours. The reaction solution was poured into EtOAc (20.0 mL) and washed three times with saturated brine (10.0 mL). The organic phase was dried over anhydrous sodium sulfate, filtered, and concentrated under reduced pressure. The obtained residues were purified by silica gel column chromatography (DCM/MeOH = 10:1) to obtain compound 30-2 (231 mg, yield 62%).

### Step 2: Preparation of 6-((1H-imidazol-2-yl)amino)-N-(1-(pyrimidin-2-yl)ethyl)-N-((5-(trifluoromethyl)pyridi n-2-yl)methyl)nicotinamide (30)

Compound 30-2 (130 mg, 0.28 mmol), *tert-*butyl 2-amino-1H-imidazole-1-carboxylate (51.3 mg, 0.28 mmol), chloro[2-(di-*tert*-butylphosphino)-2',4',6'-triisopropyl-1,1'-biphenyl][2-(2-aminoethyl)ph enyl]palladium(II) (20.6 mg, 0.03 mmol), 2-di-*tert*-butylphosphino-2',4',6'-triisopropylbiphenyl (25.5 mg, 0.06 mmol), and sodium *tert*-butoxide (24 mg, 0.25 mmol) were added to *tert*-butanol (5.0 mL) at room temperature, and was replaced with nitrogen for three times. The mixture was stirred at 90°C under a nitrogen atmosphere for 16 hours and then cooled to room temperature. The cooled reaction mixture was poured into water (50.0 mL) and extracted with EtOAc (20.0 mL × 2). The organic phases were combined, washed with saturated aqueous sodium chloride solution (10.0 mL), dried over anhydrous sodium sulfate, and filtered. The filtrate was concentrated to dryness under reduced pressure. The obtained residues were purified by Prep-HPLC (ACN/H₂O = 5-95%) to obtain compound **30** (20 mg, yield 20%).

LC-MS (ESI+): 468.2 m/z [M+H]⁺.

### Example 31: Synthesis of 6-((amino(methylamino)methylene)amino)-N-(1-(m-tolyl)ethyl)-N-((5-(trifluoromethyl) pyridin-2-yl)methyl)nicotinamide (31)

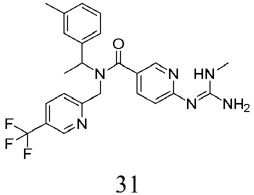

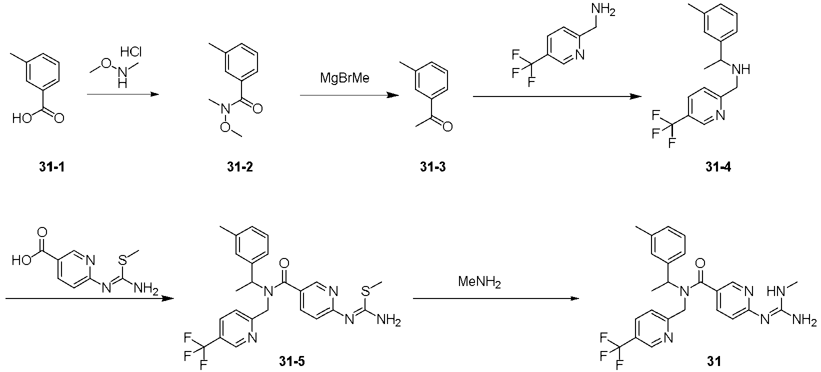

### Step 1: Preparation of N-methoxy-N,3-dimethylbenzamide (31-2)

3-Methylbenzoic acid (1.00 g, 7.34 mmol) was dissolved in DCM (10 mL) at 0°C, a solution of N,N'-carbonyldiimidazole (1.19 g, 7.34 mmol) in DCM (3.0 mL) was added thereto, and the mixture was stirred at this temperature for 0.5 hours. N,O-dimethylhydroxylamine hydrochloride (716 mg, 7.34 mmol) and triethylamine (743 mg, 7.34 mmol) were added successively at 0°C. The mixture was stirred at this temperature for 1 hour and then stirred at room temperature overnight. The reaction solution was washed once each with 1 N hydrochloric acid aqueous solution (50.0 mL), saturated sodium carbonate aqueous solution (50.0 mL), and saturated brine (50.0 mL). The organic phase was dried over anhydrous sodium sulfate, filtered, and concentrated under reduced pressure. The obtained residues were purified by silica gel column chromatography (PE/EtOAc = 4/1) to obtain compound 31-2 (1.2 g, crude yield 91%).

### Step 2: Preparation of 1-(m-tolyl)ethan-1-one (31-3)

Compound 31-2 (500 mg, 2.79 mmol) was dissolved in tetrahydrofuran (3.0 mL) at room temperature, and methylmagnesium bromide (3 M in THF, 1.86 mL, 5.58 mmol) was added thereto at 0°C. The mixture was stirred at 0°C for 3 hours. The reaction was quenched with saturated ammonium chloride aqueous solution (30.0 mL) and extracted with EtOAc (30.0 mL × 3). The organic phases were collected and washed once with saturated brine (30.0 mL). The organic phase was dried over anhydrous sodium sulfate, filtered, and concentrated under reduced pressure to obtain compound 31-3 (300 mg, crude yield 80%).

### Step 3: Preparation of 1-(m-tolyl)-N-((5-(trifluoromethyl)pyridin-2-yl)methyl)ethan-1-amine (31-4)

(5-(Trifluoromethyl)pyridin-2-yl)methanamine (360 mg, 2.04 mmol) and compound 31-3 (300 mg, 2.25 mmol) were dissolved in DCM (10 mL) at room temperature, and potassium acetate (240 mg, 2.45 mmol), acetic acid (122 mg, 2.04 mmol) and 4A molecular sieves (3.0 g) were added successively. The resulting mixture was stirred at room temperature under a nitrogen atmosphere for 0.5 hours, then cooled to 0°C, and sodium triacetoxyborohydride (1.23 g, 6.13 mmol) was added in 3 portions. After addition, the mixture was naturally warmed to room temperature and stirred overnight. The reaction solution was diluted with water (10.0 mL) and washed with saturated ammonium bicarbonate aqueous solution (20.0 mL). The resulting mixture was extracted with DCM (10.0 mL × 3). The organic phases were combined, washed with saturated brine (20.0 mL), dried over anhydrous sodium sulfate, filtered, and concentrated under reduced pressure. The obtained residues were purified by silica gel column chromatography (DCM/MeOH = 10/1) to obtain compound 31-4 (164 mg, yield 27%).

### Step 4: Preparation of methyl (Z)-N'-(5-((1-(m-tolyl)ethyl)((5-(trifluoromethyl)pyridin-2-yl)methyl)carbamoyl)pyridin -2-ylcarbamimidothioate (31-5)

Compound 1-4 (120 mg, 0.57 mmol) was dissolved in N,N-dimethylacetamide (5.0 mL) at room temperature, and compound 31-4 (167 mg, 0.57 mmol), tri-pyrrolidinylphosphonium hexafluorophosphate (318 mg, 0.68 mmol) and N,N-diisopropylethylamine (220 mg, 1.70 mmol) were added thereto successively. After stirring at room temperature for 2 hours, the reaction solution was diluted with water (10.0 mL) and extracted with EtOAc (10.0 mL × 3). The organic phases were combined, washed with saturated brine (10.0 mL), dried over anhydrous sodium sulfate, filtered, and concentrated under reduced pressure. The obtained residues were purified by silica gel column chromatography (DCM/MeOH = 10/1) to obtain compound 31-5 (300 mg, crude yield >100%).

### Step 5: Preparation of (Z)-6-((amino(methylamino)methylene)amino)-N-(1-(m-tolyl)ethyl)-N-((5-(trifluoromet hyl)pyridin-2-yl)methyl)nicotinamide (31)

Compound 31-5 (300 mg, 0.41 mmol) was dissolved in isopropanol (3.0 mL) at room temperature, and a solution of methylamine in ethanol (30%, 2.0 mL) was added thereto. The mixture was reacted in a microwave at 80°C for 1 hour. The reaction solution was purified by Prep-HPLC (ACN/H₂O = 5-95%) to obtain compound **31** (32 mg, yield 11%).

LC-MS (ESI+): 471.2 m/z [M+H]⁺.

¹H NMR (400 MHz, DMSO-d₆) δ 10.9 (s, 1H), 9.29 (s, 1H), 8.85 (s, 1H), 8.57 - 8.51 (m, 2H), 8.10 - 8.00 (m,1H), 7.56 (s, 1H), 7.22 (s, 2H), 7.10 (s, 3H),5.14 (s, 1H), 4.78 - 4.74 (m, 1H), 4.41 - 4.35 (m, 1H), 2.95 (s, 3H), 2.27 (s, 3H), 1.83 - 1.62 (m, 3H).

The following compounds were obtained according to the synthesis method of Example 31 using corresponding starting materials, wherein cyclobutanone (purchased from Shanghai Titan Scientific Exploration Platform) was used in place of compound 31-3 in Example 35, benzaldehyde (purchased from Shanghai Titan Scientific Exploration Platform) was used in place of compound 31-3 in Example 36, and 6,7-dihydro-5H-quinolin-8-one (purchased from Bide Pharmatech) was used in place of compound 31-3 in Example 44:

| Compound No. | Structure | MS data (ESI+): m/z | NMR data |
|---|---|---|---|
| 32 | 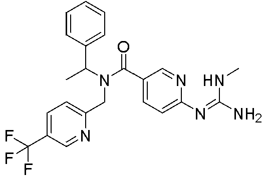 | 457.3 | ¹H NMR (400 MHz, DMSO-d₆) δ 8.86 (s, 1H), 8.42 (s, 1H), 8.37 (s, 1H), 8.06 (s, 1H), 7.90 (s, 1H), 7.50 (s, 1H), 7.37 - 7.28 (m, 5H), 6.97 (s, 1H), 5.30 (s, 1H), 4.76 (d, J = 17.3 Hz, 1H), 4.37 (s, 1H), 2.85 (s,3H), 1.59 (s, 3H). |
| 33 | 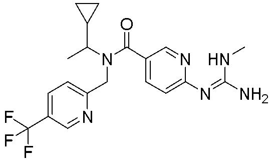 | 421.4 | ¹H NMR (400 MHz, DMSO-d₆ ) δ 8.93 (s, 1H), 8.39 (s, 1H), 8.31 (s, 1H), 8.16 (d, J = 8.6 Hz, 1H), 7.81 (d, J = 7.2 Hz, 1H), 7.65 (d, J = 7.9 Hz, 1H), 6.99 (s, 1H), 4.92 - 4.82 (m, 2H), 2.86 (s, 3H), 1.26 (s, 3H), 1.02 (s, 1H), 0.46 (s, 1H), 0.25 (s, 1H), 0.18 (s, 1H), 0.02 (s, 1H). |
| 34 | 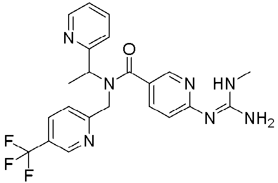 | 458.5 | ¹H NMR (400 MHz, DMSO-d₆) δ 11.20 (s, 1H), 9.47 (s, 1H), 8.83 (s, 1H), 8.57 - 8.54 (s, 3H), 8.11 - 8.06 (m, 2H), 7.78 (s, 1H), 7.51 (s, 1H), 7.37 - 7.31 (m, 2H), 7.17 - 6.99 (m, 1H), 5.21 (s, 1H), 4.81 (d, J = 16.0 Hz, 1H), 4.45 (d, J = 16.0 Hz, 1H), 2.94 (s, 3H), 1.60 (s, 3H). |
| 35 | 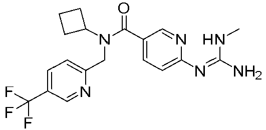 | 407.1 | ¹H NMR (400 MHz, DMSO-d₆) δ 8.96 (s, 1H), 8.39 (s, 1H), 8.31 (s, 1H), 8.19 (dd, J = 8.5, 2.4 Hz, 1H), 7.80 (s, 1H), 7.53 (d, J = 8.2 Hz, 1H), 7.00 (s, 1H), 4.91 (s, 2H), 4.46 - 4.45 (m, 1H), 2.88 (s, 3H), 2.14 (t, J = 10.1 Hz, 2H), 2.03 - 2.01 (m, 2H), 1.63 - 1.37 (m, 2H). |
| 36 | 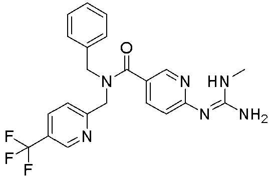 | 443.0 | |
| 37 | 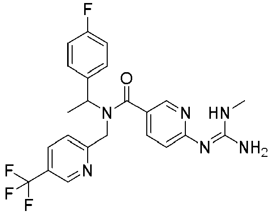 | 475.5 | ¹H NMR (400 MHz, DMSO-d₆) δ 11.05 (s, 1H), 9.36 (s, 1H), 8.81 (s, 1H), 8.53 (s, 3H), 8.05 (s, 2H), 7.50 - 7.11 (m, 5H), 5.14 - 4.41 (m, 2H), 2.92 (s, 3H), 1.56 (s, 3H). |
| 38 | 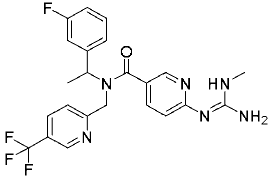 | 575.5 | ¹H NMR (400 MHz, DMSO-d₆) δ 11.17 (s, 1H), 9.42 (s, 1H), 8.83 (s, 1H), 8.57 (s, 3H), 8.11 (s, 2H), 7.53 - 7.08 (m, 5H), 5.80 - 4.44 (m, 2H), 2.92 (s, 3H), 1.58 (s, 3H). |
| 39 | 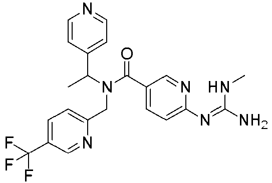 | 458.5 | |
| 40 | 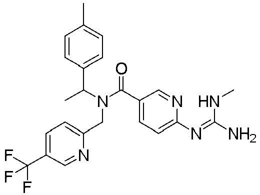 | 471.1 | ¹H NMR (400 MHz, CDCl₃) δ 9.25 (s, 1H), 8.75 (s, 2H), 8.53 (s, 2H), 8.01 (d, J = 8.5 Hz, 2H), 7.83 (s, 1H), 6.87 (s, 2H), 4.86 (s, 1H), 4.37 (d, J = 16.5 Hz, 2H), 2.97 (d, J = 5.0 Hz, 3H), 1.55 (s, 3H). |
| 41 | 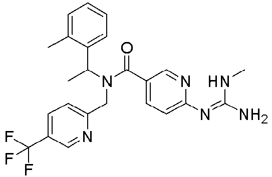 | 471.5 | |
| 42 | 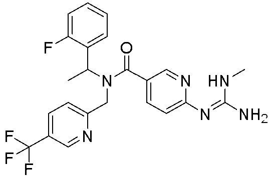 | 475.5 | ¹H NMR (400 MHz, DMSO-d₆) δ 8.81 (s, 1H), 8.41 (s, 1H), 8.05 - 8.03 (m, 1H), 7.89 (s, 1H), 7.56 - 7.49 (m, 1H), 7.41 (s, 1H), 7.34 - 7.28 (m, 1H), 7.16 (t, J = 7.6 Hz, 1H), 7.11 - 7.06 (m, 1H), 7.01 (s, 1H), 4.64 (d, J = 16.8 Hz, 1H), 4.52 (d, J = 16.8 Hz, 1H), 2.87 (s, 3H), 1.65 (s, 3H). |
| 43 | 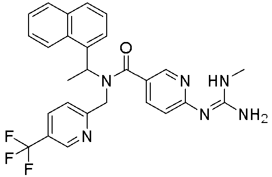 | 507.3 | |
| 44 | 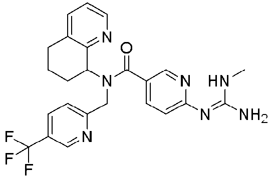 | 484.5 | ¹H NMR (400 MHz, DMSO-d₆) δ 8.86 (s, 1H), 8.54 (d, J = 2.3 Hz, 1H), 8.47 (d, J = 4.6 Hz, 1H), 8.41 (s, 1H), 8.16 (dd, J = 8.4, 2.4 Hz, 1H), 8.00 (d, J = 8.4 Hz, 1H), 7.75 (d, J = 8.3 Hz, 1H), 7.55 (d, J = 7.7 Hz, 1H), 7.25 (dd, J = 7.7, 4.7 Hz, 1H), 7.03 (d, J = 8.4 Hz, 1H), 5.19 - 5.14 (m, 1H), 4.78 (d, J = 16.8 Hz, 1H), 3.99 (d, J = 16.8 Hz, 1H), 2.86 (s, 3H), 2.82 - 2.78 (m, 1H), 2.73 - 2.63 (m, 1H), 2.29 (s, 1H), 1.98 - 1.87 (m, 2H), 1.70 - 1.66 (m, 1H). |
| 58 | 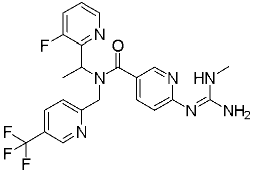 | 476.0 | |
| 59 | 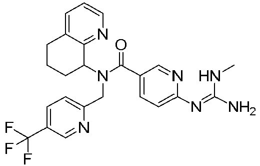 | 484.2 | |
| 60 | 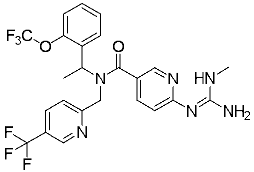 | 541.2 | |
| 61 | 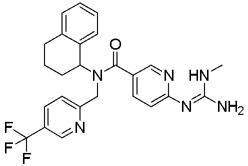 | 483.2 | |

### Example 45: Synthesis of 6-((amino(methylamino)methylene)amino)-N-(quinolin-8-yl)-N-((5-(trifluoromethyl)py ridin-2-yl)methyl)nicotinamide (45)

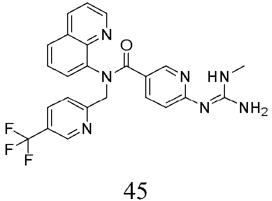

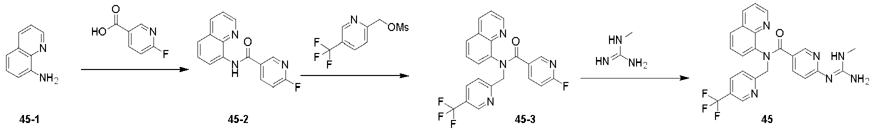

### Step 1: Preparation of 6-fluoro-N-(quinolin-8-yl)nicotinamide (45-2)

6-Fluoronicotinic acid (500 mg, 3.54 mmol) was dissolved in N,N-dimethylacetamide (10.0 mL) at room temperature, and quinolin-8-amine (511 mg, 3.54 mmol), tri-pyrrolidinylphosphonium hexafluorophosphate (2.48 g, 5.31 mmol) and N,N-diisopropylethylamine (1.08 g, 10.6 mmol) were added thereto successively. After stirring at room temperature overnight, the reaction solution was diluted with water (10 mL) and extracted with EtOAc (10 mL × 3). The organic phases were combined, washed with saturated brine (10 mL), dried over anhydrous sodium sulfate, filtered, and concentrated under reduced pressure. The obtained residues were purified by silica gel column chromatography (DCM/MeOH = 10/1) to obtain compound 45-2 (900 mg, crude yield 95%).

### Step 2: Preparation of 6-fluoro-N-(quinolin-8-yl)-N-((5-(trifluoromethyl)pyridin-2-yl)methyl)nicotinamide (45-3)

Compound 45-2 (300 mg, 1.12 mmol) was dissolved in N,N-dimethylformamide (10 mL) at room temperature,, and (5-(trifluoromethyl)pyridin-2-yl)methyl methanesulfonate (573 mg, 2.24 mmol) and potassium carbonate (465 mg, 3.36 mmol) were added thereto successively. After stirring at 50°C overnight, the reaction solution was diluted with water (10 mL) and extracted with EtOAc (10 mL × 3). The organic phases were combined, washed with saturated brine (10 mL), dried over anhydrous sodium sulfate, filtered, and concentrated under reduced pressure. The obtained residues were purified by silica gel column chromatography (DCM/MeOH = 10/1) to obtain compound 45-3 (300 mg, crude yield 55%).

### Step 3: Preparation of 6-((amino(methylamino)methylene)amino)-N-(quinolin-8-yl)-N-((5-(trifluoromethyl)py ridin-2-yl)methyl)nicotinamide (45)

Compound 45-3 (300 mg, 0.70 mmol) was dissolved in N,N-dimethylacetamide (5.0 mL) at room temperature, and 1-methylguanidine hydrochloride (153 mg, 1.40 mmol) and cesium carbonate (153 mg, 2.80 mmol) were added thereto successively. After stirring at 100°C overnight, the reaction solution was purified by Prep-HPLC (ACN/H₂O = 5-95%) to obtain compound 45 (23.8 mg, yield 7%).

LC-MS (ESI+): 480.4 m/z [M+H]⁺.

¹HNMR (400 MHz, DMSO-d₆) δ 8.91 (d, J = 3.6 Hz, 1H), 8.83 (s, 1H), 8.34 (d, J = 8.8 Hz, 1H), 8.29 (s, 1H), 8.17 (dd, J = 8.2, 2.4 Hz, 1H), 8.02 (s, 1H), 7.96 (d, J = 8.3 Hz, 1H), 7.88 (d, J = 8.5 Hz, 1H), 7.80 (d, J = 7.3 Hz, 1H), 7.56 - 7.51 (m, 3H), 6.50 (s, 1H), 5.59 (s, 1H), 5.08 (s, 1H), 2.71 (s, 3H).

The following compounds were obtained according to the synthesis method of Example 45 using corresponding starting materials:

| Compound No. | Structure | MS data (ESI+): m/z | NMR data |
|---|---|---|---|
| 46 | 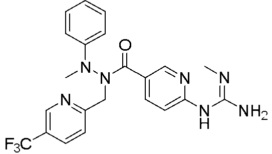 | 458.2 | |
| 47 | 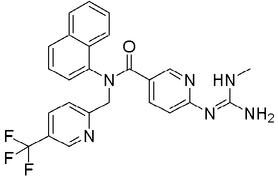 | 479.0 | ¹H NMR (400 MHz, DMSO-d₆) δ 8.90 (s, 1H), 8.32 (s, 1H), 8.22 (d, J = 8.0 Hz, 1H), 8.08 (d, J = 8.0 Hz, 1H), 8.03 (s, 1H), 7.98 (d, J = 7.6 Hz, 1H), 7.87 (d, J = 8.0 Hz, 1H), 7.82 (d, J = 8.8 Hz, 1H), 7.63 (t, J = 7.6 Hz, 1H), 7.58 (t, J = 7.6 Hz, 1H), 7.48 (d, J = 6.4 Hz, 2H), 7.42 (t, J = 8.0 Hz, 1H), 5.65 (d, J = 16.0 Hz, 1H), 4.88 (d, J = 16.0 Hz, 1H), 2.73 (s, 3H). |
| 48 | 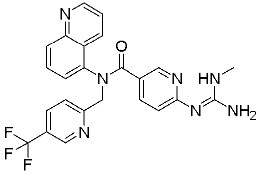 | 480.3 | ¹H NMR (400 MHz, DMSO-d₆) δ 9.01 - 8.82 (m, 2H), 8.51 (d, J = 8.4 Hz, 1H), 8.36 -8.34 (m, 2H), 8.22 - 8.20 (m, 1H), 7.99 - 7.95 (m, 2H), 7.82 - 7.90 (m,1H), 7.72 - 7.68 (m, 1H), 7.64 - 7.59 (m, 2H), 7.42 - 7.40 (m, 1H), 5.56 (d, J = 16.0 Hz, 1H), 5.01 (d, J = 15.6 Hz, 1H), 2.71 - 2.68 (m, 3H). |
| 49 | 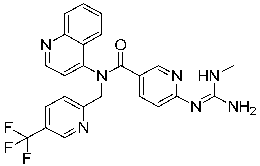 | 480.5 | ¹H NMR (400 MHz, DMSO-d₆) δ 9.13 (d, J = 2.3 Hz, 1H), 8.95 - 8.89 (m, 1H), 8.85 (dd, J = 8.2, 1.6 Hz, 1H), 8.44 - 8.42 (m, 2H), 8.38 (d, J = 7.7 Hz, 1H), 8.28 (dd, J = 8.4, 2.4 Hz, 1H), 7.80 - 7.66 (m, 4H), 7.57 - 7.51 (m, 1H), 7.10 (d, J = 8.6 Hz, 1H), 5.95 (s, 2H), 2.90 (s, 3H). |
| 50 | 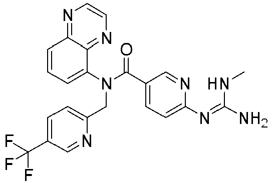 | 481.5 | ¹H NMR (400 MHz, DMSO-d₆) δ 8.94 (s, 1H),8.91 (s, 2H), 8.83 (d, J = 4.0 Hz, 1H), 8.34 - 8.32 (m, 1H), 8.19 - 8.15 (m, 1H), 8.02 - 7.99 (m, 1H), 7.94 - 7.89 (m, 2H), 7.84 - 7.79 (m, 2H), 5.58 (s, 1H), 5.14 (s, 1H), 3.16 (s, 3H). |
| 51 | 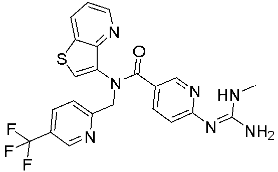 | 486.3 | ¹HNMR (400 MHz, DMSO-d₆) δ 8.88 (s, 1H), 8.68 (dd, J = 4.5, 1.4 Hz, 1H), 8.46 - 8.43 (m, 1H), 8.32 (s, 1H), 8.21 - 8.18 (m, 1H), 8.14 (d, J = 2.4 Hz, 1H), 8.06 (s, 1H), 7.91 (d, J = 8.2 Hz, 1H), 7.57 - 7.55 (m, 1H), 7.41 (dd, J = 8.2, 4.6 Hz, 1H), 6.62 - 6.60 (m, 1H), 5.34 (s, 2H), 2.76 (s, 3H). |
| 52 | 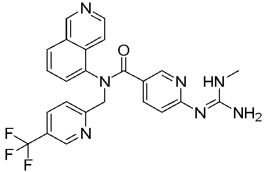 | 480.2 | ¹HNMR (400 MHz, CDCl₃) δ 9.34 (s, 1H), 8.85 (s, 1H), 8.68 (d, J = 6.0 Hz, 1H), 8.58 (s, 1H), 8.21 (s, 1H), 7.99 (t, J = 7.1 Hz, 2H), 7.88 (d, J = 6.0 Hz, 1H), 7.74 (d, J = 8.1 Hz, 1H), 7.65 (t, J = 8.5 Hz, 2H), 7.55 (d, J = 8.0 Hz, 1H), 6.82 (d, J = 8.4 Hz, 1H), 5.76 (d, J = 15.2 Hz, 1H), 4.90 (d, J = 15.2 Hz, 1H), 3.37 (s, 3H). |
| 53 | 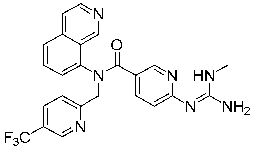 | 480.0 | |

### Example 54: Synthesis of 6-((amino(methylamino)methylene)amino)-N-methyl-N-(6-(trifluoromethyl)-2,3-dihydr obenzofuran-3-yl)nicotinamide (54)

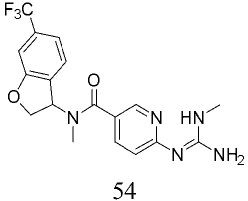

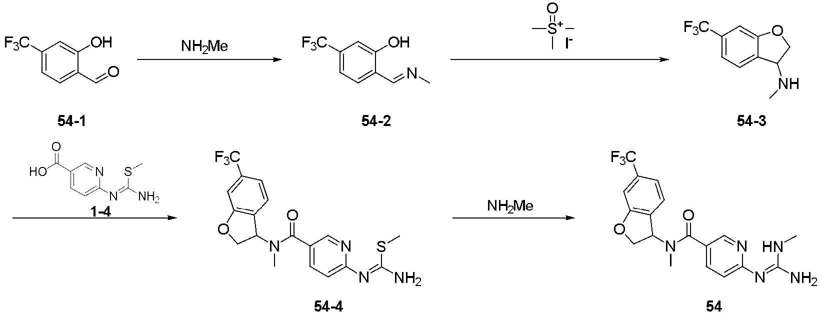

### Step 1: Preparation of 2-((methylimino)methyl)-5-(trifluoromethyl)phenol (54-2)

MgSO₄ (1.2 g, 10.5 mmol) and NH₂Me (2 M in THF, 2.6 mL, 5.2 mmol) were added to a single-neck flask containing a solution of 2-hydroxy-4-(trifluoromethyl)benzaldehyde (0.5 g, 2.6 mmol) in DCM (5 mL) successively. The resulting mixture was stirred at room temperature for 18 hours. The reaction solution was filtered, and the filter cake was dried under vacuum to obtain compound 54-2 (0.2 g, yield 37%, yellow solid).

LC-MS (ESI+): 204.0 m/z [M+H]⁺.

### Step 2: Preparation of N-methyl-6-(trifluoromethyl)-2,3-dihydrobenzofuran-3-amine (54-3)

Potassium *tert*-butoxide (1 M In THF, 2.4 mL, 2.4 mmol) was added to a solution of trimethylsulfoxonium iodide (0.5 g, 2.4 mmol) in THF (5 mL) at room temperature. After the resulting mixture was stirred at room temperature for 0.5 hours, a solution of (E)-2-((methylimino)methyl)-5-(trifluoromethyl)phenol (0.2 g, 0.9 mmol) in THF (2 mL) was slowly added dropwise, and the mixture was continued to stir at room temperature for 1 hour, and then heated to 50°C and stirred for 3 hours. The reaction solution was cooled to room temperature and filtered to remove insoluble solids. H₂O (20 mL) was added to the filtrate, and the mixture was extracted with EtOAc (20 mL × 3). The organic phases were combined, washed with saturated brine, dried over anhydrous sodium sulfate, and filtered. The filtrate was concentrated under reduced pressure. The residues were purified by silica gel column chromatography (DCM/EtOAc = 1/1) to obtain compound 54-3 (92 mg, yield 43%, colorless oil).

LC-MS (ESI+): 218.0 m/z [M+H]⁺.

### Step 3: Preparation of methyl N'-(5-(methyl(6-(trifluoromethyl)-2,3-dihydrobenzofuran-3-yl)carbamoyl)pyridin-2-yl)c arbamimidothioate (54-4)

Triethylamine (110 mg, 1.0 mmol) and compound 1-4 (90 mg, 0.4 mmol) were added to a single-neck flask containing a solution of N-methyl-6-(trifluoromethyl)-2,3-dihydrobenzofuran-3-amine (92 mg, 0.4 mmol) in DMF (2 mL) successively. The resulting mixture was cooled to 0°C, and BOPCl (0.13 g, 0.5 mmol) was slowly added thereto. The mixture was allowed to naturally warm to room temperature and stirred for 18 hours. EtOAc (50 mL) was added to the reaction solution, and the mixture was washed with saturated brine (10 mL × 3). The organic phase was dried over anhydrous sodium sulfate and filtered. The filtrate was concentrated to dryness under reduced pressure. The residues were purified by silica gel column chromatography (DCM/MeOH = 15/1) to obtain compound 54-4 (100 mg, yield 58%, colorless oil).

LC-MS (ESI+): 411.0 m/z [M+H]⁺.

### Step 4: Preparation of 6-((amino(methylamino)methylene)amino)-N-methyl-N-(6-(trifluoromethyl)-2,3-dihydr obenzofuran-3-yl)nicotinamide (54)

NH₂Me (2 M in THF, 0.3 mL, 0.6 mmol) was added to a sealed tube containing a solution of compound 54-4 (50 mg, 0.1 mmol) in isopropanol (2 mL). The resulting mixture was stirred at 90°C for 18 hours. The reaction solution was cooled to room temperature and then concentrated under reduced pressure. The residues were purified by Prep-HPLC (ACN/H₂O = 5-95%) and lyophilized to obtain compound 54 (40.3 mg, yield 85%, white solid).

LC-MS (ESI+): 394.1 m/z [M+H]⁺.

¹H NMR (400 MHz, CH₃OH-d₄) δ 8.49 (s, 1 H), 7.99 (dd, J = 8.5, 1.9 Hz, 1 H), 7.55 (s, 1 H), 7.27 (d, J = 7.9 Hz, 1 H), 7.16 - 7.01 (m, 1 H), 6.41 (s, 0.6 H), 5.76 (s, 0.4 H), 4.83 - 4.60 (m, 2 H), 3.01 (s, 3 H), 2.74 (s, 3 H).

### Example 55: Synthesis of 6-((amino(methylamino)methylene)amino)-N-methyl-N-(6-(pyridin-4-yl)-2,3-dihydrob enzofuran-3-yl)nicotinamide (55)

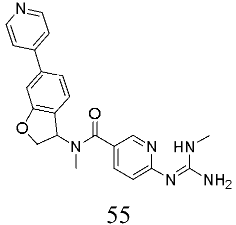

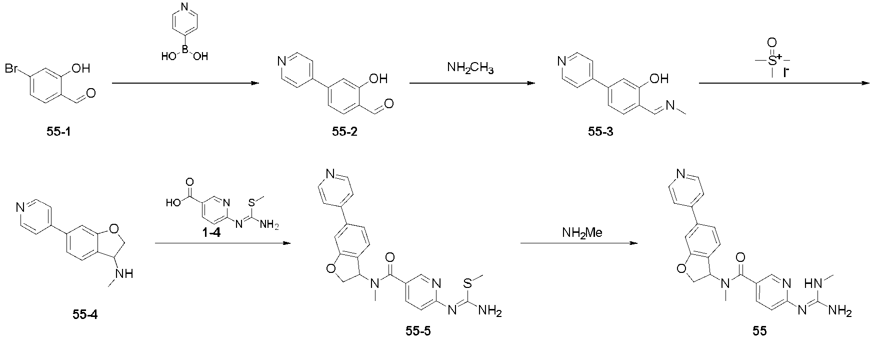

### Step 1: Preparation of 2-hydroxy-4-(pyridin-4-yl)benzaldehyde (55-2)

K₂CO₃ (66 mg, 0.6 mmol) and pyridin-4-ylboronic acid (30 mg, 0.6 mmol) were added to a single-neck flask containing a solution of 4-bromo-2-hydroxybenzaldehyde (50 mg, 0.2 mmol) in dioxane/H₂O (2 mL/0.5 mL) successively at room temperature. After the resulting mixture was purged with nitrogen, Pd(PPh₃)₄ (14 mg, 0.01 mmol) was added. After purging with nitrogen again, the mixture was heated to 90°C and stirred for 4 hours. The reaction solution was cooled to room temperature, water (20 mL) was added, and the mixture was extracted with EtOAc (20 mL × 3). The organic phases were combined, washed with saturated brine, dried over anhydrous sodium sulfate, and filtered. The filtrate was concentrated under reduced pressure. The residues were purified by Prep-TLC (PE/EtOAc = 3/1) to obtain compound 55-2 (30 mg, yield 61%, yellow solid).

LC-MS (ESI+): 200.0 m/z [M+H]⁺.

### Step 2: Preparation of 2-((methylimino)methyl)-5-(pyridin-4-yl)phenol (55-3)

Magnesium sulfate (0.48 g, 4.0 mmol) and methylamine (2 M in THF, 1 mL, 2 mmol) were added to a single-neck flask containing a solution of compound 55-2 (0.2 g, 1.0 mmol) in DCM (2 mL) successively. The resulting mixture was stirred at room temperature for 18 hours. The reaction solution was filtered through Celite, and the filtrate was concentrated under reduced pressure to obtain compound 55-3 (0.2 g, yield 99%, yellow solid), which was used directly in the next step without purification.

LC-MS (ESI+): 213.1 m/z [M+H]⁺.

### Step 3: Preparation of N-methyl-6-(pyridin-4-yl)-2,3-dihydrobenzofuran-3-amine (55-4)

t-BuOK (0.26 g, 2.36 mmol) was slowly added to a three-neck flask containing a solution of trimethylsulfoxonium iodide (0.5 g, 2.4 mmol) in THF (2 mL) under a nitrogen atmosphere. After the resulting mixture was stirred at room temperature for 0.5 hours, a solution of compound 55-3 (0.2 g, 0.9 mmol) in THF (2 mL) was slowly added dropwise. The mixture was continued to stir at room temperature for 1 hour, and then heated to 50°C and stirred for 2 hours. The reaction solution was cooled to room temperature, water (20 mL) was added, and the mixture was extracted with EtOAc (20 mL × 3). The organic phases were combined, washed with saturated brine, dried over anhydrous sodium sulfate, and filtered. The filtrate was concentrated under reduced pressure. The residues were purified by Prep-TLC (DCM/MeOH = 15/1) to obtain compound 55-4 (90 mg, yield 42%, colorless oil).

LC-MS (ESI+): 227.1 m/z [M+H]⁺.

### Step 4: Preparation of methyl N'-(5-(methyl(6-(pyridin-4-yl)-2,3-dihydrobenzofuran-3-yl)carbamoyl)pyridin-2-yl)carb amimidothioate (55-5)

Compound 1-4 (0.1 g, 0.4 mmol) and TEA (0.1 g, 0.9 mmol) were added to a single-neck flask containing a solution of compound 55-4 (90 mg, 0.3 mmol) in DMF (2 mL) successively. The resulting mixture was cooled to 0°C, and BOPCl (0.1 g, 0.4 mmol) was added thereto. The mixture was stirred at room temperature for 18 hours. EtOAc (20 mL) was added to the reaction solution, and the mixture was washed with saturated brine (10 mL × 3). The organic phase was dried over anhydrous sodium sulfate and filtered. The filtrate was concentrated under reduced pressure. The residues were purified by Prep-TLC (DCM/MeOH = 15/1) to obtain compound 55-5 (80 mg, yield 48%, white solid).

LC-MS (ESI+): 420.2 m/z [M+H]⁺.

### Step 5: Preparation of (Z)-6-((amino(methylamino)methylene)amino)-N-methyl-N-(6-(pyridin-4-yl)-2,3-dihyd robenzofuran-3-yl)nicotinamide (55)

Methylamine (2 M in THF, 0.3 mL, 0.7 mmol) was added to a sealed tube containing a solution of compound **55-5** (80 mg, 0.2 mmol) in isopropanol (2 mL). The resulting mixture was stirred at 90°C for 18 hours. The reaction solution was cooled to room temperature and concentrated under reduced pressure. The residues were purified by Prep-HPLC (ACN/H₂O = 5-95%) and lyophilized to obtain compound **55** (13 mg, yield 17%, white solid).

LC-MS (ESI+): 403.2 m/z [M+H]⁺.

¹H NMR (400 MHz, CH₃OH-d₄) δ 8.56 (d, J = 4.9 Hz, 2H), 8.47 (dd, J = 15.5, 2.1 Hz, 1H), 8.01 - 7.83 (m, 1H), 7.68 (d, J = 5.8 Hz, 2H), 7.50 (s, 1H), 7.36 (d, J = 7.6 Hz, 1H), 7.25 (s, 1H), 7.12 - 7.04 (m, 1H), 6.40 (s, 0.6H), 5.76 (s, 0.4H), 4.82 - 4.58 (m, 2H), 3.00 (s, 1.2 H), 2.77 (s, 3H), 2.50 (s, 1.7 H).

### Example 56: Synthesis of 6-((amino(methylamino)methylene)amino)-N-(1H-pyrrolo[2,3-b]pyridin-1-yl)-N-[(5-(tr ifluoromethyl)pyridin-2-yl)methyl)nicotinamide (56)

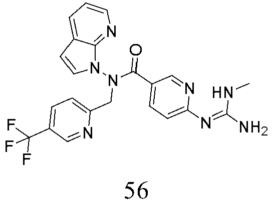

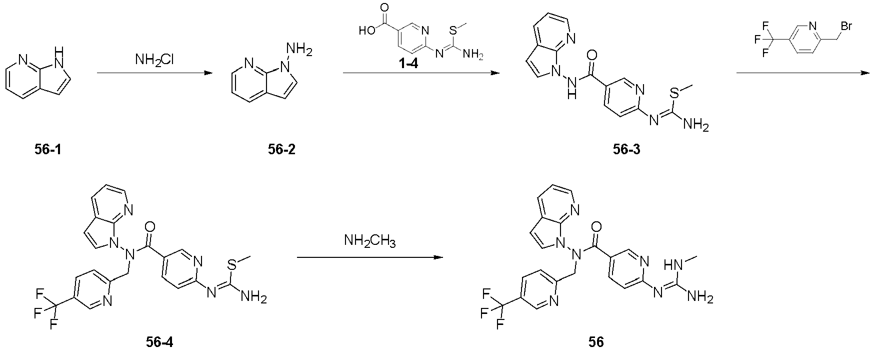

### Step 1: Preparation of 1H-pyrrolo[2,3-b]pyridin-1-amine (56-2)

Potassium *tert*-butoxide (1.9 g, 16.9 mmol) was slowly added to a single-neck flask containing a solution of 1H-pyrrolo[2,3-b]pyridine (1.0 g, 8.47 mmol) in DMF (60 mL). After the resulting mixture was stirred at room temperature for 2 hours, it was cooled to about 0°C, and a solution of NH₂Cl in ether (0.15 mol/L, 84.7 mL, 12.7 mmol) was slowly added dropwise. After the addition was complete, the reaction solution was allowed to naturally warm to room temperature and stirred overnight. Water (100 mL) was added to the reaction solution, and the mixture was extracted with methyl *tert-*butyl ether (50 mL × 3). The organic phases were combined, washed with saturated brine, dried over anhydrous sodium sulfate, and filtered. The filtrate was concentrated to dryness under reduced pressure. The residues were purified by Prep-TLC (PE/EtOAc = 1/1) to obtain compound 56-2 (322 mg, yield 26%, white solid).

LC-MS (ESI+): 134.1 m/z [M+H]⁺.

### Step 2: Preparation of methyl N'-(5-((1H-pyrrolo[2,3-b]pyridin-1-yl)carbamoyl)pyridin-2-yl)carbamimidothioate (56-3)

Compound 1-4 (416 mg, 1.96 mmol), DIEA (504 mg, 3.91 mmol), and PyBrOP (1.0 g, 2.15 mmol) were added to a single-neck flask containing a solution of compound 56-2 (260 mg, 1.96 mmol) in DMF (15 mL). The resulting mixture was stirred at room temperature overnight. Water (50 mL) was added to the reaction solution, and the mixture was extracted with EtOAc (20 mL × 3). The organic phases were combined, washed with saturated brine, dried over anhydrous sodium sulfate, and filtered. The filtrate was concentrated under reduced pressure. The residues were purified by Prep-TLC (DCM/MeOH = 15/1) to obtain compound 56-3 (500 mg, yield 78%, pale yellow oil).

LC-MS (ESI+): 327.1 m/z [M+H]⁺.

### Step 3: Preparation of methyl N'-(5-((1H-pyrrolo[2,3-b]pyridin-1-yl)((5-(trifluoromethyl)pyridin-2-yl)methyl)carbam oyl)pyridin-2-ylcarbamimidothioate (56-4)

K₂CO₃ (638 mg, 4.62 mmol) was added to a single-neck flask containing a solution of compound 56-3 (500 mg, 1.54 mmol) and 2-(bromomethyl)-5-(trifluoromethyl)pyridine (391 mg, 1.54 mmol) in DMF (10 mL). The resulting mixture was heated to 40°C and stirred for 12 hours. The reaction solution was cooled to room temperature, water (20 mL) was added, and the mixture was extracted with EtOAc (10 mL × 3). The organic phases were combined, dried over anhydrous sodium sulfate, and filtered. The filtrate was concentrated under reduced pressure. The residues were purified by Prep-TLC (DCM/MeOH = 10/1) to obtain compound 56-4 (155 mg, yield 21%, pale yellow solid).

LC-MS (ESI+): 486.1 m/z [M+H]⁺.

### Step 4: Preparation of 6-((amino(methylamino)methylene)amino)-N-(1H-pyrrolo[2,3-b]pyridin-1-yl)-N-[(5-(tr ifluoromethyl)pyridin-2-yl)methyl)nicotinamide (56)

Methylamine (3 N in THF, 0.5 mL, 0.43 mmol) was added to a microwave tube containing a solution of compound 56-4 (50 mg, 0.11 mmol) in isopropanol (3 mL). After the resulting mixture was purged with nitrogen, it was heated to 80°C and stirred overnight. The reaction solution was cooled to room temperature and concentrated to dryness under reduced pressure. The residues were purified by Prep-HPLC (ACN/H₂O = 5-95%) and lyophilized to obtain compound **56** (5.82 mg, yield 12%, white solid).

LC-MS (ESI+): 469.2 m/z [M+H]⁺.

¹H NMR (400 MHz, CH₃Cl-d) δ 8.79 (s, 1H), 8.34 - 8.27 (m, 2H), 7.89 (dd, J = 8.1, 2.3 Hz, 1H), 7.83 (dd, J = 7.8, 1.5 Hz, 1H), 7.56 (d, J = 8.2 Hz, 1H), 7.50 - 7.42 (m, 1H), 7.16 (d, J = 3.8 Hz, 1H), 7.14 - 7.09 (m, 1H), 6.82 (d, J = 8.7 Hz, 1H), 6.32 (d, J = 3.8 Hz, 1H), 5.77 (d, J = 15.6 Hz, 1H), 4.89 (d, J = 15.6 Hz, 1H), 2.95 (s, 3H).

The following compounds were obtained according to the synthesis method of Example 56 using corresponding starting materials:

| Compound No. | Structure | MS data (ESI+): m/z | NMR data |
|---|---|---|---|
| 62 | 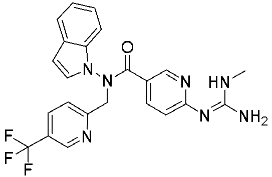 | 468.2 | ¹HNMR(400 MHz, DMSO-*d*₆) δ 8.31 (s, 1H), 8.21 - 8.12 (m, 2H), 7.72 (d, *J =* 8.3 Hz, 1H), 7.60 - 7.48 (m, 3H), 7.39 (d, *J* = 8.2 Hz, 1H), 7.18 (t, *J =* 7.6 Hz, 1H), 7.08 (t, *J* = 7.4 Hz, 1H), 6.44 (d, *J =* 3.4 Hz, 1H), 5.48 (d, *J* = 15.8 Hz, 1H), 5.04 (d, *J* = 15.8 Hz, 1H), 2.77 (s, 3H). |
| 63 | 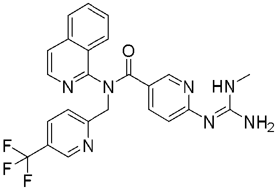 | 480.5 | |
| 64 | 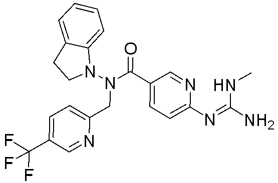 | 470.5 | 1H NMR (400 MHz, DMSO-*d*6) δ 8.92 (s, 1H), 8.54 (d, *J* = 2.4 Hz, 1H), 8.41 (s, 2H), 8.21 - 8.18 (m, 1H), 7.93 - 7.88 (m, 1H), 7.72 (d, *J =* 8.3 Hz, 1H), 7.18 (s, 1H), 7.15-7.13 (m, 1H), 6.89-6.85 (m, 1H), 6.78 (d, *J* = 7.7 Hz, 2H), 5.12 (d, *J =* 16.5 Hz, 1H), 4.54 (d, *J =* 16.5 Hz, 1H), 3.69 (d, *J* = 9.5 Hz, 4H), 2.79 (s, 3H). |

### Example 57: Synthesis of 2-(5-((2R,5S)-2-(4-fluorophenyl)-5-methylpiperidine-1-carbonyl)pyridin-2-yl)-1-methyl guanidine (57)

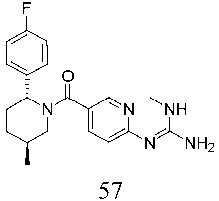

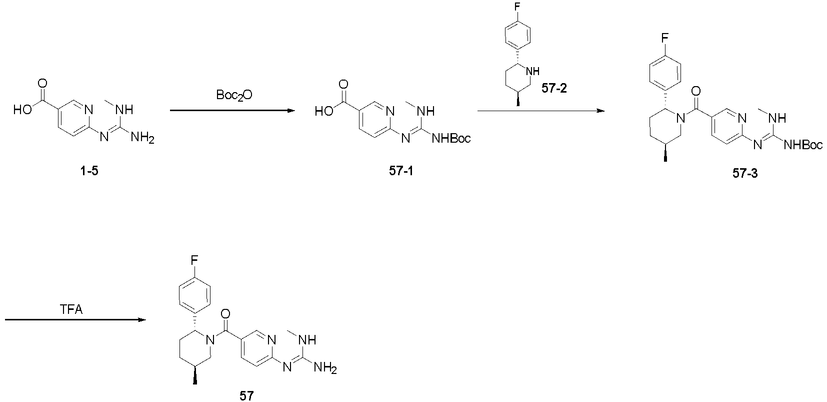

### Step 1: Synthesis of 6-((((tert-butoxycarbonyl)amino)(methylamino)methylene)amino)nicotinic acid (57-1)

Boc₂O (294 mg, 1.36 mmol), TEA (275 mg, 2.7 mmol) and DMAP (8.5 mg, 0.07 mmol) were added to a single-neck flask containing a solution of compound 1-5 (130 mg, 0.67 mmol) in THF (5 mL) successively. The resulting mixture was stirred at room temperature for 3 hours. Water (20 mL) was added to the reaction solution, and the mixture was extracted with DCM (10 mL × 3). The organic phases were combined, dried over anhydrous sodium sulfate, and filtered. The filtrate was concentrated to dryness under reduced pressure. The residues were purified by silica gel column chromatography (DCM/MeOH = 20/1) to obtain compound 57-1 (26 mg, yield 13%, brown liquid).

LC-MS (ESI+): 295.2 m/z [M+H]⁺.

### Step 2: Synthesis of tert-butyl 2-(5-((2R,5 S)-2-(4-fluorophenyl)-5-methylpiperidine-1-carbonyl)pyridin-2-yl)-N-methy 1-N'-carbamimidoylcarbamate (57-3)

(2R,5S)-2-(4-Fluorophenyl)-5-methylpiperidine (17 mg, 0.09 mmol) (57-2, referring to the synthesis method in Step 4A on page 655 of WO2022026892A1), HATU (40 mg, 0.11 mmol) and DIEA (46 mg, 0.35 mmol) were added to a single-neck flask containing a solution of compound 57-1 (26 mg, 0.09 mmol) in DMF (5 mL) successively. The resulting mixture was stirred at room temperature for 18 hours. Water (40 mL) was added to the reaction solution, and the mixture was extracted with EtOAc (20 mL × 3). The organic phases were combined, dried over anhydrous sodium sulfate, and filtered. The filtrate was concentrated to dryness under reduced pressure. The residues were purified by silica gel column chromatography (DCM/MeOH = 15/1) to obtain compound 57-3 (7 mg, yield 17%, white solid).

LC-MS (ESI+): 470.2 m/z [M+H]⁺.

### Step 3: Synthesis of 2-(5-((2R,5S)-2-(4-fluorophenyl)-5-methylpiperidine-1-carbonyl)pyridin-2-yl)-1-methyl guanidine (57)

TFA (17 mg, 0.15 mmol) was added to a single-neck flask containing a solution of compound 57-3 (7 mg, 0.015 mmol) in DCM (2 mL). The resulting mixture was stirred at room temperature for 3 hours. Water (10 mL) and aqueous NaHCO₃ solution (10 mL) were added to the reaction solution, and the mixture was extracted with EtOAc (20 mL × 3). The organic phases were combined, dried over anhydrous sodium sulfate, and filtered. The filtrate was concentrated to dryness under reduced pressure. The residues were purified by Prep-HPLC (ACN/H₂O = 5-95%) and lyophilized to obtain compound 57 (5.9 mg, yield 99%, white solid).

LC-MS (ESI+): 370.10 m/z [M+H]⁺.

¹H NMR (400 MHz, CH₃OH-d₄) δ 8.43 - 8.35 (m, 1H), 7.90 (dd, J = 8.5, 2.3 Hz, 1H), 7.40 - 7.29 (m, 2H), 7.18 -7.02 (m, 3H), 5.57 - 5.38 (m, 1H), 3.88 - 3.66 (m, 1H), 3.29 - 3.24 (m, 2H), 3.00 (s, 3H), 2.27 - 2.15 (m, 2H), 1.97 - 1.73 (m, 2H), 1.46 - 1.35 (m, 1H), 1.04 (d, J = 6.9 Hz, 3H).

### Examples 65 and 66: Preparation of rel-(S,Z)-6-((amino(methylamino)methylene)amino)-N-(1-(3-fluoropyridin-2-yl)ethyl)-N-((5-(trifluoromethyl)pyridin-2-yl)methyl)nicotinamide (65) and rel-(R,Z)-6-((amino(methylamino)methylene)amino)-N-(1-(3-fluoropyridin-2-yl)ethyl)-N-((5-(trifluoromethyl)pyridin-2-yl)methyl)nicotinamide (66)

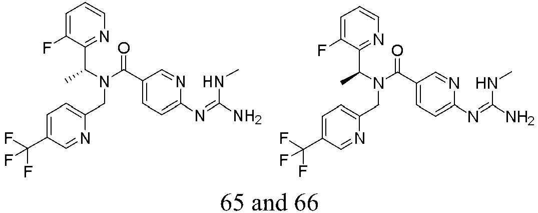

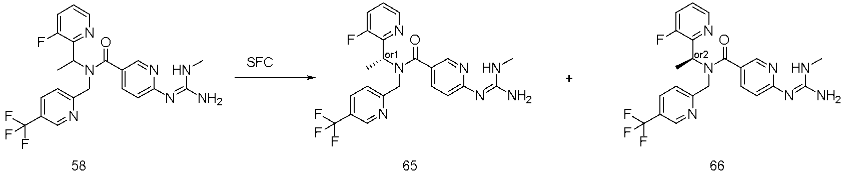

Compound 58 was subjected to chiral resolution (chiral column: AD-3, 0.46 cm × 5 cm; mobile phase: CO₂:EtOH (0.05% DEA) = 80:20; flow rate: 2.5 mL/min; column temperature: 25°C) to obtain a compound (retention time: 2.11 min), randomly defined as rel-(S,Z)-6-((amino(methylamino)methylene)amino)-N-(1-(3-fluoropyridin-2-yl)ethyl)-N-((5-(trifluoromethyl)pyridin-2-yl)methyl)nicotinamide (65), and a compound (retention time: 2.39 min), randomly defined as rel-(R,Z)-6-((amino(methylamino)methylene)amino)-N-(1-(3-fluoropyridin-2-yl)ethyl)-N-((5-(trifluoromethyl)pyridin-2-yl)methyl)nicotinamide (66).

### Compound 65:

LC-MS (ESI+): 476.2 m/z [M+H]⁺.

1H NMR (400 MHz, DMSO-*d*6) δ 8.78 (s, 1H), 8.33 (d, *J =* 3.2 Hz, 1H),8.27 (s, 1H) 8.04 (d, *J =* 7.2 Hz, 1H), 7.74 - 7.22 (m, 6H), 6.67 (s, 1H), 5.85 - 5.36 (m, 1H), 4.93 - 4.55 (m, 2H), 2.77 (s, 3H), 1.62 (s, 3H), 1.25 (s, 1H).

### Compound 66:

LC-MS (ESI+): 476.2 m/z [M+H]⁺.

¹H NMR (400 MHz, DMSO-d₆) δ 8.78 (s, 1H), 8.33 (d, *J =* 3.2 Hz, 1H), 8.28 (s, 1H), 8.04 (d, *J =* 7.6 Hz, 1H), 7.81 - 7.47 (m, 4H), 7.43 - 7.32 (m, 2H), 6.70 (s, 1H), 5.85-5.49 (m, 1H), 4.83 - 4.56 (m, 2H), 2.78 (s, 3H), 1.63 (s, 3H), 1.25 (s, 1H).

### Example 67: Preparation of (R,Z)-6-((amino(methylamino)methylene)amino)-N-(1-(2-fluorophenyl)ethyl)-N-((6'-m orpholino-[3,3'-bipyridin]-6-yl)methyl)nicotinamide (67)

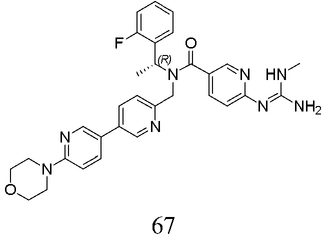

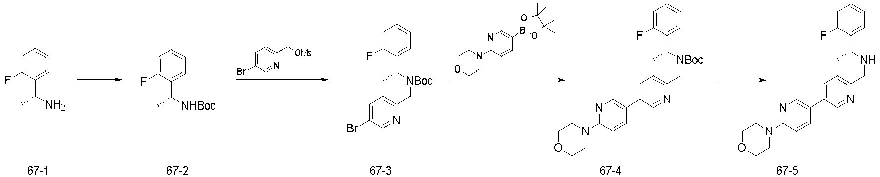

### Step 1: Preparation of tert-butyl (R)-(1-(2-fluorophenyl)ethyl)carbamate (67-2)

(R)-1-(2-fluorophenyl)ethanamine (67-1, 3.2 g, 23 mmol) and di-*tert*-butyl dicarbonate (7.5 mL) were dissolved in dichloromethane (30 mL) at room temperature, and diisopropylethylamine (6.5 mL) was added. The reaction solution was stirred at 25°C for 12 hours. The reaction was quenched by adding water (30 mL) to the reaction solution, followed by extraction with ethyl acetate (30 mL x 3). The organic phases were combined, dried over anhydrous sodium sulfate and filtered. The filtrate was concentrated under reduced pressure. The obtained residues were purified by silica gel column chromatography (eluent: ethyl acetate/petroleum ether = 1/4) to obtain compound 67-2 (4.4 g, yield: 80%).

LC-MS (ESI+): 240.2 m/z [M+H]⁺.

### Step 2: Preparation of tert-butyl (R)-((5-bromopyridin-2-yl)methyl)(1-(2-fluorophenyl)ethyl)carbamate (67-3)

Compound 67-2 (1.2 g, 5 mmol) was dissolved in N,N-dimethylformamide (10 mL), and sodium hydride (240 mg, 10 mmol) was added at 0°C. The reaction solution was stirred for 30 minutes. (5-Bromopyridin-2-yl)methyl methanesulfonate (1.3 g, 5 mmol) was added to the reaction solution. The reaction solution was stirred at 25°C for 1 hour. After completion of the reaction, the reaction was quenched by adding water (20 mL) to the reaction solution, followed by extraction with ethyl acetate (20 mL x 3). The organic phases were combined, dried over anhydrous sodium sulfate and filtered. The filtrate was concentrated under reduced pressure. The obtained residues were purified by silica gel column chromatography (eluent: ethyl acetate/petroleum ether = 1/1) to obtain compound 67-3 (1.6 g, yield 78%).

LC-MS (ESI+): 409.2 m/z [M+H]⁺.

### Step 3: Preparation of tert-butyl (R)-(1-(2-fluorophenyl)ethyl)((6'-morpholino-[3,3'-bipyridin]-6-yl)methyl)carbamate (67-4)

*tert*-Butyl (R)-((5-bromopyridin-2-yl)methyl)(1-(2-fluorophenyl)ethyl)carbamate (67-3, 100 mg, 0.24 mmol), 4-(5-(4,4,5,5-tetramethyl-1,3,2-dioxaborolan-2-yl)pyridin-2-yl)morpholine (141 mg, 0.48 mmol) and [1,1'-bis(diphenylphosphino)ferrocene]palladium dichloride (20 mg, 0.02 mmol) were dissolved in a mixed solution of dioxane (2 mL) and water (0.5 mL) at room temperature, and potassium carbonate (102 mg, 0.73 mmol) was added. The reaction solution was heated to 100°C and stirred for 12 hours under a nitrogen atmosphere. After completion of the reaction, water (10 mL) was added to the reaction solution, and the mixture was extracted with ethyl acetate (10 mL x 3). The organic phases were combined, dried over anhydrous sodium sulfate and filtered. The filtrate was concentrated under reduced pressure. The obtained residues were purified by silica gel column chromatography (eluent: methanol/dichloromethane = 1/10) to obtain compound 67-4 (100 mg, yield 83%).

LC-MS (ESI+): 493.2 m/z [M+H]⁺.

### Step 4: Preparation of (R)-1-(2-fluorophenyl)-N-((6'-morpholino-[3,3'-bipyridin]-6-yl)methyl)ethanamine (67-5)

Compound 67-4 (100 mg, 0.2 mmol) was dissolved in dry dichloromethane (2 mL) at room temperature, and trifluoroacetic acid (0.4 mL) was added. The reaction solution was stirred at 25°C for 1 hour. The reaction solution was concentrated under reduced pressure to obtain compound 67-5 (hydrochloride, 60 mg, yield 75%).

LC-MS (ESI+): 393.2 m/z [M+H]⁺.

The remaining steps were the same as Example 4, except that compound 67-5 was used instead of compound 1-6, to obtain compound 67.

LC-MS (ESI+): 569.4 m/z [M+H]⁺.

The following compounds were obtained according to the synthesis method of Example 67 using corresponding starting materials:

| Compound No. | Structure | MS data (ESI+): m/z | NMR data |
|---|---|---|---|
| 68 | 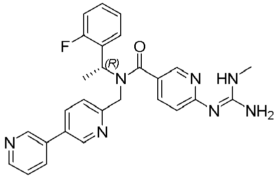 | 484.4 | |
| 69 | 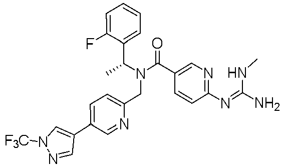 | 541.2 | |
| 70 | 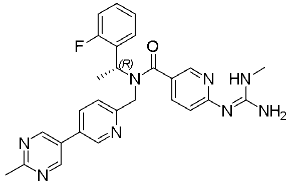 | 499.2 | ¹HNMR(400 MHz, DMSO-*d₆*) δ 9.09 - 8.98 (s, 2H), 8.84 (s, 1H), 8.38 (s, 2H), 8.05 (dd, *J =* 8.2, 2.0 Hz, 1H), 7.90 - 7.80 (m, 1H), 7.58 - 7.46 (m, 1H), 7.40 - 7.24 (m, 2H), 7.22 - 7.07 (m, 2H), 6.97 (s, 1H), 5.45 (s, 1H), 4.72 - 4.51 (m, 1H), 4.49 - 4.28 (m, 1H), 2.84 (s, 3H), 2.67 (s, 3H), 1.63 (s, 3H). |
| 71 | 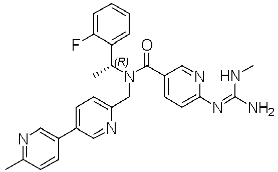 | 498.2 | ¹H NMR (400 MHz, DMSO-*d₆*) δ 8.82 - 8.71 (m, 2H), 8.49 - 8.32 (m, 2H), 8.04 - 7.93 (m, 2H), 7.95 - 7.85 (m, 1H), 7.52 (t, *J* = 7.4 Hz, 1H), 7.39 - 6.84 (m, 6H), 5.40 (s, 1H), 4.69 - 4.57 (m, 1H), 4.54 - 4.28 (m, 1H), 2.85 (s, 3H), 2.53 - 2.51 (m, 3H), 1.85 - 1.31 (s, 3H). |
| 72 | 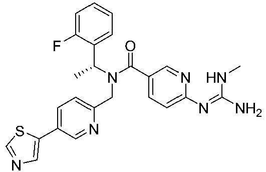 | 490.5 | ¹HNMR (400 MHz, DMSO-*d*₆) δ 9.15 (s, 1H), 8.77 (d, *J =* 2.4 Hz, 1H), 8.37 (d, *J* = 3.1 Hz, 3H), 7.96-7.93 (m, 1H), 7.86 (s, 1H), 7.54-7.49 (m, 1H), 7.34 - 7.29 (m, 1H), 7.20 - 7.07 (m, 3H), 6.98 (s, 2H), 5.44 (s, 1H), 4.61 (d, *J =* 16.9 Hz, 1H), 4.38 (d, *J* = 17.0 Hz, 1H), 2.84 (s, 4H), 1.61 (s, 3H). |
| 73 | 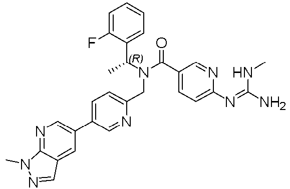 | 538.1 | ¹HNMR (400 MHz, DMSO-*d*₆) δ 8.88 (d, *J* = 2.4 Hz, 1H), 8.83 (d, *J =* 2.4 Hz, 1H), 8.53 (d, *J =* 2.4 Hz, 1H), 8.40 (s, 1H), 8.36 (s, 1H), 8.22 (s, 1H), 8.06-8.00 (m, 1H), 7.86 (s, 1H), 7.57-7.49 (m, 1H), 7.40 - 7.24 (m, 2H), 7.21 - 7.09 (m, 2H), 6.96 (s, 1H), 5.46 (s, 1H), 4.65 (d, *J =* 16.8 Hz, 1H), 4.40 (s, 1H), 4.10 (s, 3H), 2.83 (s, 3H), 1.64 (s, 3H). |

### Example 74: Preparation of 6-((Z)-(amino(methylamino)methylene)amino)-N-((5-(1-fluoroethyl)pyridin-2-yl)methy 1)-N-((R)-1-(2-fluorophenyl)ethyl)nicotinamide (74)

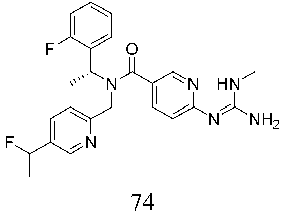

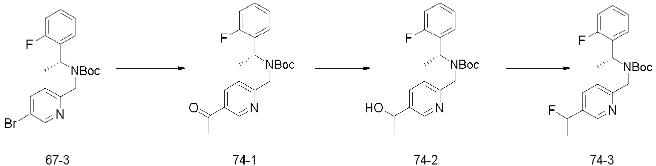

### Step 1: Preparation of tert-butyl (R)-((5-acetylpyridin-2-yl)methyl)(1-(2-fluorophenyl)ethyl)carbamate (74-1)

Compound 67-3 (1.6 g, 3.9 mmol) and tributyl(1-ethoxyvinyl)tin (2 mL) were dissolved in dry 1,4-dioxane (20 mL) at room temperature, and tetrakis(triphenylphosphine)palladium (451 mg, 0.39 mmol) was added thereto. The reaction solution was heated to 100°C and stirred for 12 hours under a nitrogen atmosphere. After completion of the reaction, 1 N hydrochloric acid (40 mL) was added to the reaction solution to quench the reaction, followed by extraction with ethyl acetate (30 mL × 3). The organic phases were combined, dried over anhydrous sodium sulfate, and filtered. The filtrate was concentrated under reduced pressure. The residues were purified by silica gel column chromatography (eluent: ethyl acetate / petroleum ether = 1/1) to obtain compound 74-1 (1.3 g, yield 89%).

LC-MS (ESI+): 373.2 m/z [M+H]⁺.

### Step 2: Preparation of tert-butyl ((R)-1-(2-fluorophenyl)ethyl)((5-(1-hydroxyethyl)pyridin-2-yl)methyl)carbamate (74-2)

Compound 74-1 (150 mg, 0.4 mmol) was dissolved in methanol (2 mL) at room temperature, and sodium borohydride (30 mg, 0.8 mmol) was added thereto. The reaction solution was stirred at 25°C for 1 hour. The reaction was quenched by water (10 mL), followed by extraction with ethyl acetate (10 mL × 3). The organic phases were combined, dried over anhydrous sodium sulfate, and filtered. The filtrate was concentrated under reduced pressure to obtain compound 74-2 (150 mg, yield 99%).

LC-MS (ESI+): 375.2 m/z [M+H]⁺.

### Step 3: Preparation of tert-butyl ((5-(1-fluoroethyl)pyridin-2-yl)methyl)((R)-1-(2-fluorophenyl)ethyl)carbamate (74-3)

Compound 74-2 (150 mg, 0.4 mmol) was dissolved in dichloromethane (5 mL) at room temperature, and diethylaminosulfur trifluoride (0.2 mL) was added thereto. The reaction solution was stirred at 25°C for 1 hour. The reaction was quenched by saturated aqueous sodium bicarbonate solution (10 mL), followed by extraction with ethyl acetate (10 mL × 3). The organic phases were combined, dried over anhydrous sodium sulfate, and filtered. The filtrate was concentrated under reduced pressure. The obtained residues were purified by silica gel column chromatography (eluent: ethyl acetate / petroleum ether = 1/5) to obtain compound 74-3 (120 mg, yield 80%).

LC-MS (ESI+): 377.4 m/z [M+H]⁺.

The remaining steps were the same as Example 67, except that compound 74-3 was used instead of compound 67-4, to obtain compound 74.

LC-MS (ESI+): 453.4 m/z [M+H]⁺.

¹H NMR (400 MHz, DMSO-*d*₆) δ 8.38 (s, 2H), 7.88 (s, 1H), 7.61 (d, *J =* 7.2 Hz, 1H), 7.43 (t, *J* = 7.2 Hz, 1H), 7.26-7.0 (m, 5H), 5.75-5.58 (m, 1H), 4.53-4.59 (m, 1H), 4.35-4.21 (m, 1H), 3.15-3.10 (m, 1H), 2.82 (s, 3H), 1.55-1.47 (m, 6H).

### Example 75: Preparation of (R,Z)-6-((amino(methylamino)methylene)amino)-N-(1-(2-fluorophenyl)ethyl)-N-((5-(2-fluoropropan-2-yl)pyridin-2-yl)methyl)nicotinamide (75)

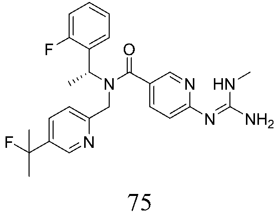

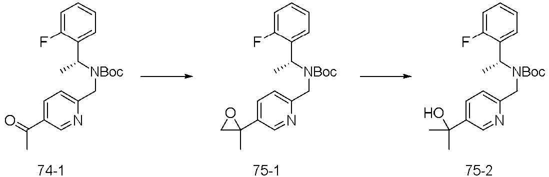

### Step 1: Preparation of tert-butyl ((R)-1-(2-fluorophenyl)ethyl)((5-(2-methyloxiran-2-yl)pyridin-2-yl)methyl)carbamate (75-1)

Trimethylsulfoxonium iodide (709 mg, 3.2 mmol) and potassium tert-butoxide (361 mg, 3.2 mmol) were dissolved in dry tetrahydrofuran (10 mL) at room temperature, and heated to 50°C and stirred for 0.5 hours. Compound 74-1 (300 mg, 0.8 mmol) was dissolved in tetrahydrofuran (2 mL) and then added to the reaction solution. The reaction solution was stirred at 50°C for 12 hours. After completion of the reaction, water (10 mL) was added to the reaction solution to quench the reaction, followed by extraction with ethyl acetate (10 mL × 3). The organic phases were combined, dried over anhydrous sodium sulfate, and filtered. The filtrate was concentrated under reduced pressure. The obtained residues were purified by silica gel column chromatography (eluent: methanol / dichloromethane = 1/10) to obtain compound 75-1 (250 mg, yield 80%).

LC-MS (ESI+): 387.4 m/z [M+H]⁺.

### Step 2: Preparation of tert-butyl (R)-(1-(2-fluorophenyl)ethyl)((5-(2-hydroxypropan-2-yl)pyridin-2-yl)methyl)carbamate (75-2)

Compound 75-1 (250 mg, 0.64 mmol) was dissolved in ethanol (2 mL) at room temperature, and sodium borohydride (49 mg, 1.3 mmol) was added thereto. The reaction solution was stirred at 25°C for 12 hours. Water (10 mL) was added to the reaction solution to quench the reaction, followed by extraction with ethyl acetate (10 mL × 3). The organic phases were combined, dried over anhydrous sodium sulfate, and filtered. The filtrate was concentrated under reduced pressure. The obtained residues were purified by silica gel column chromatography (eluent: methanol / dichloromethane = 1/10) to obtain compound 75-2 (150 mg, yield 60%). LC-MS (ESI+): 389.4 m/z [M+H]⁺.

The remaining steps were the same as Example 67, except that compound 75-2 was used instead of compound 67-4, to obtain compound 75.

LC-MS (ESI+): 467.4 m/z [M+H]⁺.

### Example 76: Preparation of (R,Z)-6-((amino(methylamino)methylene)amino)-N-((5-(1,1-difluoroethyl)pyridin-2-yl) methyl)-N-(1-(2-fluorophenyl)ethyl)nicotinamide (76)

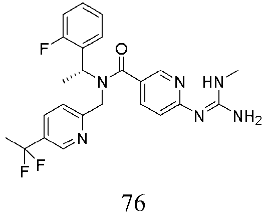

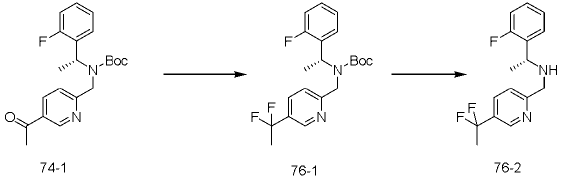

### Step 1: Preparation of tert-butyl (R)-((5-(1,1-difluoroethyl)pyridin-2-yl)methyl)(1-(2-fluorophenyl)ethyl)carbamate (76-1)

Compound 74-1 (200 mg, 0.54 mmol) and diethylaminosulfur trifluoride (173 mg, 1.07 mmol) were dissolved in 1,2-dichloroethane (3 mL), and heated to 50°C and reacted for 12 hours. The reaction mixture was poured into water and extracted with dichloromethane (10 mL × 3). The organic phases were combined, washed with brine (10 mL), dried over anhydrous sodium sulfate and filtered. The filtrate was concentrated under reduced pressure. The residues were purified by silica gel column chromatography (petroleum ether : ethyl acetate = 5:1) to obtain compound 76-1 (150 mg, yield 72%).

LC-MS (ESI+): 395.0 m/z [M+H]⁺.

### Step 2: Preparation of (R)-N-((5-(1,1-difluoroethyl)pyridin-2-yl)methyl)-1-(2-fluorophenyl)ethan-1-amine (76-2)

Compound 76-1 (140 mg, 0.35 mmol) was dissolved in trifluoroacetic acid (2 mL), reacted at room temperature for 1 hour and concentrated under reduced pressure to obtain compound 76-2 (117.0 mg, yield 99.67%).

LC-MS (ESI+): 295.0 m/z [M+H]⁺.

The remaining steps were the same as Example 67, except that compound 76-2 was used instead of compound 67-5, to obtain compound 76.

LC-MS (ESI+): 471.0 m/z [M+H]⁺.

### Example 77: Preparation of (R,Z)-6-((amino(methylamino)methylene)amino)-N-(1-(2-fluorophenyl)ethyl)-N-((5-(tr ifluoromethoxy)pyridin-2-yl)methyl)nicotinamide (77)

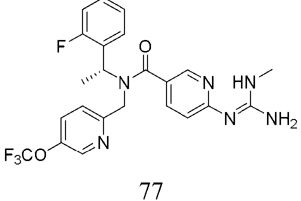

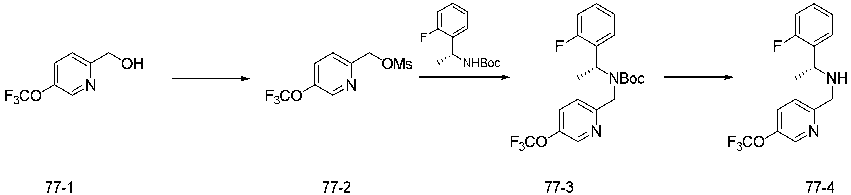

### Step 1: Preparation of (5-(trifluoromethoxy)pyridin-2-yl)methyl methanesulfonate (77-2)

(5-(Trifluoromethoxy)pyridin-2-yl)methanol (77-1, 300 mg, 1.5 mmol) and diisopropylethylamine (0.8 mL) were dissolved in dichloromethane (10 mL) at room temperature, and methanesulfonyl chloride (0.4 mL) was added thereto. The reaction solution was stirred at 25°C for 1 hour. Water (20 mL) was added to the reaction solution to quench the reaction, followed by extraction with dichloromethane (20 mL × 3). The organic phases were combined, dried over anhydrous sodium sulfate and filtered. The filtrate was concentrated under reduced pressure. The obtained residues were purified by silica gel column chromatography (eluent: ethyl acetate / petroleum ether = 1/5) to obtain compound 77-2 (300 mg, yield 71%).

LC-MS (ESI+): 272.1 m/z [M+H]⁺.

### Step 2: Preparation of tert-butyl (R)-(1-(2-fluorophenyl)ethyl)((5-(trifluoromethoxy)pyridin-2-yl)methyl)carbamate (77-3)

Sodium hydride (60%, 80 mg, 2 mmol) was added to a solution of *tert-*butyl (R)-(1-(2-fluorophenyl)ethyl)carbamate (250 mg, 1 mmol) in N,N-dimethylformamide (5 mL) under an ice-water bath, and the reaction solution was stirred at 0°C for 30 minutes. Compound 77-2 (283 mg, 1 mmol) was added thereto. The reaction solution was stirred at 25°C for 1 hour. Water (20 mL) was added to the reaction solution to quench the reaction, followed by extraction with ethyl acetate (20 mL × 3). The organic phases were combined, dried over anhydrous sodium sulfate, and filtered. The filtrate was concentrated under reduced pressure. The residues were purified by silica gel column chromatography (eluent: ethyl acetate / petroleum ether = 1/1) to obtain compound 77-3 (400 mg, yield 92%).

LC-MS (ESI+): 414.3 m/z [M+H]⁺.

### Step 3: Preparation of (R)-1-(2-fluorophenyl)-N-((5-(trifluoromethoxy)pyridin-2-yl)methyl)ethylamine (77-4)

Trifluoroacetic acid (0.2 mL) was added to a solution of compound 77-3 (100 mg, 0.24 mmol) in dichloromethane (1 mL) at room temperature. The reaction solution was stirred at 25°C for 1 hour until the starting materials disappeared. The reaction solution was concentrated under reduced pressure to obtain compound 77-4 (TFA salt, 70 mg, yield 92%).

LC-MS (ESI+): 314.2 m/z [M+H]⁺.

The remaining steps were the same as Example 4, except that compound 77-4 was used instead of compound 1-6, to obtain compound 77.

LC-MS (ESI+): 491.3 m/z [M+H]⁺.

### Example 78: Preparation of (R,Z)-6-((amino(methylamino)methylene)amino)-N-(1-(2-fluorophenyl)ethyl)-N-((2-m ethylbenzo[d]thiazol-6-yl)methyl)nicotinamide (78)

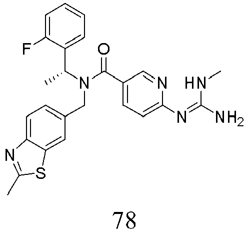

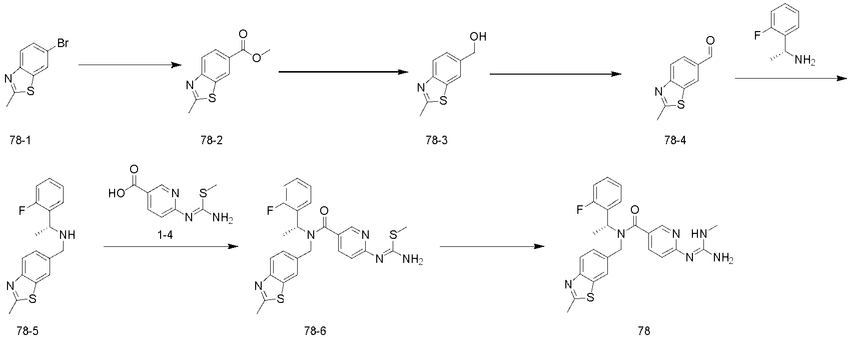

### Step 1: Preparation of methyl 2-methylbenzothiazole-6-carboxylate (78-2)

6-Bromo-2-methylbenzothiazole (78-1, 1.0 g, 4.38 mmol) was dissolved in 10 mL of ethanol at room temperature. Molybdenum hexacarbonyl (2.89 g, 11.0 mmol), triethylamine (2.66 g, 26.3 mmol), 4,5-bis(diphenylphosphino)-9,9-dimethylxanthene (1.27 g, 2.19 mmol) and [1,1'-bis(diphenylphosphino)ferrocene]palladium dichloride dichloromethane complex (801 mg, 1.10 mmol) were added thereto. The mixture was reacted at 100°C under a nitrogen atmosphere overnight. The reaction mixture was filtered through Celite, and the filtrate was concentrated under reduced pressure. The obtained residues were purified by silica gel column chromatography (dichloromethane / methanol = 0-5%) to obtain compound 78-2 (400 mg, yield 44%).

LC-MS (ESI+): 208.2 m/z [M+H]⁺.

### Step 2: Preparation of (2-methylbenzothiazol-6-yl)methanol (78-3)

Compound 78-2 (300 mg, 0.48 mmol) was dissolved in 6 mL of tetrahydrofuran at room temperature. Diisobutylaluminum hydride (172 mg, 1.21 mmol) was added dropwise in an ice bath, and the mixture was reacted under an ice bath under a nitrogen atmosphere for 2 hours. The reaction mixture was added to water to quench the reaction, and then extracted with ethyl acetate (30 mL × 2). The organic phases were combined, dried over anhydrous sodium sulfate, and filtered. The filtrate was concentrated under reduced pressure. The obtained residues were used without purification to obtain compound 78-3 (300 mg, yield 92%).

LC-MS (ESI+): 180.2 m/z [M+H]⁺.

### Step 3: Preparation of 2-methylbenzothiazole-6-carbaldehyde (78-4)

Compound 78-3 (300 mg, 1.67 mmol) was dissolved in 5 mL of dichloromethane at room temperature. Manganese dioxide (437 mg, 5.02 mmol) was added thereto, and the mixture was reacted at room temperature under a nitrogen atmosphere overnight. The reaction mixture was filtered through Celite, and the filtrate was concentrated under reduced pressure to obtain compound 78-4 (150 mg, yield 50%).

LC-MS (ESI+): 178.2 m/z [M+H]⁺.

### Step 4: Preparation of (R)-1-(2-fluorophenyl)-N-((2-methylbenzo[d]thiazol-6-yl)methyl)ethan-1-amine (78-5)

Compound 78-4 (100 mg, 0.56 mmol) was dissolved in 3 mL of dichloromethane at room temperature. (R)-1-(2-Fluorophenyl)ethan-1-amine (94 mg, 0.68 mmol), potassium acetate (55 mg, 0.56 mmol) and acetic acid (40 mg, 0.67 mmol) were added thereto. After reacting under an ice bath for half an hour, sodium triacetoxyborohydride (359 mg, 1.68 mg) was added, and the mixture was reacted at room temperature under a nitrogen atmosphere overnight. The reaction solution was quenched by adding water and extracted with dichloromethane (30 mL × 2). The organic phases were combined and concentrated under reduced pressure. The obtained residues were purified by silica gel column chromatography (dichloromethane / methanol = 0-5%) to obtain compound 78-5 (80 mg, yield 47%).

LC-MS (ESI+): 301.2 m/z [M+H]⁺.

### Step 5: Preparation of methyl (R,Z)-N'-(5-((1-(2-fluorophenyl)ethyl)((2-methylbenzo[d]thiazol-6-yl)methyl)carbamoy 1)pyridin-2-yl)carbamimidothioate (78-6)

Compound 1-4 (56 mg, 0.27 mmol), bis(2-oxo-3-oxazolidinyl)phosphinic chloride (101 mg, 0.40 mmol) and N,N-diisopropylethylamine (103 mg, 0.80 mmol) were added to compound 78-5 (80 mg, 0.27 mmol) at room temperature. The mixture was reacted at room temperature under a nitrogen atmosphere overnight. The reaction solution was quenched by adding water and extracted with ethyl acetate (30 mL × 2). The organic phases were combined, and the obtained residues were purified by silica gel column chromatography (dichloromethane / methanol = 0-5%) to obtain compound 78-6 (50 mg, yield 38%).

LC-MS (ESI+): 494.2 m/z [M+H]⁺.

### Step 6: Preparation of (R,Z)-6-((amino(methylamino)methylene)amino)-N-(1-(2-fluorophenyl)ethyl)-N-((2-m ethylbenzo[d]thiazol-6-yl)methyl)nicotinamide (78)

Compound 78-6 (50 mg, 0.10 mmol) was dissolved in 3 mL of dimethyl sulfoxide at room temperature. A solution of methylamine in ethanol (1 mL) and N,N-diisopropylethylamine (103 mg, 0.80 mmol) were added thereto. The mixture was reacted in a microwave at 100°C for 2 hours. The reaction solution was filtered, purified by Prep-HPLC (water / acetonitrile = 95%/5% - 10%/90%) and lyophilized to obtain the target compound 78 (12.3 mg, yield 25%).

LC-MS (ESI+): 477.2 m/z [M+H]⁺.

### Example 79: Preparation of (R,Z)-6-((amino(aminomethyl)methylene)amino)-N-(1-(2-fluorophenyl)ethyl)-N-((2-(1-methylpiperidin-4-yl)benzothiazol-5-yl)methyl)nicotinamide (79)

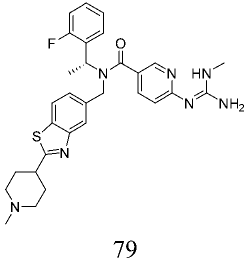

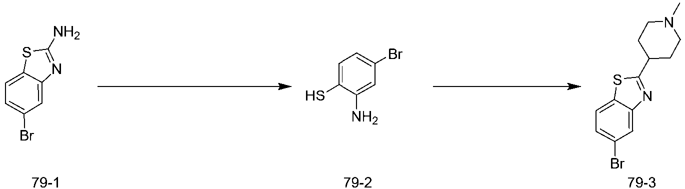

### Step 1: Preparation of 2-amino-4-bromobenzenethiol (79-2)

5-Bromobenzothiazol-2-amine (79-1, 10.0 g, 43.6 mmol) and aqueous sodium hydroxide solution (50%) (50 mL) were dissolved in ethylene glycol (50 mL) at room temperature. The mixture was reacted at room temperature for 2 hours and diluted by adding water (100 mL). The resulting mixture was extracted with ethyl acetate (100 mL × 3). The organic phases were combined, washed with saturated brine (200 mL), dried over anhydrous sodium sulfate and filtered. The filtrate was concentrated under reduced pressure. The obtained residues were purified by silica gel column chromatography (eluent: petroleum ether / ethyl acetate = 0/1) to obtain compound 79-2 (5.00 g, yield 56%).

LC-MS (ESI+): 203.8 m/z [M+H]⁺.

### Step 2: Preparation of 5-bromo-2-(1-methylpiperidin-4-yl)benzothiazole (79-3)

2-Amino-4-bromobenzenethiol (2.00 g, 869 mmol) and 1-methylpiperidine-4-carboxylic acid (2.11 g, 1.30 mol) were dissolved in phosphorus oxychloride (20 mL) at room temperature. The mixture was reacted at 90°C for 12 hours and diluted by adding water (100 mL). The resulting mixture was extracted with ethyl acetate (100 mL × 3). The organic phases were combined, washed with saturated brine (200 mL), dried over anhydrous sodium sulfate and filtered. The filtrate was concentrated under reduced pressure. The obtained residues were purified by silica gel column chromatography (eluent: petroleum ether / ethyl acetate = 0/1) to obtain compound 79-3 (2.00 g, yield 67%).

LC-MS (ESI+): 310.8 m/z [M+H]⁺.

Compound 79 was obtained according to the synthesis method of Example 78, using compound 79-3 instead of compound 78-1.

LC-MS (ESI+): 560.2 m/z [M+H]⁺.

¹H NMR (400 MHz, DMSO-*d*₆) δ 8.37 (s, 3H), 7.88 (d, *J =* 8.0 Hz, 2H), 7.67 (s, 1H), 7.55 (s, 1H), 7.30 (s, 1H), 7.19 (t, *J =* 8.0 Hz, 2H), 7.07 (s, 2H), 5.32 (s, 1H), 4.61 (d, *J* = 16.0 Hz, 1H), 4.39 (s, 1H), 3.05 (s, 1H), 2.87 (s, 5H), 2.21 (s, 3H), 2.10-2.05 (m, 5H), 1.81-1.78 (m, 2H), 1.64 (s, 2H).

### Example 80: Preparation of (R,Z)-6-((amino(methylamino)methylene)amino)-N-(1-(2-fluorophenyl)ethyl)-N-((2-(m ethylaminobenzothiazol-5-yl)methyl)nicotinamide (80)

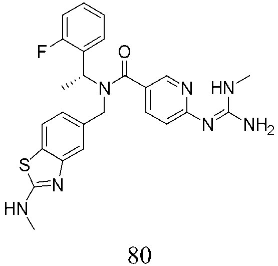

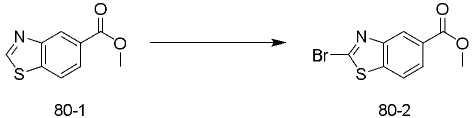

### Step 1: Preparation of methyl 2-bromobenzothiazole-5-carboxylate (80-2)

Methyl benzothiazole-5-carboxylate (80-1, 1.40 g, 7.25 mmol), carbon tetrabromide (2.64 g, 7.97 mmol) and sodium *tert*-butoxide (1.39 g, 14.49 mmol) were dissolved in tetrahydrofuran (20 mL) at room temperature. The mixture was reacted at room temperature for 2 hours and diluted by adding water (100 mL). The resulting mixture was extracted with ethyl acetate (100 mL × 3). The organic phases were combined, washed with saturated brine (200 mL), dried over anhydrous sodium sulfate and filtered. The filtrate was concentrated under reduced pressure. The obtained residues were purified by silica gel column chromatography (eluent: petroleum ether / ethyl acetate = 0/1) to obtain compound 80-2 (650 mg, yield 33%).

LC-MS (ESI+): 271.8 m/z [M+H]⁺.

Compound 80 was obtained according to the method of Example 78, using compound 80-2 instead of 78-2.

¹H NMR (400 MHz, DMSO-*d*₆) δ 8.38 (s, 2H), 7.91 (s, 1H), 7.55-7.48 (m, 3H), 7.34 - 7.31 (m, 1H), 7.20 (t, *J =* 8.0 Hz, 2H), 7.14-7.12 (m, 2H), 7.01 (s, 1H), 6.79 (s, 1H), 5.45 (s, 1H), 4.55 (d, *J =* 16.0 Hz, 1H), 4.21 (s, 1H), 2.91 (d, *J =* 4.0 Hz, 3H), 2.85 (s, 3H), 2.54 (s, 1H), 1.60 (s, 3H).

LC-MS (ESI+): 492.2 m/z [M+H]⁺.

### Example 81: Preparation of (R,Z)-6-((amino(methylamino)methylene)amino)-N-(benzothiazol-5-methylene)-N-(1-( 2-fluorophenyl)ethyl)nicotinamide (81)

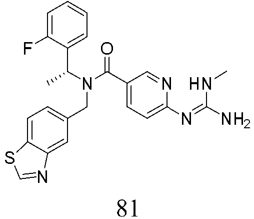

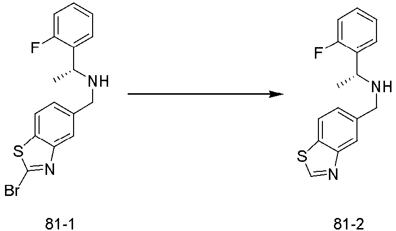

### Step 1: Preparation of (R)-N-(benzothiazol-5-methylene)-1-(2-fluorophenyl)ethan-1-amine (81-2)

(R)-N-((2-Bromobenzothiazol-5-yl)methyl)-1-(2-fluorophenyl)ethan-1-amine (obtained according to the method of Example 78 steps 2-4 using 80-2 instead of 78-2) (81-1, 100 mg, 0.27 mmol) and palladium on carbon (29 mg, 2.27 mmol) were added to methanol (5 mL) at room temperature. The mixture was reacted at room temperature under a hydrogen atmosphere for 2 hours. The reaction solution was filtered and concentrated under reduced pressure to obtain compound 81-2 (70 mg, yield 89%).

LC-MS (ESI+): 287.0 m/z [M+H]⁺.

Compound 81 was obtained according to the method of Example 7, using compound 81-2 instead of compound 1-6.

LC-MS (ESI+): 463.2 m/z [M+H]⁺.

¹H NMR (400 MHz, DMSO-*d*₆) δ 9.34 (s, 1H), 8.39 (s, 2H), 7.99 (d, *J =* 8.0 Hz, 1H), 7.89 (s, 1H), 7.77 (s, 1H), 7.55 (t, *J =* 8.0 Hz, 1H), 7.28 - 7.25 (m, 2H), 7.19-7.15 (m, 1H), 7.04 (s, 2H), 5.40 (s, 1H), 4.62 (d, *J =* 16.0 Hz, 1H), 4.46 (d, *J =* 16.0 Hz, 1H), 2.85 (s, 3H), 1.65 (d, *J =* 4.0 Hz, 3H).

### Example 82: Preparation of (R,Z)-6-((amino(methylamino)methylene)amino)-N-(1-(2,4-difluorophenyl)ethyl)-N-((5 -(trifluoromethyl)pyridin-2-yl)methyl)nicotinamide (82)

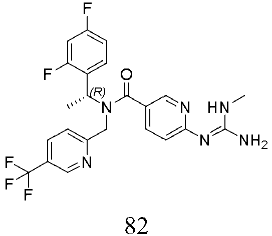

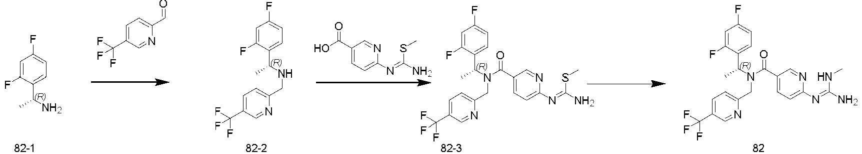

### Step 1: Preparation of (R)-1-(2,4-difluorophenyl)-N-((5-(trifluoromethyl)pyridin-2-yl)methyl)ethan-1-amine (82-2)

(R)-1-(2,4-Difluorophenyl)ethan-1-amine (82-1, 100 mg, 0.64 mmol) and 5-(trifluoromethyl)picolinaldehyde (134 mg, 0.76 mmol) were dissolved in a mixed solution of dichloromethane (2 mL) and acetic acid (1 mL) at room temperature. The reaction solution was stirred at 25°C for 1 hour. Sodium triacetoxyborohydride (60 mg, 0.95 mmol) was added to the reaction solution subsequently, and the reaction solution was stirred at 25°C for 1 hour. After completion of the reaction, water (20 mL) was added to the reaction solution to quench the reaction, followed by extraction with dichloromethane (10 mL × 3). The organic phases were combined, dried over anhydrous sodium sulfate, and filtered. The filtrate was concentrated under reduced pressure. The residues were purified by silica gel column chromatography (methanol / dichloromethane = 8%) to obtain compound 82-2 (120 mg, yield 59%).

LC-MS (ESI+): 317.2 m/z [M+H]⁺.

### Step 2: Preparation of methyl (R,Z)-N'-(5-((1-(2,4-difluorophenyl)ethyl)((5-(trifluoromethyl)pyridin-2-yl)methyl)carb amoyl)pyridin-2-yl)carbamimidothioate (82-3)

Compound 82-2 (120 mg, 0.38 mmol), compound 1-4 (160 mg, 0.76 mmol), N,N-diisopropylethylamine (147 mg, 1.14 mmol) and bis(2-oxo-3-oxazolidinyl)phosphinic chloride (145 mg, 0.57 mmol) were dissolved in N,N-dimethylacetamide (2 mL) at room temperature. The reaction solution was stirred at 25°C for 16 hours. After completion of the reaction, water (20 mL) was added to the reaction solution to quench the reaction, followed by extraction with dichloromethane (10 mL × 3). The organic phases were combined, dried over anhydrous sodium sulfate and filtered. The filtrate was concentrated under reduced pressure. The residues were purified by silica gel column chromatography (methanol / dichloromethane = 10%) to obtain compound 82-3 (50 mg, yield 26%).

LC-MS (ESI+): 510.2 m/z [M+H]⁺.

### Step 3: Preparation of (R,Z)-6-((amino(methylamino)methylene)amino)-N-(1-(2,4-difluorophenyl)ethyl)-N-((5 -(trifluoromethyl)pyridin-2-yl)methyl)nicotinamide (82)

Compound 82-3 (50 mg, 0.09 mmol), methylamine hydrochloride (13 mg, 0.20 mmol) and N,N-diisopropylethylamine (38 mg, 0.29 mmol) were dissolved in dimethyl sulfoxide (2 mL) at room temperature. The reaction solution was stirred at 90°C for 16 hours. After completion of the reaction, water (20 mL) was added to the reaction solution to quench the reaction, followed by extraction with dichloromethane (10 mL × 3). The organic phases were combined, dried over anhydrous sodium sulfate and filtered. The filtrate was concentrated under reduced pressure. The residues were purified by Prep-HPLC (water / acetonitrile = 95%/5% - 10%/90%) and lyophilized to obtain compound 82 (21.2 mg, yield 44%).

LC-MS (ESI+): 493.2 m/z [M+H]⁺.

¹H NMR (400 MHz, DMSO-*d*₆) δ 8.77 (s, 1H), 8.51 - 8.31 (m, 2H), 8.03 (d, *J =* 8.2 Hz, 1H), 7.54 (dd, *J =* 15.6, 8.4 Hz, 1H), 7.37 (s, 1H), 7.16 - 6.89 (m, 3H), 5.35 (s, 1H), 4.79 - 4.36 (m, 2H), 2.86 (s, 3H), 1.63 (s, 3H).

The following compounds were obtained according to the synthesis method of Example 82 using corresponding starting materials:

| Compound No. | Structure | MS data (ESI+): m/z | NMR data |
|---|---|---|---|
| 83 | 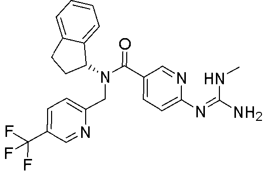 | 469.2 | |
| 84 | 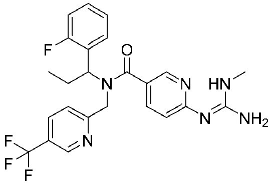 | 489.4 | |
| 85 | 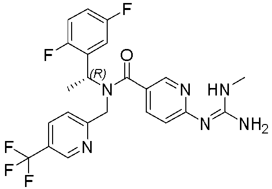 | 493.3 | |
| 86 | 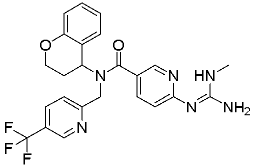 | 485.2 | ¹H NMR (400 MHz, DMSO-*d*₆) δ 8.88 (s, 1H), 8.44 (s, 1H), 8.37 (s, 1H), 8.12 (s, 1H), 7.89 (s, 1H), 7.65 (s, 1H), 7.19 - 7.15 (m, 3H), 6.93 (s, 2H), 6.78 (d, *J* = 8.0 Hz, 1H), 5.35 (s, 1H), 4.87-4.83 (m, 1H), 4.24 - 4.21 (m, 1H), 4.11 (s, 1H), 4.02 (s, 1H), 2.82 (s, 3H), 2.25 (s, 1H), 2.07 (s, 1H). |
| 87 | 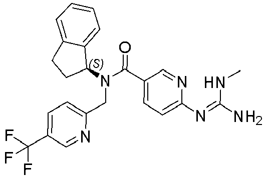 | 469.2 | |
| 88 | 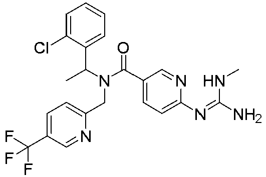 | 491.2 | ¹H NMR (400 MHz, DMSO-*d*₆) δ 8.80 (s, 1H), 8.38 (d, *J =* 11.6 Hz, 2H), 8.02 (d, *J* = 8.2 Hz, 1H), 7.62 (d, *J* = 7.6 Hz, 1H), 7.36 (s, 1H), 7.35 - 7.25 (m, 4H), 6.83 (s, 2H), 4.67 (d, *J =* 17.3 Hz, 1H), 4.53 (d, *J =* 17.6 Hz, 2H), 2.82 (s, 3H), 2.69 (s, 1H), 1.25 (s, 3H). |
| 89 | 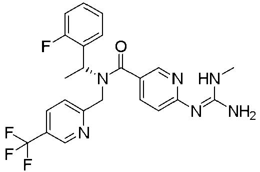 | 475.5 | ¹H NMR (400 MHz, DMSO-*d*₆) δ 8.78 (d, *J* = 2.3 Hz, 1H), 8.40-8.33 (m, 2H), 8.06 - 8.00 (m, 1H), 7.88 (s, 1H), 7.52-7.48 (m, 1H), 7.38 (s, 1H), 7.32-7.26 (m, 1H), 7.16-7.12 (m, 1H), 7.09 - 7.04 (m, 1H), 7.01 (s, 2H), 5.41 (s, 1H), 4.66 - 4.48 (m, 2H), 2.85 (s, 4H), 1.63 (s, 3H). |
| 90 | 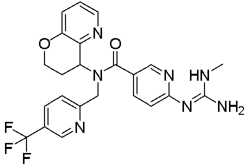 | 486.1 | ¹H NMR (400 MHz, DMSO-*d*6) δ 8.86 (s, 1H), 8.53 (s, 1H), 8.39 (s, 1H), 8.22 (s, 1H), 8.19-8.13 (m, 1H), 7.96 (s, 1H), 7.75 (d, *J* = 8.4 Hz, 1H), 7.23 (d, *J =* 8.4 Hz, 2H), 6.99 (s, 1H), 5.46-5.42 (m, 1H), 4.83 (d, *J* = 16.8 Hz, 1H), 4.35 - 4.16 (m, 2H), 3.96 (d, *J* = 16.4 Hz, 1H), 2.85 (s, 3H), 2.30 (d, *J* = 35.2 Hz, 2H). |
| 91 | 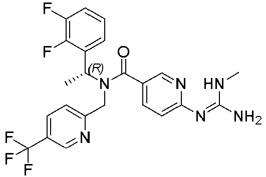 | 493.1 | ¹H NMR (400 MHz, DMSO-*d*6) δ 8.79 (s, 1H), 8.40 (s, 2H), 8.05 (d, *J =* 8.4 Hz, 1H), 7.91 (s, 1H), 7.36-7.28 (m, 3H), 7.18 - 6.94 (m, 2H), 5.50 - 5.25 (m, 1H), 4.62 (s, 2H), 2.87 (s, 3H), 1.65 (s, 3H). |

### Example 92: Preparation of (Z)-6-((amino(methylamino)methylene)amino)-N-(6,7-dihydro-5H-cyclopenta[b]pyridi n-7-yl)-N-((5-(trifluoromethyl)pyridin-2-yl)methyl)nicotinamide (92)

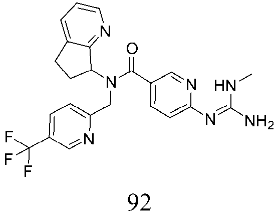

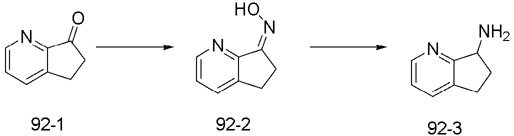

### Step 1: Preparation of (Z)-5H-cyclopenta[b]pyridin-7(6H)-one oxime (92-2)

5H-Cyclopenta[b]pyridin-7(6H)-one (92-1, 800 mg, 6 mmol) and hydroxylamine hydrochloride (835 mg, 12 mmol) were dissolved in methanol (20 mL) at room temperature, and diisopropylethylamine (4 mL) was added. The reaction solution was heated to 60°C and stirred for 12 hours. After completion of the reaction, water (20 mL) was added to the reaction solution to quench the reaction, followed by extraction with ethyl acetate (20 mL × 3). The organic phases were combined, dried over anhydrous sodium sulfate, and filtered. The filtrate was concentrated under reduced pressure to obtain compound 92-2 (700 mg, yield 78%).

LC-MS (ESI+): 149.2 m/z [M+H]⁺.

### Step 2: Preparation of 6,7-dihydro-5H-cyclopenta[b]pyridin-7-amine (92-3)

Compound 92-2 (100 mg, 0.67 mmol) was dissolved in acetic acid (2 mL) at room temperature, and zinc powder (87 mg, 1.3 mmol) was added. The reaction solution was heated to 70°C and stirred for 2 hours. After completion of the reaction, the reaction solution was directly filtered while hot, and the filtrate was concentrated under reduced pressure to obtain compound 92-3 (90 mg, yield 99%).

LC-MS (ESI+): 135.2 m/z [M+H]⁺.

Compound 92 was obtained according to the method of Example 82, using compound 92-3 instead of compound 82-1.

LC-MS (ESI+): 470.4 m/z [M+H]⁺.

The following compound was obtained according to the synthesis method of Example 92 using corresponding starting materials:

| Compound No. | Structure | MS data (ESI+): m/z | NMR data |
|---|---|---|---|
| 93 | 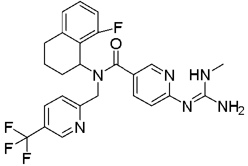 | 501.2 | |

### Example 94: Preparation of (Z)-6-((amino(methylamino)methylene)amino)-N-(cyclopropyl(2-fluorophenyl)methyl) -N-((5-(trifluoromethyl)pyridin-2-yl)methyl)nicotinamide (94)

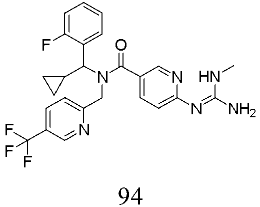

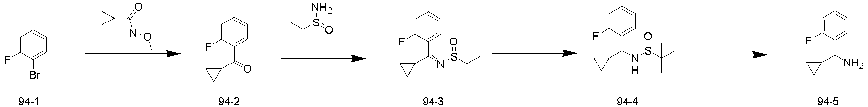

### Step 1: Preparation of cyclopropyl(2-fluorophenyl)methanone (94-2)

1-Bromo-2-fluorobenzene (94-1, 3000 mg, 17 mmol) was dissolved in tetrahydrofuran (30 mL) in a dry 100 mL three-neck flask under a nitrogen atmosphere. After the reaction solution was cooled to -78°C, n-butyllithium solution (7.2 mL, 18 mmol) was added dropwise. The mixture was stirred at -78°C for 30 minutes, and then N-methoxy-N-methylcyclopropanecarboxamide (2325 mg, 18.0 mmol) was added to the reaction solution. The reaction solution was warmed to room temperature and stirred at this temperature for another 2 hours. After completion of the reaction, water (50 mL) and saturated NH₄Cl solution (50 mL) were added to quench the reaction, followed by extraction with ethyl acetate (150 mL). The combined organic phases were dried over MgSO₄ and concentrated under reduced pressure to obtain compound 94-2 (1600 mg, yield 57%).

LC-MS (ESI+): 165.2 m/z [M+H]⁺.

### Step 2: Preparation of (Z)-N-(cyclopropyl(2-fluorophenyl)methylene)-2-methylpropane-2-sulfinamide (94-3)

Compound 94-2 (1000 mg, 6.09 mmol) and 2-methylpropane-2-sulfinamide (960 mg, 7.90 mmol) were dissolved in toluene (10 mL) at room temperature, and then titanium tetraisopropoxide (5.2 ml) was added to the reaction solution. The reaction solution was stirred at 80°C for 16 hours. After completion of the reaction, the reaction mixture was cooled to room temperature and diluted with acetonitrile (100 mL). Water (50 mL) was added dropwise to the mixture at this temperature, and the mixture was stirred for 30 minutes. The solids were filtered off, and the filtrate was concentrated under reduced pressure to obtain compound 94-3 (1000 mg, yield 61%).

LC-MS (ESI+): 268.2 m/z [M+H]⁺.

### Step 3: Preparation of N-(cyclopropyl(2-fluorophenyl)methyl)-2-methylpropane-2-sulfinamide (94-4)

Compound 94-3 (1000 mg, 3.76 mmol) was dissolved in a mixed solution of methanol (10 mL) and acetic acid (1 mL) at room temperature. Sodium cyanoborohydride (471 mg, 7.50 mmol) was added to the reaction solution subsequently, and the reaction solution was stirred at 50°C for 16 hours. After completion of the reaction, methanol was removed under reduced pressure, and water (50 mL) was added to the reaction solution to quench the reaction, followed by extraction with ethyl acetate (40 mL × 3). The organic phases were combined, dried over anhydrous sodium sulfate, and filtered. The filtrate was concentrated under reduced pressure. The residues were purified by silica gel column chromatography (petroleum ether / ethyl acetate = 60%) to obtain compound 94-4 (600 mg, yield 59%).

LC-MS (ESI+): 270.2 m/z [M+H]⁺.

### Step 4: Preparation of cyclopropyl(2-fluorophenyl)methanamine (94-5)

Compound 94-4 (600 mg, 2.23 mmol) was dissolved in methanol (10 mL) at room temperature, and then a solution of hydrochloric acid in dioxane (2 mL) was added to the reaction solution at 0°C. The reaction solution was stirred at 25°C for 16 hours. After completion of the reaction, the reaction solution was concentrated under reduced pressure to obtain compound 94-5 (300 mg, yield 81%).

LC-MS (ESI+): 166.2 m/z [M+H]⁺.

Compound 94 was obtained according to the method of Example 82, using compound 94-5 instead of compound 82-1.

LC-MS (ESI+): 501.2 m/z [M+H]⁺.

### Example 95: Preparation of (Z)-6-((amino(methylamino)methylene)amino)-N-(6-bromo-2,3-dihydrobenzofuran-3-y 1)-N-((5-(trifluoromethyl)pyridin-2-yl)methyl)nicotinamide (95)

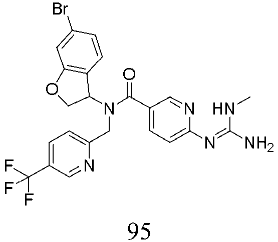

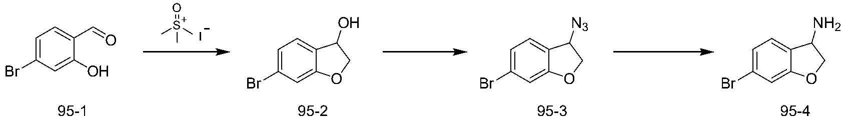

### Step 1: Preparation of 6-bromo-2,3-dihydrobenzofuran-3-ol (95-2)

4-Bromo-2-hydroxybenzaldehyde (95-1, 9.0 g, 44.8 mmol) and trimethylsulfoxonium iodide (14.8 g, 67.2 mmol) were dissolved in DMSO (90 mL). Potassium *tert*-butoxide (7.5 g, 67.2 mmol) was added at 0°C, and stirring was continued at 25°C for 12 hours. After completion of the reaction, water (150 mL) was added to the reaction mixture, followed by extraction with ethyl acetate (150 mL × 2). The organic phases were combined, washed with saturated brine (3 × 80 mL), dried over anhydrous sodium sulfate, and filtered. The filtrate was concentrated under reduced pressure. The residues were purified by silica gel column chromatography (eluent: ethyl acetate / petroleum ether = 3/10) to obtain compound 95-2 as a white solid (5.0 g, yield 52%).

LCMS(ESI+): 215.1 m/z [M+H]⁺.

¹H NMR (400 MHz, DMSO-*d*₆) δ 7.35-7.33 (m, 1H), 7.11-7.09 (m, 2H), 5.69 (d, *J* = 5.6 Hz, 1H), 5.28-5.24 (m, 1H), 4.59-4.57 (m, 1H), 4.31-4.27 (m, 1H).

### Step 2: Preparation of 3-azido-6-bromo-2,3-dihydrobenzofuran (95-3)

Compound 95-2 (3.0 g, 13.9 mmol) and diphenylphosphoryl azide (4.6 g, 16.7 mmol) were dissolved in toluene (50 mL). DBU (2.5 g, 16.7 mmol) was added at 0°C, and stirring was continued at 25°C for 12 hours. After completion of the reaction, water (60 mL) was added to the reaction mixture, followed by extraction with ethyl acetate (80 mL × 2). The organic phases were combined, washed with saturated brine (2 × 60 mL), dried over anhydrous sodium sulfate, and filtered. The filtrate was concentrated under reduced pressure. The residues were purified by silica gel column chromatography (eluent: ethyl acetate / petroleum ether = 1/10) to obtain compound 95-3 as a yellow solid (1.6 g, yield 48%).

¹H NMR (400 MHz, DMSO-*d*₆) δ 7.48 (d, *J =* 8.0 Hz, 1H), 7.25 - 7.16 (m, 2H), 5.37-5.35 (m, 1H), 4.66-4.62 (m, 1H), 4.55-4.51 (m, 1H).

### Step 3: Preparation of 6-bromo-2,3-dihydrobenzofuran-3-amine (95-4)

Compound 95-3 (1.6 g, 6.7 mmol) and triphenylphosphine (2.6 g, 10.0 mmol) were dissolved in THF (40 mL) and stirred at 25°C for 1 hour. Potassium hydroxide (1.1 g, 20.0 mmol) and water (15 mL) were added and stirred for 12 hours. After completion of the reaction, water (60 mL) was added to the reaction mixture, followed by extraction with ethyl acetate (80 mL × 2). The organic phases were combined, washed with saturated brine (60 mL), dried over anhydrous sodium sulfate, and filtered. The filtrate was concentrated under reduced pressure. The residues were purified by silica gel column chromatography (eluent: methanol / dichloromethane = 1/20) to obtain compound 95-4 as a yellow solid (1.2 g, yield 84%).

LCMS(ESI+): 214.1 m/z [M+H]⁺.

Compound 95 was obtained according to the method of Example 56, using compound 95-4 instead of compound 56-2.

LCMS(ESI⁺): 549.1 m/z [M+H]⁺.

¹HNMR (400 MHz, DMSO-*d*₆) δ 8.99 (d, *J* = 2.4 Hz, 1H), 8.90 (d, *J =* 2.4 Hz, 1H), 8.47 (s, 1H), 8.37-8.31 (m, 1H), 8.29-8.24 (m, 1H), 7.82 (d, *J =* 8.4 Hz, 1H), 7.53 (d, *J =* 8.8 Hz, 1H), 7.40 (d, *J =* 2.0 Hz, 1H), 7.28 (d, *J =* 8.0 Hz, 1H), 7.24-7.20 (m, 1H), 5.68-5.60 (m, 1H), 5.41 (d, *J =* 3.2 Hz, 2H), 4.96-4.90 (m, 1H), 4.28-4.24 (m, 1H), 3.43 (s, 3H).

### Example 96: Preparation of (R,Z)-6-((amino(methylamino)methylene)amino)-N-(1,2,3,4-tetrahydronaphthalen-1-yl) -N-((5-(trifluoromethyl)pyridin-2-yl)methyl)nicotinamide (96)

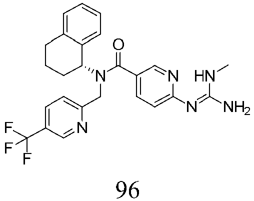

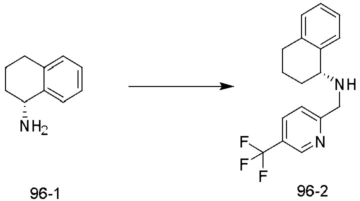

### Step 1: Preparation of (R)-N-((5-(trifluoromethyl)pyridin-2-yl)methyl)-1,2,3,4-tetrahydronaphthalen-1-amine (96-2)

(R)-1,2,3,4-Tetrahydronaphthalen-1-amine (96-1, 600 mg, 4.08 mmol) was dissolved in methanol/acetic acid (2.5 mL/0.5 mL). 5-(Trifluoromethyl)picolinaldehyde (714 mg, 4.08 mmol) was added thereto at room temperature. After stirring for 10 minutes, 2-picoline borane (873 mg, 8.16 mmol) was added thereto, and the resulting mixture was stirred at room temperature for 16 hours. After completion of the reaction, the reaction solution was poured into water (10 mL) and extracted with ethyl acetate (15 mL × 3). The organic phases were combined, washed with saturated brine, dried over anhydrous sodium sulfate, and filtered. The filtrate was concentrated under reduced pressure. The residues were purified by silica gel column chromatography (petroleum ether / ethyl acetate = 10/1~1/5) to obtain compound 96-2 (600 mg, yield 48%, yellow solid).

LC-MS (ESI+): 307.2 m/z [M+H]⁺.

Compound 96 was obtained according to the synthesis method of Example 7, using compound 96-2 instead of compound 1-6.

LC-MS (ESI+): 483.2 m/z [M+H]⁺.

### Example 97: Preparation of (R,Z)-6-((amino(methylamino)methylene)amino)-N-(1-(2-fluorophenyl)ethyl)-N-((2-m ethyl-2H-indazol-5-yl)methyl)nicotinamide (97)

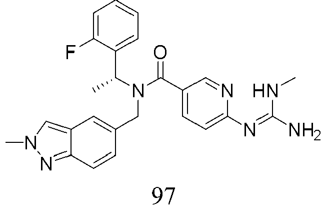

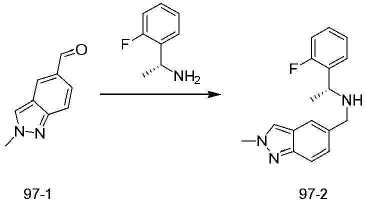

### Step 1: Preparation of (R)-1-(2-fluorophenyl)-N-((2-methyl-2H-indazol-5-yl)methyl)ethan-1-amine (97-2)

2-Methyl-2H-indazole-5-carbaldehyde (97-1, 100 mg, 0.624 mmol) was dissolved in DCM (5 mL), and (R)-1-(2-fluorophenyl)ethan-1-amine (87 mg, 0.624 mmol) and acetic acid (0.1 mL) were added. The resulting mixture was stirred at room temperature for half an hour. Sodium triacetoxyborohydride (226 mg, 0.93 mmol) was added, and the mixture was stirred at room temperature for 16 hours. Aqueous sodium bicarbonate solution was added to the reaction solution to adjust the pH to >7, followed by extraction with DCM (100 mL × 3). The organic phases were combined, washed with saturated brine, dried over anhydrous sodium sulfate, and filtered. The filtrate was concentrated under reduced pressure to obtain compound 97-2 (160 mg, yield 90%, yellow oil).

LC-MS (ESI+): 284.2 m/z [M+H]⁺.

Compound 97 was obtained according to the synthesis method of Example 7, using compound 97-2 instead of compound 1-6.

LC-MS (ESI+): 460.2 m/z [M+H]⁺.

The following compounds were obtained according to the synthesis method of Example 97 using corresponding starting materials:

| Compound No. | Structure | MS data (ESI+): m/z | NMR data |
|---|---|---|---|
| 98 | 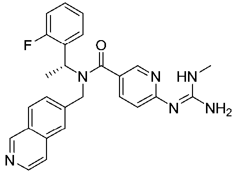 | 457.2 | |
| 99 | 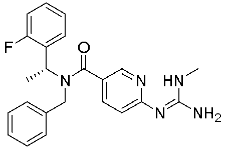 | 406.2 | |
| 100 | 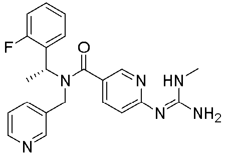 | 407.2 | ¹H NMR (400 MHz, DMSO-*d*₆) δ 8.46 - 8.21 (m, 4H), 7.90 - 7.80 (m, 1H), 7.58 - 7.40 (m, 2H), 7.35 - 7.24 (m, 1H), 7.23 - 7.12 (m, 2H), 7.09 - 6.90 (m, 2H), 5.40 (s, 1H), 4.40 (s, 2H), 2.84 (s, 3H), 1.66 (d, *J=* 6.8 Hz, 3H). |
| 101 | 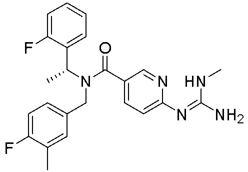 | 438.2 | ¹H NMR (400 MHz, DMSO-*d*₆) δ 8.45 - 8.18 (m, 2H), 7.85 - 7.70 (m, 1H), 7.56 - 7.43 (m, 1H), 7.36 - 7.24 (m, 1H), 7.23 - 7.12 (m, 1H), 7.11 - 7.01 (m, 1H), 7.00 - 6.76 (m, 4H), 5.42 (s, 1H), 4.46 - 4.11 (m, 2H), 2.84 (s, 3H), 2.16 - 2.07 (m, 3H), 1.60 (d, *J* = 6.8 Hz, 3H). |
| 102 | 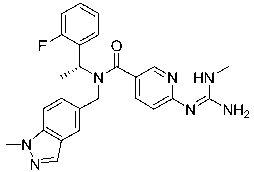 | 460.2 | |
| 103 | 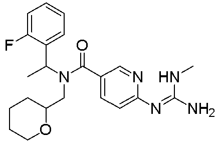 | 414.0 | |
| 104 | 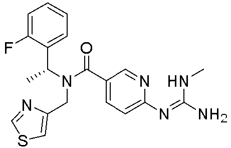 | 413.0 | |
| 105 | 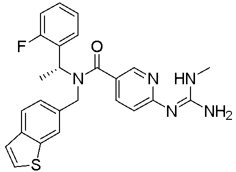 | 462.2 | ¹H NMR (400 MHz, DMSO-*d*₆) δ 8.42 (s, 2H), 7.94 (s, 1H), 7.73-7.67 (m, 3H), 7.54 (t, *J* = 8.0 Hz, 1H), 7.37 (d, *J =* 4.0 Hz, 1H), 7.32-7.27 (m, 1H), 7.18 (t, *J* = 8.0 Hz, 2H), 7.07 (d, *J =* 8.0 Hz, 2H), 5.38 (s, 1H), 4.60 (d, *J =* 16.0 Hz, 1H), 4.38 (d, *J =* 16.0 Hz, 1H), 2.86 (s, 3H), 1.63 (s, 3H). |
| 106 | 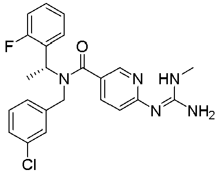 | 440.4 | ¹H NMR (400 MHz, DMSO-*d*₆) δ 8.39 (s, 2H), 7.88 (s, 1H), 7.54-7.50 (m, 1H), 7.32-7.27 (m, 1H), 7.24-7.15 (m, 3H), 7.11-7.01 (m, 4H), 4.39 (s, 2H), 3.10-3.08 (m, 1H), 2.86 (s, 3H), 1.64 (d, *J =* 6.4 Hz, 3H). |
| 107 | 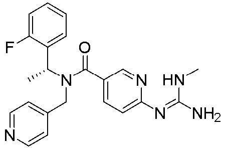 | 407.3 | |
| 108 | 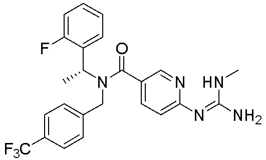 | 474.1 | ¹H NMR (400 MHz, DMSO-*d*₆) δ 8.38 (s, 2H), 7.90 (s, 1H), 7.58 - 7.48 (m, 3H), 7.37 - 7.26 (m, 3H), 7.19-7.13 (m, 1H), 7.07-7.01 (m, 2H), 5.38 (s, 1H), 4.49-4.43 (m, 2H), 2.86 (s, 3H), 1.64 (d, *J =* 7.2 Hz, 3H). |
| 109 | 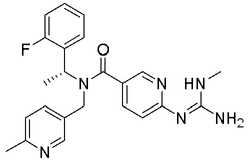 | 421.1 | ¹H NMR (400 MHz, DMSO-*d*₆) δ 8.31 (s, 2H), 8.05 (s, 1H), 7.80 (s, 1H), 7.49-7.46 (m, 1H), 7.33 - 7.21 (m, 2H), 7.16-7.08 (m, 1H), 7.00-6.94 (m, 3H), 5.29 (s, 1H), 4.32-4.24 (m, 2H), 2.79 (s, 3H), 2.30 (s, 3H), 1.57 (d, *J*= 7.2 Hz, 3H). |
| 110 | 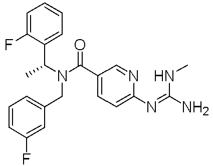 | 424.1 | ¹H NMR (400 MHz, DMSO-*d*₆) δ 8.40 (s, 2H), 7.93 (s, 1H), 7.57-7.50 (m, 1H), 7.36 - 7.16 (m, 3H), 7.13-7.01 (m, 2H), 7.0-6.94 (m, 2H), 6.88 (s, 1H), 5.50 - 5.25 (m, 1H), 4.46-4.38 (m, 2H), 2.89 (s, 3H), 1.65 (d, *J* = 7.2 Hz, 3H). |
| 111 | 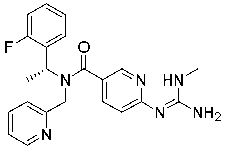 | 407.1 | ¹H NMR (400 MHz, DMSO-*d*₆) δ 8.47 - 8.36 (m, 3H), 7.89 (s, 1H), 7.68-7.64 (m, 1H), 7.55 - 7.48 (m, 1H), 7.37 - 7.29 (m, 1H), 7.22 - 7.09 (m, 4H), 7.03 (s, 1H), *5.50* - 5.25 (m, 1H), 4.60 (d, *J* = 16.8 Hz, 1H), 4.35 (d, *J =* 17.2 Hz, 1H), 2.87 (s, 3H), 1.59 (s, 3H). |
| 112 | 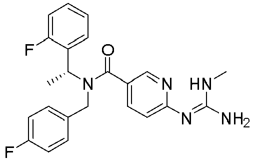 | 424.4 | |
| 113 | 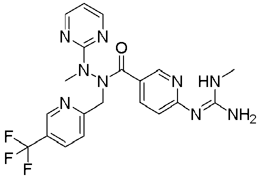 | 460.2 | ¹H NMR (400 MHz, DMSO-*d*6) δ 8.86 (s, 1H), 8.42 (d, *J* = 4.0 Hz, 1H), 8.34 (s, 1H), 8.29 (s, 1H), 8.19 (d, *J* = 8.0 Hz, 1H), 7.81 (d, *J =* 8.0 Hz, 2H), 6.85 - 6.82 (m, 2H), 5.32 (d, *J =* 16.0 Hz, 1H), 4.59 (d, *J =* 16.0 Hz, 1H), 3.15 (m, 6H), 2.80 (s, 1H). |

### Example 114: Preparation of (S,Z)-6-((amino(methylamino)methylene)amino)-N-(1,2,3,4-tetrahydronaphthalen-1-yl) -N-((5-(trifluoromethyl)pyridin-2-yl)methyl)nicotinamide (114)

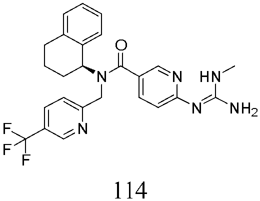

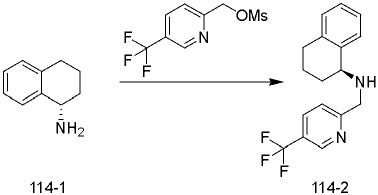

### Step 1: Preparation of (S)-N-((5-(trifluoromethyl)pyridin-2-yl)methyl)-1,2,3,4-tetrahydronaphthalen-1-amine (114-2)

(S)-1,2,3,4-Tetrahydronaphthalen-1-amine (114-1, 600 mg, 4.08 mmol) was dissolved in DMSO (2 mL). (5-(Trifluoromethyl)pyridin-2-yl)methyl methanesulfonate (1.56 g, 6.12 mmol) and potassium carbonate (1.68 g, 12.2 mmol) were added thereto at room temperature, and the resulting mixture was stirred at 55°C for 10 hours. After completion of the reaction, the reaction solution was poured into water (10 mL) and extracted with ethyl acetate (15 mL × 3). The organic phases were combined, washed with saturated brine, dried over anhydrous sodium sulfate, and filtered. The filtrate was concentrated under reduced pressure. The residues were purified by silica gel column chromatography (petroleum ether / ethyl acetate = 10/1 ~ 1/5) to obtain compound 114-2 (400 mg, yield 32%, yellow solid).

LC-MS (ESI+): 307.2 m/z [M+H]⁺.

Compound 114 was obtained according to the synthesis method of Example 31, except that compound 114-2 was used instead of compound 31-4.

LC-MS (ESI+): 483.2 m/z [M+H]⁺.

The following compounds were obtained according to the synthesis method of Example 114 using corresponding starting materials:

| Compound No. | Structure | MS data (ESI+): m/z | NMR data |
|---|---|---|---|
| 115 | 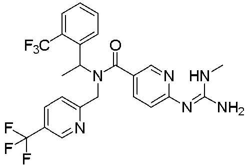 | 525.2 | |
| 116 | 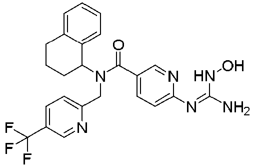 | 485.2 | |
| 117 | 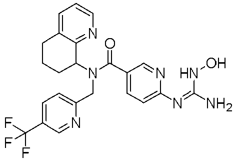 | 486.2 | |

### Example 118: Preparation of (Z)-6-((amino(methylamino)methylene)amino)-N-(1-(4-fluorophenyl)ethyl)-N-((5-(trifl uoromethyl)pyridin-2-yl)methyl)nicotinamide (118)

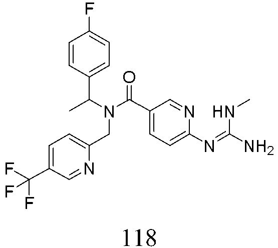

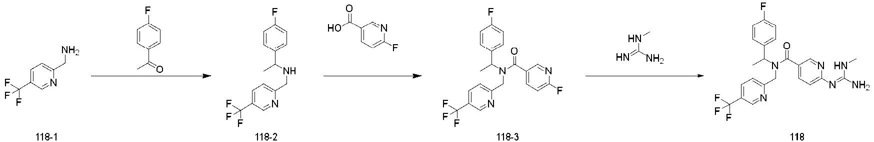

### Step 1 : Preparation of 1-(4-fluorophenyl)-N-((5-(trifluoromethyl)pyridin-2-yl)methyl)ethanamine (118-2)

(5-(Trifluoromethyl)pyridin-2-yl)methanamine (118-1, 500 mg, 3.62 mmol), 1-(4-fluorophenyl)ethanone (765 mg, 4.34 mmol), potassium acetate (532 mg, 5.43 mmol) and acetic acid (1 mL) were dissolved in dichloromethane (10 mL) at room temperature and stirred for 0.5 hours. Sodium triacetoxyborohydride (1.53 g, 7.24 mmol) was added in portions at 0°C, and the mixture was reacted at room temperature for 4 hours and diluted with water (100 mL). The resulting mixture was extracted with ethyl acetate (100 mL × 3). The organic phases were combined, washed with saturated brine (200 mL), dried over anhydrous sodium sulfate and filtered. The filtrate was concentrated under reduced pressure. The obtained residues were purified by silica gel column chromatography (petroleum ether / ethyl acetate = 0/1) to obtain compound 118-2 (270 mg, yield 25%).

LC-MS (ESI+): 298.8 m/z [M+H]⁺.

### Step 2: Preparation of 6-fluoro-N-(1-(4-fluorophenyl)ethyl)-N-((5-(trifluoromethyl)pyridin-2-yl)methyl)nicoti namide (118-3)

Compound 118-2 (211 mg, 0.71 mmol), 6-fluoronicotinic acid (100 mg, 0.71 mmol), triethylamine (144 mg, 1.42 mmol) and tri-pyrrolidinylphosphonium hexafluorophosphate (495 mg, 1.06 mmol) were dissolved in N,N-dimethylacetamide (3 mL) at room temperature. The mixture was reacted at room temperature for 12 hours and diluted with water (100 mL). The resulting mixture was extracted with ethyl acetate (100 mL × 3). The organic phases were combined, washed with saturated brine (100 mL), dried over anhydrous sodium sulfate and filtered. The filitrate was concentrated under reduced pressure. The obtained residues were purified by silica gel column chromatography (petroleum ether / ethyl acetate = 0/1) to obtain compound 118-3 (250 mg, yield 71%).

LC-MS (ESI+): 422.0 m/z [M+H]⁺.

### Step 3: Preparation of (Z)-6-((amino(methylamino)methylene)amino)-N-(1-(4-fluorophenyl)ethyl)-N-((5-(trifl uoromethyl)pyridin-2-yl)methyl)nicotinamide (118)

Compound 118-3 (250 mg, 0.59 mmol), 1-methylguanidine (86 mg, 1.19 mmol) and cesium carbonate (771 mg, 2.37 mmol) were dissolved in N,N-dimethylacetamide (6 mL) at room temperature. The mixture was reacted at 100°C for 12 hours and diluted with water (100 mL). The resulting mixture was extracted with ethyl acetate (100 mL × 3). The organic phases were combined, washed with saturated brine (100 mL), dried over anhydrous sodium sulfate and filtered. The filtrate was concentrated under reduced pressure. The obtained residues were purified by silica gel column chromatography (dichloromethane / methanol = 10/1) to obtain compound 118 (8.7 mg, yield 3%).

LC-MS (ESI+): 475.2 m/z [M+H]⁺.

¹H NMR (400 MHz, DMSO-*d*₆) δ 11.05 (s, 1H), 9.36 (s, 1H), 8.81 (s, 1H), 8.53 (s, 3H), 8.05 (s, 2H), 7.50-7.11 (m, 4H), 5.14 (s, 1H), 4.69-4.41 (m, 2H), 2.92 (s, 3H), 1.56 (s, 3H).

The following compound was obtained according to the synthesis method of Example 118 using corresponding starting materials:

| Compound No. | Structure | MS data (ESI+): m/z | NMR data |
|---|---|---|---|
| 119 | 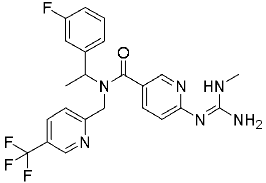 | 475.2 | ¹H NMR (400 MHz, DMSO-*d*₆) δ 11.17 (s, 1H), 9.42 (s, 1H), 8.83 (s, 1H), 8.57 (s, 2H), 8.11 (s, 2H), 7.53 - 7.08 (m, 5H), 5.80 - 4.44 (m, 3H), 2.92 (s, 3H), 1.58 (s, 3H). |

### Example 120: Preparation of (Z)-6-((amino(methylamino)methylene)amino)-N-(1-(quinoxalin-5-yl)ethyl)-N-((5-(trifl uoromethyl)pyridin-2-yl)methyl)nicotinamide (120)

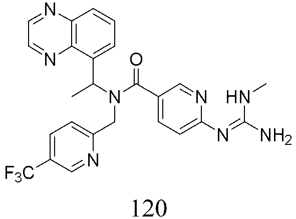

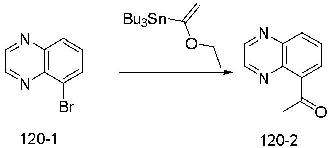

### Step 1: Preparation of 1-(quinoxalin-5-yl)ethan-1-one (120-2)

A solution of 5-bromoquinoxaline (120-1, 1.50 g, 7.18 mmol), tributyl(1-ethoxyvinyl)tin (5.18 g, 14.3 mmol), bis(triphenylphosphine)palladium dichloride (504 mg, 0.72 mmol) and triethylamine (2.99 mL, 21.5 mmol) in dioxane (6 mL)/DMF (2 mL) was heated to 120°C and reacted for 3 hours under a nitrogen atmosphere. The mixture was cooled to room temperature, 1 N aqueous hydrochloric acid solution (3 mL) was added, and stirring was continued for 30 minutes. The reaction mixture was poured into ethyl acetate for extraction (50 mL). The organic phase was washed with water 5 times (50 mL). The organic phases were combined, washed with brine (30 mL), dried over anhydrous sodium sulfate and filtered. The filtrate was concentrated under reduced pressure. The resulting residues were purified by silica gel column chromatography (petroleum ether : ethyl acetate = 2:1) to obtain compound 120-2 (1.00 g, yield 83%).

LC-MS (ESI+): 173.0 m/z [M+H]⁺.

Compound 120 was obtained according to the synthesis method of Example 118, using compound 120-2 instead of 1-(4-fluorophenyl)ethanone.

LC-MS (ESI+): 509.0 m/z [M+H]⁺.

The following compound was obtained according to the synthesis method of Example 120 using corresponding starting materials:

| Compound No. | Structure | MS data (ESI+): m/z | NMR data |
|---|---|---|---|
| 121 | 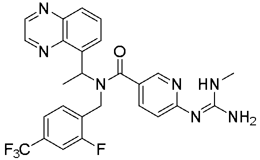 | 526.0 | |

### Example 122: Preparation of (Z)-6-((amino(methylamino)methylene)amino)-N-(5,6,7,8-tetrahydroquinoxalin-5-yl)-N -((5-(trifluoromethyl)pyridin-2-yl)methyl)nicotinamide (122)

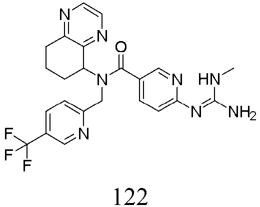

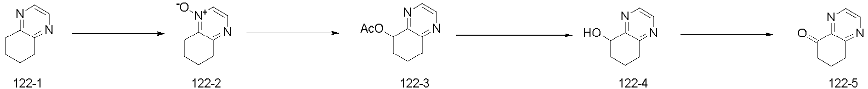

### Step 1: Preparation of 5,6,7,8-tetrahydroquinoxaline 1-oxide (122-2)

5,6,7,8-Tetrahydroquinoxaline (122-1, 3.00 g, 22.4 mmol) and m-CPBA (4.87 g, 21.2 mmol) were dissolved in DCM (15 mL) and reacted at room temperature for 16 hours. After completion of the reaction, the reaction solution was poured into saturated sodium sulfite solution (20 mL) and extracted with ethyl acetate (3 × 30 mL). The organic phases were combined, washed with sodium sulfite (3 × 30 mL) and then with saturated sodium carbonate (3 × 30 mL). The organic phase was dried over anhydrous sodium sulfate and filtered, and the filtrate was concentrated under reduced pressure to obtain a crude product, which was used directly in the next step.

LC-MS (ESI+): 151.2 m/z [M+H]⁺.

### Step 2: Preparation of 5,6,7,8-tetrahydroquinoxalin-5-yl acetate (122-3)

Compound 122-2 (240 mg, 1.60 mmol) was dissolved in acetic acid (2 mL) and acetic anhydride (2 mL), and reacted at 120°C for 16 hours. After completion of the reaction, the reaction solution was poured into saturated sodium carbonate solution (20 mL) and extracted with ethyl acetate (3 × 20 mL). The organic phases were combined, washed with water (3 × 20 mL) and brine (30 mL) successively. The organic phase was dried over anhydrous sodium sulfate and filtered, and the filtrate was concentrated under reduced pressure to obtain a crude product, which was used directly in the next step.

LC-MS (ESI+): 193.2 m/z [M+H]⁺.

### Step 3: Preparation of 5,6,7,8-tetrahydroquinoxalin-5-ol (122-4)

Compound 122-3 (306 mg, 1.59 mmol) was dissolved in MeOH (2 mL), and 4 M aqueous sodium hydroxide solution (1 mL) was added to the reaction solution. The mixture was reacted at room temperature for 1 hour. After completion of the reaction, the reaction mixture was directly concentrated under reduced pressure. The residues were purified by silica gel column chromatography (DCM/MeOH = 10/1) to obtain compound 122-4 (134 mg, yield 56%).

LC-MS (ESI+): 151.2 m/z [M+H]⁺.

### Step 4: Preparation of 7,8-dihydroquinoxalin-5(6H)-one (122-5)

Compound 122-4 (134 mg, 0.89 mmol) was dissolved in DCM (2 mL). DMP (756 mg, 1.78 mmol) was added at 0°C, and the mixture was reacted at room temperature for 1 hour. After completion of the reaction, the reaction mixture was poured into saturated sodium bicarbonate solution (20 mL) and extracted with ethyl acetate (3 × 20 mL). The organic phases were combined, dried over anhydrous sodium sulfate and filtered. The filtrate was concentrated under reduced pressure. The residues were purified by silica gel column chromatography (DCM/MeOH = 10/1) to obtain compound 122-5 (80 mg, yield 60%).

LC-MS (ESI+): 149.2 m/z [M+H]⁺.

Compound 122 was obtained according to the synthesis method of Example 120, using compound 122-5 instead of compound 120-2.

LC-MS (ESI+): 485.2 m/z [M+H]⁺.

### Example 123: Preparation of (Z)-6-((amino(methylamino)methylene)amino)-N-(1-(pyridin-2-yl)ethyl)-N-((5-(trifluor omethyl)pyridin-2-yl)methyl)pyridine-3-sulfonamide (123)

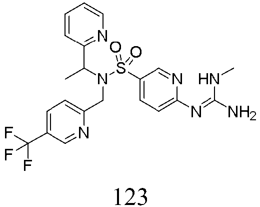

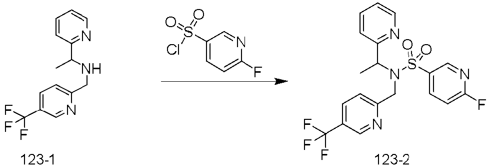

### Step 1 : Preparation of 6-fluoro-N-(1-(pyridin-2-yl)ethyl)-N-((5-(trifluoromethyl)pyridin-2-yl)methyl)pyridine-3-sulfonamide (123-2)

6-Fluoropyridine-3-sulfonyl chloride (200 mg, 0.94 mmol), 1-(pyridin-2-yl)-N-((5-(trifluoromethyl)pyridin-2-yl)methyl)ethan-1-amine (123-1, prepared according to the method of Example 118) (265 mg, 0.94 mmol) and triethylamine (390 µL, 2.83 mmol) were dissolved in dichloromethane (3 mL) at room temperature. The reaction was conducted at room temperature for 12 hours. The reaction mixture was poured into water and extracted with dichloromethane (50 mL × 3). The organic phases were combined, washed with brine (50 mL), dried over anhydrous sodium sulfate and filtered. The filtrate was concentrated under reduced pressure. The residues were purified by silica gel chromatography (petroleum ether / ethyl acetate = 2/1) to obtain compound 123-2 (100 mg, yield 23%).

LC-MS (ESI+): 441.0 m/z [M+H]⁺.

Compound 123 was obtained according to the synthesis method of Example 118, using compound 123-2 instead of compound 118-3.

LC-MS (ESI+): 494.0 m/z [M+H]⁺.

### Example 124: Preparation of (Z)-6-((amino(methylamino)methylene)amino)-N-(1,5-naphthyridin-4-yl)-N-((5-(trifluo romethyl)pyridin-2-yl)methyl)nicotinamide (124)

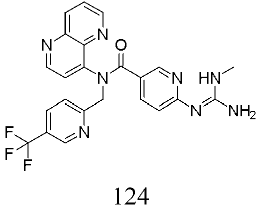

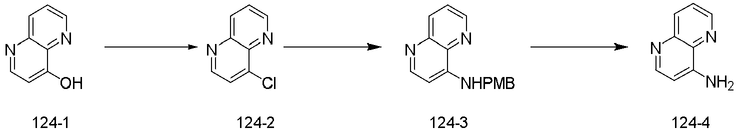

### Step 1: Preparation of 4-chloro-1,5-naphthyridine (124-2)

1,5-Naphthyridin-4-ol (124-1, 1.0 g, 6.84 mmol) was dissolved in phosphorus oxychloride (10 mL), and the resulting mixture was stirred at 110°C for half an hour. Ice water (10 mL) was added to the reaction solution and stirred for 10 minutes. Aqueous sodium bicarbonate solution was added to adjust the pH to >7, followed by extraction with DCM (100 mL × 3). The organic phases were combined, washed with saturated brine, dried over anhydrous sodium sulfate and filtered. The filtrate was concentrated under reduced pressure. The residues were purified by silica gel column chromatography (dichloromethane / methanol = 98/2) to obtain compound 124-2 (0.7 g, yield 62%, yellow oil).

LC-MS (ESI+): 165.2 m/z [M+H]⁺.

### Step 2: Preparation of N-(4-methoxybenzyl)-1,5-naphthyridin-4-amine (124-3)

Compound 124-2 (0.7 g, 4.25 mmol) was dissolved in n-butanol (10 mL), and 4-methoxybenzylamine (1.75 g, 12.7 mmol) was added. The resulting mixture was stirred at 110°C for 18 hours. Water (100 mL) was added to the reaction solution, followed by extraction with ethyl acetate (100 mL × 3). The organic phases were combined, washed with saturated brine, dried over anhydrous sodium sulfate, and filtered. The filtrate was concentrated under reduced pressure. The residues were purified by silica gel column chromatography (petroleum ether / ethyl acetate = 8/2) to obtain compound 124-3 (800 mg, yield 71%, yellow oil).

LC-MS (ESI+): 266.1 m/z [M+H]⁺.

### Step 3: Preparation of 1,5-naphthyridin-4-amine (124-4)

Compound 124-3 (0.8 g, 3.01 mmol) was dissolved in hydrobromic acid solution (10 mL), and the resulting mixture was stirred at 80°C for 2 hours. Water (10 mL) was added to the reaction solution and stirred for 10 minutes. Aqueous sodium bicarbonate solution was added to adjust the pH to >7, followed by extraction with DCM (100 mL × 3). The organic phases were combined, washed with saturated brine, dried over anhydrous sodium sulfate and filtered. The filtrate was concentrated under reduced pressure. The residues were purified by silica gel column chromatography (dichloromethane / methanol / ammonia = 97/2/1) to obtain compound 124-4 (0.4 g, yield 90%, yellow oil).

LC-MS (ESI+): 146.2 m/z [M+H]⁺.

Compound 124 was obtained according to the synthesis method of Example 45, using compound 124-4 instead of compound 45-1.

LC-MS (ESI+): 481.2 m/z [M+H]⁺.

### Example 125: Preparation of (Z)-6-((amino(methylamino)methylene)amino)-N-(6-(1-methyl-1H-pyrazol-4-yl)-2,3-di hydrobenzofuran-3-yl)-N-((5-(trifluoromethyl)pyridin-2-yl)methyl)nicotinamide (125)

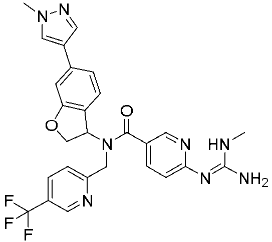

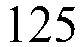

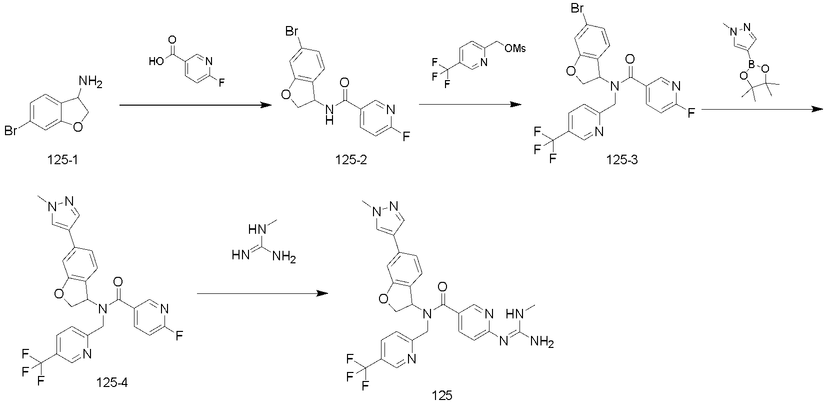

### Step 1: Preparation of N-(6-bromo-2,3-dihydrobenzofuran-3-yl)-6-fluoronicotinamide (125-2)

6-Bromo-2,3-dihydrobenzofuran-3-amine (125-1, 300 mg, 1.40 mmol), 6-fluoronicotinic acid (300 mg, 2.10 mmol), BOPCl (535 mg, 2.10 mmol) and DIEA (362 mg, 2.80 mmol) were dissolved in DMA (8 mL) and stirred at 25°C for 12 hours. After completion of the reaction, water (30 mL) was added to the reaction mixture, followed by extraction with ethyl acetate (40 mL × 2). The organic phases were combined, washed with saturated brine (2 × 30 mL), dried over anhydrous sodium sulfate and filtered. The filtrate was concentrated under reduced pressure. The residues were purified by silica gel column chromatography (eluent: ethyl acetate / petroleum ether = 5/10) to obtain compound 125-2 as a yellow solid (350 mg, yield 74%).

LCMS(ESI+): 337.1 m/z [M+H]⁺.

### Step 2: Preparation of N-(6-bromo-2,3-dihydrobenzofuran-3-yl)-6-fluoro-N-((5-(trifluoromethyl)pyridin-2-yl) methyl)nicotinamide (125-3)

Compound 125-2 (250 mg, 0.74 mmol), (5-(trifluoromethyl)pyridin-2-yl)methyl methanesulfonate (284 mg, 1.11 mmol) and potassium carbonate (205 mg, 1.48 mmol) were added to DMSO (5 mL) and stirred at 50°C for 4 hours. After completion of the reaction, water (25 mL) was added to the reaction mixture, followed by extraction with ethyl acetate (30 mL × 2). The organic phases were combined, washed with saturated brine (2 × 20 mL), dried over anhydrous sodium sulfate and filtered. The filtrate was concentrated under reduced pressure. The residues were purified by silica gel column chromatography (eluent: ethyl acetate / petroleum ether = 3/10) to obtain compound 125-3 as a yellow solid (150 mg, yield 41%).

LCMS(ESI+): 496.1 m/z [M+H]⁺.

### Step 3: Preparation of 6-fluoro-N-(6-(1-methyl-1H-pyrazol-4-yl)-2,3-dihydrobenzofuran-3-yl)-N-((5-(trifluoro methyl)pyridin-2-yl)methyl)nicotinamide (125-4)

Compound 125-3 (100 mg, 0.20 mmol), 1-methyl-4-(4,4,5,5-tetramethyl-1,3,2-dioxaborolan-2-yl)-1H-pyrazole (50 mg, 0.24 mmol), Pd(dppf)Cl₂ (15 mg, 0.02 mmol) and cesium carbonate (200 mg, 0.60 mmol) were dissolved in 1,4-dioxane (3 mL) and water (0.5 mL), and stirred at 90°C for 12 hours under a nitrogen atmosphere. After completion of the reaction, water (25 mL) was added to the reaction mixture, followed by extraction with ethyl acetate (30 mL × 2). The organic phases were combined, washed with saturated brine (2 × 20 mL), dried over anhydrous sodium sulfate and filtered. The filtrate was concentrated under reduced pressure. The crude product was purified by silica gel column chromatography (eluent: ethyl acetate / petroleum ether = 8/10) to obtain compound 125-4 as a yellow solid (70 mg, yield 70%).

LCMS(ESI+): 498.1 m/z [M+H]⁺.

### Step 4: Preparation of (Z)-6-((amino(methylamino)methylene)amino)-N-(6-(1-methyl-1H-pyrazol-4-yl)-2,3-di hydrobenzofuran-3-yl)-N-((5-(trifluoromethyl)pyridin-2-yl)methyl)nicotinamide (125)

Compound 125-4 (70 mg, 0.14 mmol), 1-methylguanidine (50 mg, 0.70 mmol) and potassium phosphate (97 mg, 0.70 mmol) were dissolved in DMSO (2 mL) and stirred at 90°C for 2 hours. After completion of the reaction, water (20 mL) was added to the reaction mixture, followed by extraction with ethyl acetate (2 × 20 mL). The organic phases were combined, washed with saturated brine (20 mL), dried over anhydrous sodium sulfate and filtered. The filtrate was concentrated under reduced pressure. The residues were purified by reverse-phase HPLC (water / acetonitrile = 95%/5% - 10%/90%) to obtain 125 as a white solid (3.0 mg, yield 3.9%).

LCMS(ESI+): 551.1 m/z [M+H]⁺.

The following compound was obtained according to the synthesis method of Example 125 using corresponding starting materials:

| Compound No. | Structure | MS data (ESI+): m/z | NMR data |
|---|---|---|---|
| 126 | 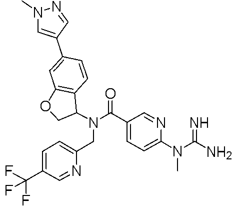 | 551.1 | |

### Example 127: Preparation of (R,Z)-N-([3,4'-bipyridin]-6-ylmethyl)-6-((amino(methylamino)methylene)amino)-N-(1-(2-fluorophenyl)ethyl)nicotinamide (127)

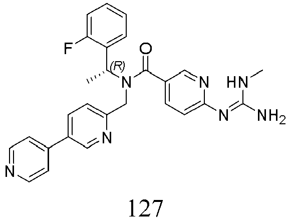

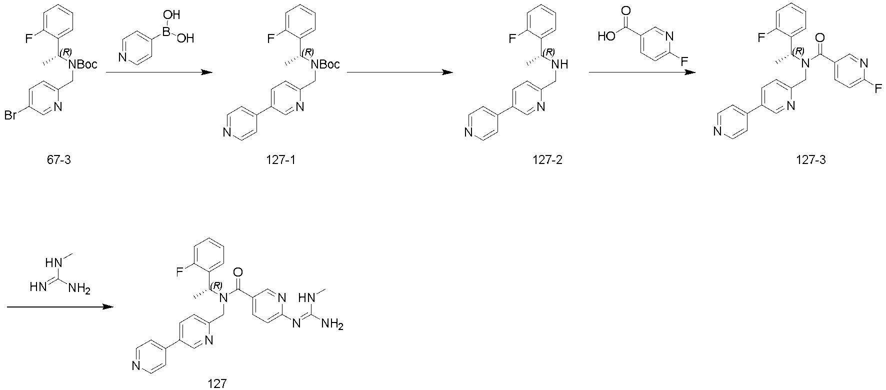

### Step 1: Preparation of tert-butyl (R)-([3,4'-bipyridin]-6-ylmethyl)(1-(2-fluorophenyl)ethyl)carbamate (127-1)

Compound 67-3 (120 mg, 0.29 mmol), pyridin-4-ylboronic acid (72 mg, 0.59 mmol), potassium carbonate (121 mg, 0.88 mmol) and Pd(dppf)Cl₂ (21 mg, 0.03 mmol) were dissolved in 1,4-dioxane (2 mL) and water (0.2 mL), and stirred at 90°C for 6 hours. After completion of the reaction, water (15 mL) was added to the reaction mixture, followed by extraction with ethyl acetate (20 mL × 2). The organic phases were combined, washed with saturated brine (20 mL), dried over anhydrous sodium sulfate and filtered. The filtrate was concentrated under reduced pressure. The residues were purified by silica gel column chromatography (eluent: ethyl acetate / petroleum ether = 7/10) to obtain compound 127-1 as a white solid (110 mg, yield 92%).

LCMS(ESI+): 408.1 m/z [M+H]⁺.

### Step 2: Preparation of (R)-N-([3,4'-bipyridin]-6-ylmethyl)-1-(2-fluorophenyl)ethan-1-amine (127-2)

Compound 127-1 (110 mg, 0.27 mmol) was dissolved in trifluoroacetic acid (1 mL) and dichloromethane (1 mL), and stirred at 25°C for 2 hours. After completion of the reaction, the reaction solution was directly concentrated under reduced pressure to obtain compound 127-2 as a yellow liquid (80 mg, yield 96%).

LCMS(ESI+): 308.1 m/z [M+H]⁺.

### Step 3: Preparation of (R)-N-([3,4'-bipyridin]-6-ylmethyl)-6-fluoro-N-(1-(2-fluorophenyl)ethyl)nicotinamide (127-3)

Compound 127-2 (100 mg, 0.33 mmol), 6-fluoronicotinic acid (69 mg, 0.49 mmol), BOPCl (125 mg, 0.49 mmol) and DIEA (84 mg, 0.66 mmol) were dissolved in DMA (2 mL), and stirred at 25°C for 2 hours. After completion of the reaction, water (20 mL) was added to the reaction mixture, followed by extraction with ethyl acetate (30 mL × 2). The organic phases were combined, washed with saturated brine (2 × 20 mL), dried over anhydrous sodium sulfate, and filtered. The filtrate was concentrated under reduced pressure. The residues were purified by silica gel column chromatography (eluent: methanol / dichloromethane = 1/20) to obtain compound 127-3 as a yellow liquid (80 mg, yield 57%).

LCMS(ESI+): 431.1 m/z [M+H]⁺.

### Step 4: Preparation of ((R,Z)-N-([3,4'-bipyridin]-6-ylmethyl)-6-((amino(methylamino)methylene)amino)-N-(1 -(2-fluorophenyl)ethyl)nicotinamide (127)

Compound 127-3 (70 mg, 0.16 mmol), 1-methylguanidine (59 mg, 0.81 mmol) and potassium carbonate (112 mg, 0.81 mmol) were dissolved in DMSO (1 mL), and stirred at 85°C for 2 hours. After completion of the reaction, water (20 mL) was added to the reaction mixture, followed by extraction with ethyl acetate (2 × 20 mL). The organic phases were combined, washed with saturated brine (20 mL), dried over anhydrous sodium sulfate, and filtered. The filtrate was concentrated under reduced pressure. The residues were purified by reverse-phase HPLC (water / acetonitrile = 95%/5% - 10%/90%) to obtain compound 127 as a white solid (3.6 mg, yield 4.6%).

LCMS(ESI+): 484.1 m/z [M+H]⁺.

¹H NMR (400 MHz, DMSO-*d*₆) δ 8.91 (d, *J =* 27.8 Hz, 1H), 8.70 - 8.67 (m, 3H), 8.47 (s, 2H), 8.12 (s, 1H), 7.78 - 7.75 (m, 2H), 7.56 - 7.52 (m, 1H), 7.42 - 7.31 (m, 3H), 7.23 - 7.10 (m, 3H), 5.35 (d, *J* = 8.0 Hz, 1H), 4.64 (d, *J =* 15.6 Hz, 1H), 4.43 (d, *J* = 16.4 Hz, 1H), 3.35 - 3.25 (m, 3H), 1.25 (s, 3H).

The following compounds were obtained according to the synthesis method of Example 127 using corresponding starting materials:

| Compound No. | Structure | MS data (ESI+): m/z | NMR data |
|---|---|---|---|
| 128 | 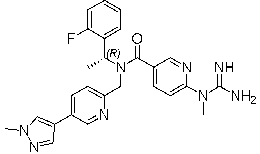 | 487.1 | ¹HNMR (400 MHz, DMSO-*d*6) δ 8.65 (s, 2H), 8.44 (s, 1H), 8.19 (s, 1H), 8.15 -7.95 (m, 1H), 7.91 (s, 1H), 7.79 (s, 1H), 7.54-7.46 (m, 1H), 7.35-7.27 (m, 1H), 7.24 - 6.98 (m, 3H), 5.32 (s, 1H),4.58 (d, *J=* 16.8 Hz, 1H), 4.29 (d, *J=* 17.2 Hz, 1H), 3.87 (s, 3H), 3.50 - 3.40 (m, 3H), 1.63 (s, 3H). |
| 129 | 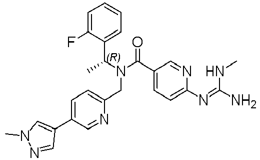 | 487.1 | 1H NMR (400 MHz, DMSO-*d*6) δ 8.67 (s, 1H), 8.41 (s, 1H), 8.34 (s, 1H), 8.19 (s, 1H), 7.91 (s, 2H), 7.82-7.76 (m, 1H), 7.54-7.48 (m, 1H), 7.36-7.28 (m, 1H), 7.23 - 6.96 (m, 4H), 5.50 - 5.10 (m, 1H),4.58 (d, *J* = 16.8 Hz, 1H), 4.32 (s, 1H), 3.87 (s, 3H), 2.87 (s, 3H), 1.58 (s, 3H). |

### Example 130: Preparation of (R,Z)-6-((amino(methylamino)methylene)amino)-N-(1-(2-fluorophenyl)ethyl)-N-((6'-(4 -methylpiperazin-1-yl)-[3,3'-bipyridin]-6-yl)methyl)nicotinamide (130)

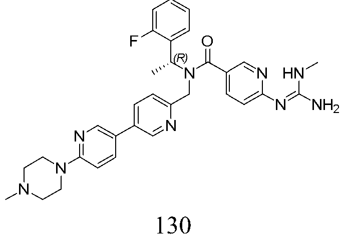

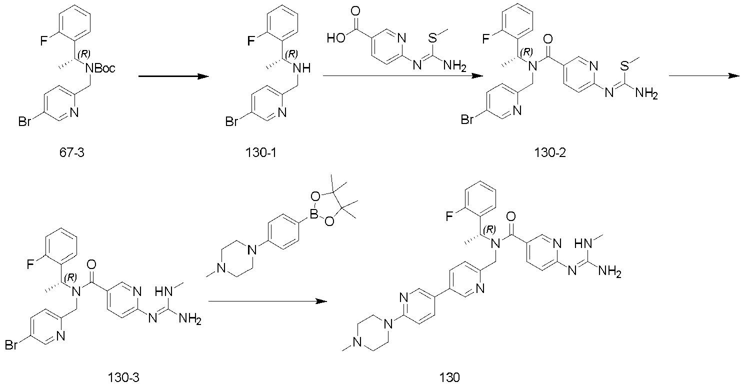

### Step 1: Preparation of (R)-N-((5-bromopyridin-2-yl)methyl)-1-(2-fluorophenyl)ethan-1-amine (130-1)

Compound 67-3 (120 mg, 0.29 mmol) was dissolved in trifluoroacetic acid (1 mL) and dichloromethane (1 mL), and stirred at 25°C for 2 hours. After completion of the reaction, the reaction solution was directly concentrated under reduced pressure to obtain compound 130-1 as a yellow liquid (80 mg, yield 88%).

LCMS(ESI+): 309.1 m/z [M+H]⁺.

### Step 2: Preparation of (R)-N-([3,4'-bipyridine]-6-ylmethyl)-6-fluoro-N-(1-(2-fluorophenyl)ethyl)nicotinamide (130-2)

Compound 130-1 (80 mg, 0.26 mmol), (Z)-6-((amino(methylthio)methylene)amino)nicotinic acid (82 mg, 0.39 mmol), BOPCl (100 mg, 0.39 mmol) and DIEA (67 mg, 0.52 mmol) were dissolved in DMA (4 mL), and stirred at 25°C for 2 hours. After completion of the reaction, water (20 mL) was added to the reaction mixture, followed by extraction with ethyl acetate (30 mL × 2). The organic phases were combined, washed with saturated brine (2 × 20 mL), dried over anhydrous sodium sulfate, and filtered. The filtrate was concentrated under reduced pressure. The residues were purified by silica gel column chromatography (eluent: methanol / dichloromethane = 1/20) to obtain compound 130-2 as a yellow liquid (80 mg, yield 62%).

LCMS(ESI+): 502.1 m/z [M+H]⁺.

### Step 3: Preparation of (R,Z)-6-((amino(methylamino)methylene)amino)-N-((5-bromopyridin-2-yl)methyl)-N-( 1-(2-fluorophenyl)ethyl)nicotinamide (130-3)

Compound 130-2 (80 mg, 0.16 mmol), 1-methylguanidine (58 mg, 0.80 mmol) and potassium phosphate (169 mg, 0.80 mmol) were dissolved in DMSO (2 mL), and stirred at 85°C for 2 hours. After completion of the reaction, water (20 mL) was added to the reaction mixture, followed by extraction with ethyl acetate (2 × 20 mL). The organic phases were combined, washed with saturated brine (20 mL), dried over anhydrous sodium sulfate, and filtered. The filtrate was concentrated under reduced pressure to obtain a crude yellow solid 130-3 (40 mg, yield 52%).

LCMS(ESI+): 485.1 m/z [M+H]⁺.

### Step 4: Preparation of (R,Z)-6-((amino(methylamino)methylene)amino)-N-(1-(2-fluorophenyl)ethyl)-N-((6'-(4 -methylpiperazin-1-yl)-[3,3'-bipyridin]-6-yl)methyl)nicotinamide (130)

Compound 130-3 (40 mg, 0.08 mmol), 1-methyl-4-(4-(4,4,5,5-tetramethyl-1,3,2-dioxaborolan-2-yl)phenyl)piperazine (37 mg, 0.12 mmol), Pd(dppf)Cl₂ (12 mg, 0.02 mmol) and potassium carbonate (23 mg, 0.16 mmol) were dissolved in 1,4-dioxane (2 mL) and water (0.2 mL), and stirred at 90°C for 12 hours under a nitrogen atmosphere. After completion of the reaction, water (15 mL) was added to the reaction mixture, followed by extraction with ethyl acetate (20 mL × 2). The organic phases were combined, washed with saturated brine (20 mL), dried over anhydrous sodium sulfate, and filtered. The filtrate was concentrated under reduced pressure. The residues were purified by reverse-phase HPLC (water / acetonitrile = 95%/5% - 10%/90%) to obtain compound 130 as a white solid (3.3 mg, yield 6.9%).

LCMS(ESI+): 582.1 m/z [M+H]⁺.

¹H NMR (400 MHz, DMSO-*d*₆) δ 8.70 (s, 1H), 8.48 - 8.30 (m, 3H), 8.08 - 7.81 (m, 3H), 7.54-7.48 (m, 1H), 7.36-7.29 (m, 1H), 7.26 - 7.04 (m, 3H), 6.93 (d, *J =* 8.8 Hz, 1H), 5.37 (s, 1H), 4.61 (d, *J =* 16.8 Hz, 1H), 4.34 (s, 1H), 3.57-3.51 (m, 4H), 2.86 (s, 3H), 2.43-2.37 (m, 4H), 2.22 (s, 3H), 1.62 (s, 3H).

### Biological Testing

Test Example 1: Test method of the enzymatic inhibition activity of the compounds against PRMT5-MTA.

The enzymatic inhibition activity of the compounds against PRMT5-MTA was detected using the AlphaScreen method. A 1× methyltransferase assay buffer IV (Signalchem) was prepared as a dilution solution for each component of the enzymatic reaction. PRMT5/MET50 protein at a final concentration of 1 nM, 5'-deoxy-5'-adenosine (Sigma) at a final concentration of 1 µM, and S-(5'-adenosyl)-L-methionine chloride dihydrochloride (Sigma) at a final concentration of 2.5 µM were mixed and incubated at room temperature for 30 minutes, and then transferred to an OptiPlate-384 plate (PerkinElmer) in a volume of 6 µL per well. Control wells did not contain PRMT5/MET50 protein. The test compounds were added to all wells except the control wells in a 3-fold dilution series of 10 concentration points starting from 1000 nM using a Tecan D300e. After centrifugation at 1000 rpm for 1 minute in a centrifuge, the plate was incubated at 23°C for 15 minutes. Histone H4 substrate at a final concentration of 5 nM was added to the plate at 4 µL per well. After centrifugation at 1000 rpm for 1 minute in a centrifuge, the plate was reacted in an incubator at 23°C for 45 minutes. Anti-Histone H4 (symmetric dimethyl R3) antibody (Abcam) was pre-incubated with 50 µg/mL AlphaScreen Protein A Acceptor beads (PerkinElmer) at room temperature for 10 minutes, and then added to the plate in a volume of 10 µL per well, followed by reaction at room temperature for 1 hour. 10 µL of 50 µg/mL AlphaScreen Streptavidin Donor beads (PerkinElmer) were added to each well, and reacted at room temperature for 1 hour. The signal values in the plate were detected using the Alpha method on an Envision multi-mode plate reader. The IC₅₀ values of the compounds for PRMT5/MET50 enzyme activity inhibition were calculated in Xlfit 5.0 software.

**Table 1: IC₅₀ values of compounds for PRMT5+MTA enzyme activity inhibition**

| Compound No. | IC₅₀ (nM) | Compound No. | IC₅₀ (nM) |
|---|---|---|---|
| 1 | 1.7 | 3 | 2.9 |
| 4 | 1.6 | 5 | 3.7 |
| 7 | 2.1 | 8 | 5.5 |
| 9 | 7.9 | 10 | 1.3 |
| 12 | 3.0 | 14 | 1.3 |
| 15 | 2.3 | | |
| 19 | 0.8 | 21 | 4.7 |
| 23 | 5.8 | 29 | 8.2 |
| 30 | 8.2 | 31 | 3.4 |
| 32 | 1.5 | 33 | 1.0 |
| 34 | 1.8 | 35 | 2.0 |
| 36 | 3.6 | 37 | 1.5 |
| 38 | 1.3 | 39 | 1.2 |
| 40 | 1.9 | 41 | 2.5 |
| 42 | 1.6 | 43 | 1.6 |
| 44 | 2.6 | 45 | 1.0 |
| 47 | 1.4 | 50 | 1.7 |
| 51 | 2.6 | 54 | 2.0 |
| 55 | 5.0 | 56 | 5.8 |
| 57 | 1.8 | 58 | 0.4 |
| 59 | 2.6 | 60 | 2.8 |
| 61 | 1.4 | 62 | 4.1 |
| 64 | 3.2 | 65 | 5.4 |
| 66 | 7.5 | 67 | 5.7 |
| 68 | 7.7 | 69 | 4.8 |
| 70 | 3.9 | 71 | 7.7 |
| 73 | 6.5 | 74 | 8.0 |
| 75 | 2.6 | 76 | 5.4 |
| 77 | 4.0 | 78 | 7.7 |
| 79 | 3.1 | 80 | 7.8 |
| 82 | 4.2 | 83 | 4.0 |
| 85 | 4.0 | 86 | 0.7 |
| 87 | 2.7 | 88 | 3.3 |
| 89 | 0.6 | 90 | 1.5 |
| 91 | 2.2 | 92 | 1.6 |
| 93 | 7.1 | 94 | 6.6 |
| 96 | 3.5 | 97 | 3.1 |
| 98 | 8.7 | 99 | 4.6 |
| 100 | 7.6 | 101 | 3.1 |
| 102 | 2.6 | 103 | 4.2 |
| 104 | 5.8 | 105 | 5.7 |
| 106 | 5.9 | 107 | 4.0 |
| 108 | 7.9 | 109 | 5.8 |
| 110 | 6.2 | 111 | 3.7 |
| 112 | 6.1 | 113 | 3.2 |
| 114 | 3.2 | 115 | 0.9 |
| 116 | 3.4 | 117 | 5.7 |
| 118 | 1.5 | 119 | 1.3 |
| 120 | 1.0 | 121 | 4.4 |
| 129 | 5.4 | 130 | 9.7 |

| | | | |
|---|---|---|---|
| Conclusion: As shown in the table above, the compounds of the present invention exhibit excellent PRMT5 enzyme inhibition activity, with the measured enzyme inhibition IC₅₀ values of most compounds being less than 10 nM. | | | |

### Test Example 2: Test of cell proliferation inhibition activity of the compounds

HCT116-WT (ATCC, CCL-247) and HCT116 MTAP KO (Cobioer Nanjing, CBP75002) cells were seeded into white low-transparency 96-well sterile cell culture plates at a density of 250 cells per well, respectively. The culture medium was McCoy's 5A Medium + 10% FBS. The plates were incubated overnight at 37°C with 5% CO₂. The cells were treated with compounds gradiently with an initial concentration of 10000 nM, 4-fold dilution, and 8 concentration points. The last two columns were control wells with only DMSO added. The treated cells were continued to be cultured for 6 days. On the seventh day, 50 µL of CellTiter Glo^{®} reagent was added to each well. After reacting on a shaker at room temperature for 10 minutes, the chemiluminescence signal in the plate was detected using the chemiluminescence detection method on a BIOTEK H1 multi-mode plate reader. The IC₅₀ values of the compounds for cell viability inhibition were calculated in Xlfit 5.0 software.

**Table 2: IC₅₀ values of compounds for HCT116 MTAP KO/WT cell growth inhibition**

| Compound No. | HCT116 MTAP KO IC₅₀ (nM) | HCT116-WT IC₅₀ (nM) | Compound No. | HCT116 MTAP KO IC50 (nM) | HCT116-WT IC₅₀ (nM) |
|---|---|---|---|---|---|
| 1 | 150 | 2250 | 7 | 422 | 1516 |
| 12 | 55 | 210 | 14 | 707 | 3546 |
| 18 | 868 | 2030 | 29 | 590 | 3732 |
| 31 | 302 | 3364 | 32 | 114 | 1890 |
| 33 | 279 | 4005 | 34 | 182 | 3475 |
| 36 | 521 | 5359 | 37 | 454 | 2697 |
| 38 | 398 | 2792 | 41 | 931 | 3711 |
| 42 | 61 | 982 | 44 | 17 | 493 |
| 45 | 24 | 275 | 47 | 31 | 593 |
| 50 | 25 | 512 | 51 | 51 | 618 |
| 56 | 36 | 536 | 58 | 34 | 971 |
| 59 | 18 | 413 | 60 | 933 | 2952 |
| 61 | 28 | 769 | 62 | 76 | 1787 |
| 64 | 63 | 1962 | 65 | 30 | 569 |
| 67 | 145 | 672 | 68 | 169 | 1986 |
| 69 | 50 | 643 | 70 | 150 | 2577 |
| 71 | 62 | 1087 | 72 | 54 | 696 |
| 73 | 72 | 1075 | 74 | 41 | 744 |
| 75 | 36 | 844 | 76 | 32 | 345 |
| 77 | 28 | 330 | 78 | 287 | 1857 |
| 79 | 782 | 3442 | 82 | 18 | 226 |
| 83 | 195 | 1714 | 84 | 25 | 428 |
| 85 | 11 | 290 | 86 | 60 | 867 |
| 87 | 975 | 3312 | 88 | 616 | 3842 |
| 89 | 12 | 421 | 90 | 81 | 1516 |
| 91 | 96 | 2013 | 92 | 134 | 1484 |
| 93 | 33 | 504 | 94 | 49 | 912 |
| 96 | 22 | 366 | 97 | 353 | 5702 |
| 98 | 64 | 1090 | 99 | 445 | 3331 |
| 101 | 352 | 1790 | 102 | 50 | 667 |
| 104 | 416 | >10000 | 105 | 263 | 2227 |
| 106 | 601 | 2893 | 107 | 474 | 8226 |
| 108 | 48 | 452 | 109 | 658 | 7719 |
| 110 | 508 | 3658 | 111 | 496 | 6452 |
| 112 | 304 | 2345 | 113 | 49 | 2653 |
| 114 | 612 | 2969 | 115 | 942 | 3181 |
| 116 | 63 | 307 | 117 | 21 | 115 |
| 118 | 454 | 2697 | 119 | 398 | 2792 |
| 122 | 192 | 1928 | 129 | 233 | 2515 |
| 130 | 329 | 1570 | | | |

| | | | | | |
|---|---|---|---|---|---|
| Conclusion: As shown in the table above, the compounds of the present invention exhibit excellent HCT116 MTAP KO cell growth inhibition activity, with the measured cell growth inhibition IC₅₀ values of some compounds being less than 100 nM. At the same time, the compounds of the present invention exhibit selectivity for HCT116-WT cells, with the selectivity of some compounds being greater than 20-fold. | | | | | |

## Claims

1. A compound of formula (I), or a tautomer, mesomer, racemate, enantiomer, diastereomer, or mixture thereof, or a pharmaceutically acceptable salt thereof, 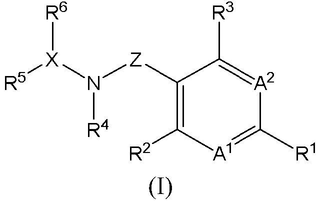 wherein:
Z is selected from the group consisting of C(=O), S(=O), S(=O)₂, 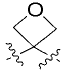 and 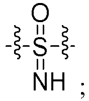
A¹ is selected from the group consisting of N and CR⁷;
A² is selected from the group consisting of N and CR⁸;
X is selected from the group consisting of N and CR⁹;
R¹ is selected from the group consisting of 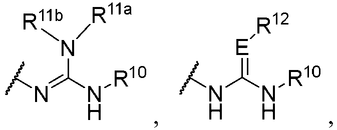 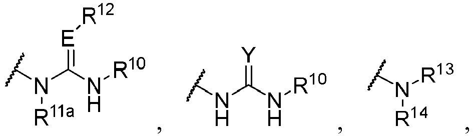 alkyl, alkenyl, alkynyl, cycloalkyl, heterocyclyl, aryl and heteroaryl, wherein the alkyl, alkenyl, alkynyl, cycloalkyl, heterocyclyl, aryl and heteroaryl are optionally further substituted by one or more groups selected from the group consisting of deuterium, halogen, amino, nitro, cyano, hydroxy, thiol, carboxy, -C(=O)OR^{a}, -C(=O)R^{a}, -C(=O)NR^{a}R^{b}, -OC(=O)R^{a}, -NR^{a}C(=O)R^{b}, -S(=O)₂R^{a}, -NR^{a}S(=O)₂R^{b}, -S(=O)₂NR^{a}R^{b}, -S(=O)R^{a}, -P(=O)R^{a}R^{b}, -NR^{a}S(=O)₂R^{b}, alkyl, -OR^{a}, haloalkyl, haloalkoxy, alkenyl, alkynyl, cycloalkyl, heterocyclyl, aryl and heteroaryl;
wherein,
E is selected from the group consisting of N and CR¹⁶;
Y is selected from the group consisting of O and S;
each R¹⁰ is independently selected from the group consisting of hydrogen, -(CH₂)ₚ-R^{a}, -OR^{a}, -(CH₂)ₚ-OR^{a}, -(CH₂)ₚ-C(=O)OR^{a}, -NR^{a}R^{b}, -(CH₂)ₚ-NR^{a}R^{b}, -C(=O)R^{a}, -C(=O)OR^{a}, -S(=O)₂R^{a}, halogen, nitro, cyano, hydroxy, thiol, alkyl, alkenyl, alkynyl, cycloalkyl, heterocyclyl, aryl and heteroaryl, wherein the alkyl, alkenyl, alkynyl, cycloalkyl, heterocyclyl, aryl and heteroaryl are optionally further substituted by one or more groups selected from the group consisting of deuterium, halogen, amino, nitro, cyano, hydroxy, thiol, carboxy, -C(=O)OR^{a}, -C(=O)R^{a}, -C(=O)NR^{a}R^{b}, -OC(=O)R^{a}, -NR^{a}C(=O)R^{b}, -S(=O)₂R^{a}, -NR^{a}S(=O)₂R^{b}, -S(=O)₂NR^{a}R^{b}, -S(=O)R^{a}, -P(=O)R^{a}R^{b}, -NR^{a}S(=O)₂R^{b}, alkyl, -OR^{a}, haloalkyl, haloalkoxy, alkenyl, alkynyl, cycloalkyl, heterocyclyl, aryl and heteroaryl;
R^{11a} and R^{11b} are each independently selected from the group consisting of hydrogen, -(CH₂)ₚ-R^{a}, -OR^{a}, -(CH₂)ₚ-OR^{a}, -(CH₂)ₚ-C(=O)OR^{a}, -NR^{a}R^{b}, -(CH₂)ₚ-NR^{a}R^{b}, -C(=O)R^{a}, -C(=O)OR^{a}, -S(=O)₂R^{a}, halogen, nitro, cyano, hydroxy, thiol, alkyl, alkenyl, alkynyl, cycloalkyl, heterocyclyl, aryl and heteroaryl, wherein the alkyl, alkenyl, alkynyl, cycloalkyl, heterocyclyl, aryl and heteroaryl are optionally further substituted by one or more groups selected from the group consisting of deuterium, halogen, amino, nitro, cyano, hydroxy, thiol, carboxy, -C(=O)OR^{a}, -C(=O)R^{a}, -C(=O)NR^{a}R^{b}, -OC(=O)R^{a}, -NR^{a}C(=O)R^{b}, -S(=O)₂R^{a}, -NR^{a}S(=O)₂R^{b}, -S(=O)₂NR^{a}R^{b}, -S(=O)R^{a}, -P(=O)R^{a}R^{b}, -NR^{a}S(=O)₂R^{b}, alkyl, -OR^{a}, haloalkyl, haloalkoxy, alkenyl, alkynyl, cycloalkyl, heterocyclyl, aryl and heteroaryl;
R¹² is selected from the group consisting of hydrogen, -(CH₂)ₚ-R^{a}, -OR^{a}, -(CH₂)ₚ-OR^{a}, -(CH₂)ₚ-C(=O)OR^{a}, -NR^{a}R^{b}, -(CH₂)ₚ-NR^{a}R^{b}, -C(=O)R^{a}, -C(=O)OR^{a}, -S(=O)₂R^{a}, halogen, nitro, cyano, hydroxy, thiol, alkyl, alkenyl, alkynyl, cycloalkyl, heterocyclyl, aryl and heteroaryl, wherein the alkyl, alkenyl, alkynyl, cycloalkyl, heterocyclyl, aryl and heteroaryl are optionally further substituted by one or more groups selected from the group consisting of deuterium, halogen, amino, nitro, cyano, hydroxy, thiol, carboxy, -C(=O)OR^{a}, -C(=O)R^{a}, -C(=O)NR^{a}R^{b}, -OC(=O)R^{a}, -NR^{a}C(=O)R^{b}, -S(=O)₂R^{a}, -NR^{a}S(=O)₂R^{b}, -S(=O)₂NR^{a}R^{b}, -S(=O)R^{a}, -P(=O)R^{a}R^{b}, -NR^{a}S(=O)₂R^{b}, alkyl, -OR^{a}, haloalkyl, haloalkoxy, alkenyl, alkynyl, cycloalkyl, heterocyclyl, aryl and heteroaryl;
or,
R^{11a} or R^{11b} together with R¹⁰, or R¹² together with R¹⁰ and the atoms to which they are attached form a heterocyclyl or heteroaryl, wherein the heterocyclyl or heteroaryl is optionally further substituted by one or more groups selected from the group consisting of halogen, amino, -NR^{a}R^{b}, nitro, cyano, hydroxy, thiol, carboxy, -C(=O)OR^{a}, -C(=O)R^{a}, -C(=O)NR^{a}R^{b}, -OC(=O)R^{a}, -NR^{a}C(=O)R^{b}, -S(=O)₂R^{a}, -S(=O)R^{a}, -P(=O)R^{a}R^{b}, -NR^{a}S(=O)₂R^{b}, alkyl, -OR^{a}, haloalkyl, haloalkoxy, alkenyl, alkynyl, cycloalkyl, heterocyclyl, aryl and heteroaryl;
R¹³ and R¹⁴ are each independently selected from the group consisting of hydrogen, -(CH₂)ₚ-R^{a}, -OR^{a}, -(CH₂)ₚ-OR^{a}, -(CH₂)ₚ-C(=O)OR^{a}, -NR^{a}R^{b}, -(CH₂)ₚ-NR^{a}R^{b}, -C(=O)R^{a}, -C(=O)OR^{a}, -S(=O)₂R^{a}, halogen, nitro, cyano, hydroxy, thiol, alkyl, alkenyl, alkynyl, cycloalkyl, heterocyclyl, aryl and heteroaryl, wherein the alkyl, alkenyl, alkynyl, cycloalkyl, heterocyclyl, aryl and heteroaryl are optionally further substituted by one or more groups selected from the group consisting of deuterium, halogen, amino, nitro, cyano, hydroxy, thiol, carboxy, -C(=O)OR^{a}, -C(=O)R^{a}, -C(=O)NR^{a}R^{b}, -OC(=O)R^{a}, -NR^{a}C(=O)R^{b}, -S(=O)₂R^{a}, -NR^{a}S(=O)₂R^{b}, -S(=O)₂NR^{a}R^{b}, -S(=O)R^{a}, -P(=O)R^{a}R^{b}, -NR^{a}S(=O)₂R^{b}, alkyl, -OR^{a}, haloalkyl, haloalkoxy, alkenyl, alkynyl, cycloalkyl, heterocyclyl, aryl and heteroaryl; or
R¹³ and R¹⁴ together with the nitrogen atom to which they are attached form a heteroaryl or heterocyclyl, wherein the heteroaryl or heterocyclyl is optionally further substituted by one or more groups selected from the group consisting of halogen, amino, -NR^{a}R^{b}, nitro, cyano, hydroxy, thiol, carboxy, -C(=O)OR^{a}, -C(=O)R^{a}, -C(=O)NR^{a}R^{b}, -OC(=O)R^{a}, -NR^{a}C(=O)R^{b}, -S(=O)₂R^{a}, -S(=O)R^{a}, -P(=O)R^{a}R^{b}, -NR^{a}S(=O)₂R^{b}, alkyl, -OR^{a}, haloalkyl, haloalkoxy, alkenyl, alkynyl, cycloalkyl, heterocyclyl, aryl and heteroaryl;
R¹⁶ is selected from the group consisting of hydrogen, -(CH₂)ₚ-R^{a}, -OR^{a}, -(CH₂)ₚ-OR^{a}, -(CH₂)ₚ-C(=O)OR^{a}, -NR^{a}R^{b}, -(CH₂)ₚ-NR^{a}R^{b}, -C(=O)R^{a}, -C(=O)OR^{a}, -S(=O)₂R^{a}, halogen, nitro, cyano, hydroxy, thiol, alkyl, alkenyl, alkynyl, cycloalkyl, heterocyclyl, aryl and heteroaryl, wherein the alkyl, alkenyl, alkynyl, cycloalkyl, heterocyclyl, aryl and heteroaryl are optionally further substituted by one or more groups selected from the group consisting of deuterium, halogen, amino, nitro, cyano, hydroxy, thiol, carboxy, -C(=O)OR^{a}, -C(=O)R^{a}, -C(=O)NR^{a}R^{b}, -OC(=O)R^{a}, -NR^{a}C(=O)R^{b}, -S(=O)₂R^{a}, -NR^{a}S(=O)₂R^{b}, -S(=O)₂NR^{a}R^{b}, -S(=O)R^{a}, -P(=O)R^{a}R^{b}, -NR^{a}S(=O)₂R^{b}, alkyl, -OR^{a}, haloalkyl, haloalkoxy, alkenyl, alkynyl, cycloalkyl, heterocyclyl, aryl and heteroaryl;
R², R³, Rand R⁸ are each independently selected from the group consisting of hydrogen, -(CH₂)ₚ-R^{a}, -OR^{a}, -(CH₂)ₚ-OR^{a}, -(CH₂)ₚ-C(=O)OR^{a}, -NR^{a}R^{b}, -(CH₂)ₚ-NR^{a}R^{b}, -C(=O)R^{a}, -C(=O)OR^{a}, -S(=O)₂R^{a}, halogen, nitro, cyano, hydroxy, thiol, alkyl, alkenyl, alkynyl, cycloalkyl, heterocyclyl, aryl and heteroaryl, wherein the alkyl, alkenyl, alkynyl, cycloalkyl, heterocyclyl, aryl and heteroaryl are optionally further substituted by one or more groups selected from the group consisting of deuterium, halogen, amino, nitro, cyano, hydroxy, thiol, carboxy, -C(=O)OR^{a}, -C(=O)R^{a}, -C(=O)NR^{a}R^{b}, -OC(=O)R^{a}, -NR^{a}C(=O)R^{b}, -S(=O)₂R^{a}, -NR^{a}S(=O)₂R^{b}, -S(=O)₂NR^{a}R^{b}, -S(=O)R^{a}, -P(=O)R^{a}R^{b}, -NR^{a}S(=O)₂R^{b}, alkyl, -OR^{a}, haloalkyl, haloalkoxy, alkenyl, alkynyl, cycloalkyl, heterocyclyl, aryl and heteroaryl;
R⁵, R⁶ and R⁹ are each independently selected from the group consisting of hydrogen, -(CH₂)ₚ-R^{a}, -OR^{a}, -(CH₂)ₚ-OR^{a}, -(CH₂)ₚ-C(=O)OR^{a}, -NR^{a}R^{b}, -(CH₂)ₚ-NR^{a}R^{b}, -C(=O)R^{a}, -C(=O)OR^{a}, -S(=O)₂R^{a}, halogen, nitro, cyano, hydroxy, thiol, alkyl, alkenyl, alkynyl, cycloalkyl, heterocyclyl, aryl and heteroaryl, wherein the alkyl, alkenyl, alkynyl, cycloalkyl, heterocyclyl, aryl and heteroaryl are optionally further substituted by one or more groups selected from the group consisting of deuterium, halogen, amino, -NR^{a}R^{b}, nitro, cyano, hydroxy, thiol, carboxy, -C(=O)OR^{a}, -C(=O)R^{a}, -C(=O)NR^{a}R^{b}, -OC(=O)R^{a}, -NR^{a}C(=O)R^{b}, -S(=O)₂R^{a}, -NR^{a}S(=O)₂R^{b}, -S(=O)₂NR^{a}R^{b}, -S(=O)R^{a}, -P(=O)R^{a}R^{b}, -NR^{a}S(=O)₂R^{b}, -B(OR^{a})₂, alkyl, -OR^{a}, haloalkyl, haloalkoxy, alkenyl, alkynyl, cycloalkyl, heterocyclyl, aryl and heteroaryl;
R⁴ is selected from the group consisting of hydrogen, -(CH₂)ₚ-R¹⁵, -NR^{a}R^{b}, -C(=O)R^{a}, -S(=O)₂R^{a}, alkyl, alkenyl, alkynyl, cycloalkyl, heterocyclyl, aryl and heteroaryl, wherein the alkyl, alkenyl, alkynyl, cycloalkyl, heterocyclyl, aryl and heteroaryl are optionally further substituted by one or more groups selected from the group consisting of deuterium, halogen, amino, nitro, cyano, hydroxy, thiol, carboxy, -C(=O)OR^{a}, -C(=O)R^{a}, -C(=O)NR^{a}R^{b}, -OC(=O)R^{a}, -NR^{a}C(=O)R^{b}, -S(=O)₂R^{a}, -NR^{a}S(=O)₂R^{b}, -S(=O)₂NR^{a}R^{b}, -S(=O)R^{a}, -P(=O)R^{a}R^{b}, -NR^{a}S(=O)₂R^{b}, alkyl, -OR^{a}, haloalkyl, haloalkoxy, alkenyl, alkynyl, cycloalkyl, heterocyclyl, aryl and heteroaryl;
or
any two of R⁴, R⁵, R⁶ and R⁹ together with the atoms to which they are attached form a cycloalkyl, heterocyclyl, aryl, or heteroaryl, wherein the cycloalkyl, heterocyclyl, aryl, or heteroaryl is optionally further substituted by one or more groups selected from the group consisting of halogen, amino, -NR^{a}R^{b}, nitro, cyano, hydroxy, thiol, carboxy, -C(=O)OR^{a}, -C(=O)R^{a}, -C(=O)NR^{a}R^{b}, -OC(=O)R^{a}, -NR^{a}C(=O)R^{b}, -S(=O)₂R^{a}, -S(=O)R^{a}, -P(=O)R^{a}R^{b}, -NR^{a}S(=O)₂R^{b}, alkyl, -OR^{a}, haloalkyl, haloalkoxy, alkenyl, alkynyl, cycloalkyl, heterocyclyl, aryl and heteroaryl, wherein the cycloalkyl, heterocyclyl, aryl and heteroaryl are optionally further substituted by one or more groups selected from the group consisting of halogen, amino, -NR^{a}R^{b}, nitro, cyano, hydroxy, thiol, carboxy, -C(=O)OR^{a}, -C(=O)R^{a}, -C(=O)NR^{a}R^{b}, -OC(=O)R^{a}, -NR^{a}C(=O)R^{b}, -S(=O)₂R^{a}, -S(=O)R^{a}, -P(=O)R^{a}R^{b}, -NR^{a}S(=O)₂R^{b}, alkyl, -OR^{a}, haloalkyl, haloalkoxy, alkenyl, alkynyl, cycloalkyl, heterocyclyl, aryl and heteroaryl;
R¹⁵ is selected from the group consisting of heteroaryl, aryl, heterocyclyl and cycloalkyl, wherein the heteroaryl, aryl, heterocyclyl and cycloalkyl are optionally further substituted by one or more groups selected from the group consisting of deuterium, halogen, amino, -NR^{a}R^{b}, nitro, cyano, hydroxy, thiol, carboxy, -C(=O)OR^{a}, -C(=O)R^{a}, -C(=O)NR^{a}R^{b}, -OC(=O)R^{a}, -NR^{a}C(=O)R^{b}, -S(=O)₂R^{a}, -NR^{a}S(=O)₂R^{b}, -S(=O)ZNR^{a}R^{b}, -S(=O)R^{a}, -P(=O)R^{a}R^{b}, -NR^{a}S(=O)₂R^{b}, alkyl, -OR^{a}, haloalkyl, haloalkoxy, alkenyl, alkynyl, cycloalkyl, heterocyclyl, aryl and heteroaryl, wherein the cycloalkyl, heterocyclyl, aryl and heteroaryl are optionally further substituted by one or more groups selected from the group consisting of deuterium, halogen, amino, -NR^{a}R^{b}, nitro, cyano, hydroxy, thiol, carboxy, -C(=O)OR^{a}, -C(=O)R^{a}, -C(=O)NR^{a}R^{b}, -OC(=O)R^{a}, -NR^{a}C(=O)R^{b}, -S(=O)₂R^{a}, -NR^{a}S(=O)₂R^{b}, -S(=O)₂NR^{a}R^{b}, -S(=O)R^{a}, -P(=O)R^{a}R^{b}, -NR^{a}S(=O)₂R^{b}, alkyl, -OR^{a}, haloalkyl, haloalkoxy, alkenyl, alkynyl, cycloalkyl, heterocyclyl, aryl and heteroaryl;
R^{a} and R^{b} are each independently selected from the group consisting of hydrogen, halogen, hydroxy, alkyl, alkenyl, alkynyl, cycloalkyl, heterocyclyl, aryl and heteroaryl, wherein the alkyl, alkenyl, alkynyl, cycloalkyl, heterocyclyl, aryl and heteroaryl are optionally further substituted by one or more groups selected from the group consisting of halogen, amino, nitro, cyano, hydroxy, thiol, carboxy, -C(=O)OR^{c}, -C(=O)R^{c}, -C(=O)NR^{c}R^{d}, -OC(=O)R^{c}, -NR^{c}C(=O)R^{d}, -S(=O)₂R^{c}, -NR^{c}S(=O)₂R^{d}, -S(=O)₂NR^{c}R^{d}, -S(=O)R^{c}, -P(=O)R^{c}R^{d}, -NR^{c}S(=O)₂R^{d}, alkyl, -OR^{c}, alkenyl, alkynyl, cycloalkyl, heterocyclyl, aryl and heteroaryl;
or, R^{a} and R^{b} together with the atoms to which they are attached form a heterocyclyl, wherein the heterocyclyl is optionally further substituted by one or more groups selected from the group consisting of halogen, amino, nitro, cyano, oxo, hydroxy, thiol, carboxy, alkoxycarbonyl, alkyl, alkoxy, alkenyl, alkynyl, cycloalkyl, heterocyclyl, aryl and heteroaryl;
R^{c} and R^{d} are each independently selected from the group consisting of hydrogen, halogen, hydroxy, alkyl, alkenyl, alkynyl, cycloalkyl, heterocyclyl, aryl and heteroaryl, wherein the alkyl, alkenyl, alkynyl, cycloalkyl, heterocyclyl, aryl and heteroaryl are optionally further substituted by one or more groups selected from the group consisting of halogen, amino, nitro, cyano, hydroxy, thiol, carboxy, alkyl, alkoxy, alkenyl, alkynyl, cycloalkyl, heterocyclyl, aryl and heteroaryl;
or, R^{c} and R^{d} together with the atoms to which they are attached form a heterocyclyl, wherein the heterocyclyl is optionally further substituted by one or more groups selected from the group consisting of halogen, amino, nitro, cyano, oxo, hydroxy, thiol, carboxy, alkoxycarbonyl, alkyl, alkoxy, alkenyl, alkynyl, cycloalkyl, heterocyclyl, aryl and heteroaryl;
p is an integer from 1 to 6.

2. The compound of formula (I) or a tautomer, mesomer, racemate, enantiomer, diastereomer, or mixture thereof, or a pharmaceutically acceptable salt thereof according to claim 1, being a compound of formula (II) or formula (III), or a tautomer, mesomer, racemate, enantiomer, diastereomer, or mixture thereof, or a pharmaceutically acceptable salt thereof, 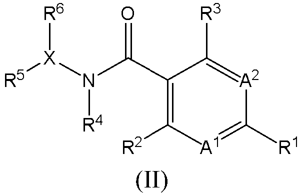 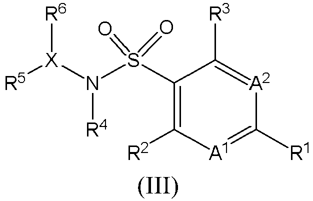 wherein, A¹, A², X, R¹~R⁶ are as defined in claim 1.

3. The compound of formula (I) or a tautomer, mesomer, racemate, enantiomer, diastereomer, or mixture thereof, or a pharmaceutically acceptable salt thereof according to claim 1 or 2, being a compound of formula (IV) or formula (V), or a tautomer, mesomer, racemate, enantiomer, diastereomer, or mixture thereof, or a pharmaceutically acceptable salt thereof, 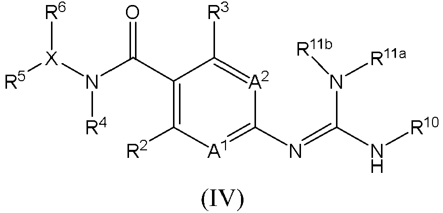 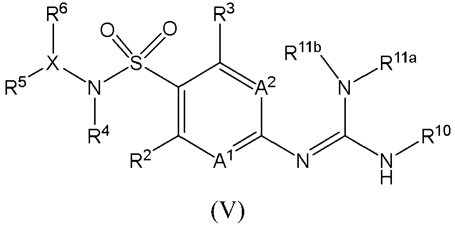 wherein,
A¹, A², X, R²~R⁶, R¹⁰, R^{11a}, R^{11b} are as defined in claim 1.

4. The compound of formula (I) or a tautomer, mesomer, racemate, enantiomer, diastereomer, or mixture thereof, or a pharmaceutically acceptable salt thereof according to claim 1 or 2, being a compound of formula (IIA) or formula (IIIA), or a tautomer, mesomer, racemate, enantiomer, diastereomer, or mixture thereof, or a pharmaceutically acceptable salt thereof, 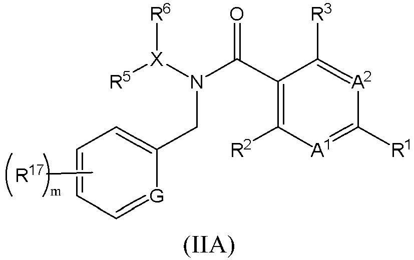 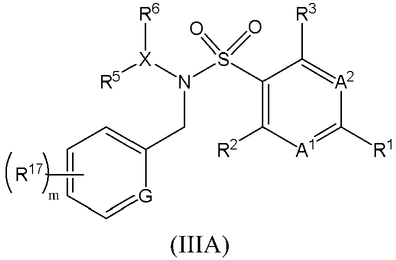 wherein:
G is selected from the group consisting of N and CH;
R¹⁷ is selected from the group consisting of hydrogen, deuterium, halogen, amino, -NR^{a}R^{b}, nitro, cyano, hydroxy, thiol, carboxy, -C(=O)OR^{a}, -C(=O)R^{a}, -C(=O)NR^{a}R^{b}, -OC(=O)R^{a}, -NR^{a}C(=O)R^{b}, -S(=O)₂R^{a}, -NR^{a}S(=O)₂R^{b}, -S(=O)₂NR^{a}R^{b}, -S(=O)R^{a}, -P(=O)R^{a}R^{b}, -NR^{a}S(=O)₂R^{b}, alkyl, -OR^{a}, haloalkyl, haloalkoxy, alkenyl, alkynyl, cycloalkyl, heterocyclyl, aryl and heteroaryl, preferably halogen, alkyl, cycloalkyl and haloalkyl; wherein the cycloalkyl, heterocyclyl, aryl and heteroaryl are optionally further substituted by one or more groups selected from the group consisting of deuterium, halogen, amino, -NR^{a}R^{b}, nitro, cyano, hydroxy, thiol, carboxy, -C(=O)OR^{a}, -C(=O)R^{a}, -C(=O)NR^{a}R^{b}, -OC(=O)R^{a}, -NR^{a}C(=O)R^{b}, -S(=O)₂R^{a}, -NR^{a}S(=O)₂R^{b}, -S(=O)₂NR^{a}R^{b}, -S(=O)R^{a}, -P(=O)R^{a}R^{b}, -NR^{a}S(=O)₂R^{b}, alkyl, -OR^{a}, haloalkyl, haloalkoxy, alkenyl, alkynyl, cycloalkyl, heterocyclyl, aryl and heteroaryl;
m is an integer from 0 to 4, preferably 1 or 2;
A¹, A², X, R¹~R³, R⁵, R⁶, R^{a}, R^{b} are as defined in claim 1.

5. The compound of formula (I) or a tautomer, mesomer, racemate, enantiomer, diastereomer, or mixture thereof, or a pharmaceutically acceptable salt thereof according to claim 1 or 4, being a compound of formula (IVA) or formula (VA), or a tautomer, mesomer, racemate, enantiomer, diastereomer, or mixture thereof, or a pharmaceutically acceptable salt thereof, 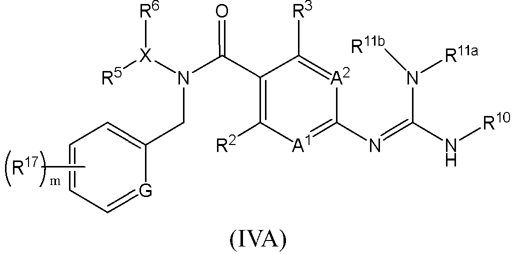 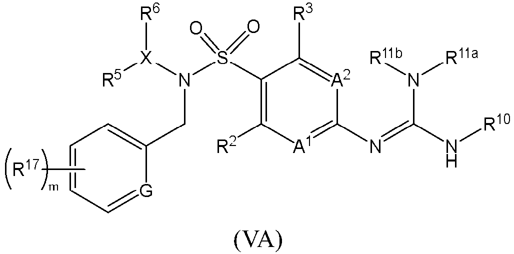 wherein,
G is selected from the group consisting of N and CH;
R¹⁷ is selected from the group consisting of hydrogen, deuterium, halogen, amino, -NR^{a}R^{b}, nitro, cyano, hydroxy, thiol, carboxy, -C(=O)OR^{a}, -C(=O)R^{a}, -C(=O)NR^{a}R^{b}, -OC(=O)R^{a}, -NR^{a}C(=O)R^{b}, -S(=O)₂R^{a}, -NR^{a}S(=O)₂R^{b}, -S(=O)₂NR^{a}R^{b}, -S(=O)R^{a}, -P(=O)R^{a}R^{b}, -NR^{a}S(=O)₂R^{b}, alkyl, -OR^{a}, haloalkyl, haloalkoxy, alkenyl, alkynyl, cycloalkyl, heterocyclyl, aryl and heteroaryl; wherein the cycloalkyl, heterocyclyl, aryl and heteroaryl are optionally further substituted by one or more groups selected from the group consisting of deuterium, halogen, amino, -NR^{a}R^{b}, nitro, cyano, hydroxy, thiol, carboxy, -C(=O)OR^{a}, -C(=O)R^{a}, -C(=O)NR^{a}R^{b}, -OC(=O)R^{a}, -NR^{a}C(=O)R^{b}, -S(=O)₂R^{a}, -NR^{a}S(=O)₂R^{b}, -S(=O)₂NR^{a}R^{b}, -S(=O)R^{a}, -P(=O)R^{a}R^{b}, -NR^{a}S(=O)₂R^{b}, alkyl, -OR^{a}, haloalkyl, haloalkoxy, alkenyl, alkynyl, cycloalkyl, heterocyclyl, aryl and heteroaryl, preferably halogen, alkyl, cycloalkyl and haloalkyl;
m is an integer from 0 to 4, preferably 1 or 2;
A¹, A², X, R²~R³, R⁵, R⁶, R¹⁰, R^{11a}, R^{11b}, R^{a}, R^{b} are as defined in claim 1.

6. The compound of formula (I) or a tautomer, mesomer, racemate, enantiomer, diastereomer, or mixture thereof, or a pharmaceutically acceptable salt thereof according to any one of claims 1 to 5, wherein A¹ is CR⁷ and A² is N; or A¹ is CR⁷ and A² is CR⁸; or A¹ is N and A² is N; R⁷ and R⁸ are as defined in claim 1, preferably, R⁷ and R⁸ are each independently selected from the group consisting of hydrogen, halogen and C₁₋₆ alkyl.

7. The compound of formula (I) or a tautomer, mesomer, racemate, enantiomer, diastereomer, or mixture thereof, or a pharmaceutically acceptable salt thereof according to any one of claims 1 to 6, wherein,
X is selected from the group consisting of N and CR⁹;
R³ and R⁶ are each independently selected from the group consisting of hydrogen, C₁₋₆ alkyl, C₃₋₆ cycloalkyl, 4- to 6-membered heterocyclyl, C₆₋₁₀ aryl and 5- to 10-membered heteroaryl, wherein the C₁₋₆ alkyl, C₃₋₆ cycloalkyl, 4- to 6-membered heterocyclyl, C₆₋₁₀ aryl and 5- to 10-membered heteroaryl are optionally further substituted by one or more groups selected from the group consisting of deuterium, halogen, C₁₋₆ alkyl and C₁₋₆ haloalkyl;
R⁹ is selected from the group consisting of hydrogen and C₁₋₆ alkyl.

8. The compound of formula (I) or a tautomer, mesomer, racemate, enantiomer, diastereomer, or mixture thereof, or a pharmaceutically acceptable salt thereof according to any one of claims 1 to 6, wherein,
X is selected from the group consisting of N and CR⁹;
any two of R⁴, R⁵, R⁶ and R⁹, together with the atoms to which they are attached, form a C₃₋₁₀ cycloalkyl, 5- to 10-membered heterocyclyl, C₆₋₁₀ aryl, or 5- to 10-membered heteroaryl, wherein the C₃₋₁₀ cycloalkyl, 5- to 10-membered heterocyclyl, C₆₋₁₀ aryl, or 5- to 10-membered heteroaryl is optionally further substituted by one or more groups selected from the group consisting of halogen, amino, -NR^{a}R^{b}, nitro, cyano, hydroxy, thiol, carboxy, -C(=O)OR^{a}, -NR^{a}C(=O)R^{b}, -OR^{a}, haloalkoxy, C₃₋₆ cycloalkyl, 5- to 10-membered heterocyclyl, C₁₋₆ alkyl, C₁₋₆ haloalkyl, C₆₋₁₀ aryl and 5-to 10-membered heteroaryl; wherein the C₆₋₁₀ aryl and 5- to 10-membered heteroaryl are optionally further substituted by C₁₋₆ alkyl;
wherein R^{a} and R^{b} are as defined in claim 1.

9. The compound of formula (I) or a tautomer, mesomer, racemate, enantiomer, diastereomer, or mixture thereof, or a pharmaceutically acceptable salt thereof according to any one of claims 1 to 6, wherein,
X is selected from the group consisting of N and CR⁹;
R⁴ and one of R⁵, R⁶, R⁹, together with the atoms to which they are attached, form a 5- to 10-membered heterocyclyl or 5- to 10-membered heteroaryl, wherein the 5- to 10-membered heterocyclyl or 5- to 10-membered heteroaryl is optionally further substituted by one or more groups selected from the group consisting of halogen, amino, -NR^{a}R^{b}, nitro, cyano, hydroxy, thiol, carboxy, -C(=O)OR^{a}, -NR^{a}C(=O)R^{b}, -OR^{a}, haloalkoxy, C₃₋₆ cycloalkyl, 5- to 10-membered heterocyclyl, C₁₋₆ alkyl, C₁₋₆ haloalkyl, C₆₋₁₀ aryl and 5- to 10-membered heteroaryl; wherein the C₆₋₁₀ aryl and 5- to 10-membered heteroaryl are optionally further substituted by C₁₋₆ alkyl;
wherein, R^{a} and R^{b} are as defined in claim 1.

10. The compound of formula (I) or a tautomer, mesomer, racemate, enantiomer, diastereomer, or mixture thereof, or a pharmaceutically acceptable salt thereof according to claim 1, being a compound of formula (IVB) or formula (VB), or a tautomer, mesomer, racemate, enantiomer, diastereomer, or mixture thereof, or a pharmaceutically acceptable salt thereof, 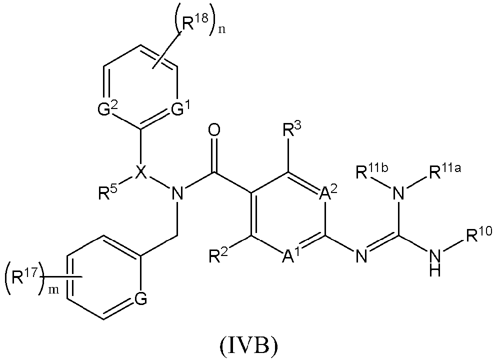 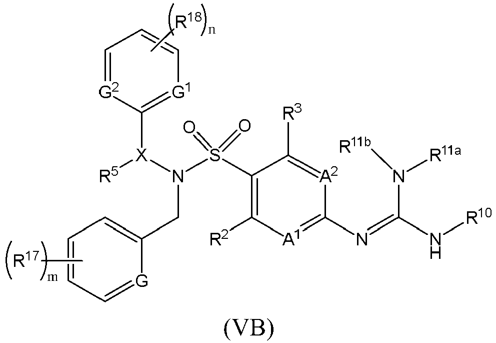 wherein,
X is selected from the group consisting of N and CR⁹;
G is selected from the group consisting of N and CH;
G¹ and G² are each independently selected from the group consisting of N and CH;
R⁵ is selected from the group consisting of hydrogen and C₁₋₆ alkyl;
R⁹ is selected from the group consisting of hydrogen and C₁₋₆ alkyl;
each R¹⁷ is independently selected from the group consisting of hydrogen, deuterium, halogen, amino, -NR^{a}R^{b}, nitro, cyano, hydroxy, thiol, carboxy, -C(=O)OR^{a}, -C(=O)R^{a}, -C(=O)NR^{a}R^{b}, -OC(=O)R^{a}, -NR^{a}C(=O)R^{b}, -S(=O)₂R^{a}, -NR^{a}S(=O)₂R^{b}, -S(=O)₂NR^{a}R^{b}, -S(=O)R^{a}, -P(=O)R^{a}R^{b}, -NR^{a}S(=O)₂R^{b}, alkyl, -OR^{a}, haloalkyl, haloalkoxy, alkenyl, alkynyl, cycloalkyl, heterocyclyl, aryl and heteroaryl, wherein the heterocyclyl, aryl and heteroaryl are optionally further substituted by one or more groups selected from the group consisting of deuterium, halogen, amino, -NR^{a}R^{b}, nitro, cyano, hydroxy, thiol, carboxy, -C(=O)OR^{a}, -C(=O)R^{a}, -C(=O)NR^{a}R^{b}, -OC(=O)R^{a}, -NR^{a}C(=O)R^{b}, -S(=O)₂R^{a}, -NR^{a}S(=O)₂R^{b}, -S(=O)₂NR^{a}R^{b}, -S(=O)R^{a}, -P(=O)R^{a}R^{b}, -NR^{a}S(=O)₂R^{b}, alkyl, -OR^{a}, haloalkyl, haloalkoxy, alkenyl, alkynyl, cycloalkyl, heterocyclyl, aryl and heteroaryl; preferably halogen, C₁₋₆ alkyl, C₁₋₆ alkoxy, C₃₋₆ cycloalkyl, C₁₋₆ haloalkyl, C₁₋₆ haloalkoxy, 5- to 6-membered heterocyclyl, phenyl and 5- to 6-membered heteroaryl, wherein the 5- to 6-membered heterocyclyl, phenyl and 5-to 6-membered heteroaryl are optionally substituted by one or more groups selected from the group consisting of -NR^{a}R^{b}, C₁₋₆ alkyl, C₁₋₆ alkoxy, C₁₋₆ haloalkyl, C₁₋₆ haloalkoxy and 5- to 6-membered heterocyclyl;
each R¹⁸ is independently selected from the group consisting of hydrogen, deuterium, halogen, amino, nitro, cyano, hydroxy, thiol, carboxy, -C(=O)OR^{a}, -C(=O)R^{a}, -C(=O)NR^{a}R^{b}, -OC(=O)R^{a}, -NR^{a}C(=O)R^{b}, -S(=O)₂R^{a}, -NR^{a}S(=O)₂R^{b}, -S(=O)₂NR^{a}R^{b}, -S(=O)R^{a}, -P(=O)R^{a}R^{b}, -NR^{a}S(=O)₂R^{b}, C₁₋₆ alkyl, -OR^{a}, haloalkyl, haloalkoxy, alkenyl, alkynyl, cycloalkyl, heterocyclyl, aryl and heteroaryl, preferably hydrogen, deuterium, C₁₋₆ alkyl, C₁₋₆ haloalkyl;
or, two adjacent R¹⁸, together with the atoms to which they are attached, form a 5-to 6-membered cycloalkyl, 5- to 6-membered heterocyclyl, 6-membered aryl, or 5- to 6-membered heteroaryl, wherein the 5- to 6-membered cycloalkyl, 5- to 6-membered heterocyclyl, 6-membered aryl, or 5- to 6-membered heteroaryl is optionally further substituted by one or more groups selected from the group consisting of deuterium, halogen, amino, -NR^{a}R^{b}, nitro, cyano, hydroxy, thiol, carboxy, -C(=O)OR^{a}, -C(=O)R^{a}, -C(=O)NR^{a}R^{b}, -OC(=O)R^{a}, -NR^{a}C(=O)R^{b}, -S(=O)₂R^{a}, -NR^{a}S(=O)₂R^{b}, -S(=O)₂NR^{a}R^{b}, -S(=O)R^{a}, -P(=O)R^{a}R^{b}, -NR^{a}S(=O)₂R^{b}, -B(OR^{a})₂, alkyl, -OR^{a}, haloalkyl, haloalkoxy, alkenyl, alkynyl, cycloalkyl, heterocyclyl, aryl and heteroaryl;
R^{a} and R^{b} are each independently selected from the group consisting of hydrogen and C₁₋₆ alkyl; or
R^{a} and R^{b}, together with the atoms to which they are attached, form a 5- to 6-membered heterocyclyl, wherein the 5- to 6-membered heterocyclyl is optionally further substituted by C₁₋₆ alkyl;
m is an integer from 0 to 4, preferably 1 or 2;
n is an integer from 0 to 4, preferably 1 or 2;
A¹, A², R², R³, R¹⁰, R^{11a}, R^{11b} are as defined in claim 1.

11. The compound of formula (I) or a tautomer, mesomer, racemate, enantiomer, diastereomer, or mixture thereof, or a pharmaceutically acceptable salt thereof according to claim 1, being a compound of formula (IVC) or formula (VC), or a tautomer, mesomer, racemate, enantiomer, diastereomer, or mixture thereof, or a pharmaceutically acceptable salt thereof, 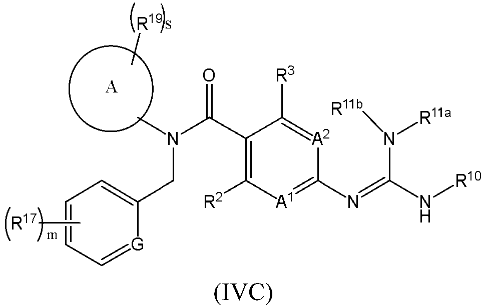 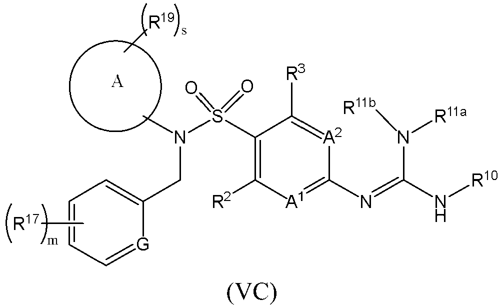 wherein,
ring A is selected from the group consisting of 5- to 10-membered heteroaryl, C₆₋₁₀ aryl, 3- to 10-membered heterocyclyl and 3- to 10-membered cycloalkyl, wherein the 5-to 10-membered heteroaryl, C₆₋₁₀ aryl, 3- to 10-membered heterocyclyl or 3- to 10-membered cycloalkyl is preferably 9- to 10-membered fused heteroaryl, naphthyl, 9-to 10-membered fused heterocyclyl, 9- to 10-membered fused cycloalkylor C₃₋₆ cycloalkyl;
each R¹⁹ is independently selected from the group consisting of hydrogen, halogen, amino, -NR^{a}R^{b}, nitro, cyano, hydroxy, thiol, carboxy, -C(=O)OR^{a}, -C(=O)R^{a}, -C(=O)NR^{a}R^{b}, -OC(=O)R^{a}, -NR^{a}C(=O)R^{b}, -S(=O)₂R^{a}, -S(=O)R^{a}, -P(=O)R^{a}R^{b}, -NR^{a}S(=O)₂R^{b}, alkyl, -OR^{a}, haloalkyl, haloalkoxy, alkenyl, alkynyl, cycloalkyl, heterocyclyl, aryl and heteroaryl, wherein the cycloalkyl, heterocyclyl, aryl and heteroaryl are optionally further substituted by one or more groups selected from the group consisting of halogen, amino, -NR^{a}R^{b}, nitro, cyano, hydroxy, thiol, carboxy, -C(=O)OR^{a}, -C(=O)R^{a}, -C(=O)NR^{a}R^{b}, -OC(=O)R^{a}, -NR^{a}C(=O)R^{b}, -S(=O)₂R^{a}, -S(=O)R^{a}, -P(=O)R^{a}R^{b}, -NR^{a}S(=O)₂R^{b}, alkyl, -OR^{a}, haloalkyl, haloalkoxy, alkenyl, alkynyl, cycloalkyl, heterocyclyl, aryl and heteroaryl; preferably hydrogen, halogen, C₁₋₆ alkyl, C₁₋₆ haloalkyl, phenyl, 5- to 6-membered heteroaryl, wherein the 5- to 6-membered heteroaryl is optionally substituted by C₁₋₆ alkyl;
G is selected from the group consisting of N and CH;
each R¹⁷ is independently selected from the group consisting of hydrogen, deuterium, halogen, amino, -NR^{a}R^{b}, nitro, cyano, hydroxy, thiol, carboxy, -C(=O)OR^{a}, -C(=O)R^{a}, -C(=O)NR^{a}R^{b}, -OC(=O)R^{a}, -NR^{a}C(=O)R^{b}, -S(=O)₂R^{a}, -NR^{a}S(=O)₂R^{b}, -S(=O)₂NR^{a}R^{b}, -S(=O)R^{a}, -P(=O)R^{a}R^{b}, -NR^{a}S(=O)₂R^{b}, alkyl, -OR^{a}, haloalkyl, haloalkoxy, alkenyl, alkynyl, cycloalkyl, heterocyclyl, aryl and heteroaryl, preferably halogen, C₁₋₆ alkyl, C₃₋₆ cycloalkyl, C₁₋₆ haloalkyl, 5- to 6-membered heterocyclyl, phenyl, or 5- to 6-membered heteroaryl;
m is an integer from 0 to 4, preferably 1 or 2;
s is an integer from 0 to 4, preferably 1 or 2;
A¹, A², R², R³, R¹⁰, R^{11a}, R^{11b} , R^{a}, R^{b} are as defined in claim 1.

12. The compound of formula (I) or a tautomer, mesomer, racemate, enantiomer, diastereomer, or mixture thereof, or a pharmaceutically acceptable salt thereof according to claim 11, wherein ring A is 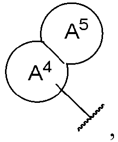 wherein ring A⁴ and A⁵ are each independently selected from the group consisting of C₅₋₆ cycloalkyl, 5- to 6-membered heterocyclyl, 5- to 6-membered heteroaryl and phenyl; specifically, ring A⁴ is selected from the group consisting of C₅₋₆ cycloalkyl, 5- to 6-membered heterocyclyl, 5- to 6-membered heteroaryl and phenyl, and ring A⁵ is selected from the group consisting of 5- to 6-membered heteroaryl and phenyl;
preferably, ring A is selected from the group consisting of: 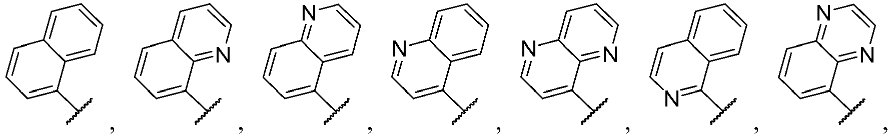 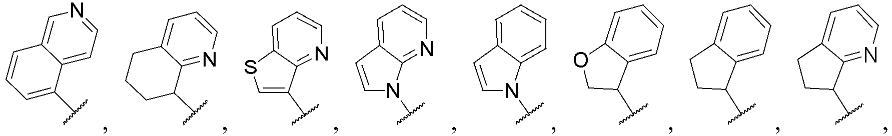 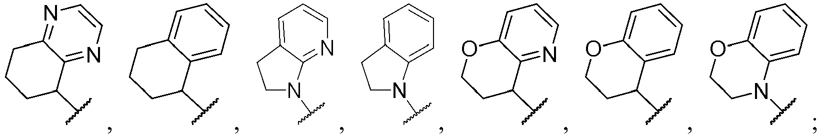
ring A is optionally substituted by one or more R¹⁹, wherein R¹⁹ is as defined in claim 11.

13. The compound of formula (I) or a tautomer, mesomer, racemate, enantiomer, diastereomer, or mixture thereof, or a pharmaceutically acceptable salt thereof according to claim 1, being a compound of formula (IVD) or formula (VD), or a tautomer, mesomer, racemate, enantiomer, diastereomer, or mixture thereof, or a pharmaceutically acceptable salt thereof, 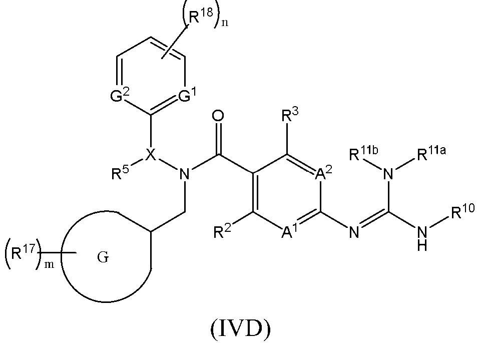 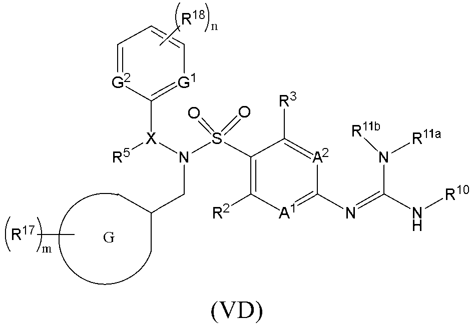 wherein,
X is selected from the group consisting of N and CR⁹;
ring G is selected from the group consisting of 5- to 10-membered heteroaryl, C₆₋₁₀ aryl and 5- to 10-membered heterocyclyl, preferably 5- to 10-membered heteroaryl, phenyl, or 5- to 6-membered heterocyclyl;
G¹ and G² are each independently selected from the group consisting of N and CH;
R⁵ is selected from the group consisting of hydrogen, C₁₋₆ alkyl and C₃₋₆ cycloalkyl;
R⁹ is selected from the group consisting of hydrogen and C₁₋₆ alkyl;
each R¹⁷ is independently selected from the group consisting of hydrogen, deuterium, halogen, amino, -NR^{a}R^{b}, nitro, cyano, hydroxy, thiol, carboxy, -C(=O)OR^{a}, -C(=O)R^{a}, -C(=O)NR^{a}R^{b}, -OC(=O)R^{a}, -NR^{a}C(=O)R^{b}, -S(=O)₂R^{a}, -NR^{a}S(=O)₂R^{b}, -S(=O)₂NR^{a}R^{b}, -S(=O)R^{a}, -P(=O)R^{a}R^{b}, -NR^{a}S(=O)₂R^{b}, alkyl, -OR^{a}, haloalkyl, haloalkoxy, alkenyl, alkynyl, cycloalkyl, heterocyclyl, aryl and heteroaryl, wherein the heterocyclyl, aryl and heteroaryl are optionally further substituted by one or more groups selected from the group consisting of deuterium, halogen, amino, -NR^{a}R^{b}, nitro, cyano, hydroxy, thiol, carboxy, -C(=O)OR^{a}, -C(=O)R^{a}, -C(=O)NR^{a}R^{b}, -OC(=O)R^{a}, -NR^{a}C(=O)R^{b}, -S(=O)₂R^{a}, -NR^{a}S(=O)₂R^{b}, -S(=O)₂NR^{a}R^{b}, -S(=O)R^{a}, -P(=O)R^{a}R^{b}, -NR^{a}S(=O)₂R^{b}, alkyl, -OR^{a}, haloalkyl, haloalkoxy, alkenyl, alkynyl, cycloalkyl, heterocyclyl, alkyl-substituted heterocyclyl, aryl and heteroaryl; preferably halogen, C₁₋₆ alkyl, C₁₋₆ alkoxy, C₃₋₆ cycloalkyl, C₁₋₆ haloalkyl, C₁₋₆ haloalkoxy, 5- to 6-membered heterocyclyl, phenyl, 5- to 6-membered heteroaryl, wherein the 5- to 6-membered heterocyclyl, phenyl and 5- to 6-membered heteroaryl are optionally substituted by one or more groups selected from the group consisting of -NR^{a}R^{b}, C₁₋₆ alkyl, C₁₋₆ alkoxy, C₁₋₆ haloalkyl, C₁₋₆ haloalkoxy and 5- to 6-membered heterocyclyl;
each R¹⁸ is independently selected from the group consisting of hydrogen, deuterium, halogen, amino, nitro, cyano, hydroxy, thiol, carboxy, -C(=O)OR^{a}, -C(=O)R^{a}, -C(=O)NR^{a}R^{b}, -OC(=O)R^{a}, -NR^{a}C(=O)R^{b}, -S(=O)₂R^{a}, -NR^{a}S(=O)₂R^{b}, -S(=O)₂NR^{a}R^{b}, -S(=O)R^{a}, -P(=O)R^{a}R^{b}, -NR^{a}S(=O)₂R^{b}, C₁₋₆ alkyl, -OR^{a}, haloalkyl, haloalkoxy, alkenyl, alkynyl, cycloalkyl, heterocyclyl, aryl and heteroaryl, preferably hydrogen, deuterium, C₁₋₆ alkyl, C₁₋₆ haloalkyl;
or, two adjacent R¹⁸, together with the atoms to which they are attached, form a 5-to 6-membered cycloalkyl, 5- to 6-membered heterocyclyl, 6-membered aryl, or 5- to 6-membered heteroaryl, wherein the 5- to 6-membered cycloalkyl, 5- to 6-membered heterocyclyl, 6-membered aryl, or 5- to 6-membered heteroaryl is optionally further substituted by one or more groups selected from the group consisting of deuterium, halogen, amino, -NR^{a}R^{b}, nitro, cyano, hydroxy, thiol, carboxy, alkyl, -OR^{a}, haloalkyl, haloalkoxy, alkenyl, alkynyl, cycloalkyl, heterocyclyl, aryl and heteroaryl;
R^{a} and R^{b} are each independently selected from the group consisting of hydrogen and C₁₋₆ alkyl; or
R^{a} and R^{b}, together with the atoms to which they are attached, form a 5- to 6-membered heterocyclyl, wherein the 5- to 6-membered heterocyclyl is optionally further substituted by C₁₋₆ alkyl;
m is an integer from 0 to 4, preferably 1 or 2;
n is an integer from 0 to 4, preferably 1 or 2;
A¹, A², R² , R³ , R¹⁰, R^{11a}, R^{11b} are as defined in claim 1.

14. The compound of formula (I) or a tautomer, mesomer, racemate, enantiomer, diastereomer, or mixture thereof, or a pharmaceutically acceptable salt thereof according to claim 13, wherein ring G is selected from the group consisting of

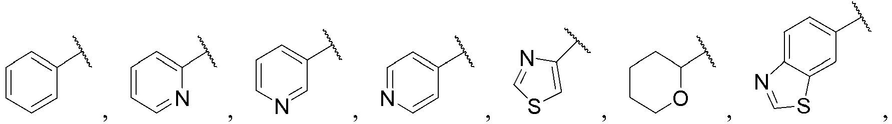

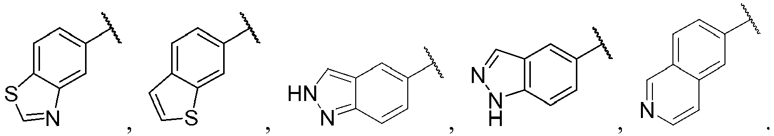

15. The compound of formula (I) or a tautomer, mesomer, racemate, enantiomer, diastereomer, or mixture thereof, or a pharmaceutically acceptable salt thereof according to any one of claims 1 to 14, wherein:
R¹⁰ is selected from the group consisting of hydrogen, -C(=O)R^{a} and C₁₋₆ alkyl;
R^{11a} is selected from the group consisting of hydrogen, -OR^{a}, -(CH₂)ₚ-OR^{a}, -(CH₂)ₚ-C(=O)OR^{a}, -(CH₂)ₚ-NR^{a}R^{b}, -C(=O)OR^{a}, nitro, C₁₋₆ alkyl, C₃₋₆ cycloalkyl, 4- to 6-membered heterocyclyl, C₆₋₁₀ aryl and 5- to 10-membered heteroaryl, wherein the C₁₋₆ alkyl, C₃₋₆ cycloalkyl, 4- to 6-membered heterocyclyl, C₆₋₁₀ aryl and 5- to 10-membered heteroaryl are optionally further substituted by one or more groups selected from the group consisting of deuterium, halogen, amino, nitro, cyano, hydroxy, thiol, carboxy, haloalkyl, haloalkoxy, alkenyl, alkynyl, cycloalkyl, heterocyclyl, aryl and heteroaryl;
R^{11b} is selected from the group consisting of hydrogen and C₁₋₆ alkyl;
R^{a} and R^{b} are each independently selected from the group consisting of hydrogen and C₁₋₆ alkyl;
or, one of R^{11a} and R^{11b}, together with R¹⁰ and the atoms to which they are attached, form a 5- to 6-membered heterocyclyl, and the other one of R^{11a} and R^{11b} is hydrogen, wherein the 5- to 6-membered heterocyclyl is optionally further substituted by one or more groups selected from the group consisting of halogen, amino, nitro, cyano, hydroxy, thiol, carboxy, alkyl, alkoxy, haloalkyl, haloalkoxy, alkenyl, alkynyl, cycloalkyl, heterocyclyl, aryl and heteroaryl;
p is an integer from 1 to 6, preferably 1.

16. The compound of formula (I) or a tautomer, mesomer, racemate, enantiomer, diastereomer, or mixture thereof, or a pharmaceutically acceptable salt thereof according to any one of claims 1 to 14, wherein:
R¹⁰ is selected from the group consisting of hydrogen and C₁₋₆ alkyl;
R^{11a} is selected from the group consisting of C₁₋₆ alkyl and hydroxy;
R^{11b} is selected from the group consisting of hydrogen and C₁₋₆ alkyl;
R^{a} and R^{b} are each independently selected from the group consisting of hydrogen and C₁₋₆ alkyl;
or, one of R^{11a} and R^{11b}, together with R¹⁰ and the atoms to which they are attached, form a 5- to 6-membered heterocyclyl, and the other one of R^{11a} and R^{11b} is hydrogen, wherein the 5- to 6-membered heterocyclyl is optionally further substituted by one or more groups selected from the group consisting of halogen, amino, nitro, cyano, hydroxy, thiol, carboxy, alkyl, alkoxy, haloalkyl, haloalkoxy, alkenyl, alkynyl, cycloalkyl, heterocyclyl, aryl and heteroaryl;
p is an integer from 1 to 6, preferably 1, 2, or 3.

17. The compound of formula (I) or a tautomer, mesomer, racemate, enantiomer, diastereomer, or mixture thereof, or a pharmaceutically acceptable salt thereof according to claim 1, being a compound of formula (VIA) or formula (VIB), or a tautomer, mesomer, racemate, enantiomer, diastereomer, or mixture thereof, or a pharmaceutically acceptable salt thereof, 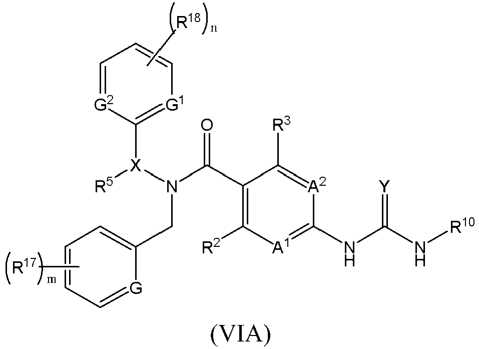 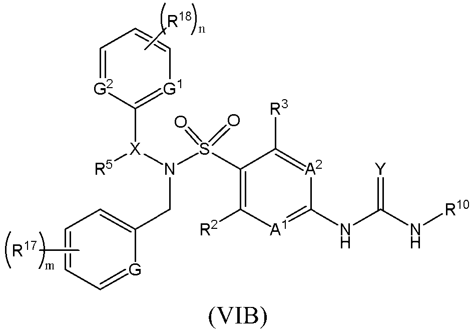 wherein,
Y is selected from the group consisting of O and S;
X is selected from the group consisting of N and CR⁹;
G is selected from the group consisting of N and CH;
G¹ and G² are each independently selected from the group consisting of N and CH;
R⁵ is selected from the group consisting of hydrogen and C₁₋₆ alkyl;
R⁹ is selected from the group consisting of hydrogen and C₁₋₆ alkyl;
R¹⁰ is selected from the group consisting of hydrogen and C₁₋₆ alkyl;
each R¹⁷ is independently selected from the group consisting of hydrogen, deuterium, halogen, amino, -NR^{a}R^{b}, nitro, cyano, hydroxy, thiol, carboxy, -C(=O)OR^{a}, -C(=O)R^{a}, -C(=O)NR^{a}R^{b}, -OC(=O)R^{a}, -NR^{a}C(=O)R^{b}, -S(=O)₂R^{a}, -NR^{a}S(=O)₂R^{b}, -S(=O)₂NR^{a}R^{b}, -S(=O)R^{a}, -P(=O)R^{a}R^{b}, -NR^{a}S(=O)₂R^{b}, alkyl, -OR^{a}, haloalkyl, haloalkoxy, alkenyl, alkynyl, cycloalkyl, heterocyclyl, aryl and heteroaryl, preferably halogen, C₁₋₆ alkyl, C₃₋₆ cycloalkyl or C₁₋₆ haloalkyl;
each R¹⁸ is independently selected from the group consisting of hydrogen, deuterium, halogen, amino, nitro, cyano, hydroxy, thiol, carboxy, -C(=O)OR^{a}, -C(=O)R^{a}, -C(=O)NR^{a}R^{b}, -OC(=O)R^{a}, -NR^{a}C(=O)R^{b}, -S(=O)₂R^{a}, -NR^{a}S(=O)₂R^{b}, -S(=O)₂NR^{a}R^{b}, -S(=O)R^{a}, -P(=O)R^{a}R^{b}, -NR^{a}S(=O)₂R^{b}, C₁₋₆ alkyl, -OR^{a}, haloalkyl, haloalkoxy, alkenyl, alkynyl, cycloalkyl, heterocyclyl, aryl and heteroaryl, preferably hydrogen, deuterium, C₁₋₆ alkyl, C₁₋₆ haloalkyl;
m is an integer from 0 to 4, preferably 1 or 2;
n is an integer from 0 to 4, preferably 1 or 2;
A¹, A², R², R³ are as defined in claim 1.

18. The compound of formula (I) or a tautomer, mesomer, racemate, enantiomer, diastereomer, or mixture thereof, or a pharmaceutically acceptable salt thereof according to claim 1, being a compound of formula (VIIA), formula (VIIB), formula (VIIC), or formula (VIID), or a tautomer, mesomer, racemate, enantiomer, diastereomer, or mixture thereof, or a pharmaceutically acceptable salt thereof, 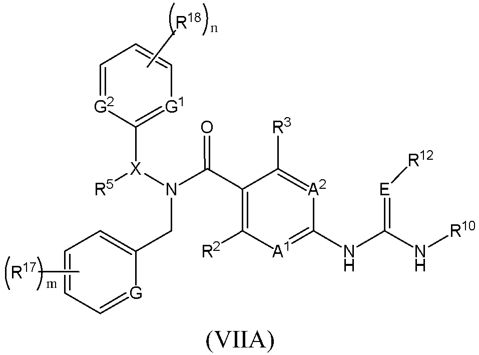 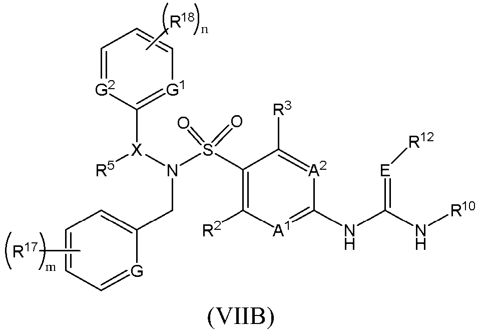 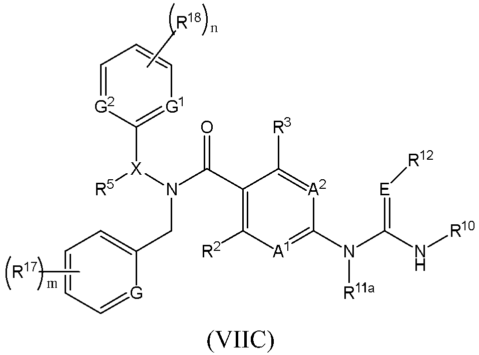 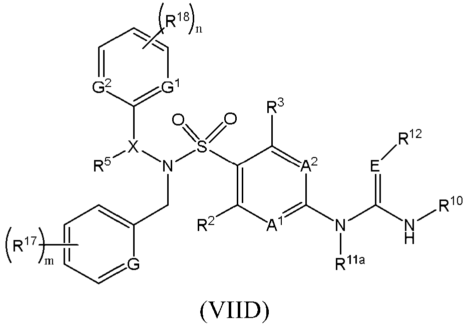 wherein,
E is selected from the group consisting of N and CR¹⁶;
X is selected from the group consisting of N and CR⁹;
G is selected from the group consisting of N and CH;
G¹ and G² are each independently selected from the group consisting of N and CH;
R⁵ is selected from the group consisting of hydrogen and C₁₋₆ alkyl;
R⁹ is selected from the group consisting of hydrogen and C₁₋₆ alkyl;
R¹⁰ is selected from the group consisting of hydrogen and C₁₋₆ alkyl;
R^{11a} is C₁₋₆ alkyl;
R¹² is selected from the group consisting of hydrogen and C₁₋₆ alkyl;
R¹⁶ is selected from the group consisting of hydrogen, C₁₋₆ alkoxy and C₁₋₆ alkyl;
each R¹⁷ is independently selected from the group consisting of hydrogen, deuterium, halogen, amino, -NR^{a}R^{b}, nitro, cyano, hydroxy, thiol, carboxy, -C(=O)OR^{a}, -C(=O)R^{a}, -C(=O)NR^{a}R^{b}, -OC(=O)R^{a}, -NR^{a}C(=O)R^{b}, -S(=O)₂R^{a}, -NR^{a}S(=O)₂R^{b}, -S(=O)₂NR^{a}R^{b}, -S(=O)R^{a}, -P(=O)R^{a}R^{b}, -NR^{a}S(=O)₂R^{b}, alkyl, -OR^{a}, haloalkyl, haloalkoxy, alkenyl, alkynyl, cycloalkyl, heterocyclyl, aryl and heteroaryl, wherein the aryl and heteroaryl are optionally substituted by C₁₋₆ alkyl; preferably halogen, C₁₋₆ alkyl, C₃₋₆ cycloalkyl, C₁₋₆ haloalkyl, 5- to 6-membered heteroaryl, wherein the 5- to 6-membered heteroaryl is optionally substituted by C₁₋₆ alkyl;
each R¹⁸ is independently selected from the group consisting of hydrogen, deuterium, halogen, amino, nitro, cyano, hydroxy, thiol, carboxy, -C(=O)OR^{a}, -C(=O)R^{a}, -C(=O)NR^{a}R^{b}, -OC(=O)R^{a}, -NR^{a}C(=O)R^{b}, -S(=O)₂R^{a}, -NR^{a}S(=O)₂R^{b}, -S(=O)₂NR^{a}R^{b}, -S(=O)R^{a}, -P(=O)R^{a}R^{b}, -NR^{a}S(=O)₂R^{b}, C₁₋₆ alkyl, -OR^{a}, haloalkyl, haloalkoxy, alkenyl, alkynyl, cycloalkyl, heterocyclyl, aryl and heteroaryl, preferably hydrogen, deuterium, C₁₋₆ alkyl, C₁₋₆ haloalkyl;
m is an integer from 0 to 4, preferably 1 or 2;
n is an integer from 0 to 4, preferably 1 or 2;
A¹, A², R², R³ are as defined in claim 1.

19. The compound of formula (I) or a tautomer, mesomer, racemate, enantiomer, diastereomer, or mixture thereof, or a pharmaceutically acceptable salt thereof according to claim 1, being a compound of formula (VIIIA) or formula (VIIIB), or a tautomer, mesomer, racemate, enantiomer, diastereomer, or mixture thereof, or a pharmaceutically acceptable salt thereof, 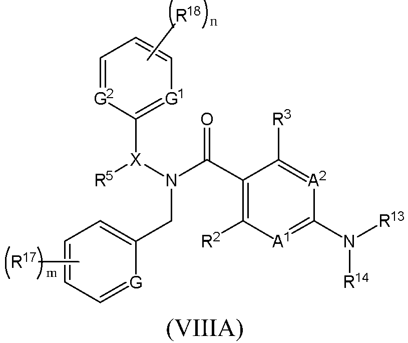 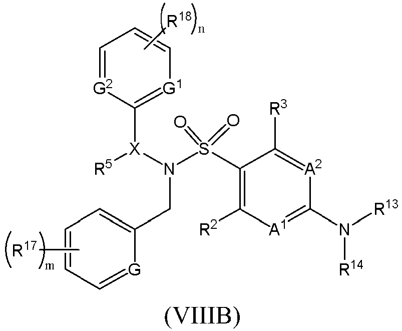 wherein,
X is selected from the group consisting of N and CR⁹;
G is selected from the group consisting of N and CH;
G¹ and G² are each independently selected from the group consisting of N and CH;
R⁵ is selected from the group consisting of hydrogen and C₁₋₆ alkyl;
R⁹ is selected from the group consisting of hydrogen and C₁₋₆ alkyl;
R¹³ is selected from the group consisting of hydrogen and C₁₋₆ alkyl;
R¹⁴ is 5- to 6-membered heteroaryl, wherein the 5- to 6-membered heteroaryl is optionally further substituted by one or more groups selected from the group consisting of halogen, amino, nitro, cyano, hydroxy, thiol, carboxy, alkyl, alkoxy, haloalkyl, haloalkoxy, alkenyl, alkynyl, cycloalkyl, heterocyclyl, aryl and heteroaryl;
or, R¹³ and R¹⁴, together with the nitrogen atom to which they are attached, form a 5- to 6-membered heteroaryl, wherein the 5- to 6-membered heteroaryl is optionally further substituted by one or more groups selected from the group consisting of halogen, amino, nitro, cyano, hydroxy, thiol, carboxy, alkyl, alkoxy, haloalkyl, haloalkoxy, alkenyl, alkynyl, cycloalkyl, heterocyclyl, aryl and heteroaryl;
each R¹⁷ is independently selected from the group consisting of hydrogen, deuterium, halogen, amino, -NR^{a}R^{b}, nitro, cyano, hydroxy, thiol, carboxy, -C(=O)OR^{a}, -C(=O)R^{a}, -C(=O)NR^{a}R^{b}, -OC(=O)R^{a}, -NR^{a}C(=O)R^{b}, -S(=O)₂R^{a}, -NR^{a}S(=O)₂R^{b}, -S(=O)₂NR^{a}R^{b}, -S(=O)R^{a}, -P(=O)R^{a}R^{b}, -NR^{a}S(=O)₂R^{b}, alkyl, -OR^{a}, haloalkyl, haloalkoxy, alkenyl, alkynyl, cycloalkyl, heterocyclyl, aryl and heteroaryl, preferably halogen, C₁₋₆ alkyl, C₃₋₆ cycloalkyl or C₁₋₆ haloalkyl;
each R¹⁸ is independently selected from the group consisting of hydrogen, deuterium, halogen, amino, nitro, cyano, hydroxy, thiol, carboxy, -C(=O)OR^{a}, -C(=O)R^{a}, -C(=O)NR^{a}R^{b}, -OC(=O)R^{a}, -NR^{a}C(=O)R^{b}, -S(=O)₂R^{a}, -NR^{a}S(=O)₂R^{b}, -S(=O)₂NR^{a}R^{b}, -S(=O)R^{a}, -P(=O)R^{a}R^{b}, -NR^{a}S(=O)₂R^{b}, C₁₋₆ alkyl, -OR^{a}, haloalkyl, haloalkoxy, alkenyl, alkynyl, cycloalkyl, heterocyclyl, aryl and heteroaryl, preferably hydrogen, deuterium, C₁₋₆ alkyl, C₁₋₆ haloalkyl;
m is an integer from 0 to 4, preferably 1 or 2;
n is an integer from 0 to 4, preferably 1 or 2;
A¹, A², R², R³ are as defined in claim 1.

20. The compound of formula (I) or a tautomer, mesomer, racemate, enantiomer, diastereomer, or mixture thereof, or a pharmaceutically acceptable salt thereof according to any one of claims 1 to 19, wherein R², R³, R⁷ and R⁸ are each independently selected from the group consisting of hydrogen, halogen and C₁₋₆ alkyl.

21. The compound of formula (I) or a tautomer, mesomer, racemate, enantiomer, diastereomer, or mixture thereof, or a pharmaceutically acceptable salt thereof according to claim 1, wherein R¹ is selected from the group consisting of C₁₋₆ alkyl, C₂₋₆ alkenyl, C₂₋₆ alkynyl, C₃₋₆ cycloalkyl, 4- to 6-membered heterocyclyl, C₆₋₁₀ aryl and 5-to 10-membered heteroaryl, wherein the C₁₋₆ alkyl, C₂₋₆ alkenyl, C₂₋₆ alkynyl, C₃₋₆ cycloalkyl, 4- to 6-membered heterocyclyl, C₆₋₁₀ aryl and 5- to 10-membered heteroaryl are optionally further substituted by one or more groups selected from the group consisting of deuterium, halogen, amino, nitro, cyano, hydroxy, thiol, carboxy, alkyl, alkoxy, haloalkyl, haloalkoxy, alkenyl, alkynyl, cycloalkyl, heterocyclyl, aryl and heteroaryl.

22. The compound of formula (I) or a tautomer, mesomer, racemate, enantiomer, diastereomer, or mixture thereof, or a pharmaceutically acceptable salt thereof according to any one of claims 1 to 21, wherein the compound is selected from the group consisting of:

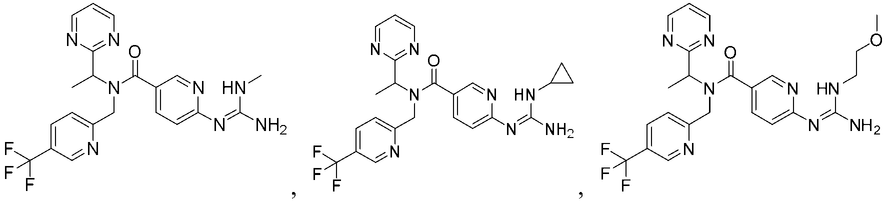

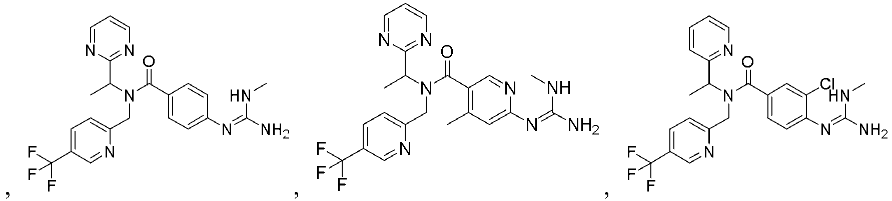

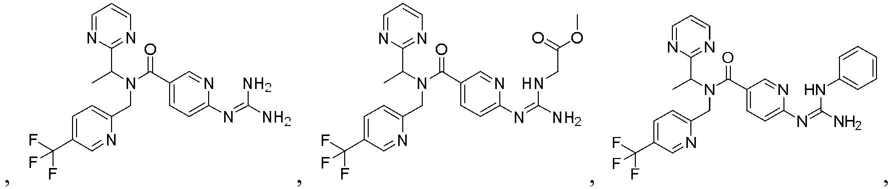

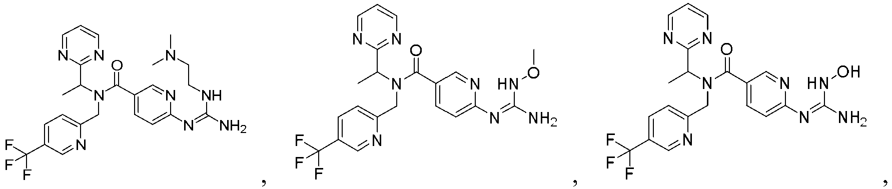

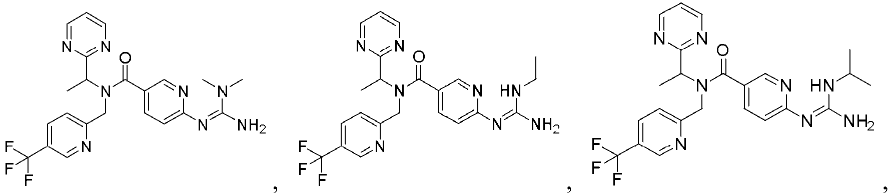

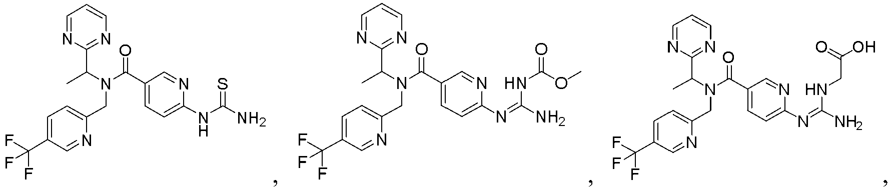

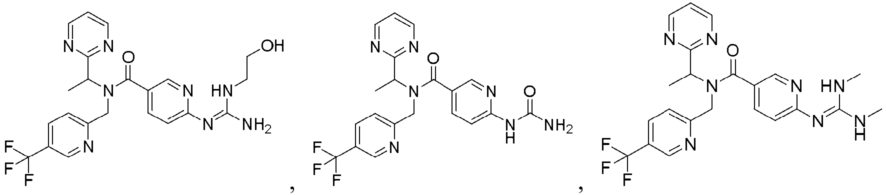

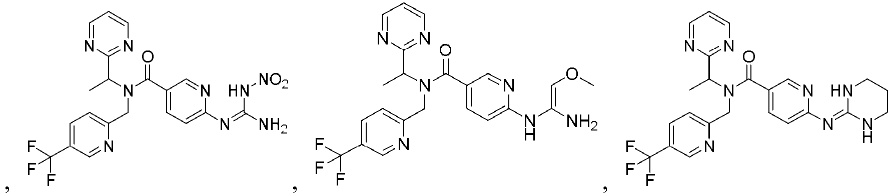

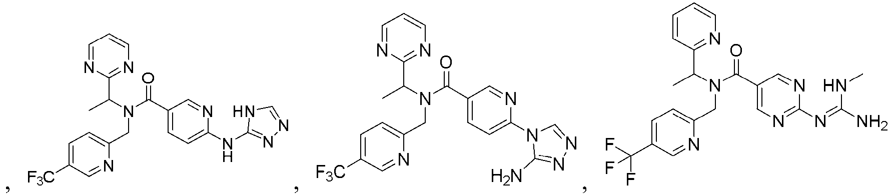

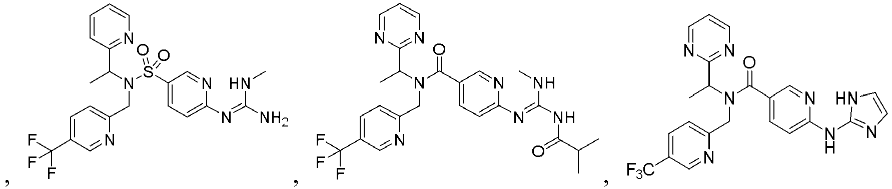

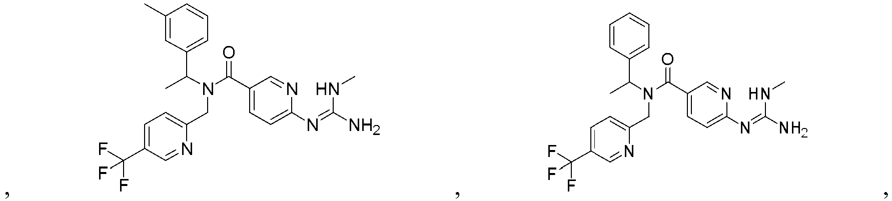

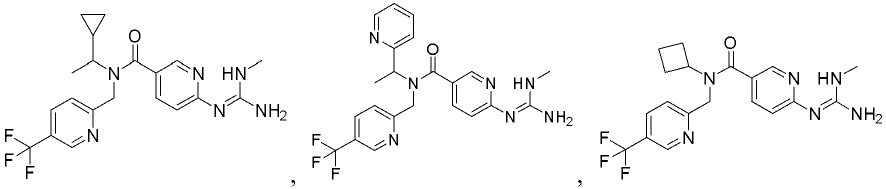

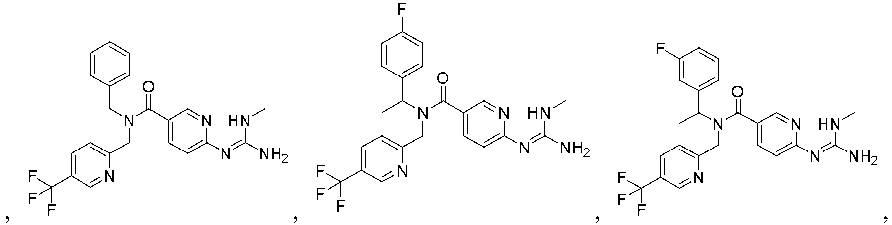

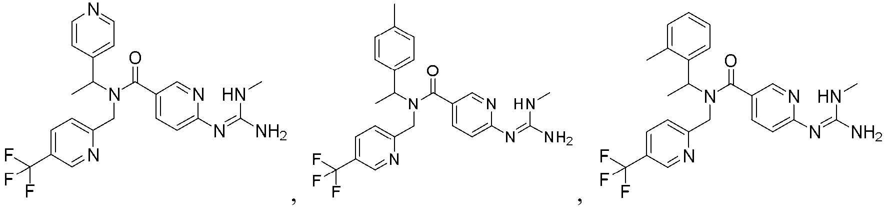

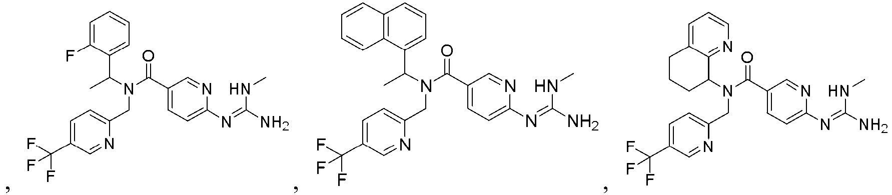

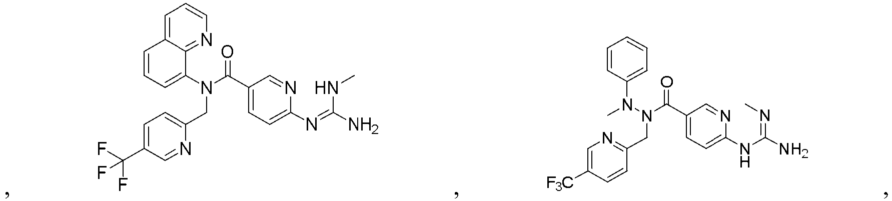

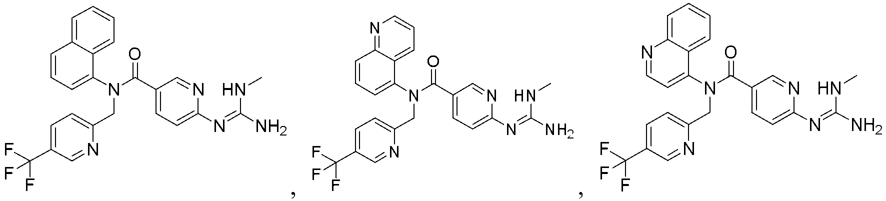

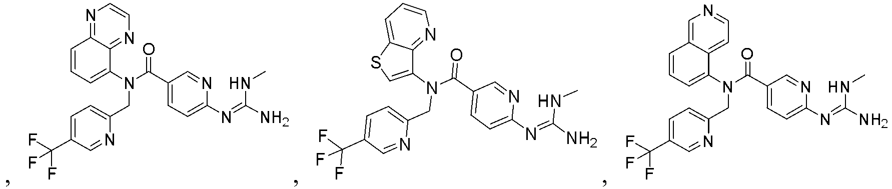

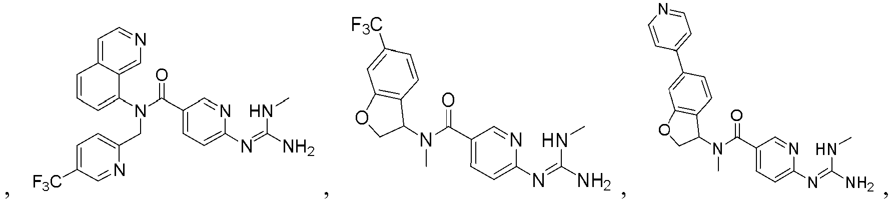

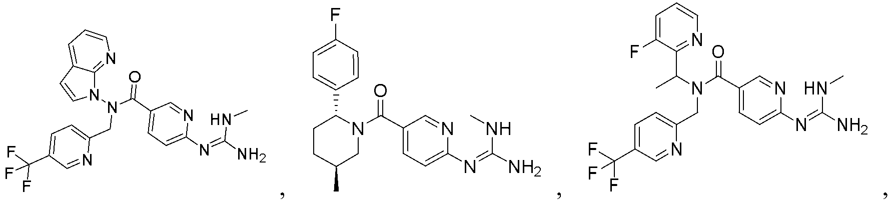

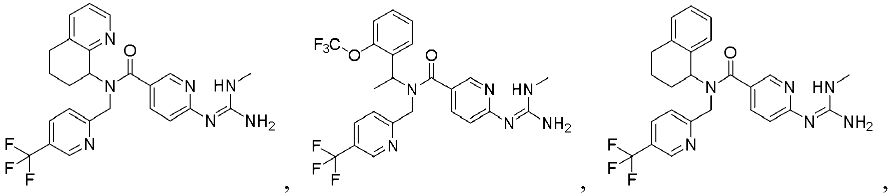

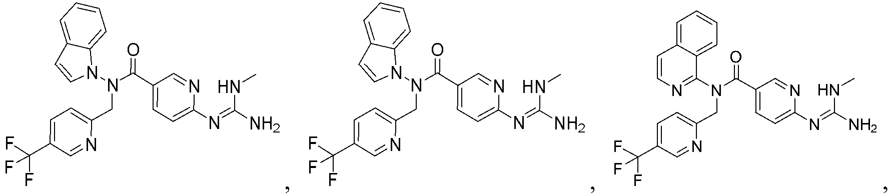

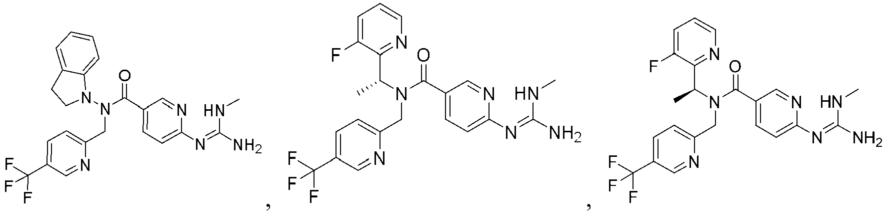

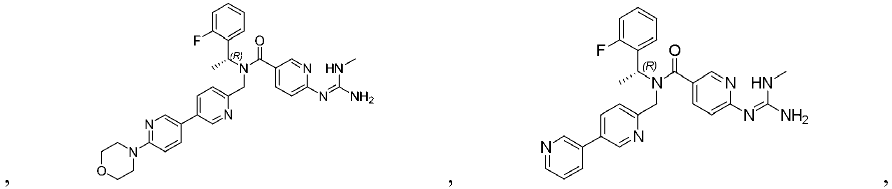

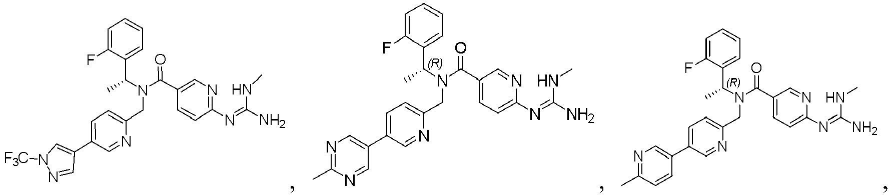

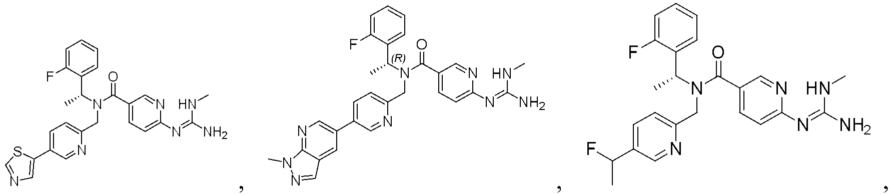

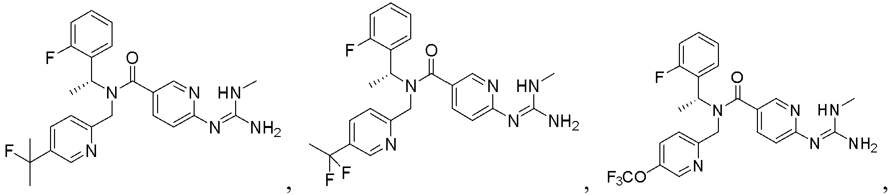

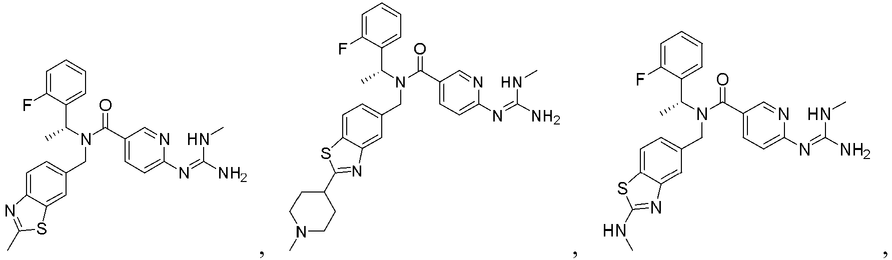

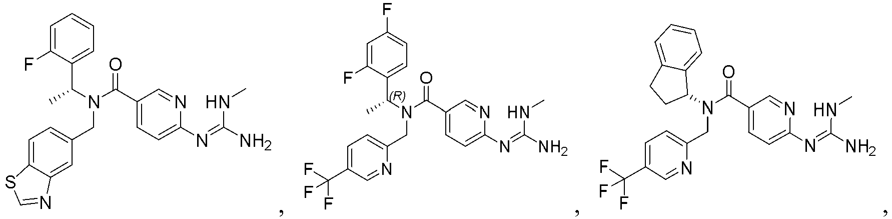

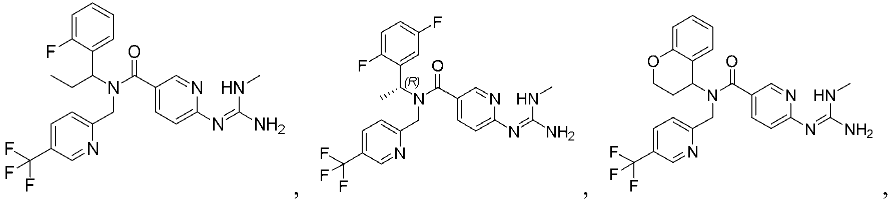

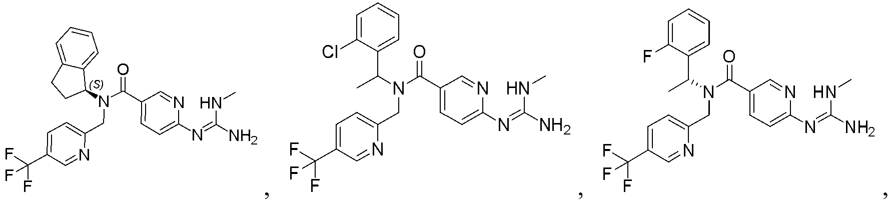

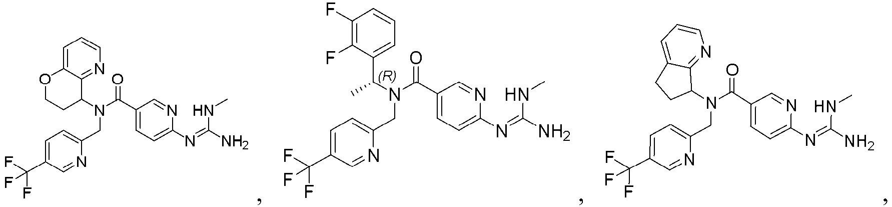

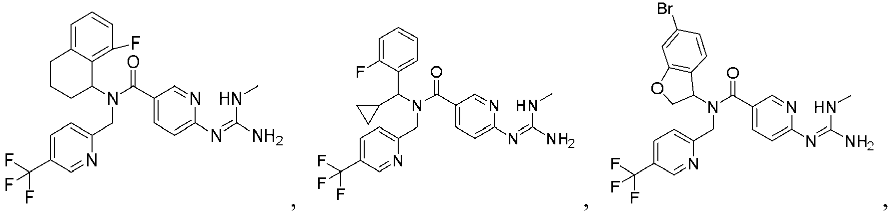

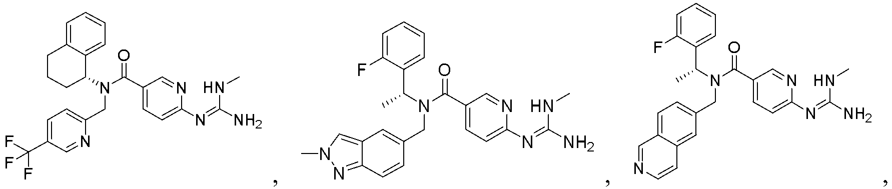

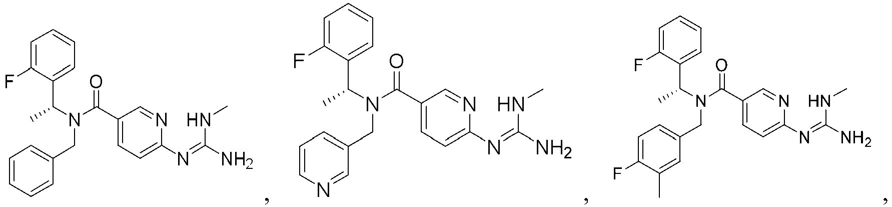

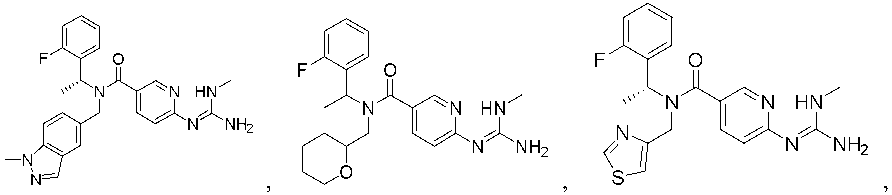

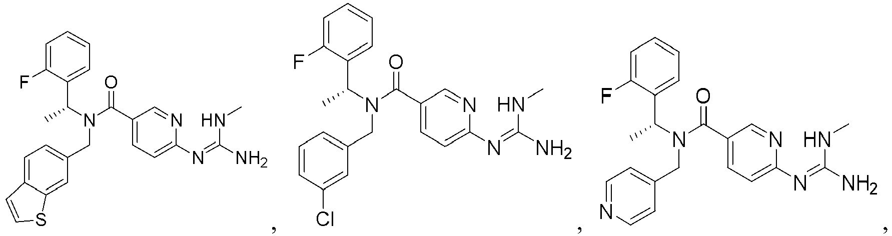

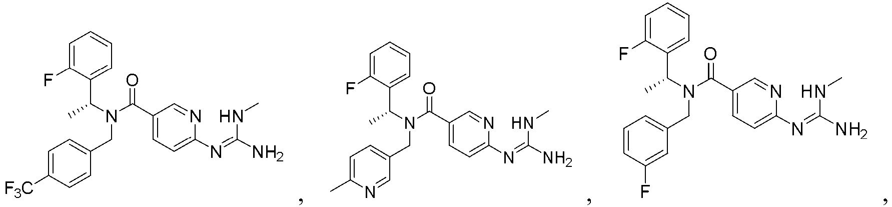

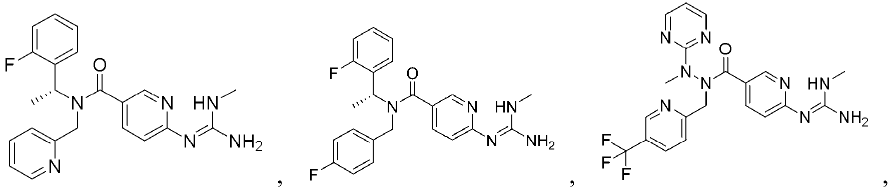

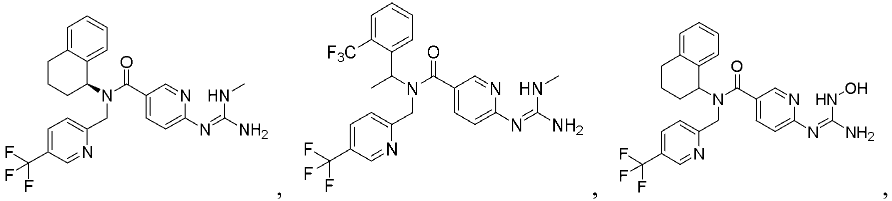

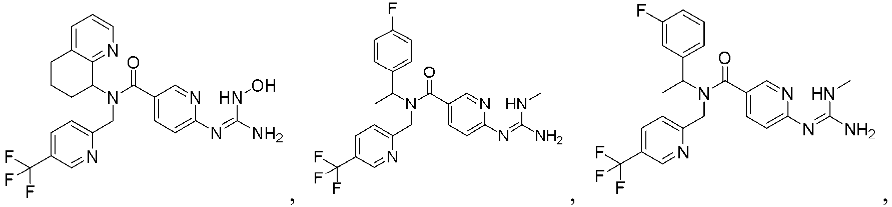

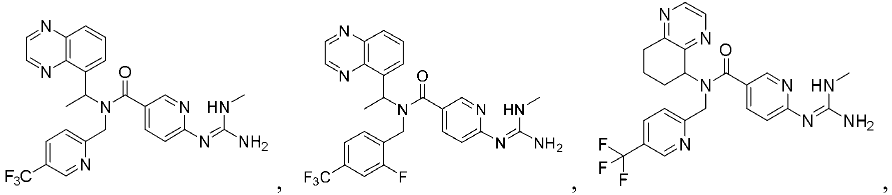

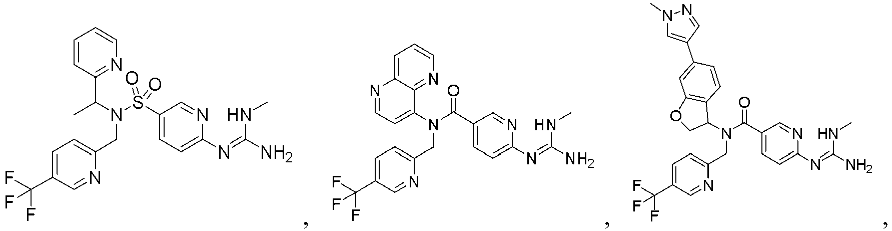

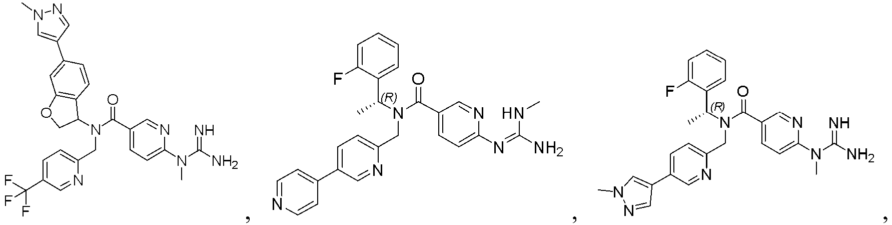

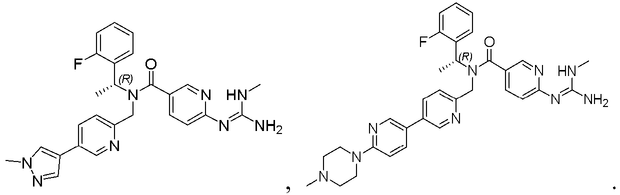

23. A pharmaceutical composition comprising the compound of formula (I) or a mesomer, racemate, enantiomer, diastereomer, or mixture thereof, or a pharmaceutically acceptable salt thereof according to any one of claims 1 to 22, and a pharmaceutically acceptable carrier or excipient.

24. Use of the compound of formula (I) or a mesomer, racemate, enantiomer, diastereomer, or mixture thereof, or a pharmaceutically acceptable salt thereof according to any one of claims 1 to 22 or the pharmaceutical composition according to claim 23 in the preparation of a PRMT5 inhibitor.

25. Use of the compound of formula (I) or a mesomer, racemate, enantiomer, diastereomer, or mixture thereof, or a pharmaceutically acceptable salt thereof according to any one of claims 1 to 22 or the pharmaceutical composition according to claim 23 in the preparation of a medicament for the prevention and/or treatment of PRMT5-mediated diseases, wherein the diseases are cancer or tumor-related diseases and preferably bladder cancer.
